(19) Europäisches Patentamt / European Patent Office / Office européen des brevets

(11) EP 1 802 572 B1

(12) **EUROPÄISCHE PATENTSCHRIFT**

(45) Veröffentlichungstag und Bekanntmachung des Hinweises auf die Patenterteilung:
**22.04.2009 Patentblatt 2009/17**

(21) Anmeldenummer: **05794403.5**

(22) Anmeldetag: **23.09.2005**

(51) Int Cl.:
*C07D 209/42* (2006.01) *C07D 401/04* (2006.01)
*C07D 401/12* (2006.01) *C07D 403/12* (2006.01)
*C07D 403/04* (2006.01) *C07D 405/04* (2006.01)
*C07D 405/14* (2006.01) *C07D 405/12* (2006.01)
*C07D 409/12* (2006.01) *C07D 409/04* (2006.01)
*C07D 417/12* (2006.01) *A61K 31/404* (2006.01)
*A61P 15/16* (2006.01)

(86) Internationale Anmeldenummer:
**PCT/EP2005/010629**

(87) Internationale Veröffentlichungsnummer:
**WO 2006/032541 (30.03.2006 Gazette 2006/13)**

(54) **INDOL DERIVATIVE ALS INHIBITOREN DER LÖSLICHEN ADENYLATZYKLASE**
INDOL DERIVATIVES AS INHIBITORS OF SOLUBLE ADENYLYL CYCLASE
DERIVES D'INDOLE EN TANT QU'INHIBITEURS DE L'ADENYLATE CYCLASE SOLUBLE

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK YU**

(30) Priorität: **24.09.2004 DE 102004047272**

(43) Veröffentlichungstag der Anmeldung:
**04.07.2007 Patentblatt 2007/27**

(73) Patentinhaber: **Bayer Schering Pharma Aktiengesellschaft**
**13353 Berlin (DE)**

(72) Erfinder:
- **NGUYEN, Duy**
  **10179 Berlin (DE)**
- **MENGEL, Anne**
  **10551 Berlin (DE)**
- **FRITSCH, Martin**
  **10555 Berlin (DE)**
- **LANGER, Gernot**
  **14612 Falkensee (DE)**
- **BOEMER, Ulf**
  **16548 Glienicke/Nordbahn (DE)**
- **KHIM, Seock-Kyu**
  **Orinda, California 94563 (US)**
- **EIS, Knut**
  **13587 Berlin (DE)**
- **MENZENBACH, Bernd**
  **07743 Jena (DE)**
- **BUCHMANN, Bernd**
  **16540 Hohen Neuendorf (DE)**

(56) Entgegenhaltungen:
**WO-A-20/04041782**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 802 572 B1

Printed by Jouve, 75001 PARIS (FR)

## Beschreibung

Die vorliegende Erfindung betrifft Inhibitoren der löslichen Adenylatzyklase, deren Herstellung sowie deren Verwendung zur Herstellung eines Arzneimittels zur Kontrazeption.

Derzeitig stehen Frauen eine Vielzahl moderner Methoden zur Kontrazeption zur Verfügung, für die männliche Fertilitätskontrolle hingegen stehen nur sehr wenige Methoden zur Verfügung (Kondom und Sterilisation). Die Entwicklung von neuen zuverlässigen Mitteln für die männliche Fertilitätskontrolle ist zwingend notwendig. Hierbei sollte die durch eine "männliche Pille" hervorgerufene Infertilität vollständig reversibel sein und genauso wirksam wie die existierenden Methoden, die der Frau zur Verfügung stehen. Die Unfruchtbarkeit sollte relativ schnell einsetzen und möglichst lang anhaltend sein. Eine derartige Verhütungsmethode sollte keine Nebenwirkungen haben, es kann sich hierbei neben hormonellen Ansätzen auch um nicht-hormonelle Ansätze handeln. Ein möglicher Ansatzpunkt ist die Regulation der Aktivität eines Enzyms, das eine wichtige Rolle bei der Befruchtung der Eizelle spielt, die lösliche Adenylatzyklase (sAC). Dieses Enzym wird hauptsächlich in den testikulären Stammzellen exprimiert und ist in reifem Sperma vorhanden.
1999 gelang es den Autoren Levin und Buck (Proc. Natl. Acad. Sci. USA 96 (1): 79-84) eine Isoform der sAC aus den Testes der Ratte zu reinigen und zu klonieren.
Das rekombinante Enzym der Ratte kann durch Bicarbonat stimuliert werden. Mittels Antikörpern konnte nachgewiesen werden, dass die katalytische Domäne des Enzyms in Testes, Sperma, Nieren und dem Choroid Plexus lokalisiert ist. Diese Offenbarungen sind Gegenstand der Anmeldung WO01/85753, die in den US zur Erteilung gekommen ist (US6544768).
In der WO01/21829 (Conti et al.*)* werden isolierte Polynukleontidesequenzen, die für die humane Isoform der sAC kodieren, ioslierte sAC Polypeptide und Testsysteme beansprucht, mit deren Hilfe Substanzen identifiziert werden können, die die Aktivität der sAC inhibieren. Die Möglichkeit, diese Substanzen zu benutzen um die Anzahl der beweglichen Samenzellen reversibel zu reduzieren, sowie deren Verwendung als Mittel zur männlichen Fertilitätskontrolle, wird offenbart.
Die Gruppe von John Herr zeigte die Isolation und Charakterisierung der humanen Isoform der sAC aus Sperma. In der WO 02/20745 werden neben Nukleinsäuren, die für die sAC kodieren auch Testsysteme beansprucht, mit deren Hilfe Substanzen identifiziert werden können, die die Expression oder die Aktivität der humanen sAC modulieren. Derartige Verbindungen könnten beispielsweise selektiv die Aktivität der sAC inhibieren, dies hätte zur Folge, dass die Samenzellen die Fähigkeit eine Eizelle zu befruchten verlieren. Diese Inhibitoren der sAC könnten daher als Arzneimittel für die nicht hormonelle Kontrazeption dienen.
Die bereits bekannten Inhibitoren der sAC zeigen jedoch spezifische Probleme: Catecholöstrogene (T. Braun, Proc Soc Exp Biol Med 1990, 194(1): 58ff) und Gossypol (KL Olgiati Arch Biochem Biophys 1984, 231(2): 411ff) sind inhärent toxisch, während Adenosinanaloga nur mit sehr schwacher Wirkung inhibieren (MA Brown und ER Casillas J Androl 1984, 5:361ff). Etwas potenter sind die Inhibitoren ($IC_{50} \leq 10\ \mu M$) der rekombinanten humanen sAC, die von Zippin *et al.* beschrieben werden (JH Zippin et al. J Cell Biol 2004, 164(4): 527ff).
Um ein Mittel für die männliche Fertilitätskontrolle zur Verfügung stellen zu können, besteht ein zunehmender Bedarf an Substanzen, die reversibel, schnell und mit gutem Erfolg zur Infertilität führen.

Diese Aufgabe wird gelöst durch die Bereitstellung der Verbindungen der allgemeinen Formel I

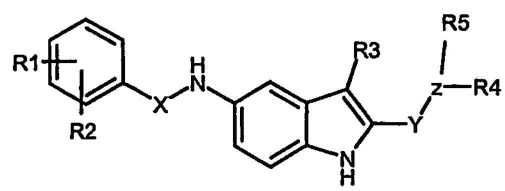

in der

$R^1$ für Wasserstoff, Halogen, $CF_3$, $C_3$-$C_6$-Cycloalkyl, welches gegebenenfalls mehrfach gesättigt und gegebenenfalls mehrfach substituiert ist, oder für die Gruppe $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl, $C_1$-$C_6$-Aryl-$C_1$-$C_6$-Alkyl oder $CF_3$, in der $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl oder $C_1$-$C_6$-Aryl-$C_1$-$C_6$-Alkyl gegebenenfalls ein- oder mehrfach, gleich oder verschieden durch Sauerstoff, Schwefel oder Stickstoff unterbrochen sein können, oder für die Gruppe Sulfonyl-$C_1$-$C_6$-Alkyl, Sulfonamid, oder Cyano steht,

$R^2$ für Halogen, $CF_3$, $C_3$-$C_6$-Cycloalkyl, welches gegebenenfalls mehrfach gesättigt und gegebenenfalls mehrfach substituiert ist, oder für die Gruppe $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl, $C_1$-$C_6$-Aryl-$C_1$-$C_6$-Alkyl oder $CF_3$, in der $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl oder $C_1$-$C_6$-Aryl-$C_1$-$C_6$-Alkyl gegebenenfalls ein- oder mehrfach, gleich oder verschieden durch Sauerstoff, Schwefel oder Stickstoff unterbrochen sein können, oder für die Gruppe Sulfonyl-$C_1$-$C_6$-Alkyl, Sulfonamid, oder Cyano steht,

$R^3$ für $C_6$-$C_{12}$-Aryl, welches gegebenenfalls ein- oder mehrfach, gleich oder verschieden mit Halogen, mit $C_1$-$C_6$-Alkyl oder $C_1$-$C_6$-Acyl, welches gegebenenfalls ein- oder mehrfach substituiert sein kann, oder mit $C_1$-$C_6$-Alkoxy, Hydroxy, Cyano, $CO_2$-($C_1$-$C_6$-Alkyl), N-($C_1$-$C_6$-Alkyl)$_2$, CO-$NR^4R^5$ oder mit $CF_3$ substituiert sein kann, für $C_5$-$C_{12}$-Heteroaryl, welches gegebenenfalls ein- oder mehrfach, gleich oder verschieden mit Halogen, mit $C_1$-$C_6$-Alkyl, $C_1$-$C_6$-Acyl, $C_1$-$C_6$-Alkoxy, Hydroxy, Cyano, $CO_2$-($C_1$-$C_6$-Alkyl), N-($C_1$-$C_6$-Alkyl)$_2$, CO-$NR^4R^5$ oder mit $CF_3$ substituiert sein kann oder für $C_3$-$C_6$-Cycloalkyl, welches gegebenenfalls ein- oder mehrfach, gleich oder verschieden mit Halogen, $CF_3$, Hydroxy, Cyano, $CO_2$-($C_1$-$C_6$-Alkyl), $C_1$-$C_6$-Alkyl, $C_1$-$C_6$-Acyl, N-($C_1$-$C_6$-Alkyl)$_2$, CO-$NR^4R^5$ oder $C_1$-$C_6$-Alkoxy substituiert sein kann, steht

$R^4$ für Wasserstoff, $C_3$-$C_6$-Cycloalkyl, welches gegebenenfalls ein- oder mehrfach, gleich oder verschieden mit $C_1$-$C_6$-Alkyl, $C_1$-$C_6$-Acyl, $C_1$-$C_6$-Alkoxy oder $CF_3$ substituiert ist, für $C_6$-$C_{12}$-Aryl, welches gegebenenfalls ein- oder mehrfach, gleich oder verschieden mit Halogen, mit $C_1$-$C_6$-Alkyl, $C_1$-$C_6$-Acyl, $C_1$-$C_6$-Alkoxy, N-$C_1$-$C_6$-Alkyl-$C_1$-$C_6$-Alkyl, $CF_3$ oder Cyano substituiert ist, oder für $C_5$-$C_{12}$-Heteroaryl, welches gegebenenfalls ein- oder mehrfach, gleich oder verschieden mit Halogen, mit $C_1$-$C_6$-Alkyl, $C_1$-$C_6$-Acyl, $C_1$-$C_6$-Alkoxy, N-$C_1$-$C_6$-Alkyl-$C_1$-$C_6$-Alkyl, $CF_3$ oder Cyano, substituiert ist, oder für $C_1$-$C_6$-Alkyl, welches beliebig substituiert sein kann, steht,

$R^5$ für Wasserstoff, $C_3$-$C_6$-Cycloalkyl, welches gegebenenfalls ein- oder mehrfach, gleich oder verschieden mit $C_1$-$C_6$-Alkyl, $C_1$-$C_6$-Acyl, $C_1$-$C_6$-Alkoxy oder $CF_3$ substituiert ist, für $C_6$-$C_{12}$-Aryl, welches gegebenenfalls ein- oder mehrfach, gleich oder verschieden mit Halogen, mit $C_1$-$C_6$-Alkyl, $C_1$-$C_6$-Acyl, $C_1$-$C_6$-Alkoxy, N-$C_1$-$C_6$-Alkyl-$C_1$-$C_6$-Alkyl, $CF_3$ oder Cyano substituiert ist, oder für $C_5$-$C_{12}$-Heteroaryl, welches gegebenenfalls ein- oder mehrfach, gleich oder verschieden mit Halogen, mit $C_1$-$C_6$-Alkyl, $C_1$-$C_6$-Acyl, $C_1$-$C_6$-Alkoxy, N-$C_1$-$C_6$-Alkyl-$C_1$-$C_6$-Alkyl, $CF_3$ oder Cyano, substituiert ist, oder für $C_1$-$C_6$-Alkyl, welches beliebig substituiert sein kann, steht,

$R^4$ und $R^5$ gemeinsam einen 5-8 gliedrigen Ring bilden, der weitere Heteroatome enthalten kann, stehen und

X für die Gruppen Sulfonyl, $(CH_2)_n$ oder Carbonyl steht,

Y für Carbonyl oder $(CH_2)_n$ steht,

Z für Stickstoff steht,

n für 0 - 4 steht,

bedeuten, sowie deren Isomere, Diastereomere, Enantiomere und Salze, die die bekannten Nachteile überwinden und verbesserte Eigenschaften aufweisen, d.h. eine gute Wirksamkeit, gute Löslichkeit und Stabilität aufweisen.
Die erfindungsgemäßen Verbindungen inhibieren die lösliche Adenylatzyklase und verhindern so die Kapazitation des Spermiums und dienen somit der männlichen Fertilitätskontrolle.

Unter Alkyl ist jeweils ein geradkettiger oder verzweigter Alkylrest, wie beispielsweise Methyl, Ethyl, Propyl, Isopropyl, Butyl, Isobutyl, sek. Butyl, tert. Butyl, Pentyl, Isopentyl und Hexyl, zu verstehen.

Unter Alkoxy ist jeweils ein geradkettiger oder verzweigter Alkoxyrest, wie beispielsweise Methoxy-, Ethoxy-, n-Propoxy-, iso-Propoxy-, n-Butoxy-, sec-Butoxy-, iso-Butoxy-, tert. Butyloxy-, Pentoxy-, iso-Pentoxy- und Hexoxy-, zu verstehen.

Unter Acyl ist jeweils ein geradkettiger oder verzweigter Rest, wie beispielsweise Formyl, Acetyl, Propionyl, Butyroyl, iso-Butyroyl, Valeroyl und Benzoyl zu verstehen.

Unter Cycloalkyl sind monocyclische Alkylringe wie Cyclopropyl, Cyclobutyl, Cyclopentyl, Cyclohexyl und zu verstehen.

Die Cycloalklyreste können anstelle der Kohlenstoffatome ein oder mehrere Heteroatome, wie Sauerstoff, Schwefel und / oder Stickstoff enthalten. Bevorzugt sind solche Heterocycloalkyle mit 3 bis 6 Ringatomen. Unter den

Ringsystemen, bei denen gegebenenfalls ein- oder mehrere mögliche Doppelbindungen im Ring enthalten sein können, sind zum Beispiel Cycloalkenyle wie Cyclopropenyl, Cyclobutenyl, Cyclopentenyl, Cyclopentadienyl, Cyclohexenyl, Cycloheptenyl zu verstehen, wobei die Anknüpfung sowohl an der Doppelbindung wie auch an den Einfachbindungen erfolgen kann.

Unter Halogen ist jeweils Fluor, Chlor, Brom oder Jod zu verstehen.

Der Arylrest umfasst jeweils 6 - 12 Kohlenstoffatome und kann beispielsweise benzokondensiert sein. Beispielsweise genannt seien: Phenyl, Tropyl, Cyclooctadienyl, Indenyl, Naphthyl, Biphenyl, Florenyl, Anthracenyl etc.

Der Heteroarylrest umfasst jeweils 5-16 Ringatome und kann anstelle des Kohlenstoffs ein- oder mehrere, gleiche oder verschiedene Heteroatome, wie Sauerstoff, Schwefel oder Stickstoff im Ring enthalten, und kann mono-, bi- oder tricyclisch sein und kann zusätzlich jeweils benzokondensiert sein.
Beispielsweise seien genannt:

Thienyl, Furanyl, Pyrrolyl, Oxazolyl, Imidazolyl, Pyrazolyl, Isoxazolyl, Isothiazolyl, Oxadiazolyl, Triazolyl, Thiadiazolyl, etc. und Benzoderivate davon, wie z.B. Benzofuranyl, Benzothienyl, Benzooxazolyl, Benzimdazolyl, Indazolyl, Indolyl, Isoindolyl, etc; oder Pyridazinyl, Pyrimidinyl, Pyrazinyl, Triazinyl, etc. und Benzoderivate davon wie z.B. Chinolyl, Isochinolyl, etc; oder Azocinyl, Indolizinyl, Purinyl, etc. und Benzoderivate davon; oder Chinolinyl, Isochinolinyl, Cinnolinyl, Phthalazinyl, Chinazolinyl, Chinoxalinyl, Naphthyridinyl, Pteridinyl, Carbazolyl, Acridinyl, Phenazinyl, Phenothiazinyl, Phenoxazinyl, Xanthenyl, Oxepinyl, etc.

Der Heteroarylrest kann jeweils benzokondensiert sein. Beispielsweise seien als 5-Ringheteroaromaten genannt: Thiophen, Furan, Oxazol, Thiazol, Imidazol, Pyrazol und Benzoderivate davon und als 6-Ring-Heteroaromaten Pyridin, Pyrimidin, Triazin, Chinolin, Isochinolin und Benzoderivate.

Unter Heteroatomen sind Sauerstoff-, Stickstoff- oder Schwefel-Atome zu verstehen.

Ist eine saure Funktion enthalten, sind als Salze die physiologisch verträglichen Salze organischer und anorganischer Basen geeignet, wie beispielsweise die gut löslichen Alkali- und Erdalkalisalze sowie N-Methyl-glukamin, Dimethylglukamin, Ethyl-glukamin, Lysin, 1,6-Hexadiamin, Ethanolamin, Glukosamin, Sarkosin, Serinol, Tris-hydroxy-methyl-amino-methan, Aminopropandiol, Sovak-Base, 1-Amino-2,3,4-butantriol.

Ist eine basische Funktion enthalten, sind die physiologisch verträglichen Salze organischer und anorganischer Säuren geeignet wie Salzsäure, Schwefelsäure, Phosphorsäure, Zitronensäure, Weinsäure u.a.

Besonders bevorzugt sind solche Verbindungen der allgemeinen Formel (I) in der

$R^1$ für Wasserstoff, Halogen, $CF_3$, $C_3$-$C_6$-Cycloalkyl, welches gegebenenfalls mehrfach gesättigt und gegebenenfalls mehrfach substituiert ist, oder für die Gruppe $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl, $C_1$-$C_6$-Aryl-$C_1$-$C_6$-Alkyl oder $CF_3$, in der $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl oder $C_1$-$C_6$-Aryl-$C_1$-$C_6$-Alkyl gegebenenfalls ein- oder mehrfach, gleich oder verschieden durch Sauerstoff, Schwefel oder Stickstoff unterbrochen sein können, oder für die Gruppe Sulfonyl-$C_1$-$C_6$-Alkyl, Sulfonamid, oder Cyano steht,

$R^2$ Halogen, $CF_3$, $C_3$-$C_6$-Cycloalkyl, welches gegebenenfalls mehrfach gesättigt und gegebenenfalls mehrfach substituiert ist, oder für die Gruppe $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl, $C_1$-$C_6$-Aryl-$C_1$-$C_6$-Alkyl oder $CF_3$, in der $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl oder $C_1$-$C_6$-Aryl-$C_1$-$C_6$-Alkyl gegebenenfalls ein- oder mehrfach, gleich oder verschieden durch Sauerstoff, Schwefel oder Stickstoff unterbrochen sein können, oder für die Gruppe Sulfonyl-$C_1$-$C_6$-Alkyl, Sulfonamid, oder Cyano steht,

$R^3$ für $C_6$-$C_{12}$-Aryl, welches gegebenenfalls ein- oder mehrfach, gleich oder verschieden mit Halogen, mit $C_1$-$C_6$-Alkyl, $C_1$-$C_3$-Acyl, $C_1$-$C_3$-Alkoxy, Cyano, Hydroxy, $N(CH_3)_2$, $CO_2$-($C_1$-$C_3$-Alkyl), CO-$NR^4R^5$ oder mit $CF_3$ substituiert sein kann, $C_5$-$C_{12}$-Heteroaryl, welches gegebenenfalls ein- oder mehrfach, gleich oder verschieden mit Chlor und / oder Fluor, mit $C_1$-$C_6$-Alkyl, $C_1$-$C_3$-Acyl, $C_1$-$C_3$-Alkoxy, Cyano, Hydroxy, N-$(CH_3)_2$, $CO_2$-($C_1$-$C_3$-Alkyl), CO-$NR^4R^5$ oder mit $CF_3$ substituiert sein kann, $C_3$-$C_6$-Cycloalkyl, welches gegebenenfalls ein- oder mehrfach, gleich oder verschieden mit Chlor und / oder Fluor, $CF_3$, Cyano, $C_1$-$C_3$-Alkyl, $C_1$-$C_3$-Acyl, Hydroxy, N-$(CH_3)_2$, $CO_2$-($C_1$-$C_3$-Alkyl), CO-$NR^4R^5$ oder $C_1$-$C_3$-Alkoxy substituiert sein kann, steht,

$R^4$ für Wasserstoff, $C_3$-$C_6$-Cycloalkyl, welches gegebenenfalls ein- oder mehrfach, gleich oder verschieden

mit $C_1$-$C_3$-Alkyl, $C_1$-$C_3$-Acyl, $C_1$-$C_3$-Alkoxy oder $CF_3$ substituiert ist, für $C_6$-$C_{12}$-Aryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano substituiert ist, oder für $C_5$-$C_{12}$-Heteroaryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano, substituiert ist, oder für $C_1$-$C_6$-Alkyl, welches beliebig substituiert sein kann, steht,

$R^5$ für Wasserstoff, $C_3$-$C_6$-Cycloalkyl, welches gegebenenfalls ein- oder mehrfach, gleich oder verschieden mit $C_1$-$C_3$-Alkyl, $C_1$-$C_3$-Acyl, $C_1$-$C_3$-Alkoxy oder $CF_3$ substituiert ist, für $C_6$-$C_{12}$-Aryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano substituiert ist, oder für $C_5$-$C_{12}$-Heteroaryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano, substituiert ist, oder für $C_1$-$C_6$-Alkyl, welches beliebig substituiert sein kann, steht,

$R^4$ und $R^5$ gemeinsam einen 5-8 gliedrigen Ring bilden, der weitere Heteroatome enthalten kann, steht,

X für die Gruppen Sulfonyl, $(CH_2)_n$ oder Carbonyl steht,

Y für Carbonyl oder $(CH_2)_n$ steht,

Z für Stickstoff steht, und

n für 0 - 2 steht,

bedeuten, sowie deren Isomere, Diastereomere, Enantiomere und Salze.

Ganz besonders bevorzugt sind solche Verbindungen der allge meinen Formel I, in der

$R^1$ für Wasserstoff steht,

$R^2$ für tertiär Butyl, Cyano, Brom, oder für die Gruppe -O-$CF_3$, -$SO_2$-$CH_3$ steht und in para-Stellung steht,

$R^3$ für die Gruppe

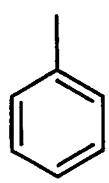 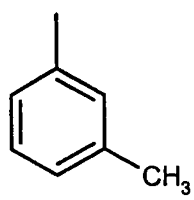 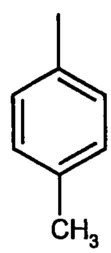 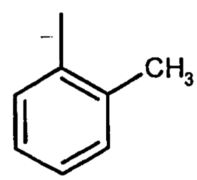 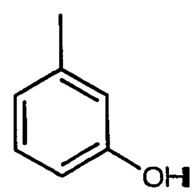

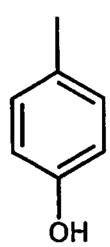 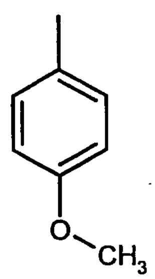 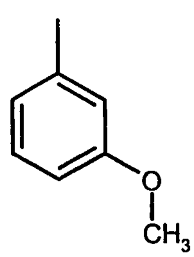 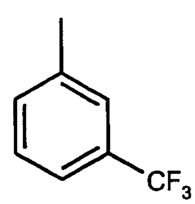 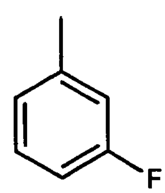

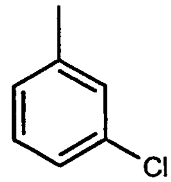 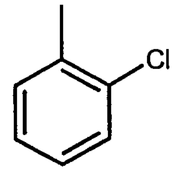 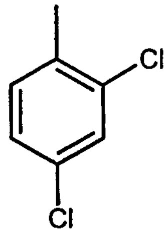 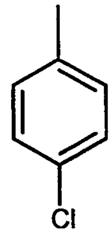

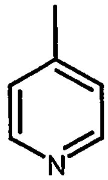 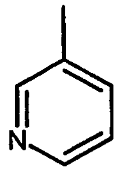 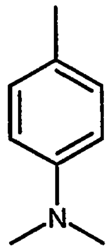

steht,

$R^4$ für Wasserstoff oder für die Gruppe $-(CH_2)_n-N-(CH_3)_2$, $-(CH_2)_2-CH_3$, $-(CH_2)_2-NH-COCH_3$, $-(CH_2)-CHCH_3-OH$, $-(CH_2)_2-O-CH_3$, $-(CH_2)_2-OH$, $-CHCH_3-CH_2-OH$,

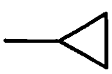 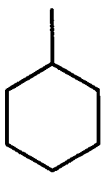 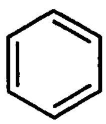 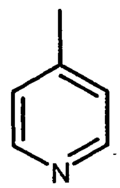

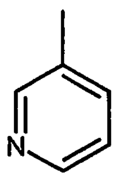 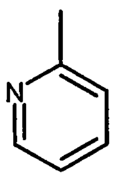 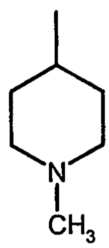 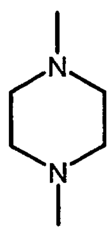 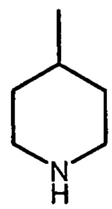

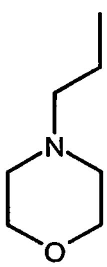 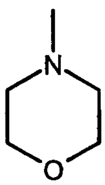 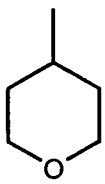 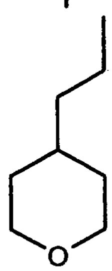 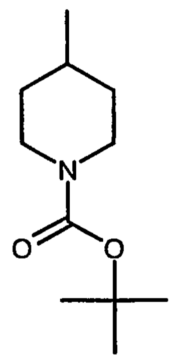

steht,

$R^5$ für Wasserstoff steht,

X für Sulfonyl, Carbonyl oder für die Gruppe $CH_2$ steht,

Y für Carbonyl oder für die Gruppe $(CH_2)n$ steht,

Z für Stickstoff bzw. für

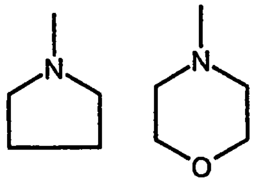

steht, und

n 1-2 steht,

bedeuten, sowie deren Isomere, Diastereomere, Enantiomere und Salze.

Ebenfalls bevorzugt sind solche Verbindungen der allgemeinen Formel I in der

$R^1$ für Wasserstoff, tertiär Butyl, Cyano, Brom, oder für die Gruppe $-O-CF_3$, $-SO_2-CH_3$ steht,

$R^2$ für tertiär Butyl, Cyano, Brom, oder für die Gruppe $-O-CF_3$, $-SO_2-CH_3$ steht, und

$R^3$ für $C_6$-$C_{12}$-Aryl, welches gegebenenfalls ein- oder mehrfach, gleich oder verschieden mit Halogen, mit $C_1$-$C_6$-Alkyl, $C_1$-$C_3$-Acyl, $C_1$-$C_3$-Alkoxy, Cyano, Hydroxy, $N-(CH_3)_2$, $CO_2$-($C_1$-$C_3$-Alkyl), CO-$NR^4R^5$ oder mit $CF_3$ substituiert sein kann, $C_5$-$C_{12}$-Heteroaryl, welches gegebenenfalls ein- oder mehrfach, gleich oder verschieden mit Chlor und / oder Fluor, mit $C_1$-$C_6$-Alkyl, $C_1$-$C_3$-Acyl, $C_1$-$C_3$-Alkoxy, Cyano, Hydroxy, $N-(CH_3)_2$, $CO_2$-($C_1$-$C_3$-Alkyl), CO-$NR^4R^5$ oder mit $CF_3$ substituiert sein kann, $C_3$-$C_6$-Cycloalkyl, welches gegebenenfalls ein- oder mehrfach, gleich oder verschieden mit Chlor und / oder Fluor, $CF_3$, Cyano, $C_1$-$C_3$-Alkyl, $C_1$-$C_3$-Acyl, Hydroxy, $N-(CH_3)_2$, $CO_2$-($C_1$-$C_3$-Alkyl), CO-$NR^4R^5$ oder $C_1$-$C_3$-Alkoxy substituiert sein kann, steht,

$R^4$ für Wasserstoff, $C_3$-$C_6$-Cycloalkyl, welches gegebenenfalls ein- oder mehrfach, gleich oder verschieden mit $C_1$-$C_3$-Alkyl, $C_1$-$C_3$-Acyl, $C_1$-$C_3$-Alkoxy oder $CF_3$ substituiert ist, für $C_6$-$C_{12}$-Aryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano substituiert ist, oder für $C_5$-$C_{12}$-Heteroaryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano, substituiert ist, oder für $C_1$-$C_6$-Alkyl, welches beliebig substituiert sein kann, steht,

$R^5$ für Wasserstoff, $C_3$-$C_6$-Cycloalkyl, welches gegebenenfalls ein- oder mehrfach, gleich oder verschieden mit $C_1$-$C_3$-Alkyl, $C_1$-$C_3$-Acyl, $C_1$-$C_3$-Alkoxy oder $CF_3$ substituiert ist, für $C_6$-$C_{12}$-Aryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl- $C_1$-$C_3$-Alkyl, $CF_3$ oder Cyano substituiert ist, oder für $C_5$-$C_{12}$-Heteroaryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano, substituiert ist, oder für $C_1$-$C_6$-Alkyl, welches beliebig substituiert sein kann, steht,

$R^4$ und $R^5$ gemeinsam einen 5-8 gliedrigen Ring bilden, der weitere Heteroatome enthalten kann, steht,

X für die Gruppen Sulfonyl, $(CH_2)_n$ oder Carbonyl steht,

Y für Carbonyl oder $(CH_2)_n$ steht,

Z für Stickstoff steht, und

n für 0 - 2 steht,

bedeuten, sowie deren Isomere, Diastereomere, Enantiomere und Salze.

Ebenfalls bevorzugt sind solche Verbindungen der allgemeinen Formel I in der

$R^1$ für Wasserstoff steht,

$R^2$ für tertiär Butyl, Cyano, Brom, oder für die Gruppe $-O-CF_3$, $-SO_2-CH_3$ steht, und in para steht, und

$R^3$ für $C_6$-$C_{12}$-Aryl, welches gegebenenfalls ein- oder mehrfach, gleich oder verschieden mit Halogen, mit $C_1$-$C_6$-Alkyl, $C_1$-$C_3$-Acyl, $C_1$-$C_3$-Alkoxy, Cyano, Hydroxy, $N-(CH_3)_2$, $CO_2$-($C_1$-$C_3$-Alkyl), CO-$NR^4R^5$ oder mit $CF_3$ substituiert sein kann, $C_5$-$C_{12}$-Heteroaryl, welches gegebenenfalls ein- oder mehrfach, gleich oder verschieden mit Chlor und / oder Fluor, mit $C_1$-$C_6$-Alkyl, $C_1$-$C_3$-Acyl, $C_1$-$C_3$-Alkoxy, Cyano, Hydroxy, $N-(CH_3)_2$, $CO_2$-($C_1$-$C_3$-Alkyl), CO-$NR^4R^5$ oder mit $CF_3$ substituiert sein kann, $C_3$-$C_6$-Cycloalkyl, welches gegebenenfalls ein- oder mehrfach, gleich oder verschieden mit Chlor und / oder Fluor, $CF_3$, Cyano, $C_1$-$C_3$-Alkyl, $C_1$-$C_3$-Acyl, Hydroxy, $N-(CH_3)_2$, $CO_2$-($C_1$-$C_3$-Alkyl), CO-$NR^4R^5$ oder $C_1$-$C_3$-Alkoxy substituiert sein kann, steht,

$R^4$ für Wasserstoff, $C_3$-$C_6$-Cycloalkyl, welches gegebenenfalls ein- oder mehrfach, gleich oder verschieden mit $C_1$-$C_3$-Alkyl, $C_1$-$C_3$-Acyl, $C_1$-$C_3$-Alkoxy oder $CF_3$ substituiert ist, für $C_6$-$C_{12}$-Aryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano substituiert ist, oder für $C_5$-$C_{12}$-Heteroaryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano, substituiert ist, oder für $C_1$-$C_6$-Alkyl, welches beliebig substituiert sein kann, steht,

$R^5$ für Wasserstoff, $C_3$-$C_6$-Cycloalkyl, welches gegebenenfalls ein- oder mehrfach, gleich oder verschieden mit $C_1$-$C_3$-Alkyl, $C_1$-$C_3$-Acyl, $C_1$-$C_3$-Alkoxy oder $CF_3$ substituiert ist, für $C_6$-$C_{12}$-Aryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano substituiert ist, oder für $C_5$-$C_{12}$-Heteroaryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano, substituiert ist, oder für $C_1$-$C_6$-Alkyl, welches beliebig substituiert sein kann, steht,

$R^4$ und $R^5$ gemeinsam einen 5-8 gliedrigen Ring bilden, der weitere Heteroatome enthalten kann, steht,

X für die Gruppen Sulfonyl, $(CH_2)_n$ oder Carbonyl steht,

Y für Carbonyl oder $(CH_2)_n$ steht,

Z für Stickstoff steht, und

n für 0 - 2 steht,

bedeuten, sowie deren Isomere, Diastereomere, Enantiomere und Salze.

Ebenfalls bevorzugt sind solche Verbindungen der allgemeinen Formel I in der

$R^1$ für Wasserstoff, Halogen, $CF_3$, $C_3$-$C_6$-Cycloalkyl, welches gegebenenfalls mehrfach gesättigt und gegebenenfalls mehrfach substituiert ist, oder für die Gruppe $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl, $C_1$-$C_6$-Aryl-$C_1$-$C_6$-Alkyl oder $CF_3$, in der $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl oder $C_1$-$C_6$-Aryl-$C_1$-$C_6$-Alkyl gegebenenfalls ein- oder mehrfach, gleich oder verschieden durch Sauerstoff, Schwefel oder Stickstoff unterbrochen sein können, oder für die Gruppe Sulfonyl-$C_1$-$C_6$-Alkyl, Sulfonamid, oder Cyano steht,

$R^2$ für Halogen, $CF_3$, $C_3$-$C_6$-Cycloalkyl, welches gegebenenfalls mehrfach gesättigt und gegebenenfalls mehrfach substituiert ist, oder für die Gruppe $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl, $C_1$-$C_6$-Aryl-$C_1$-$C_6$-Alkyl oder $CF_3$, in der $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl oder $C_1$-$C_6$-Aryl-$C_1$-$C_6$-Alkyl gegebenenfalls ein- oder mehrfach, gleich oder verschieden durch Sauerstoff, Schwefel oder Stickstoff unterbrochen sein können, oder für die Gruppe Sulfonyl-$C_1$-$C_6$-Alkyl, Sulfonamid, oder Cyano steht,

$R^3$ für $C_6$-$C_{12}$-Aryl, welches gegebenenfalls ein- oder mehrfach, gleich oder verschieden mit Halogen, mit $C_1$-$C_6$-Alkyl, $C_1$-$C_3$-Acyl, $C_1$-$C_3$-Alkoxy, Cyano, Hydroxy, N-$(CH_3)_2$, $CO_2$-($C_1$-$C_3$-Alkyl), CO-$NR^4R^5$ oder mit $CF_3$ substituiert sein kann, $C_5$-$C_{12}$-Heteroaryl, welches gegebenenfalls ein- oder mehrfach, gleich oder verschieden mit Chlor und / oder Fluor, mit $C_1$-$C_6$-Alkyl, $C_1$-$C_3$-Acyl, $C_1$-$C_3$-Alkoxy, Cyano, Hydroxy, N-$(CH_3)_2$, $CO_2$-($C_1$-$C_3$-Alkyl), CO-$NR^4R^5$ oder mit $CF_3$ substituiert sein kann, $C_3$-$C_6$-Cycloalkyl, welches gegebenenfalls ein- oder mehrfach, gleich oder verschieden mit Chlor und / oder Fluor, $CF_3$, Cyano, $C_1$-$C_3$-Alkyl, $C_1$-$C_3$-Acyl, Hydroxy, N-$(CH_3)_2$, $CO_2$-($C_1$-$C_3$-Alkyl), CO-$NR^4R^5$ oder $C_1$-$C_3$-Alkoxy substituiert sein kann, steht,

$R^4$ für Wasserstoff, $C_3$-$C_6$-Cycloalkyl, welches gegebenenfalls ein- oder mehrfach, gleich oder verschieden mit $C_1$-$C_6$-Alkyl, $C_1$-$C_6$-Acyl, $C_1$-$C_6$-Alkoxy oder $CF_3$ substituiert ist, für $C_6$-$C_{12}$-Aryl, welches gegebenenfalls ein- oder mehrfach, gleich oder verschieden mit Halogen, mit $C_1$-$C_6$-Alkyl, $C_1$-$C_6$-Acyl, $C_1$-$C_6$-Alkoxy, N-$C_1$-$C_6$-Alkyl- $C_1$-$C_6$-Alkyl, $CF_3$ oder Cyano substituiert ist, oder für $C_5$-$C_{12}$-Heteroaryl, welches gegebenenfalls ein- oder mehrfach, gleich oder verschieden mit Halogen, mit $C_1$-$C_6$-Alkyl, $C_1$-$C_6$-Acyl, $C_1$-$C_6$-Alkoxy, N-$C_1$-$C_6$-Alkyl-$C_1$-$C_6$-Alkyl, $CF_3$ oder Cyano, substituiert ist, oder für $C_1$-$C_6$-Alkyl, welches beliebig substituiert sein kann, steht,

$R^5$ für Wasserstoff, $C_3$-$C_6$-Cycloalkyl, welches gegebenenfalls ein- oder mehrfach, gleich oder verschieden mit $C_1$-$C_6$-Alkyl, $C_1$-$C_6$-Acyl, $C_1$-$C_6$-Alkoxy oder $CF_3$ substituiert ist, für $C_6$-$C_{12}$-Aryl, welches gegebenenfalls ein- oder mehrfach, gleich oder verschieden mit Halogen, mit $C_1$-$C_6$-Alkyl, $C_1$-$C_6$-Acyl, $C_1$-$C_6$-Alkoxy, N-$C_1$-$C_6$-Alkyl-$C_1$-$C_6$-Alkyl, $CF_3$ oder Cyano substituiert ist, oder für $C_5$-$C_{12}$-Heteroaryl, welches gegebenenfalls ein- oder mehrfach, gleich oder verschieden mit Halogen, mit $C_1$-$C_6$-Alkyl, $C_1$-$C_6$-Acyl, $C_1$-$C_6$-Alkoxy, N-$C_1$-$C_6$-Alkyl-$C_1$-$C_6$-Alkyl, $CF_3$ oder Cyano, substituiert ist, oder für $C_1$-$C_6$-Alkyl, welches beliebig substituiert sein kann, steht,

$R^4$ und $R^5$ gemeinsam einen 5-8 gliedrigen Ring bilden, der weitere Heteroatome enthalten kann, stehen und

X für die Gruppen Sulfonyl, $(CH_2)_n$ oder Carbonyl steht,

Y für Carbonyl oder $(CH_2)_n$ steht,

Z für Stickstoff steht,

n für 0 - 4 steht,

bedeuten, sowie deren Isomere, Diastereomere, Enantiomere und Salze.

Ebenfalls bevorzugt sind solche Verbindungen der allgemeinen Formel I in der

$R^1$ für Wasserstoff steht,

$R^2$ für tertiär Butyl, Cyano, Brom, oder für die Gruppe -O-$CF_3$, -$SO_2$-$CH_3$ steht und in para-Stellung steht, und

$R^3$ für $C_6$-$C_{12}$-Aryl, welches gegebenenfalls ein- oder mehrfach, gleich oder verschieden mit Halogen, mit $C_1$-$C_6$-Alkyl, $C_1$-$C_3$-Acyl, $C_1$-$C_3$-Alkoxy, Cyano, Hydroxy, N-$(CH_3)_2$, $CO_2$-($C_1$-$C_3$-Alkyl), CO-$NR^4R^5$ oder mit $CF_3$ substituiert sein kann, $C_5$-$C_{12}$-Heteroaryl, welches gegebenenfalls ein- oder mehrfach, gleich oder verschieden mit Chlor und / oder Fluor, mit $C_1$-$C_6$-Alkyl, $C_1$-$C_3$-Acyl, $C_1$-$C_3$-Alkoxy, Cyano, Hydroxy, N-$(CH_3)_2$, $CO_2$-($C_1$-$C_3$-Alkyl), CO-$NR^4R^5$ oder mit $CF_3$ substituiert sein kann, $C_3$-$C_6$-Cycloalkyl, welches gegebenenfalls ein- oder mehrfach, gleich oder verschieden mit Chlor und / oder Fluor, $CF_3$, Cyano, $C_1$-$C_3$-Alkyl, $C_1$-$C_3$-Acyl, Hydroxy, N-$(CH_3)_2$, $CO_2$-($C_1$-$C_3$-Alkyl), CO-$NR^4R^5$ oder $C_1$-$C_3$-Alkoxy substituiert sein kann, steht,

$R^4$ für Wasserstoff oder für die Gruppe $-(CH_2)_n-N-(CH_3)_2$, $-(CH_2)_2-CH_3$, $-(CH_2)_2-NH-COCH_3$, $-(CH_2)-CHCH_3-OH$, $-(CH_2)_2-O-CH_3$,$-(CH_2)_2-OH$, $-CHCH_3-CH_2-OH$,

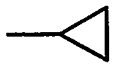
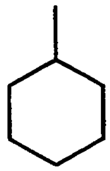
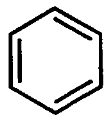
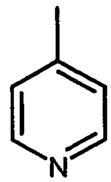
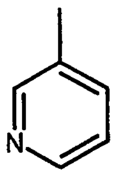
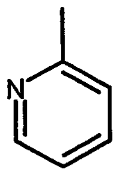
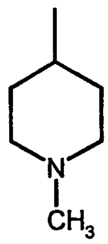
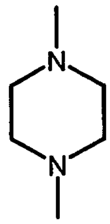
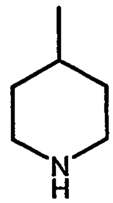
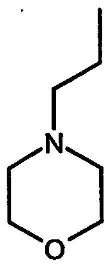
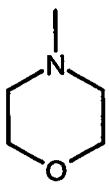
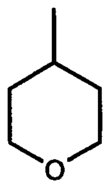
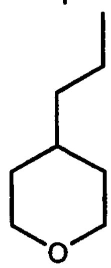
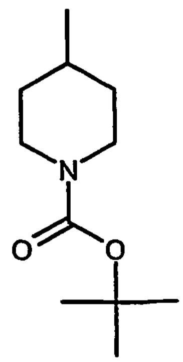

steht,

$R^5$ für Wasserstoff steht,

X für Sulfonyl, Carbonyl oder für die Gruppe $CH_2$ steht,

Y für Carbonyl oder für die Gruppe $(CH_2)n$ steht,

Z fürStickstoff bzw. für

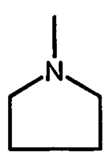
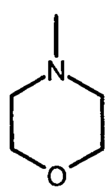

steht, und

n 1-2 steht,

bedeuten, sowie deren Isomere, Diastereomere, Enantiomere und Salze.

Ebenfalls bevorzugt sind solche Verbindungen der allgemeinen Formel I in der

$R^1$ für Wasserstoff, Halogen, $CF_3$, $C_3$-$C_6$-Cycloalkyl, welches gegebenenfalls mehrfach gesättigt und gegebenenfalls mehrfach substituiert ist, oder für die Gruppe $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl, $C_1$-$C_6$-Aryl-$C_1$-$C_6$-Alkyl oder $CF_3$, in der $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl oder $C_1$-$C_6$-Aryl-$C_1$-$C_6$-Alkyl gegebenenfalls ein- oder mehrfach, gleich oder verschieden durch Sauerstoff, Schwefel oder Stickstoff unterbrochen sein können, oder für die Gruppe Sulfonyl-$C_1$-$C_6$-Alkyl, Sulfonamid, oder Cyano steht,

$R^2$ Halogen, $CF_3$, $C_3$-$C_6$-Cycloalkyl, welches gegebenenfalls mehrfach gesättigt und gegebenenfalls mehrfach substituiert ist, oder für die Gruppe $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl, $C_1$-$C_6$-Aryl-$C_1$-$C_6$-Alkyl oder $CF_3$, in der $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl oder $C_1$-$C_6$-Aryl-$C_1$-$C_6$-Alkyl gegebenenfalls ein- oder mehrfach, gleich oder verschieden durch Sauerstoff, Schwefel oder Stickstoff unterbrochen sein können, oder für die Gruppe Sulfonyl-$C_1$-$C_6$-Alkyl, Sulfonamid, oder Cyano steht,

$R^3$ für die Gruppe

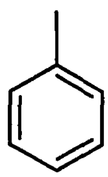 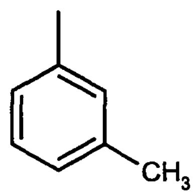 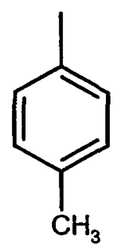 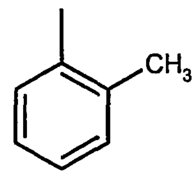 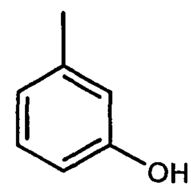

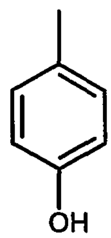 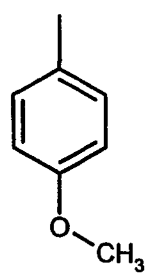 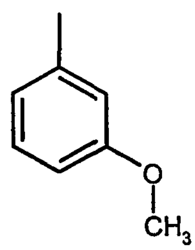 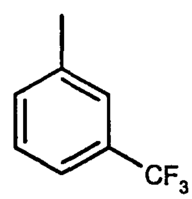 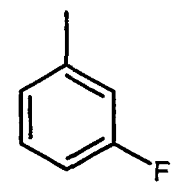

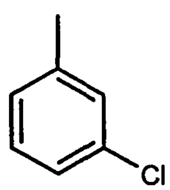 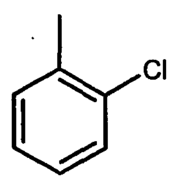 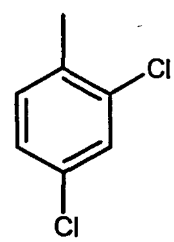 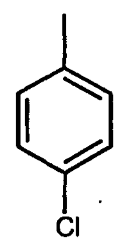

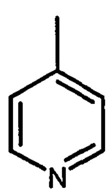 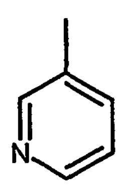 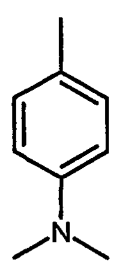

steht,

$R^4$ für Wasserstoff, $C_3$-$C_6$-Cycloalkyl, welches gegebenenfalls ein- oder mehrfach, gleich oder verschieden mit $C_1$-$C_3$-Alkyl, $C_1$-$C_3$-Acyl, $C_1$-$C_3$-Alkoxy oder $CF_3$ substituiert ist, für $C_6$-$C_{12}$-Aryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano substituiert ist, oder für $C_5$-$C_{12}$-Heteroaryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano, substituiert ist, oder für $C_1$-$C_6$-Alkyl, welches beliebig substituiert sein kann, steht,

$R^5$ für Wasserstoff, $C_3$-$C_6$-Cycloalkyl, welches gegebenenfalls ein- oder mehrfach, gleich oder verschieden mit $C_1$-$C_3$-Alkyl, $C_1$-$C_3$-Acyl, $C_1$-$C_3$-Alkoxy oder $CF_3$ substituiert ist, für $C_6$-$C_{12}$-Aryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano substituiert ist, oder für $C_5$-$C_{12}$-Heteroaryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano, substituiert ist, oder für $C_1$-$C_6$-Alkyl, welches beliebig substituiert sein kann, steht,

$R^4$ und $R^5$ gemeinsam einen 5-8 gliedrigen Ring bilden, der weitere Heteroatome enthalten kann, steht,

X für die Gruppen Sulfonyl, $(CH_2)_n$ oder Carbonyl steht,

Y für Carbonyl oder $(CH_2)_n$ steht,

Z für Stickstoff steht, und

n für 0 - 2 steht,

bedeuten, sowie deren Isomere, Diastereomere, Enantiomere und Salze.

Ebenfalls bevorzugt sind solche Verbindungen der allgemeinen Formel I in der

$R^1$ für Wasserstoff steht,

$R^2$ für tertiär Butyl, Cyano, Brom, oder für die Gruppe -O-$CF_3$, -$SO_2$-$CH_3$ steht und in para-Stellung steht, und

$R^3$ für die Gruppe

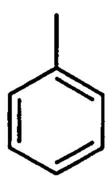 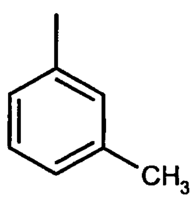 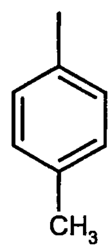 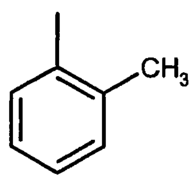 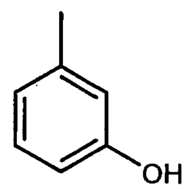

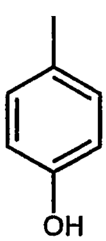 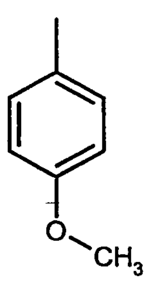 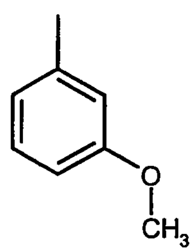 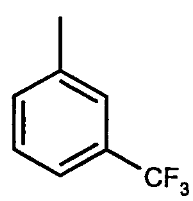 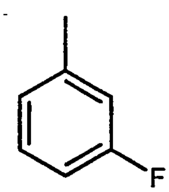

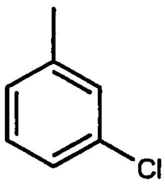 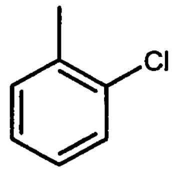 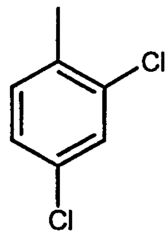 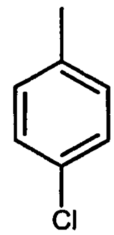

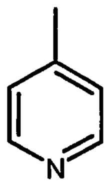 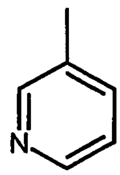 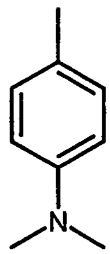

steht,

$R^4$ für Wasserstoff, $C_3$-$C_6$-Cycloalkyl, welches gegebenenfalls ein- oder mehrfach, gleich oder verschieden mit $C_1$-$C_3$-Alkyl, $C_1$-$C_3$-Acyl, $C_1$-$C_3$-Alkoxy oder $CF_3$ substituiert ist, für $C_6$-$C_{12}$-Aryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano substituiert ist, oder für $C_5$-$C_{12}$-Heteroaryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano, substituiert ist, oder für $C_1$-$C_6$-Alkyl, welches beliebig substituiert sein kann, steht,

$R^5$ für Wasserstoff, $C_3$-$C_6$-Cycloalkyl, welches gegebenenfalls ein- oder mehrfach, gleich oder verschieden mit $C_1$-$C_3$-Alkyl, $C_1$-$C_3$-Acyl, $C_1$-$C_3$-Alkoxy oder $CF_3$ substituiert ist, für $C_6$-$C_{12}$-Aryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano substituiert ist, oder für $C_5$-$C_{12}$-Heteroaryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano, substituiert ist, oder für $C_1$-$C_6$-Alkyl, welches beliebig substituiert sein kann, steht,

X für Sulfonyl, Carbonyl oder für die Gruppe $CH_2$ steht,

Y für Carbonyl oder für die Gruppe $(CH_2)n$ steht,

Z für Stickstoff bzw. für

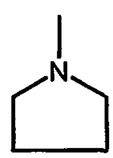 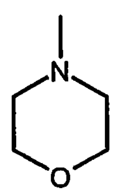

steht, und

n 1-2 steht,

bedeuten, sowie deren Isomere, Diastereomere, Enantiomere und Salze.

Ebenfalls bevorzugt sind solche Verbindungen der allgemeinen Formel I in der

$R^1$ für Wasserstoff, Halogen, $CF_3$, $C_3$-$C_6$-Cycloalkyl, welches gegebenenfalls mehrfach gesättigt und gegebenenfalls mehrfach substituiert ist, oder für die Gruppe $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl, $C_1$-$C_6$-Aryl-$C_1$-$C_6$-Alkyl oder $CF_3$, in der $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl oder $C_1$-$C_6$-Aryl-$C_1$-$C_6$-Alkyl gegebenenfalls ein- oder mehrfach, gleich oder verschieden durch Sauerstoff, Schwefel oder Stickstoff unterbrochen sein können, oder für die Gruppe Sulfonyl-$C_1$-$C_6$-Alkyl, Sulfonamid, oder Cyano steht,

$R^2$ Halogen, $CF_3$, $C_3$-$C_6$-Cycloalkyl, welches gegebenenfalls mehrfach gesättigt und gegebenenfalls mehrfach substituiert ist, oder für die Gruppe $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl, $C_1$-$C_6$-Aryl-$C_1$-$C_6$-Alkyl oder $CF_3$, in der $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl oder $C_1$-$C_6$-Aryl-$C_1$-$C_6$-Alkyl gegebenenfalls ein- oder mehrfach, gleich oder verschieden durch Sauerstoff, Schwefel oder Stickstoff unterbrochen sein können, oder für die Gruppe Sulfonyl-$C_1$-$C_6$-Alkyl, Sulfonamid, oder Cyano steht,

$R^3$ für $C_6$-$C_{12}$-Aryl, welches gegebenenfalls ein- oder mehrfach, gleich oder verschieden mit Halogen, mit $C_1$-$C_6$-Alkyl, $C_1$-$C_3$-Acyl, $C_1$-$C_3$-Alkoxy, Cyano, Hydroxy, $N(CH_3)_2$, $CO_2$-($C_1$-$C_3$-Alkyl), CO-$NR^4R^5$ oder mit $CF_3$ substituiert sein kann, $C_5$-$C_{12}$-Heteroaryl, welches gegebenenfalls ein- oder mehrfach, gleich oder verschieden mit Chlor und / oder Fluor, mit $C_1$-$C_6$-Alkyl, $C_1$-$C_3$-Acyl, $C_1$-$C_3$-Alkoxy, Cyano, Hydroxy, $N(CH_3)_2$, $CO_2$-($C_1$-$C_3$-Alkyl), CO-$NR^4R^5$ oder mit $CF_3$ substituiert sein kann, $C_3$-$C_6$-Cycloalkyl, welches gegebenenfalls ein- oder mehrfach, gleich oder verschieden mit Chlor und / oder Fluor, $CF_3$, Cyano, $C_1$-$C_3$-Alkyl, $C_1$-$C_3$-Acyl, Hydroxy, $N(CH_3)_2$, $CO_2$-($C_1$-$C_3$-Alkyl), CO-$NR^4R^5$ oder $C_1$-$C_3$-Alkoxy substituiert sein kann, steht,

$R^4$ für Wasserstoff oder für die Gruppe $-(CH_2)_n-N-(CH_3)_2$, $-(CH_2)_2-CH_3$, $-(CH_2)_2-NH-COCH_3$, $-(CH_2)-CHCH_3-OH$, $-(CH_2)_2-O-CH_3$,$-(CH_2)_2-OH$, $-CHCH_3-CH_2-OH$,

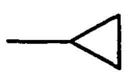 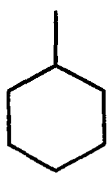 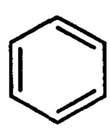 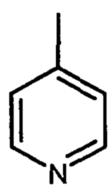

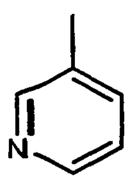 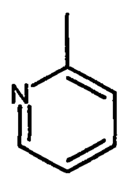 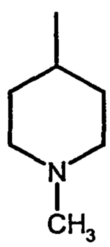 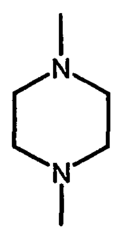 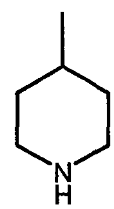

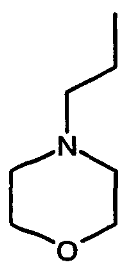 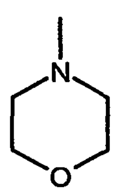 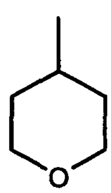 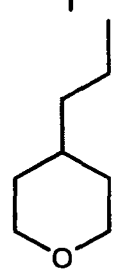 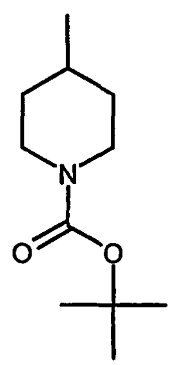

steht,

$R^5$ für Wasserstoff steht,

X für die Gruppen Sulfonyl, $(CH_2)_n$ oder Carbonyl steht,

Y für Carbonyl oder $(CH_2)_n$ steht,

Z für Stickstoff steht, und

n für 0 - 2 steht,

bedeuten, sowie deren Isomere, Diastereomere, Enantiomere und Salze.

Ebenfalls bevorzugt sind solche Verbindungen der allgemeinen Formel I in der

$R^1$ für Wasserstoff, Halogen, $CF_3$, $C_3$-$C_6$-Cycloalkyl, welches gegebenenfalls mehrfach gesättigt und gegebenenfalls mehrfach substituiert ist, oder für die Gruppe $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl, $C_1$-$C_6$-Aryl-$C_1$-$C_6$-Alkyl oder $CF_3$, in der $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl oder $C_1$-$C_6$-Aryl-$C_1$-$C_6$-Alkyl gegebenenfalls ein- oder mehrfach, gleich oder verschieden durch Sauerstoff, Schwefel oder Stickstoff unterbrochen sein können, oder für die Gruppe Sulfonyl-$C_1$-$C_6$-Alkyl, Sulfonamid, oder Cyano steht,

$R^2$ Halogen, $CF_3$, $C_3$-$C_6$-Cycloalkyl, welches gegebenenfalls mehrfach gesättigt und gegebenenfalls mehrfach substituiert ist, oder für die Gruppe $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl, $C_1$-$C_6$-Aryl-$C_1$-$C_6$-Alkyl oder $CF_3$, in der $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl oder $C_1$-$C_6$-Aryl-$C_1$-$C_6$-Alkyl gegebenenfalls ein- oder mehrfach, gleich oder verschieden durch Sauerstoff, Schwefel oder Stickstoff unterbrochen sein können, oder für die Gruppe Sulfonyl-$C_1$-$C_6$-Alkyl, Sulfonamid, oder Cyano steht,

$R^3$ für $C_6$-$C_{12}$-Aryl, welches gegebenenfalls ein- oder mehrfach, gleich oder verschieden mit Halogen, mit $C_1$-$C_6$-Alkyl, $C_1$-$C_3$-Acyl, $C_1$-$C_3$-Alkoxy, Cyano, Hydroxy, $N(CH_3)_2$, $CO_2$-($C_1$-$C_3$-Alkyl), CO-$NR^4R^5$ oder mit $CF_3$ substituiert sein kann, $C_5$-$C_{12}$-Heteroaryl, welches gegebenenfalls ein- oder mehrfach, gleich oder verschieden mit Chlor und / oder Fluor, mit $C_1$-$C_6$-Alkyl, $C_1$-$C_3$-Acyl, $C_1$-$C_3$-Alkoxy, Cyano, Hydroxy, $N(CH_3)_2$, $CO_2$-($C_1$-$C_3$-Alkyl), CO-$NR^4R^5$ oder mit $CF_3$ substituiert sein kann, $C_3$-$C_6$-Cycloalkyl, welches gegebenenfalls ein- oder mehrfach, gleich oder verschieden mit Chlor und / oder Fluor, $CF_3$, Cyano, $C_1$-$C_3$-Alkyl, $C_1$-$C_3$-Acyl, Hydroxy, $N(CH_3)_2$, $CO_2$-($C_1$-$C_3$-Alkyl), CO-$NR^4R^5$ oder $C_1$-$C_3$-Alkoxy substituiert sein kann, steht,

$R^4$ für Wasserstoff, $C_3$-$C_6$-Cycloalkyl, welches gegebenenfalls ein- oder mehrfach, gleich oder verschieden mit $C_1$-$C_3$-Alkyl, $C_1$-$C_3$-Acyl, $C_1$-$C_3$-Alkoxy oder $CF_3$ substituiert ist, für $C_6$-$C_{12}$-Aryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano substituiert ist, oder für $C_5$-$C_{12}$-Heteroaryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano, substituiert ist, oder für $C_1$-$C_6$-Alkyl, welches beliebig substituiert sein kann, steht,

$R^5$ für Wasserstoff, $C_3$-$C_6$-Cycloalkyl, welches gegebenenfalls ein- oder mehrfach, gleich oder verschieden mit $C_1$-$C_3$-Alkyl, $C_1$-$C_3$-Acyl, $C_1$-$C_3$-Alkoxy oder $CF_3$ substituiert ist, für $C_6$-$C_{12}$-Aryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano substituiert ist, oder für $C_5$-$C_{12}$-Heteroaryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano, substituiert ist, oder für $C_1$-$C_6$-Alkyl, welches beliebig substituiert sein kann, steht,

$R^4$ und $R^5$ gemeinsam einen 5-8 gliedrigen Ring bilden, der weitere Heteroatome enthalten kann, steht,

X für die Gruppen Sulfonyl, $(CH_2)_n$ oder Carbonyl steht,

Y für Carbonyl oder $(CH_2)_n$ steht,

Z für Stickstoff bzw. für

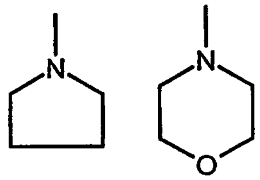

steht und

n für 0 - 2 steht,

bedeuten, sowie deren Isomere, Diastereomere, Enantiomere und Salze.

Die folgenden Verbindungen entsprechend der vorliegenden Erfindung sind ganz besonders bevorzugt:

1. 5-(4-tert-Butylbenzolsulfonylamino)-3-phenyl-1*H*-indol-2-carbonsäurecyclopropylamid
2. 5-(4-tert-Butylbenzolsulfonylamino)-3-phenyl-1*H*-indol-2-carbonsäure-pyridin-3-ylamid
3. 5-(4-tert-Butylbenzolsulfonylamino)-3-phenyl-1*H*-indol-2-carbonsäurecyclohexylamid
4. 4-tert-Butyl-N-[3-phenyl-2-(pyrrolidin-1-carbonyl)-1*H*-indol-5-yl]-benzolsulfonamid
5. 4-tert-Butyl-N-[2-(morpholin-4-carbonyl)-3-phenyl-1*H*-indol-5yl]-benzolsulfonamid
6. 5-(4-tert-Butylbenzolsulfonylamino)-3-phenyl-1*H*-indol-2-carbonsäure-(2-dimethylaminoethyl)amid
7. 5-(4-Cyanobenzolsulfonylamino)-3-phenyl-1*H*-indol-2-carbonsäurepropylamid
8. 5-(4-Brombenzolsulfonylamino)-3-phenyl-1*H*-indol-2-carbonsäurepropylamid
9. 5-(4-tert-Butylbenzolsulfonylamino)-3-phenyl-1*H*-indol-2-carbonsäurepropylamid
10. 5-(4-(Trifluormethoxy)benzolsulfonylamino)-3-phenyl-1*H*-indol-2-carbonsäurepropylamid
11. 5-(4-(Methylsulfonyl)benzolsulfonylamino)-3-phenyl-1 H-indol-2-carbonsäurepropylamid
12. 5-(4-tert-Butylbenzolsulfonylamino)-3-phenyl-1*H*-indol-2-carbonsäurephenylamid
13. 5-(4-Cyanobenzolsulfonylamino)-3-phenyl-1*H*-indol-2-carbonsäurephenylamid
14. 5-(4-Brombenzolsulfonylamino)-3-phenyl-1*H*-indol-2-carbonsäurephenylamid
15. 5-(4-(Trifluormethoxy)benzolsulfonylamino)-3-phenyl-1H-indol-2-carbonsäurephenylamid
16. 5-(4-(Methylsulfonyl)benzolsulfonylamino)-3-phenyl-1*H*-indol-2-carbonsäurephenylamid
17. 5-(4-tert-Butylbenzolsulfonylamino)-3-phenyl-1*H*-indol-2-carbonsäure-pyridin-2-ylamid
18. 5-(4-Cyanobenzolsulfonylamino)-3-phenyl-1*H*-indol-2-carbonsäure-pyfidin-2-ylamid
19. 5-(4-tert-Butylbenzolsulfonylamino)-3-phenyl-1*H*-indol-2-carbonsäure-(2-morpholin-4-ylethyl)amid
20. 5-(4-tert-Butylbenzolsulfonylamino)-3-phenyl-1*H*-indol-2-carbonsäure-(4-methylpiperazin-1-yl)amid
21. 5-(4-tert-Butylbenzolsulfonylamino)-3-phenyl-1*H*-indol-2-carbonsäure-pyrindin-4-ylamid
22. 5-(4-tert-Butylbenzylamino)-3-phenyl-1*H*-indol-2carbonsäurepyridin-4-ylamid
23. 5-(4-tert-Butylbenzoylamino)-3-phenyl-1*H*-indol-2-carbonsäure-(2-dimethylaminoethyl)amid
24. 5-(4-tert-Butylbenzolsulfonylamino)-3-(2-chlorophenyl)-1H-indol-2-carbonsäure-(2-dimethylaminoethyl)amid
25. 5-(4-tert-Butylbenzolsulfonylamino)-3-(3-chlorphenyl)-1*H*-indol-2-carbonsäure-(2-dimethylaminoethyl)amid
26. 5-(4-tert-Butylbenzolsulfonylamino)-3-(4-chlorphenyl)-1*H*-indol-2-carbonsäure-(2-dimethylaminoethyl)amid
27. 5-(4-tert-Butylbenzolsulfonylamino)-3-(2,4-dichlorphenyl)-1*H*-indol-2-carbonsäure-(2-dimethylaminoethyl)amid
28. 5-(4-tert-Butylbenzolsulfonylamino)-3-(2-methylphenyl)-1*H*-indol-2-carbonsäure-(2-dimethylaminoethyl)amid
29. 5-(4-tert-Butylbenzolsulfonylamino)-3-(4-methylphenyl)-1*H*-indol-2-carbonsäure-(2-dimethylaminoethyl)amid
30. 5-(4-tert-Butylbenzolsulfonylamino)-3-(4-methylphenyl)-1*H*-indol-2-carbonsäurepyridin-4ylamid
31. 5-(4-tert-Butylbenzolsulfonylamino)-3-(4-methoxyphenyl)-1*H*-indol-2-carbonsäure-(2-dimethylaminoethyl)amid
32. 5-(4-tert-Butylbenzolsulfonylamino)-3-pyridin-3-yl-1*H*-indol-2-carbon-säure-(2-dimethylaminoethyl)amid
33. 5-(4-tert-Butylbenzolsulfonylamino)-3-(4-hydroxyphenyl)-1H-indol-2-carbonsäure-(2-dimethylaminoethyl)amid
34. 5-(4-tert-Butylbenzolsulfonylamino)-3-(3-fluorphenyl)-1*H*-indol-2-carbonsäure-(2-dimethylaminoethyl)amid
35. 5-(4-tert-Butylbenzolsulfonylamino)-3-phenyl-1*H*-indol-2-carbonsäure-(2-hydroxypropyl)amid
36. 5-(4-tert-Buiylbenzolsulfonylamino)-3-phenyl-1*H*-indol-2-carbonsäure-(2-methoxyethyl)amid
37. 5-(4-tert-Butylbenzolsulfonylamino)-3-phenyl-1*H*-indol-2-carbonsäure-(2-hydroxyethyl)amid

38. 5-(4-tert-Butylbenzolsulfonylamino)-3-phenyl-1*H*-indol-2-carbonsäure-(2-hydroxy-1-methylethyl)amid
39. 5-(4-tert-Butylbenzolsulfonylamino)-3-phenyl-1*H*-indol-2-carbonsäure-(2-acetylaminoethyl)amid
40. 5-(4-tert-Butylbenzolsulfonylamino)-3-phenyl-1*H*-indol-2-carbonsäure-(tetrahydropyran-4-yl)amid
41. 5-(4-tert-Butylbenzolsulfonylamino)-3-phenyl-1*H*-indol-2-carbonsäure-(1-methylpiperidin-4-yl)amid
42. 5-(4-tert-Butylbenzolsulfonylamino)-3-(4-N,N-dimethylaminophenyl)-1*H*-indol-2-carbonsäure-(2-dimethylaminoethyl)amid
43. 5-(4-tert-Butylbenzolsulfonylamino)-3-(3-methoxyphenyl)-1*H*-indol-2-carbonsäure-(2-dimethylaminoethyl)amid
44. 5-(4-tert-Butylbenzolsulfonylamino)-3-(3-trifluormethylphenyl)-1*H*-indol-2-carbonsäure-(2-dimethylaminoethyl)amid
45. 5-(4-tert-Butylbenzolsulfonylaminor3-(3-fluorphenyl)-1*H*-indol-2-carbonsäure-(2-hydroxyethyl)amid
46. 5-(4-tert-Butylbenzolsulfonylamino)-3-(3-fluorphenyl)-1*H*-indol-2-carbonsäure-(tetrahydropyran-4-yl)amid
47. 5-(4-tert-Butylbenzolsulfonylamino)-3-(3-fluorphenyl)-1*H*-indol-2-carbonsäure-(2-acetylaminoethyl)amid
48. 5-(4-tert-Butylbenzolsulfonylamino)-3-(3-fluorphenyl)-1*H*-indol-2-carbonsäure-(2-morpholin-4-ylethyl)amid
49. 5-(4-tert-Butylbenzolsulfonylamino)-3-(3-methoxyphenyl)-1*H*-indol-2-carbonsäure-(2-hydroxyethyl)amid
50. 5-(4-tert-Butylbenzolsulfonylamino)-3-(3-methoxyphenyl)-1*H*-indol-2-carbonsäure-(2-acetylaminoethyl)amid
51. 5-(4-tert-Butylbenzolsulfonylamino)-3-pyridin-3-yl-1*H*-indol-2-carbon-säure-(2-acetylaminoethyl)amid
52. 5-(4-tert-Butylbenzolsulfonylamino)-3-pyridin-3-yl-1*H*-indol-2-carbon-säure-(tetrahydropyran-4yl)amid
53. 5-(4-tert-Butylbenzolsulfonylamino)-3-pyridin-4-yl-1*H*-indol-2-carbon-säure-(2-acetylaminoethyl)amid
54. 5-(4-tert-Butylbenzolsulfonylamino)-3-pyridin-4-yl-1*H*-indol-2-carbon-säure-(2-morpholin-4-ylethyl)amid
55. 5-(4-tert-Butylbenzolsulfonylamino)-3-pyridin-4-yl-1*H*-indol-2-carbon-säure-(tetrahydropyran-4-yl)amid
56. 5-(4-tert-Butylbenzolsulfonylamino)-3-(3-methylphenyl)-1*H*-indol-2-carbonsäure-(2-acetylaminoethyl)amid
57. 5-(4-tert-Butylbenzolsulfonylamino)-3-(3-methylphenyl)-1*H*-indol-2-carbonsäure-(2-hydroxyethyl)amid
58. 5-(4-tert-Butylbenzolsulfonylamino)-3-(3-methylphenyl)-1*H*-indol-2-carbonsäure-(2-morpholin-4-ylethyl)amid
59. 5-(4-tert-Butylbenzolsulfonylamino)-3-(3-methylphenyl)-1*H*-indol-2-carbonsäure-(tetrahydropyran-4-yl)amid
60. 5-(4-tert-Butylbenzolsulfonylamino)-3-pyridin-3-yl-1*H*-indol-2-carbon-säure-(2-morpholin-4-ylethyl)amid
61. 5-(4-tert-Butylbenzolsulfonylamino)-3-(3-methoxyphenyl)-1*H*-indol-2-carbonsäure-(tetrahydropyran-4-yl)amid
62. 5-(4-tert-Butylbenzolsulfonylamino)-3-(3-methoxyphenyl)-1*H*-indol-2-carbonsäure-(2-morpholin-4-ylethyl)amid
63. 5-(4-tert-Butylbenzolsulfonylamino)-3-phenyl-1*H*-indol-2-carbonsäurepiperidin-4-ylamid
64. 5-(4-tert-Butylbenzolsulfonylamino)-3-phenyl-1*H*-indol-2-carbonyl]-piperidin-1-carbonsäure-tert-butylester
65. 5-(4-tert-Butyl-phenylsulfonyl-amino)-3-naphthalin-1-yl-1*H*-indol-2-carbonsäure-(2-morpholin-4-yl-ethyl)-amid
66. 5-(4-tert-Butyl-phenylsulfonyl-amino)-3-m-tolyl-1*H*-indol-2-carbonsäure-(2-morpholin-4-yl-ethyl)-amid
67. 5-(4-tert-Butyl-phenylsulfonyl-amino)-3-thiophen-2-yl-1*H*-indol-2-carbonsäure-(2-morpholin-4-yl-ethyl)-amid
68. 5-(4-tert-Butyl-phenylsulfonyl-amino)-3-thiophen-3-yl-1*H*-indol-2-carbonsäure-(2-morpholin-4-yl-ethyl)-amid
69. 3-Benzofuran-2-yl-5-(4-tert-butyl-phenylsulfonylamino)-1*H*-indol-2-carbonsäure-(2-morpholin-4-yl-ethyl)-amid
70. 5-(4-tert-Butyl-phenylsulfonyl-amino)-3-(5-chlor-thiophen-2-yl)-1*H*-indol-2-carbonsäure-(2-morpholin-4-yl-ethyl)-amid
71. 5-(4-tert-Butyl-phenylsulfonyl-amino)-3-furan-2-yl-1 H-indol-2-carbonsäure-(2-morpholin-4-yl-ethyl)-amid
72. 5-(4-tert-Butyl-phenylsulfonyl-amino)-3-(3-fluor-4-methoxy-phenyl)-1H-indol-2-carbonsäure-(2-morpholin-4-yl-ethyl)-amid
73. 3-Benzo[1,3]dioxol-5-yl-5-(4-tert-butyl-phenylsulfonylamino)-1H-indol-2-carbonsäure-(2-morpholin-4-yl-ethyl)-amid
74. 3-(4-Acetyl-phenyl)-5-(4-tert-butyl-phenylsulfonylamino)-1H-indol-2-carbonsäure-(2-morpholin-4-yl-ethyl)-amid
75. 3-(3-Acetyl-phenyl)-5-(4-tert-butyl-phenylsulfonylamino)-1H-indol-2-carbonsäure-(2-morpholin-4-yl-ethyl)-amid
76. 3-Benzo[b]thiophen-2-yl-5-(4-tert-butyl-phenylsulfonylamino)-1H-indol-2-carbonsäure-(2-morpholin-4-yl-ethyl)-amid
77. 3-Benzo[b]thiophen-3-yl-5-(4-tert-butyl-phenylsulfonylamino)-1H-indol-2-carbonsäure-(2-morpholin-4-yl-ethyl)-amid
78. 5-(4-tert-Butyl-phenylsulfonyl-amino)-3-(5-methyl-thiophen-2-yl)-1H-indol-2-carbonsäure-(2-morpholin-4-yl-ethyl)-amid
79. 3-[5-(4-tert-Butyl-phenylsulfonyl-amino)-2-(2-morpholin-4-yl-ethylcarbamoyl)-1 H-indol-3-yl]-benzoesäure methylester
80. 5-(4-tert-Butyl-phenylsulfonyl-amino)-3-(2-fluor-3-methoxy-phenyl)-1H-indol-2-carbonsäure-(2-morpholin-4-yl-ethyl)-amid
81. 5-(4-tert-Butyl-phenylsulfonyl-amino)-3-(3-chlor-4-methyl-phenyl)-1H-indol-2-carbonsäure-(2-morpholin-4-yl-ethyl)-amid
82. 5-(4-tert-Butyl-phenylsulfonyl-amino)-3-(2,4-dimethoxy-pyrimidin-5-yl)-1H-indol-2-carbonsäure-(2-morpholin-4-yl-ethyl)-amid
83. 5-(4-tert-Butyl-phenylsulfonyl-amino)-3-(2,5-difluor-phenyl)-1H-indol-2-carbonsäure-(2-morpholin-4-yl-ethyl)-amid
84. 5-(4-tert-Butyl-phenylsulfonyl-amino)-3-(2,4-difluor-phenyl)-1H-indol-2-carbonsäure-(2-morpholin-4-yl-ethyl)-amid
85. 5-(4-tert-Butyl-phenylsulfonyl-amino)-3-(2,3-difluor-phenyl)-1H-indol-2-carbonsäure-(2-morpholin-4-yl-ethyl)-amid
86. 5-(4-tert-Butyl-phenylsulfonyl-amino)-3-(2,6-difluor-phenyl)-1H-indol-2-carbonsäure-(2-morpholin-4-yl-ethyl)-amid
87. 5-(4-tert-Butyl-phenylsulfonyl-amino)-3-(3-hydroxy-phenyl)-1H-indol-2-carbonsäure-(2-morpholin-4-yl-ethyl)-amid
88. 5-(4-tert-Butyl-phenylsulfonyl-amino)-3-(4-hydroxy-phenyl)-1H-indol-2-carbonsäure-(2-morpholin-4-yl-ethyl)-amid
89. 5-(4-tert-Butyl-phenylsulfonyl-amino)-3-(3-fluor-4-methyl-phenyl)-1H-indol-2-carbonsäure-(2-morpholin-4-yl-ethyl)-amid
90. 5-(4-tert-Butyl-phenylsulfonyl-amino)-3-(4-trifluormethyl-phenyl)-1H-indol-2-carbonsäure-(2-morpholin-4-yl-ethyl)-amid
91. 5-(4-tert-Butyl-phenylsulfonyl-amino)-3-(4-cyanomethyl-phenyl)-1 H-indol-2-carbonsäure-(2-morpholin-4-yl-ethyl)-amid
92. 5-(4-tert-Butyl-phenylsulfonyl-amino)-1H,1'H-[3,4']biindolyl-2-carbonsäure-(2-morpholin-4-yl-ethyl)-amid
93. 5-(4-tert-Butyl-phenylsulfonyl-amino)-3-(3-cyano-4-fluor-phenyl)-1H-indol-2-carbonsäure-(2-morpholin-4-yl-ethyl)-amid
94. 5-(4-tert-Buty1-phenylsulfonyl-amino)-3-(2-fluor-phenyl)-1H-indol-2-carbonsäure-(2-morpholin-4-yl-ethyl)-amid
95. 5-(4-tert-Butyl-phenylsulfonyl-amino)-3-(3,4-difluor-phenyl)-1H-indol-2-carbonsäure-(2-morpholin-4-yl-ethyl)-amid
96. 5-(4-tert-Butyl-phenylsulfonyl-amino)-3-(3-cyano-phenyl)-1H-indol-2-carbonsäure-(2-morpholin-4-yl-ethyl)-amid
97. 5-(4-tert-Butyl-phenylsulfonyl-amino)-3-(4-cyano-phenyl)-1H-indol-2-carbonsäure-(2-morpholin-4-yl-ethyl)-amid
98. 5-(4-tert-Butyl-phenylsulfonyl-amino}-3-(4-methyl-thiophen-2-yl)-1H-indol-2-carbonsäure-(2-morpholin-4-yl-ethyl)-amid
99. 5-(4-tert-Butyl-phenylsulfonyl-amino)-3-phenyl-1H-indol-2-carbonsäure-(3-chloro-phenyl)-amid
100. 5-(4-tert-Butyl-phenylsulfonyl-amino)-3-phenyl-1H-indol-2-carbonsäure-(3-methyl-isoxazol-5-yl)-amid
101. 5-(4-tert-Butyi-phenylsulfonyl-amino)-3-phenyl-1H-indol-2-carbonsäure-(4-fluoro-phenyl)-amid
102. 5-(4-tert-Butyl-phenylsulfonyl-amino)-3-phenyl-1H-indol-2-carbonsäure-(2-fluoro-phenyl)-amid
103. 5-(4-tert-Butyl-phenylsulfonyl-amino)-3-phenyl-1H-indol-2-carbonsäure-(6-methyl-pyridin-2-yl)-amid
104. 5-(4-tert-Butyl-phenylsulfonyl-amino)-3-phenyl-1H-indol-2-carbonsäure-(5-carbamoyl-pyridin-2-yl)-amid
105. 5-(4-tert-Butyl-phenylsulfonyl-amino)-3-phenyl-1 H-indol-2-carbonsäure-(4-hydroxy-phenyl)-amid
106. 5-(4-tert-Butyl-phenylsulfonyl-amino)-3-phenyl-1 H-indol-2-carbonsäure-(2-methoxy-phenyl)-amid
107. 5-(4-tert-Butyl-phenylsulfonyl-amino)-3-phenyl-1H-indol-2-carbonsäure-(3-methoxy-phenyl)-amid
108. 5-(4-tert-Butyl-phenylsulfonyl-amino)-3-phenyl-1H-indol-2-carbonsäure-(4-methoxy-phenyl)-amid
109. 5-(4-tert-Butyl-phenylsulfonyl-amino)-3-phenyl-1H-indol-2-carbonsäure-(4-chloro-phenyl)-amid
110. 5-(4-tert-Butyl-phenylsulfonyl-amino)-3-phenyl-1H-indol-2-carbonsäure-(4-dimethylamino-phenyl)-amid
111. 5-(4-tert-Butyl-phenylsulfonyl-amino)-3-phenyl-1 H-indol-2-carbonsäure-(5-chlor-pyridin-2-yl)-amid
112. 5-(4-tert-Butyl-phenylsulfonyl-amino)-3-phenyl-1 H-indol-2-carbonsäure-p-tolylamid
113. 5-(4-tert-Butyl-phenylsulfonyl-amino)-3-phenyl-1H-indol-2-carbonsäurepyrazin-2-ylamid
114. 5-(4-tert-Butyl-phenylsulfonyl-amino)-3-phenyl-1 H-indol-2-carbonsäure-(4-cyano-phenyl)-amid
115. 5-(4-tert-Butyl-phenylsulfonyl-amino)-3-phenyl-1H-indol-2-carbonsäure-(3-methyl-isothiazol-5-yl)-amid
116. 5-(4-tert-Butyl-phenylsulfonyl-amino)-3-phenyl-1H-indol-2-carbonsäure-(4-brom-phenyl)-amid
117. 5-(4-tert-Butyl-phenylsulfonyl-amino)-3-phenyl-1 H-indol-2-carbonsäure-(4-carbamoyl-phenyl)-amid
118. 5-(4-tert-Butyl-phenylsulfonyl-amino)-3-phenyl-1H-indol-2-carbonsäure-(3-methyl-pyridin-2-yl)-amid
119. 5-(4-tert-Butyl-phenylsulfonyl-amino)-3-phenyl-1H-indol-2-carbonsäure-(2-chlor-phenyl)-amid
120. 5-(4-tert-Butyl-phenylsulfonyl-amino)-3-phenyl-1H-indol-2-carbonsäure-(5-methyl-2H-pyrazol-3-yl)-amid
121. 5-(4-tert-Butyl-phenylsulfonyl-amino)-3-phenyl-1H-indol-2-carbonsäurequinolin-5-ylamid
122. 5-(4-tert-Butyl-phenylsulfonyl-amino)-3-phenyl-1H-indol-2-carbonsäurequinolin-6-ylamid
123. 5-(4-tert-Butyl-phenylsulfonyl-amino)-3-phenyl-1H-indol-2-carbonsäure-(2,6-dichlor-pyridin-4-yl)-amid
124. 5-(4-tert-Butyl-phenylsulfonyl-amino)-3-phenyl-1H-indol-2-carbonsäure-(3-fluor-phenyl)-amid
125. 5-(4-tert-Butyl-phenylsulfonyl-amino)-3-phenyl-1H-indol-2-carbonsäure-(2-methyl-pyridin-4-yl)-amid
126. 5-(4-tert-Butyl-phenylsulfonyl-amino)-3-phenyl-1H-indol-2-carbonsäure-(3-fluor-pyridin-4-yl)-amid
127. 5-(4-tert-Butyl-phenylsulfonyl-amino)-3-phenyl-1H-indol-2-carbonsäure-(3-methyl-pyridin-4-yl)-amid

128. 5-(4-tert-Butyl-phenylsulfonyl-amino)-3-phenyl-1H-indol-2-carbonsäure-(3-brom-pyridin-4-yl)-amid
129. 5-(4-tert-Butyl-phenylsulfonyl-amino)-3-phenyl-1H-indol-2-carbonsäure-(2,3-dihydroxy-propyl)-amid
130. 5-(4-tert-Butyl-phenylsulfonyl-amino)-3-phenyl-1H-indol-2-carbonsäure-(2-oxo-tetrahydro-thiophen-3-yl)-amid
131. 5-(4-tert-Butyl-phenylsulfonyl-amino)-3-phenyl-1H-indol-2-carbonsäure-[2-(2-oxo-imidazolidin-1-yl)-ethyl]-amid
132. 5-(4-tert-Butyl-phenylsulfonyl-amino)-3-phenyl-1H-indol-2-carbonsäure-(2,2-diethoxy-ethyl)-amid
133. 5-(4-tert-Butyl-phenylsulfonyl-amino)-3-phenyl-1 H-indol-2-carbonsäure-(3-ethoxy-propyl)-amid
134. 5-(4-tert-Butyl-phenylsulfonyl-amino)-3-phenyl-1H-indol-2-carbonsäure-(3-isopropoxy-propyl)-amid
135. 5-(4-tert-Butyl-phenylsulfonyl-amino)-3-phenyl-1H-indol-2-carbonsäure-(3-morpholin-4-yl-propyl)-amid
136. 5-(4-tert-Butyl-phenylsulfonyl-amino)-3-phenyl-1 H-indol-2-carbonsäure-(3-diethylamino-propyl)-amid
137. 5-(4-tert-Butyl-phenylsulfonyl-amino)-3-phenyl-1H-indol-2-carbonsäure-(3-dimethylamino-propyl)-amid
138. 5-(4-tert-Butyl-phenylsulfonyl-amino)-3-phenyl-1H-indol-2-carbonsäure-(furan-2-ylmethyl)-amid
139. 5-(4-tert-Butyl-phenylsulfonyl-amino)-3-phenyl-1H-indol-2-carbonsäure-(2-methylsulfanyl-ethyl)-amid
140. 5-(4-tert-Butyl-phenylsulfonyl-amino)-3-phenyl-1 H-indol-2-carbonsäure-(2-diethylamino-ethyl)-amid
141. 5-(4-tert-Butyl-phenylsulfonyl-amino)-3-phenyl-1H-indol-2-carbonsäure-[2-(3,4-dimethoxy-phenyl)-ethyl]-amid
142. 5-(4-tert-Butyl-phenylsulfonyl-amino)-3-phenyl-1H-indol-2-carbonsäure-(2-piperidin-1-yl-ethyl)-amid
143. 5-(4-tert-Butyl-phenylsulfonyl-amino)-3-phenyl-1H-indol-2-carbonsäure-(3-pyrrolidin-1-yl-propyl)-amid
144. 5-(4-tert-Butyl-phenylsulfonyl-amino)-3-phenyl-1H-indol-2-carbonsäurephenethyl-amid
145. 5-(4-tert-Butyl-phenylsulfonyl-amino)-3-phenyl-1H-indol-2-carbonsäure-(2-methoxy-1-methyl-ethyl)-amid
146. 5-(4-tert-Butyl-phenylsulfonyl-amino)-3-phenyl-1H-indol-2-carbonsäure-(pyridin-2-ylmethyl)-amid
147. 5-(4-tert-Butyl-phenylsulfonyl-amino)-3-phenyl-1H-indol-2-carbonsäure-(pyridin-3-ylmethyl)-amid
148. 5-(4-tert-Butyl-phenylsulfonyl-amino)-3-phenyl-1H-indol-2-carbonsäure-(pyridin-4-ylmethyl)-amid
149. 5-(4-tert-Butyl-phenylsulfonyl-amino)-3-phenyl-1H-indol-2-carbonsäure-(4-diethylamino-1-methyl-butyl)-amid
150. 5-(4-tert-Butyl-phenylsulfonyl-amino)-3-phenyl-1H-indol-2-carbonsäure-(2-imidazol-1-yl-ethyl)-amid
151. 5-(4-tert-Butyl-phenylsulfonyl-amino)-3-phenyl-1H-indol-2-carbonsäurebenzylamid
152. 5-(4-tert-Butyl-phenylsulfonyl-amino)-3-phenyl-1H-indol-2-carbonsäure-(2,2,2-trifluor-ethyl)-amid
153. 5-(4-tert-Butyl-phenylsulfonyl-amino)-3-phenyl-1H-indol-2-carbonsäure-4-methoxy-benzylamid
154. 5-(4-tert-Butyl-phenylsulfonyl-amino)-3-phenyl-1H-indol-2-carbonsäurecyclopentylamid
155. 5-(4-tert-Butyl-phenylsulfonyl-amino)-3-phenyl-1H-indol-2-carbonsäure-(3-methyl-butyl)-amid
156. 5-(4-tert-Butyl-phenylsulfonyl-amino)-3-phenyl-1 H-indol-2-carbonsäure-[3-(4-methyl-piperazin-1-yl)-propyl]-amid
157. 5-(4-tert-Butyl-phenylsulfonyl-amino)-3-phenyl-1H-indol-2-carbonsäure-[2-(4-hydroxy-phenyl)-ethyl]-amid
158. 5-(4-tert-Butyl-phenylsulfonyl-amino)-3-phenyl-1H-indol-2-carbonsäure-[2-(4-chlor-phenyl)-ethyl]-amid
159. 5-(4-tert-Butyl-phenylsulfonyl-amino)-3-phenyl-1 H-indol-2-carbonsäurecyclopropylamid
160. 5-(4-tert-Butyl-phenylsulfonyl-amino)-3-phenyl-1H-indol-2-carbonsäurecyclohexylmethyl-amid
161. 5-(4-tert-Butyl-phenylsulfonyl-amino)-3-phenyl-1H-indol-2-carbonsäure-(tetrahydro-furan-2-ylmethyl)-amid
162. 5-(4-tert-Butyl-phenylsulfonyl-amino)-3-phenyl-1H-indol-2-carbonsäure-(thiophen-2-ylmethyl)-amid
163. 5-(4-tert-Butyl-phenylsulfonyl-amino)-3-phenyl-1H-indol-2-carbonsäure-4-fluoro-benzylamid
164. 5-(4-tert-Butyl-phenylsulfonyl-amino)-3-phenyl-1H-indol-2-carbonsäure-(2-thiophen-2-yl-ethyl)-amid
165. 5-(4-tert-Butyl-phenylsulfonyl-amino)-3-phenyl-1H-indol-2-carbonsäure-(2-pyrrolidin-1-yl-ethyl)-amid
166. 5-(4-tert-Butyl-phenylsulfonyl-amino)-3-phenyl-1H-indol-2-carbonsäure-4-methyl-benzylamid
167. 5-(4-tert-Butyl-phenylsulfonyl-amino)-3-phenyl-1H-indol-2-carbonsäure-(1-ethyl-pyrrolidin-2-ylmethyl)-amid
168. 5-(4-tert-Butyl-phenylsulfonyl-amino)-3-phenyl-1 H-indol-2-carbonsäure-(2-pyridin-3-yl-ethyl)-amid
169. 5-(4-tert-Butyl-phenylsulfonyl-amino)-3-phenyl-1H-indol-2-carbonsäure-3-chlor-benzylamid
170. 5-(4-tert-Butyl-phenylsulfonyl-amino)-3-phenyl-1H-indol-2-carbonsäure-[2-(3-chlor-phenyl)-ethyl]-amid
171. 5-(4-tert-Butyl-phenylsulfonyl-amino)-3-phenyl-1 H-indol-2-carbonsäure-((R)-2-hydroxy-1-phenyl-ethyl)-amid
172. 5-(4-tert-Butyl-phenylsulfonyl-amino)-3-phenyl-1H-indol-2-carbonsäure-[3-(2-methyl-piperidin-1-yl)-propyl]-amid
173. 5-(4-tert-Butyl-phenylsulfonyl-amino)-3-phenyl-1H-indol-2-carbonsäure-(3-phenyl-propyl)-amid
174. 5-(4-tert-Butyl-phenylsulfonyl-amino)-3-phenyl-1H-indol-2-carbonsäure-(2-carbamoyl-ethyl)-amid
175. 5-(4-tert-Butyl-phenylsulfonyl-amino)-3-phenyl-1H-indol-2-carbonsäure-[3-(5-methyl-1H-pyrazol-4-yl)-propyl]-amid
176. 5-(4-tert-Butyl-phenylsulfonyl-amino)-3-phenyl-1H-indol-2-carbonsäure-(4-methyl-cyclohexyl)-amid
177. 5-(4-tert-Butyl-phenylsulfonyl-amino)-3-phenyl-1H-indol-2-carbonsäure-((S)-2-methoxy-1-methyl-ethyl)-amid
178. 5-(4-tert-Butyl-phenylsulfonyl-amino)-3-phenyl-1H-indol-2-carbonsäurecyclopropylmethyl-amid
179. 5-(4-tert-Butyl-phenylsulfonyl-amino)-3-phenyl-1H-indol-2-carbonsäurecarbamoylmethyl-amid

180. 5-(4-tert-Butyl-phenylsulfonyl-amino)-3-phenyl-1H-indol-2-carbonsäurecycloheptylamid
181. 5-(4-tert-Butyl-phenylsulfonyl-amino)-3-phenyl-1H-indol-2-carbonsäure-((R)-2-methoxy-1-methyl-ethyl)-amid
182. 5-(4-tert-Butyl-phenylsulfonyl-amino)-3-phenyl-1H-indol-2-carbonsäure-(furan-3-ylmethyl)-amid
183. 5-(4-tert-Butyl-phenylsulfonyl-amino)-3-phenyl-1H-indol-2-carbonsäure-3-fluor-benzylamid
184. 5-(4-tert-Butyl-phenylsulfonyl-amino)-3-phenyl-1H-indol-2-carbonsäure-(5-methyl-pyrazin-2-ylmethyl)-amid
185. 5-(4-tert-Butyl-phenylsulfonyl-amino)-3-phenyl-1H-indol-2-carbonsäure-(2-pyridin-2-yl-ethyl)-amid
186. 5-(4-tert-Butyl-phenylsulfonyl-amino)-3-phenyl-1H-indol-2-carbonsäure-(2-phenoxy-ethyl)-amid
187. 5-(4-tert-Butyl-phenylsulfonyl-amino)-3-phenyl-1H-indol-2-carbonsäure-(2-benzoimidazol-1-yl-ethyl)-amid
188. 5-(4-tert-Butyl-phenylsulfonyl-amino)-3-phenyl-1H-indol-2-carbonsäure-(3-imidazol-1-yl-propyl)-amid
189. 5-(4-tert-Butyl-phenylsulfonyl-amino)-3-phenyl-1H-indol-2-carbonsäure-(1-benzyl-piperidin-4-yl)-amid
190. 5-(4-tert-Butyl-phenylsulfonyl-amino)-3-phenyl-1H-indol-2-carbonsäure-[3-(2-oxo-pyrrolidin-1-yl)-propyl]-amid
191. 5-(4-tert-Butyl-phenylsulfonyl-amino)-3-phenyl-1H-indol-2-carbonsäure-[2-(1-methyl-pyrrolidin-2-yl)-ethyl]-amid
192. 5-(4-tert-Butyl-phenylsulfonyl-amino)-3-phenyl-1H-indol-2-carbonsäure methyl-(2-morpholin-4-yl-ethyl)-amid

Die erfindungsgemäßen Verbindungen inhibieren die lösliche Adenylatzyklase, worauf auch deren Wirkung zum Beispiel bei der männlichen Fertilitätskontrolle beruht.

Adenylatzyklasen sind die Effektormoleküle für einen der am meist genutzten Signaltransduktionswege, sie synthetisieren das second messenger Molekül zyklisches Adenosinmonophosphat (cAMP) aus Adenosintriphosphat (ATP) unter Abspaltung von Pyrophosphat (PP). cAMP vermittelt zahlreiche zelluläre Antworten für eine Vielzahl von Neurotransmittern und Hormonen. Die lösliche, spermienspezifische Adenylatzyklase (sAC, humane mRNA-Sequenz (GenBank) NM_018417, humanes Gen ADCY X) ist eine von zehn beschriebenen Adenylatzyklasen im menschlichen Genom. sAC zeigt dabei einige spezifische Eigenschaften, die sie von den anderen Adenylatzyklasen unterscheidet. sAC wird, im Gegensatz zu allen anderen Adenylatzyklasen, durch die Konzentration an Bicarbonat im sie umgebenden Medium stimuliert und nicht durch G-Proteine. sAC besitzt keine Transmembranregionen in ihrer Aminosäuresequenz, sie ist nicht durch Forskolin inhibierbar, ist durch Mangan viel stärker stimulierbar als durch Magnesium, und zeigt nur geringe Sequenzhomologien zu den anderen Adenylatzyklasen ($\leq$ 26% Identität der katalytischen Domänen I und II der sAC mit anderen Adenylatzyklasen auf Aminosäureebene).

Spezifische, Mangan-abhängige Aktivität von sAC wurde zuerst von T. Braun et al. (1975, PNAS 73:1097ff) in Rattentestis und Spermien beschrieben. N. Okamura et al. (1985, J. Biol. Chem 260(17):9699ff) zeigten, dass es sich bei der Substanz, die die Aktivität der sAC in Eberseminalflüssigkeit stimuliert um Bicarbonat handelt. Ebenfalls konnte gezeigt werden, dass nur in Rattentestis und Spermien, aber nicht in anderen Geweben, Bicarbonat -stimulierbare AC-Aktivität nachgewiesen werden kann. sAC wurde von der Gruppe Buck und Levin aus Rattentestis gereinigt und erstmalig sequenziert (J. Buck et al. 1999 PNAS 96:79ff, WO 01/85753). Die zu erwartenden Eigenschaften (e.g. Bikarbonat- und Magnesiumstimulierbarkeit) wurden am rekombinant exprimierten Protein bestätigt (Y. Chen et al. 2000 Science 289: 625ff).

Daten zur Verteilung der sAC mRNA und zu Bicarbonat -stimulierbarer sAC Aktivität lassen auf eine Testis- und Spermienspezifische Expression der Enzyms schließen (ML Sinclair et al. 2000 Mol Reprod Develop 56:6ff; N Okamura et al. 1985 J. Biol. Chem 260(17):9699ff; J. Buck et al. 1999 PNAS 96:79ff). Im Hoden wird sAC mRNA dabei nur in späteren Stadien, der sich zu Spermien entwickelnden Keimzellen, exprimiert, nicht aber in somatischen Zellen (ML Sinclair et al. 2000 Mol Reprod Develop 56:6ff).

Zur Funktion der sAC in Spermien in Säugetieren gibt es eine Reihe pharmakologischer Untersuchungen. Spermien müssen, bevor sie die Zona Pellucida des Eies durchdringen können um anschließend mit dem Oolemma des Eies zu verschmelzen, für diese Funktionalität vorbereitet sein. Dieser Prozess, die Spermienkapazitation, ist recht gut untersucht. Ein kapazitiertes Spermium zeichnet sich durch ein verändertes Bewegungsmuster aus und durch die Fähigkeit, durch einen geeigneten Stimulus den Prozess der akrosomalen Reaktion (einer Ausschüttung lytischer Enzyme die vermutlich dem Durchdringen der Zona Pellucida durch das Spermium dienen) zu durchlaufen. Die Spermienkapazitation erfolgt *in vivo* und *in vitro* u.a. abhängig von einer erhöhten Bikarbonatkonzentration im Medium (PE Visconti & GS Kopf (1998) Biol Reprod 59:1ff; E de Lamirande et al. 1997 Mol Hum Reprod 3(3):175ff). Die Spermienkapazitation kann auch stimuliert werden durch die Zugabe geeigneter membrangängiger cAMP-Analoga, z.B. db-cAMP, und einem Inhibitor, der deren Abbau hemmt (z.B. IBMX). Die vermutete Abhängigkeit der Spermienfunktion von sAC wurde erst kürzlich durch ein genetisches Deletionsmodell, eine sog. Knock-out Maus, bestätigt (G Esposito et al. 2004 PNAS 101 (9):2993ff). Männliche Mäuse, denen das Gen für sAC fehlt, zeigen eine normal verlaufende Spermatogenese, sind aber infertil. Die Spermien haben Bewegungsdefekte und sind nicht in der Lage ein Ei zu befruchten. Die Tier zeigten keine sonstigen Defekte oder abnorme Befunde, was gegen andere hypothetisierte Funktionen der sAC spricht (JH Zippin et al 2003 FASEB 17:82ff)).

Die sAC hat eine einzigartige Sequenz und nur eine geringe Homologie zu anderen somatischen Adenylatzyklasen.

Sie ist die einzige Adenylatzyklase im Säugetiersperma und die Aktivität ist für die Beweglichkeit der Spermien und die Kapazitation essentiell. Spezifische Inhibitoren der sAC stellen demnach eine wichtige Möglichkeit dar, die männliche Fertilität zu regulieren.
Arzneimittel, die mindestens eine der Verbindungen gemäß den Ansprüchen 1-3 enthalten, sind daher Gegenstand der vorliegenden Erfindung.
Ebenfalls Gegenstand der vorliegenden Erfindung ist die Verwendung der Verbindungen gemäß Anspruch 1-3

Zur Verwendung der erfindungsgemäßen Verbindungen als Arzneimittel werden diese in die Form eines pharmazeutischen Präparats gebracht, das neben dem Wirkstoff für die enterale oder parenterale Applikation geeignete pharmazeutische, organische oder anorganische inerte Trägermaterialien, wie zum Beispiel, Wasser, Gelatine, Gummi arabicum, Milchzucker, Stärke, Magnesiumstearat, Talk, pflanzliche Öle, Polyalkylenglykole usw. enthält. Die pharmazeutischen Präparate können in fester Form, zum Beispiel als Tabletten, Dragees, Suppositorien, Kapseln oder in flüssiger Form, zum Beispiel als Lösungen, Suspensionen oder Emulsionen vorliegen. Gegebenenfalls enthalten sie darüber hinaus Hilfsstoffe, wie Konservierungs-, Stabilisierungs-, Netzmittel oder Emulgatoren; Salze zur Veränderung des osmotischen Drucks oder Puffer. Diese pharmazeutischen Präparate sind ebenfalls Gegenstand der vorliegenden Erfindung.

Für die parenterale Anwendung sind insbesondere Injektionslösungen oder Suspensionen, insbesondere wässrige Lösungen der aktiven Verbindungen in polyhydroxyethoxyliertem Rizinusöl, geeignet.

Als Trägersysteme können auch grenzflächenaktive Hilfsstoffe wie Salze der Gallensäuren oder tierische oder pflanzliche Phospholipide, aber auch Mischungen davon sowie Liposomen oder deren Bestandteile verwendet werden.

Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt ist.

Für die vaginale Applikation sind z. B. Zäpfchen geeignet und üblich.

Die enteralen, parenteralen, vaginalen und oralen Applikationen sind ebenfalls Gegenstand der vorliegenden Erfindung.

Die Dosierung der Wirkstoffe kann je nach Verabfolgungsweg, Alter und Gewicht des Patienten, Art und Schwere der zu behandelnden Erkrankung und ähnlichen Faktoren variieren. Die tägliche Dosis beträgt 0,5-1000 mg, vorzugsweise 50-200 mg, wobei die Dosis als einmal zu verabreichende Einzeldosis oder unterteilt in 2 oder mehreren Tagesdosen gegeben werden kann.

Die erfindungsgemäßen Verbindungen der allgemeinen Formel I sind unter anderem hervorragende Inhibitoren der löslichen Adenylatzyklase. Inhibitoren der löslichen Adenylatzyklase führen zu einer Erniedrigung des cAMP Signals. Der cAMP-Spiegel ist entscheidend für die Kontrolle der Prozesse, die bei der Zellproliferation, der Zelldifferenzierung und der Apoptose eine wichtige Rolle spielen. Erkrankungen, wie z.B. Krebs, bei denen die Erniedrigung des cAMP-Spiegels entscheidend sind, können durch Inhibitoren der löslichen Adenylatzyklase moduliert werden. Diese Modulation kann prophylaktische und therapeutische Effekte haben für die Patienten, die an einer derartigen Erkrankung leiden. Im Moment werden Erkrankungen, die wie Krebs mit einer erhöhten Zellproliferation einhergehen z.B. durch Strahlentherapie und Chemotherapie behandelt. Diese Verfahren sind unspezifisch und haben ein hohes Nebenwirkungspotential. Die Bereitstellung von neuen Substanzen, die direkt an bestimmten Zielorten eingreifen sind deshalb vorteilhaft. Gegenstand der vorliegenden Erfindung sind Substanzen, die die cAMP-Produktion durch die Inhibition der löslichen Adenylatzyklase modulieren. So kann beispielsweise die anomale Zellproliferation erniedrigt oder gehemmt werden durch eine Regulation oder Inhibition der cAMP Produktion. Durch den Einsatz der erfindungsgemäßen Substanzen kann die lösliche Adenylatzyklase inhibiert werden, dies hat eine Erniedrigung der Zellproliferation zur Folge. Gegenstand der vorliegenden Erfindung sind Arzneimittel zur Behandlung von Erkrankungen, die mindestens eine Verbindung gemäß der allgemeinen Formel I enthalten, sowie Arzneimittel mit geeigneten Formulierungs- und Trägerstoffen. Die Erkrankungen sind dadurch gekennzeichnet, dass sie verursacht werden durch Störungen des Stoffwechsels des second messengers cAMP.

Eine Erniedrigung der cAMP-Konzentration durch Inhibition der löslichen Adenylatzyklase kann Mittel zur Verfügung stellen zur Modulation der Spermienkapazitation. Gegenstand der vorliegenden Erfindung ist die Verwendung der erfindungsgemäßen Substanzen zur Erniedrigung und / oder zur Hemmung der männlichen Keimzell-Fertilität vermittelt durch die Reduktion oder Inhibition der löslichen Adenylatzyklase Aktivität und dadurch resultierend der Spermienkapazitation.

Durch die Administration einer wirksamen Menge einer Substanz, die zur Inhibition der cAMP-Produktion führt, kann die Befruchtung des Ovums verhindert werden. Ebenfalls Gegenstand der vorliegenden Erfindung ist die Verwendung der Verbindung der allgemeinen Formel I zur Herstellung eines Arzneimittels für die nicht hormonelle Kontrazeption.

Soweit die Herstellung der Ausgangsverbindungen nicht beschrieben wird, sind diese bekannt oder analog zu bekannten Verbindungen oder hier beschriebenen Verfahren herstellbar. Es ist ebenfalls möglich, alle hier beschriebenen Umsetzungen in Parallel-Reaktoren oder mittels kombinatorischer Arbeitstechniken durchzuführen.
Die Isomerengemische können nach üblichen Methoden wie beispielsweise Kristallisation, Chromatographie oder Salzbildung in die Enantiomeren bzw. E/Z-Isomeren aufgetrennt werden.
Die Herstellung der Salze erfolgt in üblicher Weise, indem man eine Lösung der Verbindung der Formel I mit der äquivalenten Menge oder einem Überschuss einer Base oder Säure, die gegebenenfalls in Lösung ist, versetzt und den Niederschlag abtrennt oder in üblicher Weise die Lösung aufarbeitet.

**Herstellung der erfindungsgemäßen Verbindungen**

Die nachfolgenden Beispiele erläutern die Herstellung der erfindungsgemäßen Verbindungen der allgemeinen Formel (I), ohne den Umfang der beanspruchten Verbindungen auf diese Beispiele zu beschränken.
Die erfindungsgemäßen Verbindungen der allgemeinen Formel (I) lassen sich wie nachstehend beschrieben herstellen.

**Vorschrift 1: *Amidkupplung*:**

Eine Carbonsäure (1.0 äquivalent) wird in N,N-Dimethylformamid (DMF) (10 ml/1 mmol) gelöst, mit N-[(Dimethylamino)-1*H*-1,2,3-triazolo[4,5-*b*]pyridin-1-ylmethylen]-*N*-Methylmethanaminiumhexafluorophosphat- N-oxid (HATU) (1.1 äquivalent) und dem zu kuppelnden Amin (1.0 äquivalent) versetzt. Anschließend wird Ethyldiisopropylamin (1.1 äquivalent) bei 0˚C dazugegeben und die Mischung für 22 Stunden bei Raumtemperatur gerührt. Danach wird das Gemisch mit Eiswasser versetzt (35 ml/1 mmol Carbonsäure), 30 Min. bei Raumtemperatur gerührt. Die ausgefallenen Kristalle werden abgesaugt und an der Luft getrocknet. Das Produkt wird entweder ohne weitere Reinigung in der nachfolgenden Stufe umgesetzt oder chromatographisch gereinigt.

**Vorschrift 2: *Reduktion der Nitrogruppe*:**

Die Nitroverbindung (1.0 äquivalent) wird in Methanol (10 ml/1 mmol) und Wasser (0.03 ml/1 mmol) vorgelegt, mit Ammoniumformiat (5 äquivalent) und mit katalytischen Mengen Palladium auf Kohle (10%) versetzt und 3 Stunden bei 90˚C am Rückfluss gekocht. Anschließend wird über Celite abgesaugt und mit kochendem Methanol nachgewaschen. Nach Entfernen des Lösungsmittels wird der Rückstand mit Wasser (7 ml/1 mmol Amid) versetzt und die ausgefallenen Kristalle abgesaugt. Bilden sich keine Kristalle, wird die wässrige Phase mit Ethylacetat oder Dichlormethan extrahiert. Die vereinigten organischen Phasen werden mit gesättigter Natriumchloridlösung gewaschen und über Natriumsulfat getrocknet. Anschließend wird das Lösungsmittel unter reduziertem Druck entfernt.

**Vorschrift 3: *Kupplung mit Arylsulfonylchloriden:***

Das entstandene Amin (1.0 äquivalent) wird in DMF (10 ml/1 mmol) gelöst, bei 0˚C mit Ethyldiisopropylamin (1.5 äquivalent) und Arylsulfonsäurechlorid (1.0 äquivalent) versetzt und eine Stunde bei Raumtemperatur gerührt. Das Lösungsmittel wird unter reduziertem Druck entfernt und der Rückstand chromatographisch gereinigt.

**Vorschrift 4: *Bromierung:***

5-Nitroindol-2-carbonsäureethylester (1.0 äquivalent) wird in Tetrahydrofuran (5 ml/1 mmol) gelöst und mit N-Bromsuccinimid (1.0 äquivalent) versetzt. Nach 30 Min. wird Wasser hinzugefügt und 20 Min. später die ausgefallenen Kristalle abgesaugt. Falls sich keine Kristalle bilden, wird die wässrige Phase mit Ethylacetat extrahiert und die vereinigten organischen Phasen über Natriumsulfat getrocknet. Nach Entfernen des Lösungsmittels erfolgt die chromatographische Reinigung des Rückstands.

**Vorschrift 5 *Verseifung:***

Die Esterverbindungen (1.0 äquivalent) werden mit 19 äquivalent einer 1 M Natronlauge-Lösung in Ethanol/Wasser (1/1) versetzt. Nach 6 Stunden bei Raumtemperatur wird Ethanol unter reduziertem Druck entfernt, mit Wasser verdünnt und mit 10%iger wässriger Schwefelsäure auf pH 2 eingestellt.
Anschließend werden die ausgefallenen Kristalle abgesaugt.

**Vorschrift 6 *Kupplung mit Arylboronsäuren*:**

3-Bromindol-2-carbonsäureester (1.0 äquivalent) wird mit einer Arylboronsäure (1.5 äquivalent) in Toluol/Ethanol 1:1 (40 ml/1 mmol Ester) suspendiert und mit 1M-Natriumcarbonatlösung (2.5 äquivalent) sowie Lithiumchlorid (2.8 äquivalent) versetzt. Nach Zugabe von Tetrakis(triphenylphosphin)-palladium (0.08 äquivalent) wird die Reaktionsmischung 8 Stunden refluxiert. Nach Abkühlen auf Raumtemperatur wird mit Ethylacetat (70 ml/ 1mmol Ester) verdünnt

und 10 Min. später über Celite abgesaugt. Das Filtrat wird mit gesättigter Natriumhydrogencarbonat- und gesättigter Natriumchloridlösung gewaschen und über Natriumsulfat getrocknet. Nach Entfernen des Lösungsmittels erfolgt die chomatographische Reinigung des Rückstands.

**Beispiel 1:** 5-(4-tert-Butylbenzolsulfonylamino)-3-phenyl-1*H*-indol-2-carbonsäure-cyclopropylamid

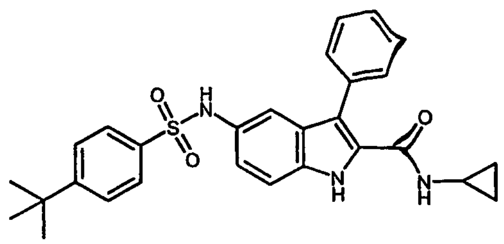

Gemäß Vorschrift 1 werden nach Umsetzung von 5-Nitro-3-phenyl-1*H*-indol-2-carbonsäure (423 mg, 1.5 mmol) mit Cyclopropylamin (0.105 ml, 1.5 mmol) 445 mg (93%) des Produkts *5-Nitro-3-phenyl-1H-indol2-carbonsäurecyclopropylamid* erhalten, das ohne weitere Reinigung in der nachfolgenden Stufe umgesetzt wird.
Gemäß Vorschrift 2 werden nach Umsetzung von *5-Nitro-3-phenyl-1H-indol-2-carbonsäurecyclopropylamid* (445mg, 1.39 mmol) mit Ammoniumformiat (438 mg, 6.95 mmol) in Gegenwart von Palladium auf Kohle (44mg) 289 mg (72%) des Produkts *5-Amino-3-phenyl-1H-indol-2-carbonsäurecyclopropylamid* erhalten.
NMR (300 MHz, DMSO-d6): δ 0.28-0.36 (m, 2H), 0.60-0.68 (m, 2H), 2.72 (m, 1 H), 5.00 (br, 2H), 6.60-6.68 (m, 2H), 7.15 (d, 1 H), 7.22-7.38 (m, 2H), 7.40-7.50 (m, 4H), 11.19 (s, 1 H).
Gemäß Vorschrift 3 werden nach Umsetzung von *5-Amino-3-phenyl-1H-indol-2-carbonsäurecyclopropylamid* (145 mg, 0.5 mmol) mit 4-tert-Butylbenzolsulfonsäurechlorid (116 mg, 0.5 mmol) und chromatographischer Reinigung (Kieselgel, Hexan/Ethylacetat (0-100% Ethylacetat)) 190 mg (78%) des Produkts *5-(4-tert-Butylbenzolsulfonylamino)-3-phenyl-1H-indol-2-carbonsäure-cyclopropylamid* erhalten.
NMR (300 MHz, DMSO-d6): δ 0.32-0.37 (m,2H), 0.59-0.65 (m, 2H), 1.25 (s, 9H), 2.67-2.76 (m, 1 H), 7.02 (dd, 1 H), 7.10 (s, 1 H), 7.25 (d, 2H), 7.31-7.36 (m, 2H), 7.40-7.45 (m, 2H), 7.51-7.59 (m, 5H), 9.79 (s, 1 H), 11.66 (s, 1 H).

**Beispiel 2:** 5-(4-tert-Butylbenzolsulfonylamino)-3-phenyl-1H-indol-2-carbonsäure-pyridin-3-ylamid

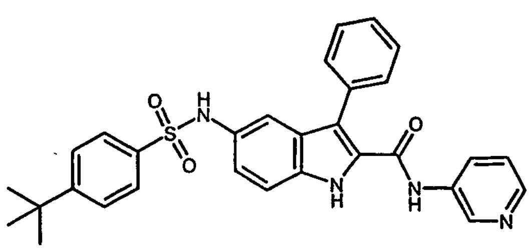

Gemäß Vorschrift 1 werden nach Umsetzung von 5-Nitro-3-phenyl-1*H*-indol-2-carbonsäure (423 mg, 1.5 mmol) mit 3-Aminopyridin (141 mg, 1.5 mmol) 494 mg (93%) des Produkts *5-Nitro-3-phenyl-1H-indol-2-carbonsäurepyridin-3-ylamid* erhalten , das ohne weitere Reinigung in der nachfolgenden Stufe umgesetzt wird.
Gemäß Vorschrift 2 werden nach Umsetzung von *5-Nitro-3-phenyl-1H-indol-2-carbonsäurepyridin-3-ylamid* (494mg, 1.39 mmol) mit Ammoniumformiat (435 mg, 6.90 mmol) in Gegenwart von Palladium auf Kohle (49 mg) 257 mg (72%) des Produkts *5-Amino-3-phenyl-1H-indol-2-carbonsäurepyrldin-3-ylamid* erhalten.
NMR (300 MHz, DMSO-d6): δ 4.98 (br, 2H), 6.70-6.78 (m, 2H), 7.23 (d , 1 H), 7.30-7.38 (m, 2H), 7.40-7.58 (m, 4H), 8.00 (d, 1 H), 8.25 (dd, 1 H), 8.64 (d , 1H), 9.75 (s, 1 H), 11.49 (s, 1 H).

Gemäß Vorschrift 3 werden nach Umsetzung von (128 mg, 0.39 mmol) mit 4-tert-Butylbenzolsulfonsäurechlorid (90 mg, 0.39 mmol) und chromatographischer Reinigung (Kieselgel, Hexan/Ethylacetat (0-100% Ethylacetat)) 140 mg (68%) des Produkts *5-(4-tert-Butylbenzolsulfonylamino)-3-phenyl-1H-indol-2-carbonsäure-pyridin-3-ylamid* erhalten.
NMR (300 MHz, DMSO-d6): δ 1.26 (s, 9H), 7.09 (dd, 1 H), 7.19 (s, 1 H), 7.34-7.43 (m, 7H), 7.54 (d, 2H), 7.60 (d, 2H), 7.95-7.97 (m, 1 H), 8.27 (d, 1 H), 8.65 (s, 1 H), 9.86 (s, 1 H), 9.98 (s, 1 H), 11.97 (s, 1 H).

**Beispiel 3:** 5-(4-tert-Butylbenzolsulfonylamino)-3-phenyl-1*H*-indol-2-carbonsäure-cyclohexylamid

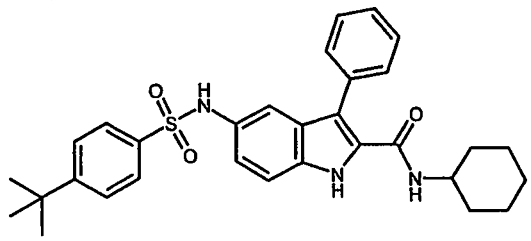

Gemäß Vorschrift 1 werden nach Umsetzung von 5-Nitro-3-Phenyl-1*H*-indol-2-carbonsäure (423 mg, 1.5 mmol) mit Cyclohexylamin (149 mg, 1.5 mmol) 504 mg (93%) des Produkts *5-Nitro-3-phenyl-1H-indol-2-carbonsäurecyclohexylamid* erhalten , das ohne weitere Reinigung in der nachfolgenden Stufe umgesetzt wird.
Gemäß Vorschrift 2 werden nach Umsetzung von *5-Nitro-3-phenyl-1H-indol-2-carbonsäurecyclohexylamid* (504mg, 1.39 mmol) mit Ammoniumformiat (438 mg, 6.95 mmol) in Gegenwart von Palladium auf Kohle (50 mg) 367 mg (79%) des Produkts *5-Amino-3-phenyl-1H-indol-2-carbonsäurecyclohexylamid* erhalten

NMR (300 MHz, DMSO-d6): δ 0.92-1.16 (m, 3H), 1.18-1.31 (m, 2H), 1.38-1.55 (m, 3H), 1.62-1.76 (m, 2H), 3.61-3.78 (m, 1 H), 4.82 (br, 2H), 6.58-6.74 (m, 3H), 7.18 (d, 1 H), 7.32-7.40 (m, 1 H), 7.43-7.54 (m, 4H), 11.25 (s, 1 H).
Gemäß Vorschrift 3 werden nach Umsetzung von *5-Amino-3-phenyl-1H-indol-2-carbonsäurecyclohexylamid* (183 mg, 0.55 mmol) mit 4-tert-Butylbenzolsulfonsäurechlorid (128 mg, 0.55 mmol) und chromatographischer Reinigung (Kieselgel, Hexan/Ethylacetat (0-100% Ethylacetat)) 231 mg (79%) des Produkts *5-(4-ted-Butylbenzolsulfonylamino)-3-phenyl-1H-indol-2-carbonsäure-cyclohexylamid* erhalten.
NMR (300 MHz, DMSO-d6): δ 0.98-1.09 (m, 3H), 1.19-1.32 (m, 11 H), 1.50-1.53 (m, 3H), 1.66-1.70 (m, 2H), 3.65-3.74 (m, 1 H), 6.97-7.06 (m, 3H), 7.29-7.48 (m, 6H), 7.51 (d, 2H), 7.57(d, 2H), 9.79 (s, 1 H), 11.71 (s, 1 H).

**Beispiel 4:** 4-tert-Butyl-N-[3-phenyl-2-(pyrrolidin-1-carbonyl)-1 H-indol-5-yl]-benzolsulfonamid

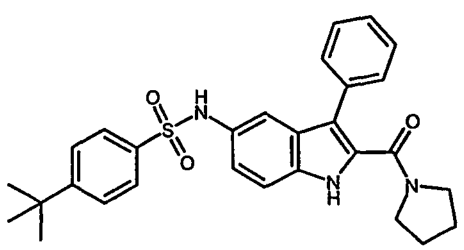

Gemäß Vorschrift 1 werden nach Umsetzung von 5-Nitro-3-phenyl-1*H*-indol-2-carbonsäure (423 mg, 1.5 mmol) mit Pyrrolidin (0.124 mg, 1.5 mmol) 430 mg (85%) des Produkts *5-Nitro-3-phenyl-1H-indol-2-yl)pyrrolidin-1-yl-methanon* erhalten, das ohne weitere Reinigung in der nachfolgenden Stufe umgesetzt wird.
Gemäß Vorschrift 2 werden nach Umsetzung von *5-Nitro-3-phenyl-1H-indol-2-yl)pyrrolidin-1-yl-methanon* (430 mg, 1.28 mmol) mit Ammoniumformiat (404 mg, 6.41 mmol) in Gegenwart von Palladium auf Kohle (43 mg) 255 mg (65%) des Produkts *(5-Amino-3-phenyl-1H-indol-2-yl)pyrrolidin-1-yl-methanon* erhalten.
NMR (300 MHz, DMSO-d6): δ 1.44-1.60 (m, 2H), 1.62-1.78 (m, 2H), 2.84 (t, 2H), 3.41 (t, 2H), 4.90 (br, 2H), 6.61 (dd,

1H), 6.88 (d, 1H), 7.12 (d, 1 H), 7.22-7.30 (m, 1 H), 7.38-7.50 (m, 4H), 11.25 (s, 1H).
Gemäß Vorschrift 3 werden nach Umsetzung von *(5-Amino-3-phenyl-1H-indol-2-yl)pyrrolidin-1-yl-methanon* (128 mg, 0.42 mmol) mit 4-tert-Butylbenzolsulfonsäurechlorid (98 mg, 0.42 mmol) und chromatographischer Reinigung (Kieselgel, Hexan/Ethylacetat (0-100% Ethylacetat)) 160 mg (76%) des Produkts *4-terl-Butyl-N-[3-phenyl-2-(pyrrolidin-1-carbonyl)-1H-indol-5-yl]-benzolsulfonamid* erhalten.
NMR (300 MHz, DMSO-d6): δ 1.24 (s, 9H), 1.49-1.55 (m, 2H), 1.65-1.73 (m, 2H), 2.81 (t, 2H), 3.41 (t, 2H), 7.01 (dd, 1H), 7.24-7.34 (m, 5H), 7.42-7.46 (m, 2H), 7.54 (d, 2H), 7.60 (d, 2H), 9.85 (s, 1 H), 11.72 (s, 1 H).

**Beispiel 5:** 4-tert-Butyl-N-[2-(morpholin-4-carbonyl)-3-phenyl-1*H*-indol-5yl]-benzol-sulfonamid

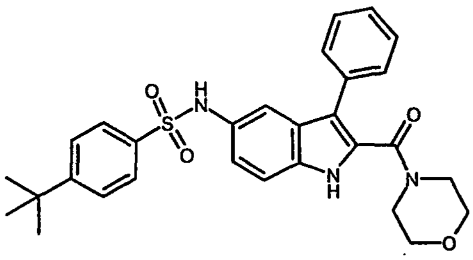

Gemäß Vorschrift 1 werden nach Umsetzung von 5-Nitro-3-phenyl-1*H*-indol-2-carbonsäure (423 mg, 1.5 mmol) mit Morpholin (0.131 ml, 1.5 mmol) 487 mg (92%) des Produkts *Morpholin-4-yl-(5-nitro-3-phenyl-1H-indol-2-yl)-methanon* erhalten, das ohne weitere Reinigung in der nachfolgenden Stufe umgesetzt wird.
Gemäß Vorschrift 2 werden nach Umsetzung von *Morpholin-4-yl-(5-nitro-3-phenyl-1H-indol2-yl)-methanon* (487 mg, 1.39 mmol) mit Ammoniumformiat (438 mg, 6.95 mmol) in Gegenwart von Palladium auf Kohle (49 mg) 393 mg (88%) des Produkts *(5-Amino-3-phenyl-1H-indol-2yl)-morpholin-4-ylmethanon* erhalten.
NMR (300 MHz, DMSO-d6): δ 2.78-3.70 (m, 8H), 4.70 (br, 2H), 6.63 (dd, 1 H), 6.82 (d, 1 H), 7.12 (d, 1 H), 7.29-7.51 (m, 5H), 11.30 (s, 1H).
Gemäß Vorschrift 3 werden nach Umsetzung von *(5-Amino-3-phenyl-1H-indol-2yl)-morpholin-4-ylmethanon* (196 mg, 0.61 mmol) mit 4-tert-Butylbenzolsulfonsäurechlorid (142 mg, 0.61 mmol) und chromatographischer Reinigung (Kieselgel, Hexan/Ethylacetat (0-100% Ethylacetat)) 280 mg (88%) des Produkts *4-tert-Butyl-N-[2-(morpholin-4-carbonyl)-3-phenyl-1H-indol-5yl]-benzolsulfonamid* erhalten.
NMR (300 MHz, DMSO-d6): δ 1.24 (s, 9H), 2.75-3.10 (br, 4H), 3.30-3.60 (br, 4H), 7.02 (dd, 1 H), 7.21-7.30 (m, 5H), 7.42-7.61 (m, 6H), 9.85 (s, 1H), 11.78 (s, 1 H).

**Beispiel 6:** 5-(4-tert-Butylbenzolsulfonylamino)-3-phenyl-1*H*-indol-2-carbonsäure-(2-dimethylaminoethyl)amid

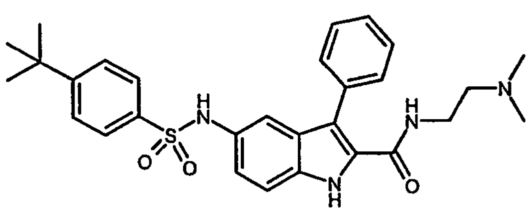

Gemäß Vorschrift 1 werden nach Umsetzung von 5-Nitro-3-phenyl-1*H*-indol-2-carbonsäure (1.0 g, 3.54 mmol) mit N,N-Dimethylethylendiamin (312 mg, 3.54 mmol) 557 mg (44%) des Produkts *5-Nitro-3-phenyl-1H-indol-2-carbonsäure-(2-dimethylaminoethyl)amid* erhalten, das ohne weitere Reinigung in der nachfolgenden Stufe umgesetzt wird.
Gemäß Vorschrift 2 werden nach Umsetzung von *5-Nitro-3-phenyl-1H-indol-2-carbonsäure-(2-dimethylaminoethyl) amid* (557 mg, 1.58 mmol) mit Ammoniumformiat (498 mg, 7.90 mmol) in Gegenwart von Palladium auf Kohle (87 mg) 386 mg (75%) des Produkts *5-Amino-3-phenyl-1H-indol-2-carbonsäure-(2-dimethylaminoethyl)amid* erhalten.
NMR (300 MHz, DMSO-d6): δ 2.03 (s, 6H), 2.21 (t, 2H), 3.24 (q, 2H), 4.95 (br, 2H), 6.55 (d, 1 H), 6.64 (dd, 1 H), 6.86

(t, 1 H), 7.17 (d, 1H), 7.34-7.50 (m, 5H), 11.25 (s, 1H).
Gemäß Vorschrift 3 werden nach Umsetzung von *5-Amino-3-phenyl-1H-indol-2-carbonsäure-(2-dimethylaminoethyl) amid* (229 mg, 0.71 mmol) mit 4-tert-Butylbenzolsulfonsäurechlorid (165 mg, 0.71 mmol), chromatographischer Reinigung (Kieselgel, Dichlormethan/Methanol (0-10% Methanol)) 300 mg Feststoff, der in Dichlormethan gelöst und mit wässriger 1 N KOH Lösung gewaschen wird. Nach Trocknen über Natriumsulfat und Entfernen des Lösungsmittels werden 90 mg (24%) des Produkts *5-(4-tert Butylbenzolsulfonylamino)-3-phenyl-1H-indol-2-carbonsäure-(2-dimethylaminoethyl)amid*.
NMR (300 MHz, DMSO-d6): δ 1.25 (s, 9H), 2.00 (s, 6H), 2.25 (t, 2H), 3.24 (q, 2H), 7.00-7.10 (m, 3H), 7.21-7.60 (m, 10H), 9.80 (s, 1 H), 11.71 (s, 1 H).

**Beispiel 7** 5-(4-Cyanobenzolsulfonylamino)-3-phenyl-1H-indol-2-carbonsäurepropyl-amid

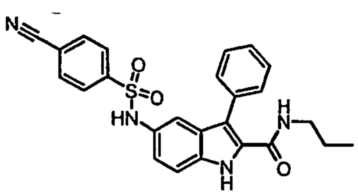

Gemäß Vorschrift 1 werden nach Umsetzung von 5-Nitro-3-phenyl-1*H*-indol-2-carbonsäure (423 mg, 1.5 mmol) mit Propylamin (89 mg, 1.5 mmol) 450 mg (93%) des Produkts *5-Nitro-3-phenyl-1H-indol-2-carbonsäurepropylamid* erhalten, das ohne weitere Reinigung in der nachfolgenden Stufe umgesetzt wird.
Gemäß Vorschrift 2 werden nach Umsetzung von *5-Nitro-3-phenyl-1H-indol-2-carbonsäurepropylamid* (400 mg, 1.24 mmol) mit Ammoniumformiat (390 mg, 6.18 mmol) in Gegenwart von Palladium auf Kohle (40 mg) 281 mg (77%) des Produkts *5-Amino-3-phenyl-1H-indol-2-carbonsäurepropylamid* erhalten.
NMR (300 MHz, DMSO-d6): δ 0.76 (t, 3H), 1.33-1.40 (m, 2H), 3.11 (q, 2H), 4.64 (br, 2H), 6.62-66 (m, 2H), 7.00 (t, 1H), 7.17 (d, 1 H), 7.33-7.36 (m, 1 H), 7.43-7.46 (m, 4H), 11.19 (s, 1H).
Gemäß Vorschrift 3 werden nach Umsetzung von *5-Amino-3-phenyl-1H-indol-2-carbonsäurepropylamid* (146 mg, 0.5 mmol) mit 4-Cyanobenzolsulfonylchlorid (121 mg, 0.60 mmol) und chromatographischer Reinigung (Kieselgel, Hexan/Ethylacetat (0-100% Ethylacetat)) 41 mg (18%) des Produkts 5-(4-*Cyanobenzolsulfonylamino)-3-phenyl-1H-indol-2-carbonsäurepropylamid* erhalten.
NMR (300 MHz, DMSO-d6): δ 0.76 (t, 3H), 1.31-1.43 (m, 2H), 3.11 (q, 2H), 6.96 (dd, 1H), 7.00 (s, 1H), 7.27-.749 (m, 7H), 7.77 (d, 2H), 8.03 (d, 2H), 10.08 (s, 1 H), 11.75 (s, 1 H).

**Beispiel 8** 5-(4-Brombenzolsulfonylamino)-3-phenyl-1*H*-indol-2-carbonsäurepropyl-amid

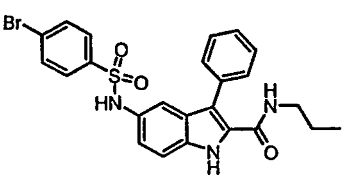

Gemäß Vorschrift 3 werden nach Umsetzung von *5-Amino-3-phenyl-1H-indol-2-carbonsäurepropylamid* (146 mg, 0.5 mmol) mit 4-Brombenzolsulfonylchlorid (153 mg, 0.60 mmol) und chromatographischer Reinigung (Kieselgel, Hexan/Ethylacetat (0-100% Ethylacetat)) 91 mg (35%) des Produkts erhalten.
NMR (300 MHz, DMSO-d6): δ 0.76 (t, 3H), 1.31-1.43 (m, 2H), 3.11 (q, 2H), 6.96-7.03 (m, 2H), 7.26-7.49 (m, 7H), 7.53 (d, 2H), 7.77 (d, 2H), 9.86 (s, 1 H), 11.73 (s, 1H).

**Beispiel 9** 5-(4-tert-Butylbenzolsulfonylamino)-3-phenyl-1H-indol-2-carbonsäure-propylamid

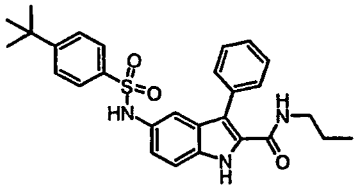

Gemäß Vorschrift 3 werden nach Umsetzung von *5-Amino-3-phenyl-1H-indol-2-carbonsäurepropylamid* (146 mg, 0.5 mmol) mit 4-tert-Butylbenzolsulfonsäurechlorid (139 mg, 0.60 mmol) und chromatographischer Reinigung (Kieselgel, Hexan/Ethylacetat (0-100% Ethylacetat)) 52 mg (21%) des Produkts erhalten.
NMR (300 MHz, $CDCl_3$): δ 0.73 (t, 3H), 1.29-1.41 (m, 11 H), 3.22 (q, 2H), 5.92 (t, 1 H), 6.47 (s, 1 H), 7.04 (s, 1 H), 7.07 (dd, 1 H), 7.34-7.61 (m, 10H), 9.55 (s, 1 H).

**Beispiel 10** 5-(4-(Trifluormethoxy)benzolsulfonylamino)-3-phenyl-1*H*-indol-2-carbonsäurepropylamid

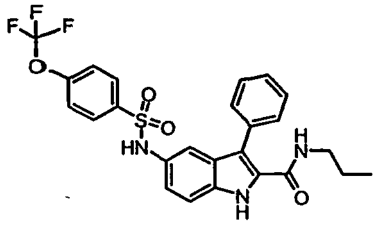

Gemäß Vorschrift 3 werden nach Umsetzung von *5-Amino-3-phenyl-1H-indol-2-carbonsäurepropylamid* (146 mg, 0.5 mmol) mit 4-(Trifluormethoxy)benzolsulfonylchlorid (156 mg, 0.60 mmol) und chromatographischer Reinigung (Kieselgel, Hexan/Ethylacetat (0-100% Ethylacetat)) 106 mg (41 %) des Produkts erhalten.
NMR (300 MHz, $CDCl_3$): δ 0.73 (t, 3H), 1.28-1.38 (m, 2H), 3.22 (q, 2H), 5.95 (t, 1 H), 6.76 (s, 1 H), 7.02 (s, 1 H), 7.06 (dd, 1 H), 7.20 (d, 2H), 7.37-7.51 (m, 6H), 7.71 (d, 2H), 9.81 (s, 1H).

**Beispiel 11** 5-(4-(Methylsulfonyl)benzolsulfonylamino)-3-phenyl-1*H*-indol-2-ca rbon-säurepropylamid

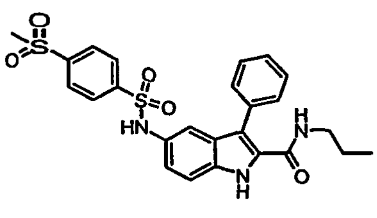

Gemäß Vorschrift 3 werden nach Umsetzung von *5-Amino-3-phenyl-1H-indol-2-carbonsäurepropylamid* (146 mg, 0.50 mmol) mit 4-Methylsulfonylbenzolsulfonylchlorid (153 mg, 0.60 mmol) und chromatographischer Reinigung (Kieselgel, Hexan/Ethylacetat (0-100% Ethytacetat)) 87 mg (39%) des Produkts erhalten.
NMR (300 MHz, DMSO-d6): δ 0.76 (t, 3H), 1.31-1.43 (m, 2H), 3.11 (q, 2H), 3.25 (s, 3H), 7.00 (dd, 1 H), 7.05 (s, 1 H), 7.27 (d, 2H), 7.34-7.38 (m, 3H), 7.43-7.49 (m, 2H), 7.87 (d, 2H), 8.10 (d, 2H), 10.10 (s, 1 H), 11.75 (s, 1 H).

**Beispiel 12** 5-(4-tert-Butylbenzolsulfonylamino)-3-phenyl-1*H*-indol-2-carbonsäurephenylamid

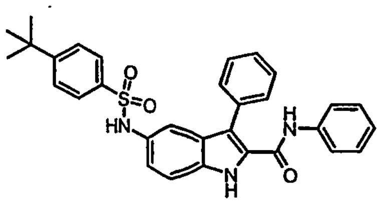

Gemäß Vorschrift 1 werden nach Umsetzung von 5-Nitro-3-phenyl-1*H*-indol-2-carbonsäure (1.27 g mg, 4.5 mmol) mit Anilin (419 mg, 1.5 mmol) das Produkt *5-Nitro-3-phenyl-1H-indol-2-carbonsäurephenylamid* in quantitativer Ausbeute erhalten, das ohne weitere Reinigung in der nachfolgenden Stufe umgesetzt wird.
Gemäß Vorschrift 2 werden nach Umsetzung von *5-Nitro-3-phenyl-1H-indol-2-carbonsäurephenylamid* (1.61 g, 4.5 mmol) mit Ammoniumformiat (1.42 g, 22.5 mmol) in Gegenwart von Palladium auf Kohle (160 mg) 1.19 mg (81%) des Produkts *5-Amino-3-phenyl-1H-indot-2-carbonsäurephenylamid* erhalten.
NMR (300 MHz, DMSO-d6): δ 4.90 (br, 2H), 6.68-6.72 (m, 2H), 7.15 (t, 1H), 7.20-7.40 (m, 4H), 7.44-7.60 (m, 6H), 9.38 (s, 1 H), 11.5 (s,1 H).
Gemäß Vorschrift 3 werden nach Umsetzung von *5-Amino-3-phenyl-1H-indol-2-carbonsäurephenylamid* (164 mg, 0.5 mmol) mit 4-tert-Butylbenzolsulfonsäurechlorid (116 mg, 0.50 mmol) und chromatographischer Reinigung (Kieselgel, Hexan/Ethylacetat (0-100% Ethylacetat)) 117 mg (44%) des Produkts *5-(4-tert-Butylbenzolsulfonylamino)-3-phenyl-1H-indol-2-carbonsäurephenylamid* erhalten.
NMR (300 MHz, DMSO-d6): δ 1.26 (s, 9H), 7.08 (m, 2H), 7.17 (d, 1 H), 7.28-7.51 (m, 10H), 7.55 (d, 2H), 7.59 (d, 2H), 9.65 (s, 1 H), 9.85 (s, 1 H), 11.94 (s, 1 H).

**Beispiel 13** 5-(4-Cyanobenzolsulfonylamino)-3-phenyl-1H-indol-2-carbonsäurephenylamid

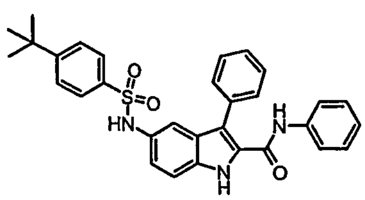

Gemäß Vorschrift 3 werden nach Umsetzung von *5-Amino-3-phenyl-1H-indol-2-carbonsäurephenylamid* (164 mg, 0.50 mmol) mit 4-Cyanobenzolsulfonylchlorid (100 mg, 0.50 mmol) und chromatographischer Reinigung (Kieselgel, Hexan/Ethylacetat (0-100% Ethylacetat)) 35 mg (14%) des Produkts erhalten.
NMR (300 MHz, DMSO-d6): δ 7.04-7.11 (m, 3H), 7.46-7.51 (m, 10H), 7.80 (d, 2H), 8.05 (d, 2H), 9.66 (s, 1 H), 10.12 (s, 1H), 11.99 (s, 1 H).

**Beispiel 14** 5-(4-Brombenzolsulfonylamino)-3-phenyl-1H-indol-2-carbonsäurephenylamid

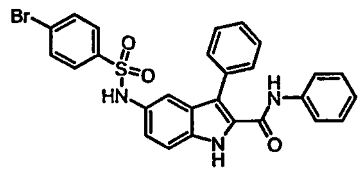

Gemäß Vorschrift 3 werden nach Umsetzung von *5-Amino-3-phenyl-1H-indol-2-carbonsäurephenylamid* (164 mg, 0.50 mmol) mit 4-Brombenzolsulfonylchlorid (127 mg, 0.50 mmol) und chromatographischer Reinigung (Kieselgel, Hexan/Ethylacetat (0-100% Ethylacetat)) 76 mg (27%) des Produkts erhalten.
NMR (300 MHz, DMSO-d6): δ 7.02-7.10 (m, 3H), 7.28-7.51 (m, 10H), 7.57 (d, 2H), 7.78 (d, 2H), 9.67 (s, 1 H), 9.92 (s, 1 H), 11.98 (s, 1 H).

**Beispiel 15** 5-(4-(Trifluormethoxy)benzolsulfonylamino)-3-phenyl-1H-indol-2-carbon-säurephenylamid

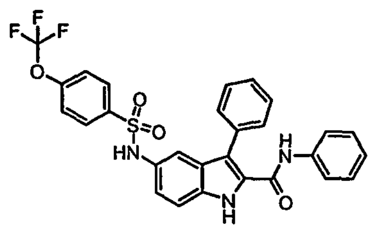

Gemäß Vorschrift 3 werden nach Umsetzung von *5-Amino-3-phenyl-1H-indol-2-carbonsäurephenylamid* (164 mg, 0.50 mmol) mit 4-(Trifluormethoxy)benzolsulfonylchlorid (130 mg, 0.50 mmol) und chromatographischer Reinigung (Kieselgel, Hexan/Ethylacetat (0-100% Ethylacetat)) 161 mg (58%) des Produkts erhalten.
NMR (300 MHz, DMSO-d6): δ 7.02-7.09 (m, 2H), 7.14 (d, 1 H), 7.28-7.57 (m, 12H), 7.77 (d, 2H), 7.78 (d, 2H), 9.67 (s, 1 H), 9.98 (s, 1H), 11.98 (s, 1 H).

**Beispiel 16** 5-(4-(Methylsulfonyl)benzolsulfonylamino)-3-phenyl-1H-indol-2-carbon-säurephenylamid

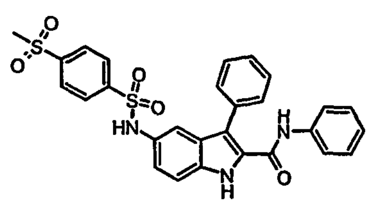

Gemäß Vorschrift 3 werden nach Umsetzung von *5-Amino-3-phenyl-1H-indol-2-carbonsäurephenylamid* (164 mg, 0.5 mmol) mit 4-Methylsulfonylbenzolsulfonylchlorid (127 mg, 0.50 mmol) und chromatographischer Reinigung (Kieselgel, Hexan/Ethylacetat (0-100% Ethylacetat)) 140 mg (51%) des Produkts erhalten.
NMR (300 MHz, DMSO-d6): δ 3.27 (s, 3H), 7.03-7.09 (m, 2H), 7.16 (d, 1 H), 7.28-7.51 (m, 10H), 7.91 (d, 2H), 8.12 (d, 2H), 9.66 (s, 1 H), 10.15 (s, 1 H), 11.99 (s, 1H).

**Beispiel 17** 5-(4-tert-Butylbenzolsulfonylamino)-3-phenyl-1*H*-indol-2-carbonsäure-pyridin-2-ylamid

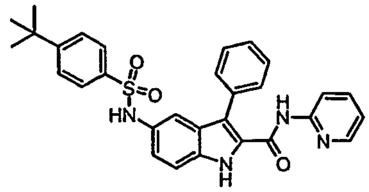

Gemäß Vorschrift 1 werden nach Umsetzung von 5-Nitro-3-phenyl-1H-indol-2-carbonsäure (1.27 g, 4.5 mmol) mit 2-Aminopyridin (423 mg, 4.5 mmol) 1.46 g (90%) des Produkts erhalten *5-Nitro-3-phenyl-1H-indol-2-carbonsäure-pyridin-2-ylamid*, das ohne weitere Reinigung in der nachfolgenden Stufe umgesetzt wird.
Gemäß Vorschrift 2 werden nach Umsetzung von erhalten *5-Nitro-3-phenyl-1H-indol-2-carbonsäurepyridin-2-ylamid* (1.45 g, 4.05 mmol) mit Ammoniumformiat (1.27 g, 20.23 mmol) in Gegenwart von Palladium auf Kohle (145 mg) 490 mg (37%) des Produkts *5-Amino-3-phenyl-1H-indol-2-carbonsäurepyridin-2-ylamid* erhalten.
NMR (300 MHz, DMSO-d6): δ 5.40 (br, 2H), 6.57 (s, 1 H), 6.73 (dd, 1 H), 7.06-7.10 (m, 1H), 7.23 (d, 1H), 7.43-7.53 (m, 5H), 7.79 (td, 1H), 8.15-8.23 (m, 2H), 9.00 (s, 1 H), 11.60 (s, 1 H).
Gemäß Vorschrift 3 werden nach Umsetzung von *5-Amino-3-phenyl-1H-indol-2-carbonsäurepyridin-2-ylamid* (98 mg, 0.3 mmol) mit 4-tert-Butylbenzolsulfonylchlorid (70 mg, 0.3 mmol) und chromatographischer Reinigung (Kieselgel, Hexan/Ethylacetat (0-100% Ethylacetat)) 72 mg (45%) des Produkts *5-(4-tert-Butylbenzolsulfonylamino)-3-phenyl-1H-indol-2-carbonsäure-pyridin-2-ylamid* erhalten.
NMR (300 MHz, DMSO-d6): δ 1.26 (s, 9H), 7.06-7.12 (m, 3H), 7.36-7.60 (m, 10H), 7.80 (td, 1 H), 8.14 (d, 1 H), 8.23 (d, 1 H), 9.31 (s, 1 H), 9.86 (s, 1 H), 12.1 (s, 1 H).

**Beispiel 18** 5-(4-Cyanobenzolsulfonylamino)-3-phenyl-1*H*-indol-2-carbonsäurepyridin-2-ylamid

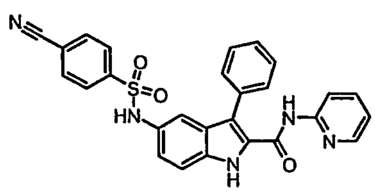

Gemäß Vorschrift 3 werden nach Umsetzung von *5-Amino-3-phenyl-1H-indol-2-carbonsäurepyridin-2-ylamid* (98 mg, 0.3 mmol) mit 4-Cyanobenzolsulfonylchlorid (60 mg, 0.3 mmol) und chromatographischer Reinigung (Kieselgel, Hexan/Ethylacetat (0-100% Ethylacetat)) 49 mg (33%) des Produkts erhalten.
NMR (300 MHz, DMSO-d6): δ 6.99 (s, 1H), 7.04 (dd, 1 H), 7.11 (dd, 1H), 7.36-7.54 (m, 6H), 7.77-7.84 (m, 3H), 8.05 (d, 2H), 8.14 (d, 1 H), 8.25 (d, 1 H), 9.34 (s, 1 H), 10.12 (s,1 H), 12.09 (s, 1 H).

**Beispiel 19** 5-(4-tert-Butylbenzolsulfonylamino)-3-phenyl-1H-indol-2-carbonsäure-(2-morpholin-4-ylethyl)amid

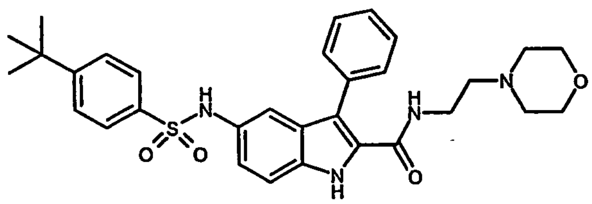

Gemäß Vorschrift 1 werden nach Umsetzung von 5-Nitro-3-Phenyl-1*H*-indol-2-carbonsäure (423 mg, 1.5 mmol)

mit 4-(2-Aminoethyl)morpholin (0.197 ml, 1.5 mmol) 528 mg (89%) des Produkts *5-Nitro-3-phenyl-1H-indol-2-carbonsäure-(2-morpholin-4-ylethyl)amid* erhalten, das ohne weitere Reinigung in der nachfolgenden Stufe umgesetzt wird.

Gemäß Vorschrift 2 werden nach Umsetzung von *5-Nitro-3-phenyl-1H-indol-2-carbonsäure-(2-morpholin-4-ylethyl) amid* (521 mg, 1.32 mmol) mit Ammoniumformiat (416 mg, 6.60 mmol) in Gegenwart von Palladium auf Kohle (52 mg) 337 mg (70%) des Produkts *5-Amino-3-phenyl-1H-indol-2-carbonsäure-(2-morpholin-4-ylethyl)amid* erhalten.

NMR (300 MHz, DMSO-d6): δ 2.16-2.22 (m, 4H), 2.25 (t, 2H), 3.26 (q, 2H), 3.40-3.48 (m, 4H), 4.75 (br, 2H), 6.55 (d, 1 H), 6.64 (dd, 1 H), 6.80 (t, 1H), 7.20 (d, 1 H), 7.32-7.56 (m, 5H), 11.28 (s, 1 H).

Gemäß Vorschrift 3 werden nach Umsetzung von *5-Amino-3-phenyl-1H-indol-2-carbonsäure-(2-morpholin-4-ylethyl) amid* (330 mg, 0.91 mmol) mit 4-tert-Butylbenzolsulfonsäurechlorid (212 mg, 0.91 mmol) und chromatographischer Reinigung (Kieselgel, Hexan/Ethylacetat (0-100% Ethylacetat)) 130 mg (25%) des Produkts *5-(4-tert-Butylbenzolsulfonylamino)-3-phenyl-1H-indol-2-carbonsäure-(2-morpholin-4-ylethyl)amid* erhalten.

NMR (300 MHz, DMSO-d6): δ 1.25 (s, 9H), 2.25-2.36 (m, 6H), 3.27 (q, 2H), 3.40-3.41 (m, 4H), 6.98 (t, 1H), 7.02-7.05 (m, 2H), 7.29-7.51 (m, 6H), 7.53 (d, 2H), 7.57 (d, 2H), 9.79 (s, 1 H), 11.73 (s, 1H).

**Beispiel 20** 5-(4-tert-Butylbenzolsulfonylamino)-3-phenyl-1H-indol-2-carbonsäure-(4-methylpiperazin-1-yl)amid

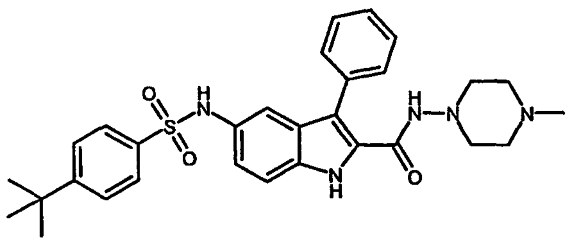

Gemäß Vorschrift 1 entsteht nach Umsetzung von 5-Nitro-3-Phenyl-1*H*-indol-2-carbonsäure (423 mg, 1.5 mmol) mit 1-Amino-4-methylpiperazin (0.282 ml, 1.5 mmol) das Produkt *5-Nitro-3-phenyl-1H-indol-2-carbonsäure-(4-methylpiperazin-1-yl)amid* nach chromatographischer Reinigung (Kieselgel, Dichlormethan/Methanol (0-30% Methanol)) in quantitativer Ausbeute.

NMR (300 MHz, DMSO-d6): δ 2.60 (s, 3H), 2.85-3.10 (m, 8H), 7.40-7.60 (m, 5H), 7.64 (d, 1 H), 8.23 (dd, 1 H), 8.48 (d, 1 H), 9.20 (s, 1 H), 12.60 (s, 1 H).

Gemäß Vorschrift 2 werden nach Umsetzung von *5-Nitro-3-phenyl-1H-indol-2-carbonsäure-(4-methylpiperazin-1-yl)amid* (830 mg, 2.19 mmol) mit Ammoniumformiat (688 mg, 10.91 mmol) in Gegenwart von Palladium auf Kohle (83 mg) und chromatographischer Reinigung (Kieselgel, Dichlormethan/Methanol (0-40% Methanol)) 720 mg (94%) des Produkts 5-*Amino-3-phenyl-1H-indol-2-carbonsäure-(4-methylpiperazin-1-yl)amid* erhalten.

NMR (300 MHz, DMSO-d6): δ 2.35 (s, 3H), 2.60-2.80 (m, 8H), 6.60-6.70 (m, 2H), 7.14 (d, 1H), 7.25-7.35 (m, 1 H), 7.38-7.50 (m, 4H), 8.42 (s, 1H), 11.25 (s, 1 H).

Gemäß Vorschrift 3 werden nach Umsetzung von *5-Amino-3-phenyl-1H-indol-2-carbonsäure-(4-methylpiperazin-1-yl) amid* (720 mg, 2.06 mmol) mit 4-tert-Butylbenzolsulfonsäurechlorid (476 mg, 2.06 mmol) und chromatographischer Reinigung (Kieselgel, Dichlormethan/Methanol (0-30% Methanol)) 170 mg (15%) des Produkts *5-(4-tert-Butylbenzolsulfonylamino)-3-phenyl-1H-indol-2-carbonsäure-(4-methyl-piperazin-1-yl)amid* erhalten.

NMR (300 MHz, DMSO-d6): δ 1.25 (s, 9H), 2.65 (s, 3H), 3.03-3.16 (m, 8H), 7.03 (d, 1H), 7.16 (s, 1H), 7.29-7.35 (m, 4H), 7.43-7.46 (m, 2H), 7.50-7.60 (m, 4H), 9.08 (s, 1 H), 9.85 (s, 1 H), 11.80 (s, 1 H).

**Beispiel 21** 5-(4-tert-Butylbenzolsulfonylamino)-3-phenyl-1H-indol-2-carbonsäure-pyrindin-4-ylamid

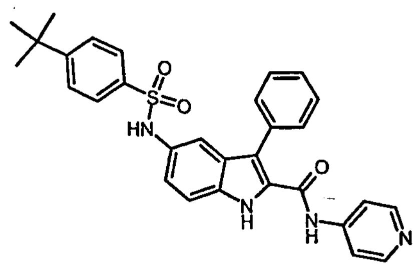

Gemäß Vorschrift 1 werden nach Umsetzung von 5-Nitro-3-phenyl-1*H*-indol-2-carbonsäure (1.0 g, 3.54 mmol) mit 4-Aminopyridin (382 mg, 3.54 mmol) 1.16 g (91%) des Produkts *5-Nitro-3-phenyl-1H-indol-2-carbonsäurepyrindin-4-ylamid* erhalten, das ohne weitere Reinigung in der nachfolgenden Stufe umgesetzt wird.
Gemäß Vorschrift 2 werden nach Umsetzung von *5-Nitro-3-phenyl-1H-indol-2-carbonsäurepyrindin-4-ylamid* (280 mg, 0.75 mmol) mit Ammoniumformiat (237 mg, 3.76 mmol) in Gegenwart von Palladium auf Kohle (28 mg) 110 mg (43%) des Produkts *5-Amino-3-phenyl-1H-indol-2-carbonsäurepyrindin-4-ylamid* erhalten.
NMR (300 MHz, DMSO-d6): δ 5.05 (br, 2H), 6.70-6.74 (m, 2H), 7.24 (d, 1H), 7.33 (t, 1 H), 7.41-7.50 (m, 6H), 8.41 (d, 2H), 9.95 (s, 1 H), 11.16 (s, 1 H).
Gemäß Vorschrift 3 werden nach Umsetzung von *5-Amino-3-phenyl-1H-indol-2-carbonsäurepyrindin-4-ylamid* (33 mg, 0.10 mmol) mit 4-tert-Butylbenzolsulfonsäurechlorid (28 mg, 0.12 mmol) und chromatographischer Reinigung (Kieselgel, Hexan/Ethylacetat (0-100% Ethylacetat)) 17 mg (32%) des Produkts *5-(4-tert-Butylbenzolsulfonylamino)-3-phenyl-1H-indol-2-carbonsäure-pyrindin-4-ylamid* erhalten.
NMR (300 MHz, DMSO-d6): δ 1.26 (s, 9H), 7.11 (dd, 1H), 7.18 (s, 1H), 7.31-7.61 (m, 12H), 8.43 (d, 2H), 9.87 (s, 1 H), 10.14 (s, 1 H), 12.00 (1 H).

**Beispiel 22** 5-(4-tert-Butylbenzylamino)-3-phenyl-1 H-indol-2carbonsäurepyridin-4-ylamid

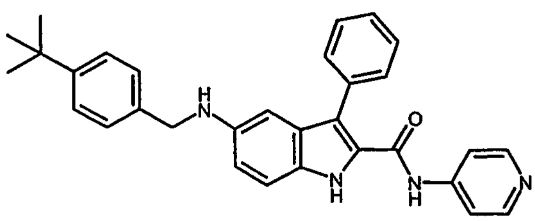

5-Amino-3-phenyl-1*H*-indol-2-carbonsäurepyrindin-4-ylamid (270 mg, 0.82 mmol) und 4-tert-Butylbenzaldehyd (146 mg, 0.90 mmol) werden in 34 ml Xylol vorgelegt, mit Titantetraethylat (0.34 ml, 1.64 mmol) versetzt und 9 Stunden refluxiert. Nach Entfernen des Lösungsmittels und chromatographischer Reinigung des Rückstands (Kieselgel, Hexan/Ethylacetat (0-100% Ethylacetat)) entstehen 130 mg (33%) des Produkts *5-{[1-(4-tert-Butylphenyl)-methyliden]-amino}-3-phenyl-1H-indol-2-carbonsäurepyridin-4-ylamid.*
NMR (300 MHz, DMSO-d6): δ 1.30 (s, 9H), 7.38-7.63 (m, 12H), 7.88 (d, 2H), 8.44 (d, 2H), 8.69 (s, 1 H), 10.17 (s, 1 H), 12.10 (s, 1 H).
5-{[1-(4-tert-Butylphenyl)-methyliden]-amino}-3-phenyl-1H-indol-2-carbonsäurepyridin-4-ylamid (70 mg, 0.15 mmol) wird in 5 ml Methanol gelöst und bei 0°C mit Natriumborhydrid (44 mg, 1.14 mmol) versetzt. Der Abbruch der Reaktion erfolgt durch Zugabe von Wasser. Nach Extraktion mit Ethylacetat werden die vereinigten organischen Phasen mit gesättigter Natriumchloridlösung gewaschen und über Natriumsulfat getrocknet. Nach Entfernen des Lösungsmittels und chromatographischer Reinigung des Rückstands (Kieselgel, Hexan/Ethylacetat (0-100% Ethylacetat)) entstehen 50 mg (71%) des Produkts *5-(4-tert-Butylbenzylamino)-3-phenyl-1H-indol-2-carbonsäurepyridin-4-ylamid* .
NMR (300 MHz, DMSO-d6): δ 1.27 (s, 9H), 4.18 (d, 2H), 5.91 (t, 1H), 6.56 (d, 1 H), 6.84 (dd, 1 H), 7.20-7.45 (m, 10H), 7.48 (d, 2H), 8.43 (d, 2H), 9.90 (s, 1 H), 11.56 (s, 1H).

**Beispiel 23** 5-(4-tert-Butylbenzoylamino)-3-phenyl-1*H*-indol-2-carbonsäure-(2-dimethylaminoethyl)amid

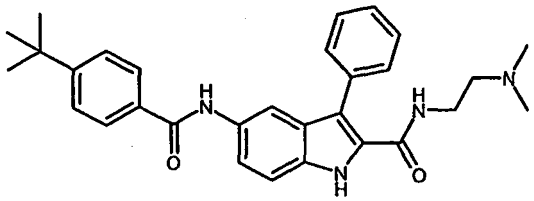

4-tert-Butylbenzoesäure (185 mg, 1.04 mmol) wird in 5ml DME vorgelegt und bei 0°C mit Thionychlorid (0.09 ml, 1.24 mmol) versetzt. Nach 30 Min. wird 5-Amino-3-phenyl-1*H*-indol-2-carbonsäure-(2-dimethylaminoethyl)amid (500 mg, 1.55 mmol) hinzugefügt und anschließend 20 Stunden bei Raumtemperatur gerührt. Der Abbruch der Reaktion erfolgt durch Zugabe 5 ml einer 10%igen wässrigen Zitronensäurelösung, anschließend wird die Lösung mit gesättigter Natriumhydrogencarbonatlösung in einen basischen Bereich gebracht. Nach Extraktion mit Ethylacetat folgt die Trocknung der vereinigten organischen Phasen über Natriumsulfat. Nach Entfernen des Lösungsmittels unter reduziertem Druck und chromatographischer Reinigung (Kieselgel, Dichlormethan/Methanol (0-10% Methanol)).beträgt die Ausbeute 22 mg (3%) an Produkt.
NMR (300 MHz, DMSO-d6): δ 1.31 (s, 9H), 2.00 (s, 6H), 2.22 (t, 2H), 3.38 (q, 2H), 7.00 (t, 1 H), 7.38-7.56 (m, 8H), 7.61 (dd, 1 H), 7.88 (d, 2H), 7.93 (s, 1 H), 10.08 (s, 1 H), 11.70 (s, 1 H).

**Beispiel 24** 5-(4-tert-Butylbenzolsulfonylamino)-3-(2-chlorophenyl)-1H-indol-2-carbonsäure-(2-dimethylaminoethyl) amid

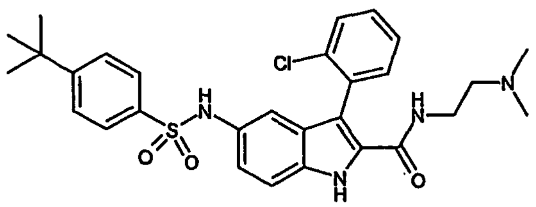

Gemäß Vorschrift 2 entstehen nach Umsetzung von 5-Nitro-1*H*-indol-2-carbonsäureethylester (500 mg, 2.13 mmol) mit Ammoniumformiat (671 mg, 10.65 mmol) in Gegenwart von Palladium auf Kohle (50 mg) 330 mg (76%) 5-Amino-1*H*-indol-2-carbonsäureethylester..
NMR (300 MHz, DMSO-d6): δ 1.32 (t, 3H), 4.30 (q, 2H), 4.70 (br, 2H), 6.64-6.72 (m, 2H), 6.83 (d, 1 H), 7.25 (d, 1 H), 11.40 (s, 1H).
Gemäß Vorschrift 3 werden nach Umsetzung von 5-Amino-1*H*-indol-2-carbonsäureethylester (160 mg, 0.78 mmol) mit 4-tert-Butylbenzolsulfonsäurechlorid (181 mg, 0.78 mmol) und chromatographischer Reinigung (Kieselgel, Hexan/ Ethylacetat (0-100% Ethylacetat)) 270 mg (86%) des Produkts *5-(4-tert-Butylbenzolsulfonylamino)-1H-indol-2-carbonsäureethylester* erhalten.
NMR (300 MHz, DMSO-d6): δ 1.25 (s, 9H), 1.32 (t, 3H), 4.32 (q, 2H), 7.02-7.08 (m, 2H), 7.30 (d, 1 H), 7.35 (d, 1 H), 7.52 (d, 2H), 7.62 (d, 2H), 9.97 (s, 1 H), 11.35 (s, 1H).
Gemäß Vorschrift 4 werden nach der Umsetzung von *5-(4-tert-Butylbenzolsulfonylamino)-1H-indol-2-carbonsäureethylester* (270 mg, 067 mmol) mit N-Bromsuccinimid (120 mg, 0.67 mmol) und chromatographischer Reinigung (Kieselgel, Hexan/Ethylacetat (0-100% Ethylacetat)) 240 mg (75%) des Produkts *3-Brom-5-(4-tert-butylbenzolsulfonylamino)-1H-indol-2-carbonsäureethylester* erhalten.
NMR (300 MHz, DMSO-d6): δ 1.25 (s, 9H), 1.32 (t, 3H), 4.34 (q, 2H), 7.12-7.18 (m, 2H), 7.35 (d, 1 H), 7.52 (d, 2H), 7.65 (d, 2H), 10.10 (s, 1 H), 12.1 (s, 1 H).
Gemäß Vorschrift 6 werden nach Umsetzung von *3-Brom-5-(4-tert-butylbenzolsulfonylamino)-1H-indol-2-carbonsäureethylester* (400 mg, 0.83 mmol) mit 2-Chlorphenylboronsäure (186 mg, 1.19 mmol) und chromatographischer Reinigung (Kieselgel, Hexan/Ethylacetat (0-100% Ethylacetat)) 320 mg (70%) des Produkts *5-(4-tert-Butylbenzolsulfonylamino)-3-(2-chlorophenyl)-1H-indol-2-carbonsäureethylester* erhalten.
NMR (300 MHz, DMSO-d6): δ 1.02 (t, 3H), 1.25 (s, 9H), 4.10-4.20 (m, 2H), 6.82 (s, 1H), 7.14 (d, 1 H), 7.22 (dd, 1H), 7.32-7.44 (m, 3H), 7.46-7.58 (m, 5H), 9.80 (s, 1H), 12.08 (s, 1 H).
Gemäß Vorschrift 5 werden nach Umsetzung von (320 mg, 0.63 mmol) mit 11.5 ml einer 1 M NaOH Lösung in Ethanol/Wasser (1/1) 290 mg (95%) des Produkts *5-(4-tert-Butylbenzolsulfonylamino)-3-(2-chlorphenyl)-1H-indot-2-carbonsäure* erhalten.
NMR (300 MHz, DMSO-d6): δ 1.25 (s, 9H), 6.77 (d, 1H), 7.12 (d, 1H), 7.25 (dd, 1H), 7.32-7.44 (m, 3H), 7.48-7.56 (m, 5H), 9.80 (s, 1H), 11.90 (s, 1H), 12.75 (br, 1H).
Gemäß Vorschrift 1 werden nach Umsetzung *5-(4-tert-Butylbenzolsulfonylamino)-3-(2-chlorphenyl)-1H-indol-2-carbonsäure* (290 mg, 0.6 mmol) mit N,N-Dimethylethylendiamin (0.066 ml, 0.6 mmol) und chromatographischer Reinigung (Kieselgel, Dichlormethan/Methanol (0-20% Methanol)) 180 mg (54%) des Produkts *5-(4-tert-Butylbenzolsulfonylamino)-3-(2-chlorphenyl)-1H-indol-2-carbonsäure-(2-dimethylaminoethyl)amid* erhalten..
NMR (300 MHz, DMSO-d6): δ 1.25 (s, 9H), 1.96 (s, 6H), 2.19 (t, 2H), 3.18-3.23 (m, 2H), 6.73-6.74 (m, 2H), 7.05 (dd, 1H), 7.28 (dd, 1H), 7.35 (d, 1H), 7.40-7.53 (m, 6H), 7.59 (dd, 1H), 9.75 (s, 1H), 11.80 (s, 1H).

**Beispiel 25** 5-(4-tert-Butylbenzolsulfonylamino)-3-(3-chlorphenyl)-1*H*-indol-2-carbonsäure-(2-dimethylaminoethyl) amid

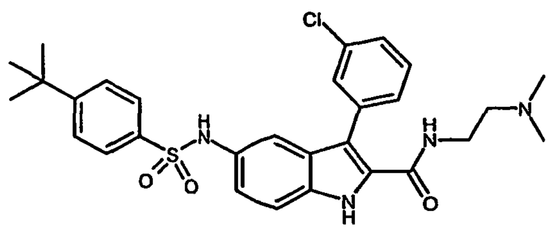

Gemäß Vorschrift 6 werden nach Umsetzung von *3-Brom-5-(4-tert butylbenzolsulfonylamino)-1H-indol-2-carbonsäureethylester* (400 mg, 0.83 mmol) mit 3-Chlorphenylboronsäure (186 mg, 1.19 mmol) und chromatographischer Reinigung (Kieselgel, Hexan/Ethylacetat (0-100% Ethylacetat)) 300 mg (66%) des Produkts *5-(4-tert-Butylbenzolsulfonylamino)-3-(3-chlorphenyl)-1H-indol-2-carbonsäureethylester* erhalten.
NMR (300 MHz, DMSO-d6): δ 1.15 (t, 3H), 1.25 (s, 9H), 4.22 (q, 2H), 7.08 (s, 1H), 7.13 (dd, 1H), 7.20-7.28 (m,1H), 7.34-7.45 (m, 4H), 7.52 (d, 2H), 7.60 (d, 2H), 9.92 (s, 1H), 12.05 (s, 1H).
Gemäß Vorschrift 5 werden nach Umsetzung von *5-(4-tert-Butylbenzolsulfonylamino)-3-(3-chlorphenyl)-1H-indol-2-carbonsäureethylester* (300 mg, 0.58 mmol) mit 10.9 ml einer 1M NaOH Lösung in Ethanol/Wasser (1/1) 240 mg (85%) des Produkts *5-(4-tert-Butylbenzolsulfonylamino)-3-(3-chlorphenyl)-1H-indol-2-carbonsäure* erhalten.
NMR (300 MHz, DMSO-d6): δ 1.23 (s, 9H), 7.06 (s, 1H), 7.11 (dd, 1H), 7.25 (d, 1H), 7.34-7.45 (m, 4H), 7.51 (d, 2H), 7.10 (d, 2H), 9.89 (s, 1H), 11.92 (s, 1H), 12.20 (br, 1H).
Gemäß Vorschrift 1 werden nach Umsetzung *5-(4-tert-Butylbenzolsulfonylamino)-3-(3-chlorphenyl)-1H-indol-2-carbonsäure* (240 mg, 0.5 mmol) mit N,N-Dimethylethylendiamin (0.054 ml, 0.5 mmol) und chromatographischer Reinigung (Kieselgel, Dichlormethan/Methanol (0-20% Methanol)) 90 mg (33%) des Produkts *5-(4-tert-Butylbenzolsulfonylamino)-3-(3-chlorphenyl)-1H-indol-2-carbonsäure-(2-dimethylaminoethyl)amid* erhalten.
NMR (300 MHz, DMSO-d6): δ 1.23 (s, 9H), 2.06 (s, 6H), 2.25 (t, 2H), 3.24 (q, 2H), 7.04-7.07 (m, 2H), 7.24 (d, 1H), 7.32-7.35 (m, 3H), 7.45-7.59 (m, 6H), 9.86 (s, 1H), 11.82 (s, 1H).

**Beispiel 26** 5-(4-tert-Butylbenzolsulfonylamino)-3-(4-chlorphenyl)-1H-indol-2-carbonsäure-(2-dimethylaminoethyl) amid

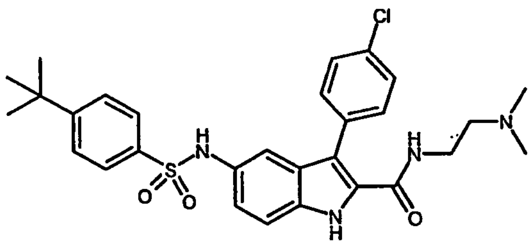

Gemäß Vorschrift 6 werden nach Umsetzung von *3-Brom-5-(4-tert-butylbenzolsulfonylamino)-1H-indol-2-carbonsäureethylester* (500 mg, 1.05 mmol) mit 4-Chlorphenylboronsäure (236 mg, 1.5 mmol) und chromatographischer Reinigung (Kieselgel, Hexan/Ethylacetat (0-100% Ethylacetat)) 396 mg (74%) des Produkts *5-(4-tert-Butylbenzolsulfonylamino)-3-(4-chlorphenyl)-1H-indol-2-carbonsäure-ethylester* erhalten.
NMR (300 MHz, DMSO-d6): δ 1.15 (t, 3H), 1.25 (s, 9H), 4.22 (q, 2H), 7.07 (s,1H), 7.14 (dd, 1H), 7.34 (d, 2H), 7.39 (d, 1H), 7.44-7.60 (m, 6H), 9.90 (s, 1H), 11.98(s,1H).
Gemäß Vorschrift 5 werden nach Umsetzung von Produkts *5-(4-tert-Butylbenzolsulfonylamino)-3-(4-chlorphenyl)-1H-indol-2-carbonsäure-ethylester* Azb SV 148 (396 mg, 0.77 mmol) mit 14.5 ml einer 1M NaOH Lösung in Ethanol/Wasser (1/1) 302 mg (81%) des Produkts *5-(4-tert-Butylbenzolsulfonylamino)-3-(4-chlorphenyl)-1H-indol-2-carbonsäure* erhalten.
NMR (300 MHz, DMSO-d6): δ 1.23 (s, 9H), 7.14 (s, 1H), 7.19 (dd, 1H), 7.38-7.48 (m, 3H), 7.47 (d, 2H), 7.53 (d, 2H), 7.60 (d, 2H), 9.89 (s, 1H), 11.90 (s, 1H), 12.90 (br, 1H).
Gemäß Vorschrift 1 werden nach Umsetzung *5-(4-tert-Butylbenzolsulfonylamino)-3-(4-chlorphenyl)-1H-indol-2-carbonsäure* (335 mg, 0.69 mmol) mit N,N-Dimethylethylendiamin (0.08 ml, 0.69 mmol) und chromatographischer Reinigung (Kieselgel, Dichlormethan/Methanol (0-20% Methanol)) 112 mg (29%) des Produkts *5-(4-tert-Butylbenzolsulfonylamino)-3-(4-chlorphenyl)-1H-indol-2-carbonsäure-(2-dimethylaminoethyl)amid* erhalten.
NMR (300 MHz, DMSO-d6): δ 1.24 (s, 9H), 2.03 (s, 6H), 2.23 (t, 2H), 3.23 (q, 2H), 7.01-7.04 (m, 2H), 7.17 (t, 1H). 7.29-7.35 (m, 3H), 7.51-7.60 (m, 6H), 9.83 (s, 1H), 11.78 (s, 1H).

**Beispiel 27** 5-(4-tert-Butylbenzolsulfonylamino)-3-(2,4-dichlorphenyl)-1H-indol-2-carbonsäure-(2-dimethylaminoethyl) amid

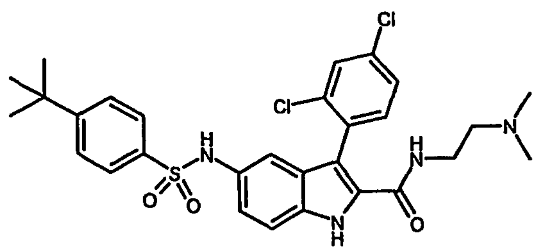

Gemäß Vorschrift 6 werden nach Umsetzung von *3-Brom-5-(4-tert-butylbenzolsulfonylamino)-1H-indol-2-carbonsäureethylester* (400 mg, 0.83 mmol) mit 2,4-Dichlorphenylboronsäure (227 mg, 1.19 mmol) und chromatographischer Reinigung (Kieselgel, Hexan/Ethylacetat (0-100% Ethylacetat)) 370 mg (81%) des Produkts *5-(4-tert-Butylbenzolsulfonylamino)-3-(2,4-dichlorphenyl)-1H-indol-2-carbonsäure-ethylester* erhalten..
NMR (300 MHz, DMSO-d6): δ 1.15 (t, 3H), 1.23 (s, 9H), 4,22 (q, 2H), 6.80 (d, 1H), 7.14 (dd, 1H). 7.30 (d, 1H), 7.40 (d, 1H). 7.42-7.56 (m, 5H), 7.70 (d, 1H), 9.89 (s, 1H), 12.12 (s, 1H).
Gemäß Vorschrift 5 werden nach Umsetzung von *5-(4-tert-Butylbenzolsulfonylamino)-3-(2,4-dichlorphenyl)-1H-indol-2-carbonsäureethylester* (370 mg, 0.68 mmol) mit 12.6 ml einer 1M NaOH Lösung in Ethanol/Wasser (1/1) 330 mg (94%) des Produkts *5-(4-tert-Butylbenzolsulfonylamino)-3-(2,4-dichlorphenyl)-1H-indol-2-carbonsäure* erhalten.
NMR (300 MHz, DMSO-d6): δ 1.23 (s, 9H), 6.78 (d, 1H). 7.12 (dd, 1H), 7.30 (d, 1H), 7.38 (d, 1H). 7.42-7.58 (m, 5H), 7.70 (d, 1H), 9.85 (s, 1H), 12.02 (s, 1H). Gemäß Vorschrift 1 werden nach Umsetzung *5-(4-tert-Butylbenzolsulfonylamino)-3-(2,4-dichlorphenyl)-1H-indol-2-carbonsäure* (330 mg, 0.64 mmol) mit N,N-Dimethylethylendiamin (0.07 ml,

0.64 mmol) und chromatographischer Reinigung (Kieselgel, Dichlormethan/Methanol (0-20% Methanol)) 170 mg (45%) des Produkts *5-(4-tert-Butylbenzolsulfonylamino)-3-(2,4-dichlorphenyl)-1H-indol-2-carbonsäure-(2-dimethylaminoethyl)amid* erhalten

NMR (300 MHz, DMSO-d6): δ 1.24 (s, 9H), 2.00 (s, 6H), 2.21 (t, 2H), 3.21-3.24 (m, 2H), 6.73 (d, 1H), 6.85 (t, 1H), 7.06 (dd, 1H), 7.31-7.39 (m, 2H), 7.47-7.56 (m, 5H), 7.77 (d, 1H), 9.79 (s, 1H), 11.87 (s, 1H).

**Beispiel 28** 5-(4-tert-Butylbenzolsulfonylamino)-3-(2-methylphenyl)-1H-indol-2-carbonsäure-(2-dimethylaminoethyl) amid

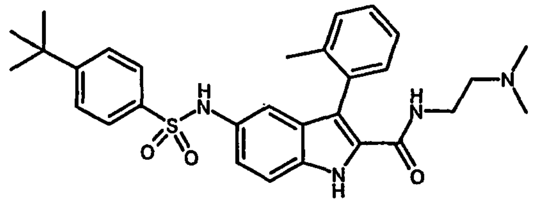

Gemäß Vorschrift 6 werden nach Umsetzung von *3-Brom-5-(4-tert-butylbenzolsulfonylamino)-1H-indol-2-carbonsäureethylester* (400 mg, 0.83 mmol) mit o-Toluylboronsäure (161 mg, 1.19 mmol) und chromatographischer Reinigung (Kieselgel, Hexan/Ethylacetat (0-100% Ethylacetat)) 250 mg (55%) des Produkts *5-(4-tert-Butylbenzolsulfonylamino)-3-(2-methylphenyl)-1H-indol-2-carbonsäureethyl-ester* erhalten.
NMR (300 MHz, DMSO-d6): δ 1.02 (s, 3H), 1.25 (s, 9H), 1.88 (s, 3H), 4.11 (q, 2H), 6.70 (d, 1H), 7.02 (d, 1H), 7.12-7.24 (m, 2H), 7.28-7.32 (m, 2H), 7.38 (d, 1H), 7.45-7.53 (m, 4H), 9.80 (s, 1H), 11.90 (s, 1H).
Gemäß Vorschrift 5 werden nach Umsetzung von Produkts *5-(4-tert-Butylbenzolsulfonylamino)-3-(2-methylphenyl)-1H-indol-2-carbonsäureethylester* (250 mg, 0.51 mmol) mit 9.4 ml einer 1M NaOH Lösung in Ethanol/Wasser (1/1) das Produkt *5-(4-tert-Butylbenzolsulfonylamino)-3-(2-methylphenyl)-1H-indol-2-carbonsäure* in quantitativer Ausbeute erhalten.
NMR (300 MHz, DMSO-d6): δ 1.23 (s, 9H), 1.88 (s, 3H), 6.68 (s, 1H), 7.00 (d, 1H), 7.12 (dd, 1H), 7.14-7.20 (m, 1H), 7.24-7.28 (m, 2H), 7.35 (d, 1H), 7.50 (m, 4H), 9.73 (s, 1H), 11.78 (s, 1H).
Gemäß Vorschrift 1 werden nach Umsetzung *5-(4-tert-Butylbenzolsulfonylamino)-3-(2-methylphenyl)-1H-indol-2-carbonsäure* (320 mg, 0.69 mmol) mit N,N-Dimethylethylendiamin (0.076 ml, 0.69 mmol) und chromatographischer Reinigung (Kieselgel, Dichlormethan/Methanol (0-20% Methanol)) 140 mg (38%) des Produkts *5-(4-tert-Butylbenzolsulfonylamino)-3-(2-methylphenyl)-1H-indol-2-carbonsäure-(2-dimethylaminoethyl)amid* erhalten.
NMR (300 MHz, DMSO-d6): δ 1.25 (s, 9H), 1.88 (s, 3H), 1.89 (s, 6H), 2.08-2.12 (m, 2H), 3.13-3.18 (m, 2H), 6.42 (t, 1H), 6.62 (d, 1H), 7.04-7.10 (m, 2H), 7.24-7.30 (m, 1H), 7.34-7.37 (m, 3H), 7.49 (2d, 4H), 9.70 (s, 1H), 11.71 (s, 1H).

**Beispiel 29** 5-(4-tert-Butylbenzolsulfonylamino)-3-(4-methylphenyl)-1H-indol-2-carbonsäure-(2-dimethylaminoethyl) amid

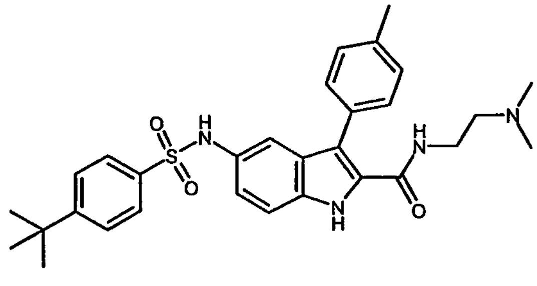

Gemäß Vorschrift 6 werden nach Umsetzung von *3-Brom-5-(4-tert-butylbenzolsulfonylamino)-1H-indol-2-car-*

*bonsäureethylester* (500 mg, 1.05 mmol) mit p-Toluylboronsäure (204 mg, 1.5 mmol) und chromatographischer Reinigung (Kieselgel, Hexan/Ethylacetat (0-100% Ethylacetat)) 420 mg (82%) des Produkts *5-(4-tert-Butylbenzolsulfonylamino)-3-(4-methylphenyl)-1H-indol-2-carbonsäureethyl-ester* erhalten.
NMR (300 MHz, DMSO-d6): δ 1.14 (t, 3H), 1.24 (s, 9H), 2.38 (s, 3H), 4.22 (q, 2H), 7.05 (d, 1H), 7.10 (dd, 1H), 7.18 (d, 2H), 7.22 (d, 2H), 7.36 (d, 1H), 7.52-7.60 (m, 4H), 9.84 (s, 1H), 11.82 (s, 1H).
Gemäß Vorschrift 5 werden nach Umsetzung von *5-(4-tert-Butylbenzolsulfonylamino)-3-(4-methylphenyl)-1H-indol-2-carbonsäureethylester* (420 mg, 0.86 mmol) mit 16 ml einer 1M NaOH Lösung in Ethanol/Wasser (1/1) 340 mg (85%) des Produkts *5-(4-tert-Butylbenzolsulfonylamino)-3-(4-methylphenyl)-1H-indol-2-carbonsäure* erhalten.
NMR (300 MHz, DMSO-d6): δ. 1.23 (s, 9H), 2.34 (s, 3H), 6.98-7.04 (m, 2H), 7.12 (d, 2H), 7.21 (d, 2H), 7.30 (d, 1H), 7.50-7.60 (m, 4H), 9.75 (s, 1H), 11.48 (s, 1H).
Gemäß Vorschrift 1 werden nach Umsetzung *5-(4-tert-Butylbenzolsulfonylamino)-3-(4-methylphenyl)-1H-indol-2-carbonsäure* (340 mg, 0.74 mmol) mit N,N-Dimethylethylendiamin (0.082 ml, 0.74 mmol) und chromatographischer Reinigung (Kieselgel, Amin-Phase, Dichlormethan/Methanol (0-20% Methanol)) 260 mg (66%) des Produkts 5-(4-*tert-Butylbenzolsulfonylamino)-3-(4-methylphenyl)-1H-indol-2-carbonsäure-(2-dimethylaminoethyl)amid* erhalten.
NMR (300 MHz, DMSO-d6): δ 1.25 (s, 9H), 1.99 (s, 6H), 2.19 (t, 2H), 2.38 (s, 3H), 3.21 (q, 2H), 6.94-7.03 (m, 3H), 7.17 (d, 2H), 7.26-7.33 (m, 3H), 7.51 (d, 2H), 7.56 (d, 2H), 9.76 (s, 1H), 11.67 (s, 1H).

**Beispiel 30** 5-(4-tert-Butylbenzolsulfonylamino)-3-(4-methylphenyl)-1H-indol-2-carbonsäurepyridin-4ylamid

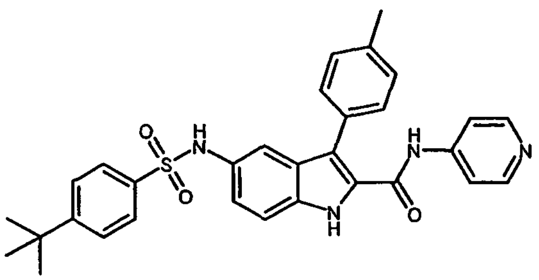

Gemäß Vorschrift 1 werden nach Umsetzung von *5-(4-tert-Butylbenzolsulfonylamino)-3-(4-methylphenyl)-1H-indol-2-carbonsäure* (170 mg, 0.37 mmol) mit 4-Aminopyridin (35 mg, 0.37 mmol) 130 mg (65%) des Produkts erhalten.
NMR (300 MHz, DMSO-d6): δ 1.25 (s, 9H), 2.36 (s, 3H), 7.09 (dd, 1H), 7.16-7.25 (m, 5H), 7.38 (d, 1H), 7.49 (d, 2H), 7.53 (d, 2H), 7.59 (d, 2H), 8.43 (d, 2H), 9.85 (s, 1H), 10.09 (s, 1H), 11.94 (s, 1H).

**Beispiel 31** 5-(4-tert-Butylbenzolsulfonylamino)-3-(4-methoxyphenyl)-1H-indol-2-carbonsäure-(2-dimethylaminoethyl) amid

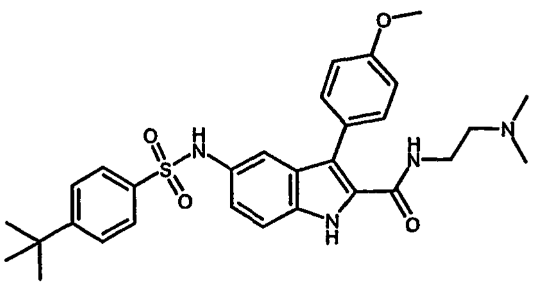

Gemäß Vorschrift 6 werden nach Umsetzung von *3-Brom-5-(4-tert-butylbenzolsulfonylamino)-1H-indol-2-carbonsäureethylester* (500 mg, 1.05 mmol) mit 4-Methoxyphenylboronsäure (228 mg, 1.5 mmol) und chromatographischer

Reinigung (Kieselgel, Hexan/Ethylacetat (0-100% Ethylacetat)) 400 mg (75%) des Produkts *5-(4-tert-Butylbenzolsulfonylamino)-3-(4-methoxyphenyl)-1H-indol-2-carbonsäureethylester* erhalten.

NMR (300 MHz, DMSO-d6): δ 1.14 (t, 3H), 1.24 (s, 9H), 3.82 (s, 3H), 4.22 (q, 2H), 6.96 (d, 2H), 7.08-7.12 (m, 2H), 7.24 (d, 2H), 7.35 (d, 1H), 7.53 (d, 2H), 7.64 (d, 2H), 9.85 (s, 1H), 11.80 (s, 1H).

Gemäß Vorschrift 5 entsteht nach Umsetzung von *5-(4-tert-Butylbenzolsulfonylamino)-3-(4-methoxyphenyl)-1H-indol-2-carbonsäureethylester* (420 mg, 0.86 mmol) mit 15 ml einer 1M NaOH Lösung in Ethanol/Wasser (1/1) das Produkt *5-(4-tert-Butylbenzolsulfonylamino)-3-(4-methoxyphenyl)-1H-indol-2-carbonsäure* in quantitativer Ausbeute..

NMR (300 MHz, DMSO-d6): δ 1.24 (s, 9H), 3.82 (s, 3H), 6.96 (d, 2H), 7.08-7.12 (m, 2H), 7.22 (d, 2H), 7.34 (d, 1H), 7.52 (d, 2H), 7.60 (d, 2H), 9.80 (s, 1H), 11.70 (s, 1H), 12.25 (br, 1H).

Gemäß Vorschrift 1 werden nach Umsetzung *5-(4-tert-Butylbenzolsulfonylamino)-3-(4-methoxyphenyl)-1H-indol-2-carbonsäure* (390 mg, 0.82 mmol) mit N,N-Dimethylethylendiamin (0.091 ml, 0.82 mmol) und chromatographischer Reinigung (Kieselgel, Dichlormethan/Methanol (0-20% Methanol)) 200 mg (44%) des Produkts *5-(4-tert-Butylbenzolsulfonylamino)-3-(4-methoxyphenyl)-1H indol-2-carbonsäure-(2-dimethylaminoethyl)amid* erhalten

NMR (300 MHz, DMSO-d6): δ 1.25 (s, 9H), 2.01 (s, 6H), 2.22 (t, 2H), 3.22 (q, 2H), 3.83 (s, 3H), 6.93 (t, 1H), 6.98-6.99 (m, 2H), 7.03 (d, 2H), 7.21 (d, 2H), 7.31 (d, 1H), 7.52 (d, 2H), 7.56 (d, 2H), 9.77 (s, 1H), 11.63 (s, 1H).

**Beispiel 32** 5-(4-tert-Butylbenzolsulfonylamino)-3-pyridin-3-yl-1H-indol-2-carbon-säure-(2-dimethylaminoethyl)amid

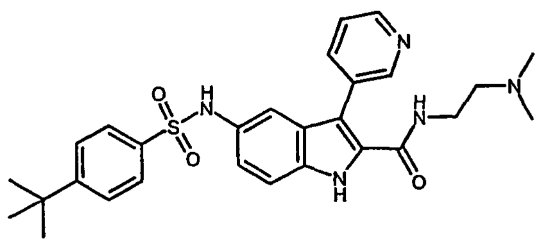

Gemäß Vorschrift 6 werden nach Umsetzung von *3-Brom-5-(4-tert-butylbenzolsulfonylamino)-1H-indol-2-carbonsäureethylester* (500 mg, 1.05 mmol) mit Pyridin-3-boronsäure (184 mg, 1.5 mmol) und chromatographischer Reinigung (Kieselgel, Hexan/Ethylacetat (0-100% Ethylacetat)) 280 mg (56%) des Produkts *5-(4-tert-Butylbenzolsulfonylamino)-3-pyridin-3-yl-1H-indol-2-carbonsäureethylester* erhalten..

NMR (300 MHz, DMSO-d6): δ 1.14 (t, 3H), 1.24 (s, 9H), 4.21 (q, 2H), 7.05 (d, 1H), 7.12 (dd, 1H), 7.40-7.60 (m, 6H), 7.72 (m, 1H), 8.50 (d, 1H), 8.58 (dd, 1H), 9.90 (s, 1H), 12.10 (s, 1H).

Gemäß Vorschrift 5 werden nach Umsetzung von *5-(4-tert-Butylbenzolsulfonylamino)-3-pyridin-3-yl-1H-indol-2-carbonsäureethylester* (280 mg, 0.59 mmol) mit 11 ml einer 1M NaOH Lösung in Ethanol/Wasser (1/1) 260 mg (98%) des Produkts *5-(4-tert-Butylbenzolsulfonylamino)-3-pyridin-3-yl-1H-indol-2-carbonsäure* erhalten.

NMR (300 MHz, DMSO-d6): δ 1.22 (s, 9H), 7.08-7.12 (m, 2H), 7.40 (d, 1H), 7.52 (d, 2H), 7.58 (d, 2H), 7.70-7.76 (m, 1H), 8.05 (d, 1H), 8.68 (dd, 1H), 8.72 (d, 1H), 9.95 (s, 1H), 12.12 (s, 1H).

Gemäß Vorschrift 1 werden nach Umsetzung *5-(4-tert-Butylbenzolsulfonylamino)-3-pyridin-3-yl-1H-indol-2-carbonsäure* (260 mg, 0.58 mmol) mit N,N-Dimethylethylendiamin (0.064 ml, 0.58 mmol) und chromatographischer Reinigung (Kieselgel, Dichlormethan/Methanol (0-20% Methanol)) sowie anschließender Umkristallisation aus Dichlormethan 130 mg (43%) des Produkts *5-(4-tert-Butylbenzolsulfonylamino)-3-pyridin-3-yl-1H-indol-2-carbonsäure-(2-dimethylaminoethyl)amid* erhalten.

NMR (300 MHz, DMSO-d6): δ 1.25 (s, 9H), 2.06 (s, 6H), 2.25 (t, 2H), 3.24 (q, 2H), 7.04-7.08 (m, 2H), 7.36 (d, 1H), 7.44-7.58 (m, 6H), 7.68-7.71 (m, 1H), 8.46 (d, 1H), 8.56 (dd, 1H), 9.82 (s, 1H), 11.85 (s, 1H).

**Beispiel 33:** 5-(4-tert-Butylbenzolsulfonylamino)-3-(4-hydroxyphenyl)-1H-indol-2-carbonsäure-(2-dimethylaminoethyl) amid

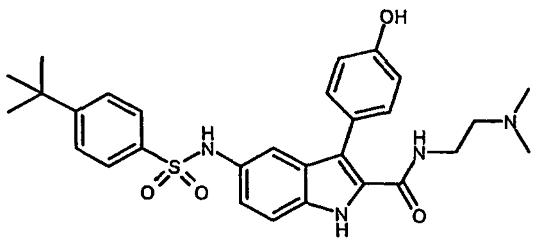

5-(4- tert- Butylbenzolsulfonylamino)- 3-(4- methoxyphenyl)- 1*H*-indol- 2- carbonsäure-(2- dimethylaminoethyl) amid (180 mg, 0.33 mmol) wird mit 9.70 ml einer 1M Bortribromid-Lösung in Dichlormethan (9.70 mmol) versetzt und 20 Stunden bei Raumtemperatur gerührt. Der Abbruch der Reaktion erfolgt durch Zugabe gesättigter Natriumhydrogencarbonat-Lösung. Nach Extraktion mit Ethylacetat, werden die vereinigten organischen Phasen mit 2N Natronlauge und gesättigter Natriumchloridlösung gewaschen. Nach Trocknen über Natriumsulfat, Entfernen des Lösungsmittels sowie chromatogaphischer Reinigung (Kieselgel, Dichlormethan/Methanol (0-50% Methanol)) werden 60 mg (34% der Theorie) des Produkts erhalten.
NMR (300 MHz, DMSO-d6): δ 1.26 (s, 9H), 2.00 (s, 6H), 2.19 (t, 2H), 3.23 (q, 2H), 6.81-6.87 (m, 3H), 6.94 (s, 1H), 6.99-7.08 (m, 3H), 7.31 (d, 1H), 7.50-7.56 (AA'BB', 4H), 9.58 (s, 1H), 9.75 (s, 1H), 11.58 (s, 1H).

**Beispiel 34:** 5-(4-tert-Butylbenzolsulfonylamino)-3-(3-fluorphenyl)-1H-indol-2-carbon-säure-(2-dimethylaminoethyl) amid

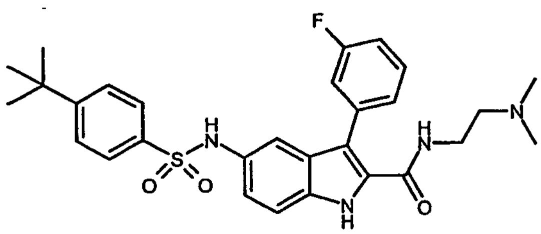

*5-(4-ted-Butylbenzolsulfonylamino)-3-(3-fluorphenyl)-1H-indol-2-carbonsäureethyl-ester:* Gemäß Vorschrift 6 werden nach Umsetzung von 3-Brom-5-(4-tert-butylbenzolsulfonylamino)-1H-indol-2-carbonsäureethylester (400 mg, 0.83 mmol) mit 3-Fluorphenylboronsäure (167 mg, 1.19 mmol) und chromatographischer Reinigung (Kieselgel, Hexan/Ethylacetat (0-100% Ethylacetat)) 290 mg (71 %) des Produkts erhalten.
NMR (300 MHz, DMSO-d6): δ 1.17 (t, 3H), 1.24 (s, 9H), 4.23 (q, 2H), 7.05-7.65 (m, 11H), 9.88 (s, 1H), 12.01 (s, 1H)
*5-(4-tert-Butylbenzolsulfonylamino)-3-(3-fluorphenyl)-1H-indol-2-carbonsäure:* Gemäß Vorschrift 5 werden nach Umsetzung von 5-(4-tert-Butylbenzolsulfonylamino)-3-(3-fluorphenyl)-1H-indol-2-carbonsäureethylester (280 mg, 0.59 mmol) mit 9.1 ml einer 1M NaOH Lösung in Ethanol/Wasser (1/1) das Produkt in quantitativer Ausbeute erhalten.
NMR (300 MHz, DMSO-d6): δ 1.24 (s, 9H), 6.98-7.21 (m, 5H), 7.34-7.46 (m, 6H), 9.86 (s, 1H), 11.89 (s, 1H), 12.90 (br, 1H).
*5-(4-tert-Butylbenzolsulfonylamino)-3-(3-fluorphenyl)-1H-indol-2-carbonsäure*-(2-*dimethylaminoethyl)amid:* Gemäß Vorschrift 1 werden nach Umsetzung von 5-(*4*-tert-Butylbenzolsulfonylamino)-3-(3-fluorphenyl)-1*H*-indol-2-carbonsäure (240 mg, 0.51 mmol) mit N,N-Dimethylethylendiamin (0.056 mL, 0.51 mmol) und chromatographischer Reinigung (Kieselgel, Dichlormethan/Methanol (0-30% Methanol)) 160 mg (59%) des Produkts erhalten.
NMR (300 MHz, DMSO-d6): δ 1.24 (s, 9H), 2.06 (s, 6H), 2.26 (t, 2H), 3.25 (q, 2H), 7.04-7.13 (m, 4H), 7.19-7.30 (m, 2H), 7.35 (d, 1H), 7.36-7.59 (m, 5H), 9.81 (s, 1H), 11.79 (s, 1H).

**Beispiel 35:** 5-(4-tert-Butylbenzolsulfonylamino)-3-phenyl-1H-indol-2-carbonsäure-(2-hydroxypropyl)amid

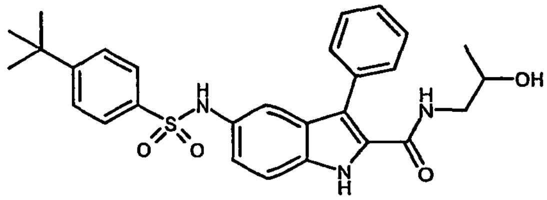

*5-(4-tert-Butylbenzolsulfonylamino)-3-phenyl-1H-indol-2-carbonsäureethylester.* Gemäß Vorschrift 6 werden nach Umsetzung von 3-Brom-5-(4-tert-butylbenzolsulfonylamino)-1*H*-indol-2-carbonsäureethylester (2.0 g, 4.15 mmol) mit Phenylboronsäure (725 mg, 5.9 mmol) und chromatographischer Reinigung (Kieselgel, Hexan/Ethylacetat (0-50% Ethylacetat)) 1.35 g (68%) des Produkts erhalten.
NMR (300 MHz, DMSO-d6): δ 1.17 (t, 3H), 1.25 (s, 9H), 4.19 (q, 2H), 7.07 (d, 1H), 7.12 (dd, 1H), 7.23-7.44 (m, 6H), 7.50-7.59 (m, 4H), 9.85 (s, 1H), 11.90 (s, 1H).
*5-(4-tert-Butylbenzolsulfonylamino)-3-phenyl-1H-indol-2-carbonsäure:* Gemäß Vorschrift 5 werden nach Umsetzung von 5-(4-tert-Butylbenzolsulfonylamino)-3-phenyl-1*H*-indol-2-carbonsäureethylester (1.35 g, 2.83 mmol) mit 55 mL einer 1M NaOH Lösung in Ethanol/Wasser (1/1) 1.17 g (92%) des Produkts erhalten. NMR (300 MHz, DMSO-d6): δ 1.25 (s, 9H), 7.05 (d, 1H), 7.09 (dd, 1H), 7.27-7.43 (m, 6H), 7.55 (d, 2H), 7.60 (d, 2H), 9.82 (s, 1H), 11.80 (s,1H), 12.5 (br, 1H).
*5-(4-tert-Butylbenzolsulfonylamino)-3-phenyl-1H-indol-2-carbonsäure-(2-hydroxy-propyl)amid:* Gemäß Vorschrift 1 werden nach Umsetzung von 5-(4-tert-Butylbenzolsulfonylamino)-3-phenyl-1*H*-indol-2-carbonsäure (199 mg, 0.44 mmol) mit 1-Amino-2-propanol (0.035 mL, 0.44 mmol) und präparativer Dünnschichtchromatographie (Kieselgel, Dichlormethan/Methanol 95:5) 33 mg (15%) des Produkts erhalten (AP 3795).

NMR (300 MHz, DMSO-d6): δ 0.94 (d, 3H), 1.25 (s, 9H), 2.99-3.23 (m, 2H), 3.54-3.66 (m, 1H), 4.60 (d, 1H), 7.02-7.04 (m, 2H), 7.16 (t, 1H), 7.28-7.51 (m, 6H), 7.52 (d, 2H), 7.57 (d, 2H), 9.79 (s, 1H), 11.71 (s, 1H).

**Beispiel 36:** 5-(4-tert-Butylbenzolsulfonylamino)-3-phenyl-1*H*-indol-2-carbonsäure-(2-methoxyethyl)amid

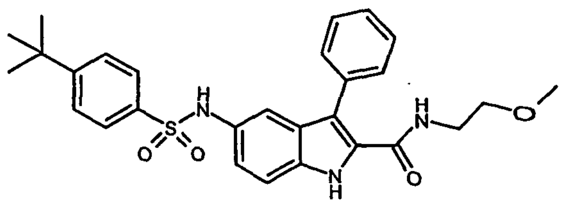

Gemäß Vorschrift 1 werden nach Umsetzung von 5-(4-tert-Butylbenzolsulfonylamino)-3-phenyl-1H-indol-2-carbonsäure (199 mg, 0.44 mmol) mit 2-Methoxyethylamin (0.039 mL, 0.44 mmol) und präparativer Dünnschichtchromatographie (Kieselgel, Dichlormethan/Methanol 95:5) 20 mg (9%) des Produkts erhalten.
NMR (300 MHz, DMSO-d6): δ 1.25 (s, 9H), 3.16 (s, 3H), 3.31 (m, 4H), 7.02-7.05 (m, 2H), 7.20 (br, 1H), 7.30-7.48 (m, 6H), 7.52 (d, 2H), 7.57 (d, 2H), 9.79 (s, 1H), 11.71 (s, 1H).

**Beispiel 37:** 5-(4-tert-Butylbenzolsulfonylamino)-3-phenyl-1*H*-indol-2-carbonsäure-(2-hydroxyethyl)amid

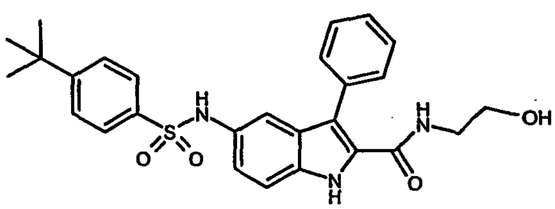

Gemäß Vorschrift 1 werden nach Umsetzung von 5-(4-tert-Butylbenzolsulfonylamino)-3-phenyl-1*H*-indol-2-carbonsäure (199 mg, 0.44 mmol) mit Ethanolamin (0.027 mL, 0.44 mmol) und präparativer Dünnschichtchromatographie (Kieselgel, Dichlormethan/Methanol 95:5) 23 mg (11 %) des Produkts erhalten.

NMR (300 MHz, DMSO-d6): δ 1.25 (s, 9H), 3.22 (q, 2H), 3.35-3.41 (m, 2H), 4.61 (t, 1H), 7.02-7.07 (m, 2H), 7.30-7.47 (m, 7H), 7.52 (d, 2H), 7.57 (d, 2H), 9.79 (s,1H), 11.69 (s, 1H).

**Beispiel 38:** 5-(4-tert-Butylbenzolsulfonylamino)-3-phenyl-1*H*-indol-2-carbonsäure-(2-hydroxy-1-methylethyl)amid

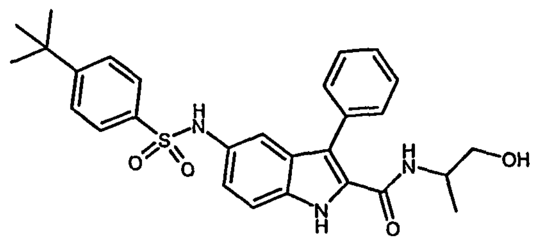

Gemäß Vorschrift 1 werden nach Umsetzung von 5-(4-tert-Butylbenzolsulfonylamino)-3-phenyl-1H-indol-2-carbonsäure (199 mg, 0.44 mmol) mit 2-Amino-propanol (0.035 mL, 0.44 mmol) und präparativer Dünnschichtchromatographie (Kieselgel, Dichlormethan/Methanol 95:5) 32 mg (14%) des Produkts erhalten.
NMR (300 MHz, DMSO-d6): δ 0.96 (d, 3H), 1.25 (s, 9H), 3.16-3.33 (m, 2H), 3.87-3.96 (m, 1H), 4.63 (t, 1H), 6.95-7.06 (m, 3H), 7.28-7.47 (m, 6H), 7.52 (d, 2H), 7.57 (d, 2H), 9.79 (s, 1H), 11.71 (s, 1H).

**Beispiel 39:** 5-(4-tert-Butylbenzolsulfonylamino)-3-phenyl-1*H*-indol-2-carbonsäure-(2-acetylaminoethyl)amid

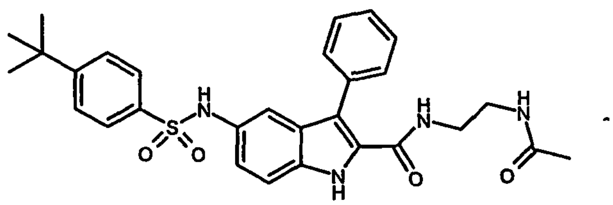

Gemäß Vorschrift 1 werden nach Umsetzung von 5-(4-tert-Butylbenzolsulfonylamino)-3-phenyl-1*H*-indol-2-carbonsäure (199 mg, 0.44 mmol) mit N-Acetylethylendiamin (0.047 mL, 0.44 mmol) und präparativer Dünnschichtchromatographie (Kieselgel, Dichlormethan/Methanol 95:5) 42 mg (18%) des Produkts erhalten.
NMR (300 MHz, DMSO-d6): δ 1.25 (s, 9H), 1.76 (s, 3H), 3.07-3.10 (m, 2H), 3.17-3.26 (m, 2H), 7.03 (dd, 1H), 7.08 (s, 1H), 7.27-7.49 (m, 7H), 7.52 (d, 2H), 7.57 (d, 2H), 7.80 (t, 1H), 9.79 (s, 1H), 11.66 (s, 1H).

**Beispiel 40:** 5-(4-tert-Butylbenzolsulfonylamino)-3-phenyl-1*H*-indol-2-carbonsäure-(tetrahydropyran-4-yl)amid

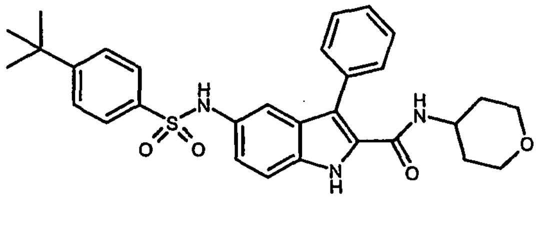

Gemäß Vorschrift 1 werden nach Umsetzung von 5-(4-tert-Butylbenzolsulfonylamino)-3-phenyl-1*H*-indol-2-carbonsäure (199 mg, 0.44 mmol) mit 4-Aminotetrahyropyran (45 mg, 0.44 mmol) und präparativer Dünnschichtchromatographie (Kieselgel, Dichlormethan/Methanol 95:5) 45 mg (19%) des Produkts erhalten.
NMR (300 MHz, DMSO-d6): δ 1.13-1.44 (m, 11H), 1.66-1.70 (m, 2H), 3.30-3.37 (m, 2H), 3.69-3.73 (m, 2H), 3.84-3.89 (m,1H), 7.03 (dd, 1H), 7.09 (d, 1H), 7.29-7.47 (m, 7H), 7.52 (d, 2H), 7.58 (d, 2H), 9.80 (s, 1H), 11.73 (s, 1H).

**Beispiel 41:** 5-(4-tert-Butylbenzolsulfonylamino)-3-phenyl-1*H*-indol-2-carbonsäure-(1-methylpiperidin-4-yl)amid

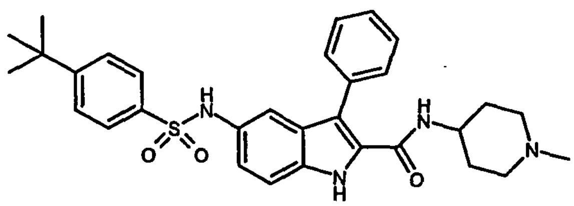

Gemäß Vorschrift 1 werden nach Umsetzung von 5-(4-tert-Butylbenzolsulfonylamino)-3-phenyl-1*H*-indol-2-carbonsäure (199 mg, 0.44 mmol) mit 4-Amino-1-methylpiperidin (51 mg, 0.44 mmol) und chromatographischer Reinigung 154 mg (64%) des Produkts erhalten.
NMR (300 MHz, DMSO-d6): δ 1.25 (s, 9H), 1.37-1.53 (br, 2H), 1.85-1.91 (m, 2H), 2.60 (s, 3H) ,2.69-2.89 (br, 2H), 3.07-3.10 (br, 2H), 3.87-3.89 (br, 1H), 7.03 (dd, 1H), 7.12 (s, 1H), 7.29-7.47 (m, 7H), 7.52 (d, 2H), 7.57 (d, 2H), 9.82 (s, 1H), 11.71 (s, 1H).

**Beispiel 42**: 5-(4-tert-Butylbenzolsulfonylamino)-3-(4-N,N-dimethylaminophenyl)-1*H*-indol-2-carbonsäure-(2-dimethylaminoethyl)amid

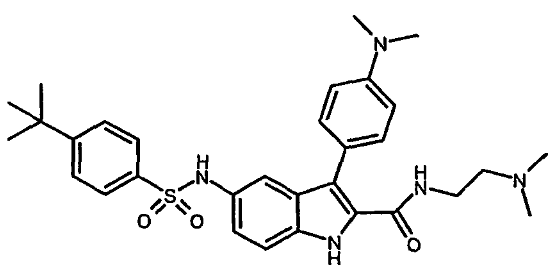

*5-(4-tert-Butylbenzolsulfonylamino)-3-(4-N,N-dimethylaminophenyl)-1H-indol-2-carbonsäureethylester:* Gemäß Vorschrift 6 werden nach Umsetzung von 3-Brom-5-(4-tert-butylbenzolsulfonylamino)-1*H*-indol-2-carbonsäureethylester (400 mg, 0.83 mmol) mit 4-*N,N*-Dimethylaminophenylboronsäure (196 mg, 1.19 mmol) und chromatographischer Reinigung (Kieselgel, Hexan/Ethylacetat (0-100% Ethylacetat)) 90 mg (21 %) des Produkts erhalten.
NMR (300 MHz, DMSO-d6): δ 1.23 (t, 3H), 1.25 (s, 9H), 2.96 (s, 6H), 4.20 (q, 2H), 6.74 (d, 2H), 7.06-7.17 (m, 4H), 7.34 (d, 1H), 7.53 (d, 2H), 7.58 (d, 2H), 9.81 (s. 1H), 11.69 (s, 1H).
*5-(4-tert-Butylbenzolsulfonylamino)-3-(4-N,N-dimethylaminophenyl)-1H-indol-2-carbonsäure*: Gemäß Vorschrift 5 werden nach Umsetzung von 5-(4-tert-Butylbenzolsulfonylamino)-3-(4-N,N-dimethylaminophenyl)-1H-indol-2-carbonsäure-ethylester (90 mg, 0.59 mmol) mit 3.2 mL einer 1M NaOH Lösung in Ethanol/Wasser (1/1) das Produkts in quantitativer Ausbeute erhalten.
*5-(4-tert-Butylbenzolsulfonylamino)-3-(4-N,N-dimethylaminophenyl)-1H-indol-2-carbonsäure-(2-dimethylaminoethyl)amid:* Gemäß Vorschrift 1 werden nach Umsetzung von *5*-(*4*-tert-Butylbenzolsulfonylamino)-3-(4, N,N-dimethylaminophenyl)-1*H*-indol-2-carbonsäure (220 mg, 0.45 mmol) mit N,N-Dimethylethylendiamin (0.049 mL, 0.45 mmol) und chromatographischer Reinigung (Kieselgel, Dichlormethan/Methanol (0-30% Methanol)) 22 mg (9%) des Produkts erhalten.
NMR (300 MHz, DMSO-d6): δ 1.24 (s, 9H), 2.05 (s, 6H), 2.27 (t, 2H), 2.97 (s, 6H), 3.24 (q, 2H), 6.81 (d, 2H), 6.89 (t,

1H), 6.98-7.01 (m, 2H), 7.11 (d, 2H), 7.29 (d, 1H), 7.50-7.57 (AA'BB', 4H), 9.74 (s, 1H), 11.54 (s, 1H).

**Beispiel 43:** 5-(4-tert-Butylbenzolsulfonylamino)-3-(3-methoxyphenyl)-1*H*-indol-2-carbonsäure-(2-dimethylaminoethyl) amid

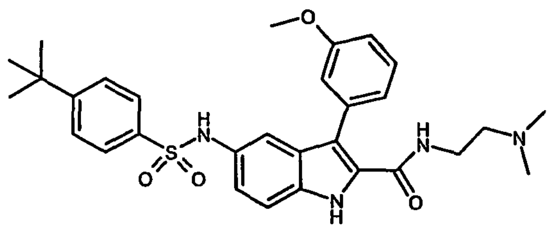

*5-(4-tert-Butylbenzolsulfonylamino)-3-(3-methoxyphenyl)-1H-indol-2-carbonsäure-ethylester:* Gemäß Vorschrift 6 werden nach Umsetzung von 3-Brom-5-(4-tert-butylbenzolsulfonylamino)-1*H*-indol-2-carbonsäureethylester (400 mg, 0.83 mmol) mit 3-Methoxyphenylboronsäure (181 mg, 1.19 mmol) und chromatographischer Reinigung (Kieselgel, Hexan/Ethylacetat (0-100% Ethylacetat)) 250 mg (60%) des Produkts erhalten.
NMR (300 MHz, DMSO-d6): δ 1.16 (t, 3H), 1.24 (s, 9H), 3.79 (s, 3H), 4.20 ( q, 2H), 6.84 (d, 1H), 6.92-6.96 (m, 2H), 7.11-7.14 (m, 2H), 7.30-7.42 (m, 2H), 7.51 (d, 2H), 7.58 (d, 2H), 9.88 (s, 1H), 11.90 (s, 1H).

*5-(4-tert-Butylbenzolsulfonylamino)-3-(3-methoxyphenyl)-1H-indol-2-carbonsäure*: Gemäß Vorschrift 5 werden nach Umsetzung von 5-(4-tert-Butylbenzol-sulfonylamino)-3-(3-methoxyphenyl)-1H-indol-2-carbonsäureethylester (250 mg, 0.49 mmol) mit 9.1 mL einer 1M NaOH Lösung in Ethanol/Wasser (1/1) 190 mg (81%) des Produkts erhalten.
NMR (300 MHz, DMSO-d6): δ 1.24 (s, 9H), 3.78 (s, 3H), 6.84 (d, 1H), 6.90-6.92 (m, 2H), 7.07-7.12 (m, 2H), 7.28-7.36 (m, 2H), 7.51 (d, 2H), 7.57 (d, 2H), 9.85 (s, 1H), 11.79 (s, 1H), 12.90 (br, 1H).

*5-(4-tert-Butylbenzolsulfonylamino)-3-(3-methoxyphenyl)-1H-indol-2-carbonsäure-(2-dimethylaminoethyl) amid:* Gemäß Vorschrift 1 werden nach Umsetzung von *5-(4*-tert-Butylbenzolsulfonylamino)-3-(3-methoxyphenyl)-1*H*-indol-2-carbonsäure (190 mg, 0.40 mmol) mit N,N-Dimethylethylendiamin (0.044 mL, 0.40 mmol) und chromatographischer Reinigung (Kieselgel, Dichlormethan/Methanol (0-20% Methanol)) 120 mg (55%) des Produkts erhalten.
NMR (300 MHz, DMSO-d6): δ 1.24 (s, 9H), 2.02 (s, 6H), 2.23 (t, 2H), 3.23 (q, 2H), 3.79 (s, 3H), 6.83-6.97 (m, 2H), 6.96-7.09 (m, 4H), 7.31-7.40 (m, 2H), 7.50 (d, 2H), 7.56 (d, 2H), 9.80 (br, 1H), 11.71 (s, 1H).

**Beispiel 44:** 5-(4-tert-Butylbenzolsulfonylamino)-3-(3-trifluormethylphenyl)-1*H*-indol-2-carbonsäure-(2-dimethylaminoethyl)amid

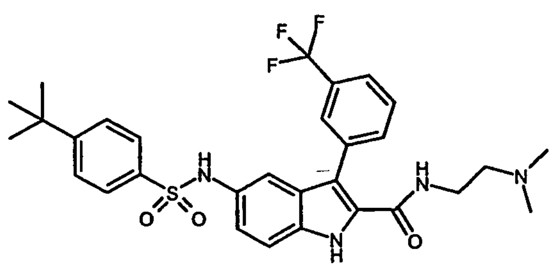

*5-(4-tert-Butylbenzolsulfonylamino)-3-(3-trifluormethylphenyl)-1H-indol-2-carbonsäureethylester:* Gemäß Vorschrift 6 werden nach Umsetzung von 3-Brom-5-(4-tert-butylbenzolsulfonylamino)-1*H*-indol-2-carbonsäureethylester (400 mg, 0.83 mmol) mit 3-Trifluormethylphenylboronsäure (226 mg, 1.19 mmol) und chromatographischer Reinigung (Kieselgel, Hexan/Ethylacetat (0-100% Ethylacetat)) 300 mg (66%) des Produkts erhalten.
NMR (300 MHz, DMSO-d6): δ 1.16 (t, 3H), 1.22 (s, 9H), 4.21 (q, 2H), 7.09 (s, 1H), 7.15 (dd, 1H), 7.41 (d, 1H), 7.49 (d, 2H), 7.56-7.59 (m, 3H), 7.65-7.75 (m, 3H), 9.95 (s, 1H), 12.08 (s, 1H).

*5-(4-tert Butylbenzolsulfonylamino)-3-(3-trifluormethylphenyl)-1H-indol-2-carbonsäure*: Gemäß Vorschrift 5

werden nach Umsetzung von 5-(4-tert-Butylbenzolsulfonylamino)-3-(3-trifluormethylphenyl)-1*H*-indol-2-carbonsäureethyl-ester (300 mg, 0.55 mmol) mit 10.2 mL einer 1M NaOH Lösung in Ethanol/Wasser (1/1) 270 mg (95%) des Produkts erhalten

NMR (300 MHz, DMSO-d6): δ 1.24 (s, 9H), 7.08-7.18 (m, 2H), 7.40 (d, 1H), 7.52 (d, 2H), 7.58-7.75 (m, 6H), 9.91 (s, 1H), 11.99 (s,1H), 12.40 (br, 1H).

*5-(4-terf-Butylbenzolsulfonylamino)-3-(3-trifluormethylphenyl)-1H-indol-2-carbonsäure-(2-dimethylaminoethyl)amid:* Gemäß Vorschrift 1 werden nach Umsetzung von *5-(4*-tert-Butylbenzolsulfonylamino)-3-(3-trifluormethylphenyl)-1*H*-indol-2-carbonsäure (270 mg, 0.52 mmol) mit N,N-Dimethylethylendiamin (0.057 mL, 0.52 mmol) und chromatographischer Reinigung (Kieselgel, Dichlormethan/Methanol (0-20% Methanol)) 180 mg (59%) des Produkts erhalten.

NMR (300 MHz, DMSO-d6): δ 1.22 (s, 9H), 2.05 (s, 6H), 2.25 (t, 2H), 3.24 (q, 2H), 7.05-7.09 (m, 2H), 7.35 (d, 1H), 7.44-7.51 (m, 3H), 7.57-7.61 (m, 4H), 7.66-7.75 (m, 2H), 9.89 (s, 1H), 11.86 (s,1H).

**Beispiel 45:** 5-(4-tert-Butylbenzolsulfonylamino)-3-(3-fluorphenyl)-1*H*-indol-2-carbon-säure-(2-hydroxyethyl)amid

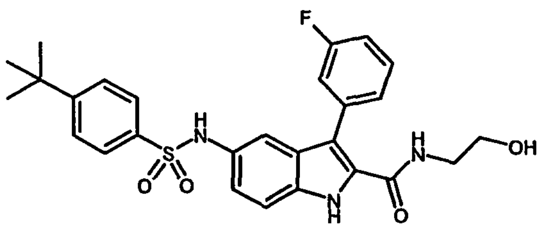

Gemäß Vorschrift 1 werden nach Umsetzung von *5-(4*-tert-Butylbenzolsulfonylamino)-3-(3-fluorphenyl)-1H-indol-2-carbonsäure (150 mg, 0.32 mmol) mit Ethanolamin (0.019 mL, 0.32 mmol) und chromatographischer Reinigung (Kieselgel, Dichlormethan/Methanol (0-20% Methanol)) 27 mg (16%) des Produkts erhalten.

NMR (300 MHz, DMSO-d6): δ 1.24 (s, 9H), 3.24 (q, 2H), 3.40 (q, 2H), 4.64 (t, 1H), 7.05-7.21 (m, 5H), 7.34 (d, 1H), 7.42-7.61 (m, 6H), 9.83 (s, 1H), 11.79 (s, 1H).

**Beispiel 46:** 5-(4-tert-Butylbenzolsulfonylamino)-3-(3-fluorphenyl)-1H-indol-2-carbon-säure-(tetrahydropyran-4-yl) amid

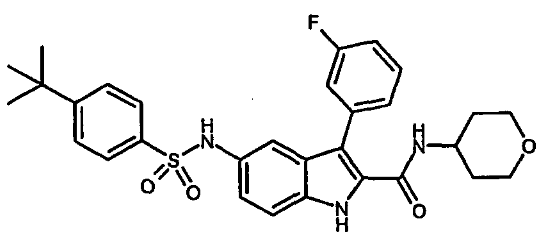

Gemäß Vorschrift 1 werden nach Umsetzung von 5-(4-tert-Butylbenzolsulfonylamino)-3-(3-fluorphenyl)-1H-indol-2-carbonsäure (150 mg, 0.32 mmol) mit 4-Aminotetrahydropyran (33 mg, 0.32 mmol) und chromatographischer Reinigung (Kieselgel, Dichlormethan/Methanol (0-20% Methanol)) 57 mg (33%) des Produkts erhalten.

NMR (300 MHz, DMSO-d6): δ 1.24 (s, 9H), 1.32-1.36 (m, 2H), 1.67-1.71 (m, 2H), 3.33-3.38 (m, 2H+$H_2O$), 3.74-3.78 (m, 2H), 3.87-3.94 (m, 1H), 7.04-7.20 (m, 5H), 7.34 (d, 1H), 7.43-7.73 (m, 6H), 9.84 (s, 1H), 11.81 (s, 1H).

**Beispiel 47:** 5-(4-tert-Butylbenzolsulfonylamino)-3-(3-fluorphenyl)-1*H*-indol-2-carbon-säure-(2-acetylaminoethyl)amid

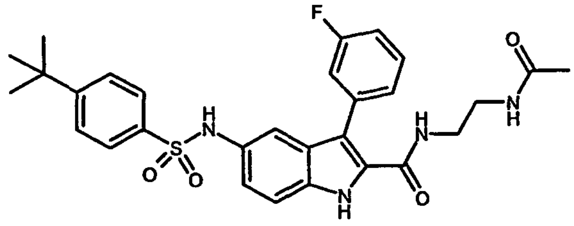

Gemäß Vorschrift 1 werden nach Umsetzung von *5-(4*-tert-Butylbenzolsulfonylamino)-3-(3-fluorphenyl)-1*H*-indol-2-carbonsäure (150 mg, 0.32 mmol) mit N-Acetylethylendiamin (0.03 mL, 0.32 mmol) und chromatographischer Reinigung (Kieselgel, Dichlormethan/Methanol (0-20% Methanol)) 45 mg (26%) des Produkts erhalten.
NMR (300 MHz, DMSO-d6): δ 1.24 (s, 9H), 1,77 (s, 3H), 3.10 (q, 2H), 3.20 (q, 2H), 7.08-7.20 (m, 5H), 7.35 (d, 1H), 7.43-7.59 (m, 5H), 7.76 (t, 1H), 7.84 (t, 1H), 9.83 (s, 1H), 11.76 (s, 1H).

**Beispiel 48:** 5-(4-tert-Butylbenzolsulfonylamino)-3-(3-fluorphenyl)-1*H*-indol-2-carbon-säure-(2-morpholin-4-ylethyl) amid

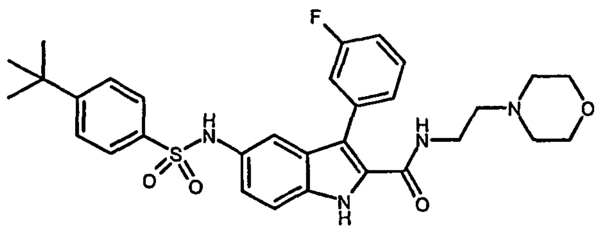

Gemäß Vorschrift 1 werden nach Umsetzung von 5-(4-tert-Butylbenzolsulfonylamino)-3-(3-fluorphenyl)-1*H*-indol-2-carbonsäure (150 mg, 0.32 mmol) mit 4-(2-Aminoethyl)morpholin (0.042 mL, 0.32 mmol) und chromatographischer Reinigung (Kieselgel, Dichlormethan/Methanol (0-20% Methanol)) 58 mg (32%) des Produkts erhalten.
NMR (300 MHz, DMSO-d6): δ 1.24 (s, 9H), 2.26-2.33 (m, 6H), 3.27-3.33 (m, 2H+$H_2O$), 3.44-3.46 (m, 4H), 7.05-7.15 (m, 4H), 7.19-7.26 (m, 2H), 7.34 (d, 1H), 7.47-7.62 (m, 5H), 9.82 (s, 1H), 11.82 (s, 1H).

**Beispiel 49:** 5-(4-tert-Butylbenzolsulfonylamino)-3-(3-methoxyphenyl)-1*H*-indol-2-carbonsäure-(2-hydroxyethyl)amid

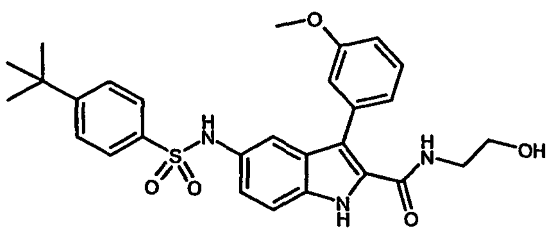

Gemäß Vorschrift 1 werden nach Umsetzung von *5-(4*-tert-Butylbenzolsulfonylamino)-3-(3-methoxyphenyl)-1*H*-indol-2-carbonsäure (200 mg, 0.42 mmol) mit Ethanolamin (0.025 mL, 0.42 mmol) und chromatographischer Reinigung (Kieselgel, Dichlormethan/Methanol (0-20% Methanol)) 48 mg (22%) des Produkts erhalten.
NMR (300 MHz, DMSO-d6): δ 1.24 (s, 9H), 3.21-3.31 (m, 2H), 3.36-3.42 (m, 2H), 3.79 (s, 3H), 4.62 (t, 1H), 6.85-6.95 (m, 3H), 7.04 (dd, 1H), 7.14 (d, 1H), 7.29-7.38 (m, 3H), 7.50 (d, 2H), 7.57 (d, 2H), 9.82 (s, 1H), 11.70 (s, 1H).

**Beispiel 50:** 5-(4-tert-Butylbenzolsulfonylamino)-3-(3-methoxyphenyl)-1*H*-indol-2-carbonsäure-(2-acetylaminoethyl) amid

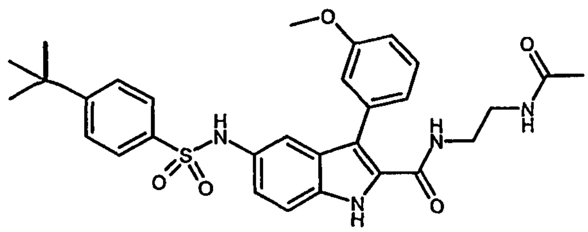

Gemäß Vorschrift 1 werden nach Umsetzung von *5-(4*-tert-Butylbenzolsulfonylamino)-3-(3-methoxyphenyl)-1*H*-indol-2-carbonsäure (200 mg, 0.42 mmol) mit N-Acetylethylenamin (0.04 mL, 0.42 mmol) und chromatographischer Reinigung (Kieselgel, Dichlormethan/Methanol (0-20% Methanol)) 110 mg (47%) des Produkts erhalten.
NMR (300 MHz, DMSO-d6): δ 1.24 (s, 9H), 1.76 (s, 3H), 3.07-3.11 (m, 2H), 3.18-3.24 (m, 2H), 3.79 (s, 3H), 6.85-6.95 (m, 3H), 7.04 (dd, 1H), 7.17 (s, 1H), 7.31-7.37 (m, 2H), 7.50-7.62 (m, 5H), 7.81 (t, 1H), 9.83 (s, 1H), 11.68 (s, 1H).

**Beispiel 51:** 5-(4-tert-Butylbenzolsulfonylamino)-3-pyridin-3-yl-1*H*-indol-2-carbon-säure-(2-acetylaminoethyl)amid

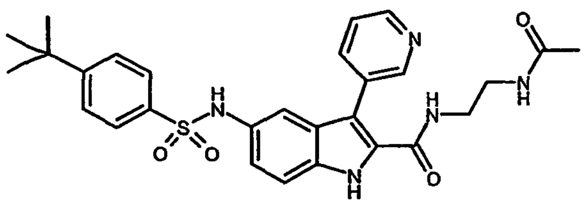

Gemäß Vorschrift 1 werden nach Umsetzung von 5-(4-tert-Butylbenzolsulfonylamino)-3-pyridin-3-yl-1*H*-indol-2-carbonsäure (210 mg, 0.47 mmol) mit N-Acetylethylendiamin (0.045 mL, 0.47 mmol) und chromatographischer Reinigung (Kieselgel, Dichlormethan/Methanol (0-20% Methanol)) 66 mg (26%) des Produkts erhalten.
NMR (300 MHz, DMSO-d6): δ 1.25 (s, 9H), 1.78 (s, 3H), 3.09-3.14 (m, 2H), 3.16-3.24 (m, 2H), 7.06-7.09 (m, 2H), 7.37 (d, 1H), 7.44 (dd, 1H), 7,52 (d, 2H), 7.57 (d, 2H), 7.67-7.69 (m, 1H), 7.86 (t, 1H), 7.93 (t, 1H), 8.47 (d, 1H), 8.53 (dd,1H), 9.83 (s, 1H), 11.85 (s, 1H).

**Beispiel 52:** 5-(4-tert-Butylbenzolsulfonylamino)-3-pyridin-3-yl-1*H*-indol-2-carbon-säure-(tetrahydropyran-4yl)amid

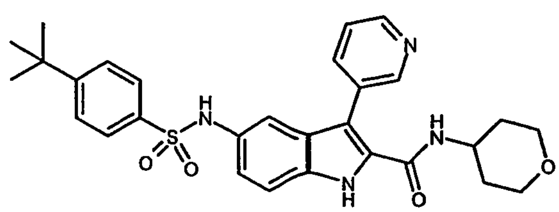

Gemäß Vorschrift 1 werden nach Umsetzung von 5-(4-tert-Butylbenzolsulfonylamino)-3-pyridin-3-yl-1*H*-indol-2-carbonsäure (140 mg, 0.31 mmol) mit 4-Aminotetrahydropyran (32 mg, 0.31 mmol) und chromatographischer Reinigung (Kieselgel, Dichlormethan/Methanol (0-50% Methanol)) 30 mg (18%) des Produkts erhalten.
NMR (300 MHz, DMSO-d6): δ 1.24 (s, 9H), 1.34-1.39 (m, 2H), 1.67-1.71 (m, 2H), 3.32-3.38 (m, 2H+$H_2O$), 3.76-3.80

(m, 2H), 3.87-3.97 (m, 1H), 7.07 (dd, 1H), 7.11 (s, 1H), 7.37 (d, 1H), 7.45 (dd, 1H), 7.52 (d, 2H), 7.58 (d, 2H), 7.66-7.69 (m, 1H), 7.82 (d, 1H), 8.46 (d, 1H), 8.51 (d, 1H), 9.83 (s, 1H), 11.87 (s, 1H).

**Beispiel 53:** 5-(4-tert-Butylbenzolsulfonylamino)-3-pyridin-4-yl-1*H*-indol-2-carbon-säure-(2-acetylaminoethyl)amid

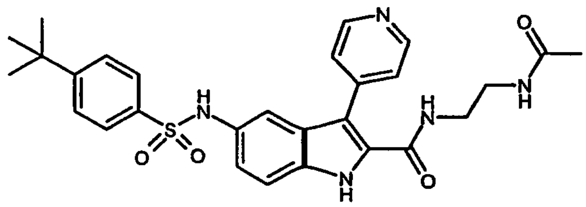

*5-(4-tert-Butylbenzolsulfonylamino)-3-pyridin-4-yl-1H-indol-2-carbonsäureethylester:*

Gemäß Vorschrift 6 werden nach Umsetzung von 3-Brom-5-(4-tert-butylbenzolsulfonylamino)-1*H*-indol-2-carbonsäureethylester (500 mg, 1.05 mmol) mit Pyridin-4-boronsäure (184 mg, 1.5 mmol) und chromatographischer Reinigung (Kieselgel, Hexan/Ethylacetat (0-100% Ethylacetat)) 310 mg (62%) des Produkts erhalten.
NMR (300 MHz, DMSO-d6): δ 1.18 (t, 3H), 1.24 (s, 9H), 4.23 (q, 2H), 7.11-7.16 (m, 2H), 7.32 (d, 2H), 7.42 (d, 1H), 7.53 (d, 2H), 7.59 (d, 2H), 8.62 (d, 2H), 9.95 (s, 1H), 12.17 (s, 1H).

*5-(4-tert-Butylbenzolsulfonylamino)-3-pyridin-4-yl-1H-indol-2-carbonsäure:*

Gemäß Vorschrift 5 werden nach Umsetzung von 5-(4-tert-Butylbenzolsulfonylamino)-3-pyridin-4-yl-1*H*-indol-2-carbonsäureethylester (310 mg, 0.65 mmol) mit 12 mL einer 1M NaOH Lösung in Ethanol/Wasser (1/1) 290 mg (99%) des Produkts erhalten.
NMR (300 MHz, DMSO-d6): δ 1.24 (s, 9H), 7.10 (dd, 1H), 7.20 (d, 1H), 7.41 (d, 1H), 7.52-7.63 (m, 6H), 8.73 (d, 2H), 9.99 (s, 1H), 12.24 (s, 1H).

*5-(4-tert-Butylbenzolsulfonylamino)-3-pyridin-4-yl-1H-indol-2-carbonsäure-(2-acetyl-aminoethyl)amid:* Gemäß Vorschrift 1 werden nach Umsetzung von 5-(4-tert-Butylbenzolsulfonylamino)-3-pyridin-4-yl-1H-indol-2-carbonsäure (180 mg, 0.4 mmol) mit N-Acetylethylendiamin (0.038 mL, 0.4 mmol) und chromatographischer Reinigung (Kieselgel, Dichlormethan/Methanol (0-20% Methanol)) 22 mg (10%) des Produkts erhalten.
NMR (300 MHz, DMSO-d6): δ 1.24 (s, 9H), 1.77 (s, 3H), 3.12-3.16 (m, 2H), 3.20-3.27 (m, 2H), 7.04 (dd, 1H), 7.19 (d, 1H), 7.27 (d, 2H), 7.36 (d, 1H), 7.54 (d, 2H), 7.59 (d, 2H), 7.89 (t, 1H), 8.09 (t, 1H), 8.58 (d, 2H), 9.95 (br, 1H), 11.97 (br, 1H).

**Beispiel 54:** 5-(4-tert-Butylbenzolsulfonylamino)-3-pyridin-4-yl-1*H*-indol-2-carbon-säure-(2-morpholin-4-ylethyl)amid

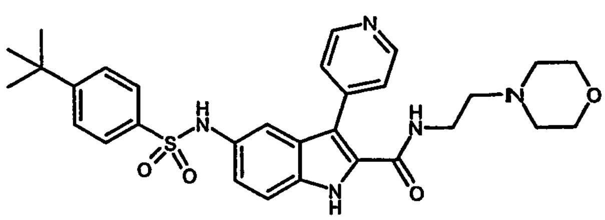

Gemäß Vorschrift 1 werden nach Umsetzung von 5-(4-tert-Butylbenzolsulfonylamino)-3-pyridin-4-yl-1H-indol-2-carbonsäure (145 mg, 0.32 mmol) mit 4-(2-Aminoethyl)morpholin (0.042 mL, 0.32 mmol) und chromatographischer Reinigung (Kieselgel, Dichlormethan/Methanol (0-20% Methanol)) 58 mg (32%) des Produkts erhalten.
NMR (300 MHz, DMSO-d6): δ 1.24 (s, 9H), 2.30-2.38 (m, 6H), 3.30-3.34 (m, 2H+$H_2O$), 3.45-3.47 (m, 4H), 7.05 (dd, 1H), 7.16 (d, 1H), 7.30 (d, 2H), 7.35 (d, 1H), 7.49-7.65 (m, 5H), 8.61 (d, 2H), 9.89 (br, 1H), 11.97 (br, 1H).

**Beispiel 55:** 5-(4-tert-Butylbenzolsulfonylamino)-3-pyridin-4-yl-1*H*-indol-2-carbon-säure-(tetrahydropyran-4-yl)amid

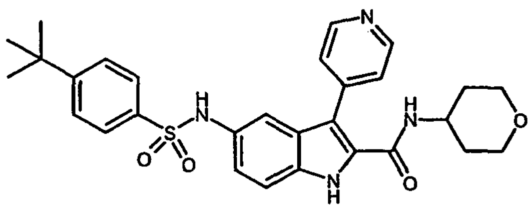

Gemäß Vorschrift 1 werden nach Umsetzung von 5-(4-tert-Butylbenzolsulfonylamino)-3-pyridin-4-yl-1*H*-indol-2-carbonsäure (145 mg, 0.32 mmol) mit 4-Aminotetrahydropyran (33 mg, 0.32 mmol) und chromatographischer Reinigung (Kieselgel, Dichlormethan/Methanol (0-20% Methanol)) 90 mg (53%) des Produkts erhalten.
NMR (300 MHz, DMSO-d6): δ 1.24 (s, 9H), 1.31-1.49 (m, 2H), 1.69.1.74 (m, 2H), 3.39-3.50 (m, 2H), 3.79-3.82 (m, 2H), 3.89-4.00 (m, 1H), 7.06 (dd, 1H), 7.24 (d, 1H), 7.27 (d, 2H), 7.37 (d, 1H), 7.54 (d, 2H), 7.60 (d, 2H), 8.03 (d, 1H), 8.57 (d, 2H), 9.87 (s, 1H), 11.96 (s, 1H).

**Beispiel 56:** 5-(4-tert-Butylbenzolsulfonylamino)-3-(3-methylphenyl)-1*H*-indol-2-carbonsäure-(2-acetylaminoethyl) amid

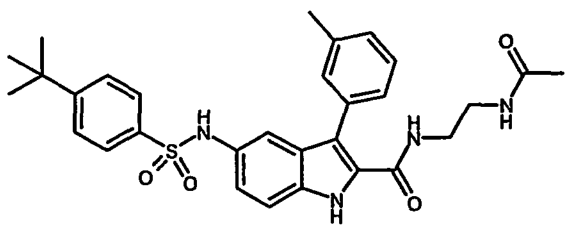

*5-(4-tert-Butylbenzolsulfonylamino)-3-(3-methylphenyl)-1H-indol-2-carbonsäureethyl-ester*: Gemäß Vorschrift 6 werden nach Umsetzung von 3-Brom-5-(4-tert-butylbenzolsulfonylamino)-1*H*-indol-2-carbonsäureethylester (500 mg, 1.05 mmol) mit m-Toluylboronsäure (204 mg, 1.5 mmol) und chromatographischer Reinigung (Kieselgel, Hexan/Ethylacetat (0-100% Ethylacetat)) 330 mg (67%) des Produkts erhalten.
NMR (300 MHz, DMSO-d6): δ 1.18 (t, 3H), 1.24 (s, 9H), 2.55 (s, 3H), 4.19 (q, 2H), 7.06-7.21 (m, 5H), 7.30 (t, 1H), 7.37 (d, 1H), 7.52 (d, 2H), 7.58 (d, 2H), 9.88 (s, 1H), 11.88 (s, 1H).

*5-(4-tert-Butylbenzolsulfonylamino)-3-(3-methylphenyl)-1H-indol-2-carbonsäure:*

Gemäß Vorschrift 5 werden nach Umsetzung von 5-(4-tert-Butylbenzolsulfonylamino)-3-(3-methylphenyl)-1H-indol-2-carbonsäureethylester (330 mg, 0.67 mmol) mit 12 mL einer 1M NaOH Lösung in Ethanol/Wasser (1/1) 300 mg (97%) des Produkts erhalten.
NMR (300 MHz, DMSO-d6): δ 1.24 (s, 9H), 2.35 (s, 3H), 7.05-7.15 (m, 5H), 7.29 (t, 1H), 7.34 (d, 1H), 7.51 (d, 2H), 7.57 (d, 2H), 9.85 (s, 1H), 11.77 (s, 1H).

*5-(4-tert-Butylbenzolsulfonylamino)-3-(3-methylphenyl)-1H-indol-2-carbonsäure-(2-acetylaminoethyl)amid:* Gemäß Vorschrift 1 werden nach Umsetzung von 5-(4-tert-Butylbenzolsulfonylamino)-3-(3-methylphenyl)-1H-indol-2-carbonsäure (210 mg, 0.45 mmol) mit N-Acetylethylendiamin (0.043 mL, 0.45 mmol) und chromatographischer Reinigung (Kieselgel, Dichlormethan/Methanol (0-20% Methanol)) 120 mg (49%) des Produkts erhalten.
NMR (300 MHz, DMSO-d6): δ 1.24 (s, 9H), 1.75 (s, 3H), 2.36 (s, 3H), 3.06-3.10 (m, 2H), 3.19-3.23 (m, 2H), 7.02-7.09 (m, 2H), 7.13-7.23 (m, 3H), 7.33-7.35 (m, 2H), 7.41 (t, 1H), 7.52 (d, 2H), 7.57 (d, 2H), 7.81 (t, 1H), 9.82 (s, 1H), 11.65 (s, 1H).

**Beispiel 57:** 5-(4-tert-Butylbenzolsulfonylamino)-3-(3-methylphenyl)-1*H*-indol-2-carbonsäure-(2-hydroxyethyl)amid

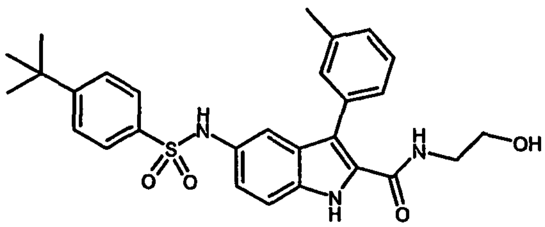

Gemäß Vorschrift 1 werden nach Umsetzung von 5-(4-tert-Butylbenzolsulfonylamino)-3-(3-methylphenyl)-1*H*-indol-2-carbonsäure (210 mg, 0.45 mmol) mit Ethanolamin (0.027 mL, 0.45 mmol) und chromatographischer Reinigung (Kieselgel, Dichlormethan/Methanol (0-20% Methanol)) 22 mg (10%) des Produkts erhalten.
NMR (300 MHz, DMSO-d6): δ 1.24 (s, 9H), 2.36 (s, 3H), 3.16-3.26 (m, 2H), 3.35-3.39 (m, 2H), 4.62 (t, 1H), 7.02-7.09 (m, 3H), 7.17-7.25 (m, 3H), 7.31-7.36 (m, 2H), 7.52 (d, 2H), 7.58 (d, 2H), 9.81 (s, 1H), 11.68 (s, 1H).

**Beispiel 58:** 5-(4-tert-Butylbenzolsulfonylamino)-3-(3-methylphenyl)-1*H*-indol-2-carbonsäure-(2-morpholin-4-ylethyl) amid

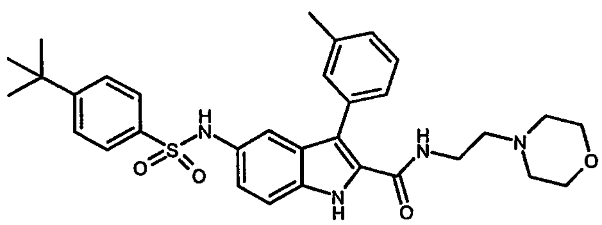

Gemäß Vorschrift 1 werden nach Umsetzung von 5-(4-tert-Butylbenzolsulfonylamino)-3-(3-methylphenyl)-1*H*-indol-2-carbonsäure (150 mg, 0.32 mmol) mit 4-(2-Aminoethyl)morpholin (0.042 mL, 0.32 mmol) und chromatographischer Reinigung (Kieselgel, Dichlormethan/Methanol (0-20% Methanol)) 90 mg (49%) des Produkts erhalten.
NMR (300 MHz, DMSO-d6): δ 1.24 (s, 9H), 2.13-2.19 (m, 4H), 2.27 (t, 2H), 2.37 (s, 3H), 3.27-3.39 (m, 2H), 3.39 (br, 4H), 6.92 (t, 1H), 7.02-7.10 (m, 3H), 7.15(s, 1H), 7.22 (d, 1H), 7.34-7.40 (m, 2H), 7.52 (d, 2H), 7.57 (d, 2H), 9.80 (s, 1H), 11.70 (s, 1H).

**Beispiel 59:** 5-(4-tert-Butylbenzolsulfonylamino)-3-(3-methylphenyl)-1*H*-indol-2-carbonsäure-(tetrahydropyran-4-yl) amid

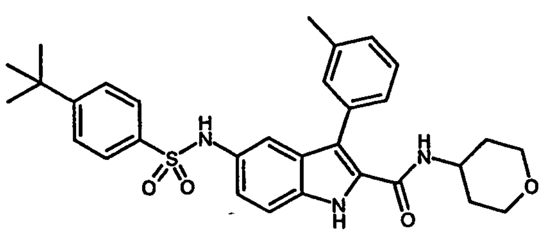

Gemäß Vorschrift 1 werden nach Umsetzung von 5-(4-tert-Butylbenzolsulfonylamino)-3-(3-methylphenyl)-1H-indol-2-carbonsäure (150 mg, 0.32 mmol) mit 4-Aminotetrahydropyran (33 mg, 0.32 mmol) und Reinigung mittels HPLC 50 mg (29%) des Produkts erhalten.
NMR (300 MHz, DMSO-d6): δ 1.20-1.35 (m,11H), 1.63-1.71 (m, 2H), 2.36 (s, 3H), 3.33-3.38 (m, 2H+$H_2O$), 3.68-3.72 (m, 2H), 3.87-3.99 (m, 1H), 7.02 (dd, 1H), 7.08-7.11 (m, 2H), 7.17-7.20 (m, 3H), 7.30-7.37 (m, 2H), 7.51 (d, 2H), 7.57 (d, 2H), 9.83 (s, 1H), 11.70 (s, 1H).

**Beispiel 60:** 5-(4-tert-Butylbenzolsulfonylamino)-3-pyridin-3-yl-1*H*-indol-2-carbon-säure-(2-morpholin-4-ylethyl)amid

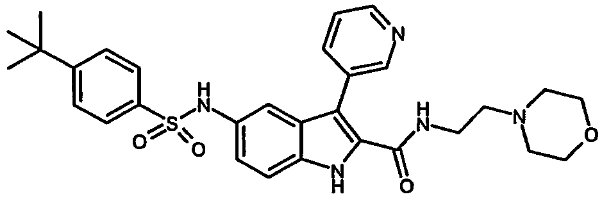

Gemäß Vorschrift 1 werden nach Umsetzung von 5-(4-tert-Butylbenzolsulfonylamino)-3-pyridin-3-yl-1*H*-indol-2-carbonsäure (140 mg, 0.31 mmol) mit 4-(2-Aminoethyl)morpholin (0.04 mL, 0.31 mmol) und chromatographischer Reinigung (Kieselgel, Dichlormethan/Methanol (0-50% Methanol)) 30 mg (17%) des Produkts erhalten.
NMR (300 MHz, DMSO-d6): δ 1.24 (s, 9H), 2.31-2.40 (m, 6H), 3.48 (br, 4H), 3.59-3.60 (m, 2H), 7.05-7.11 (m, 2H), 7.37 (d, 1H), 7.46-7.59 (m, 6H), 7.70 (d, 1H), 8.50 (d, 1H), 8.55 (dd, 1H), 9.83 (s, 1H), 11.86 (s, 1H).

**Beispiel 61:** 5-(4-tert-Butylbenzolsulfonylamino)-3-(3-methoxyphenyl)-1*H*-indol-2-carbonsäure-(tetrahydropyran-4-yl) amid

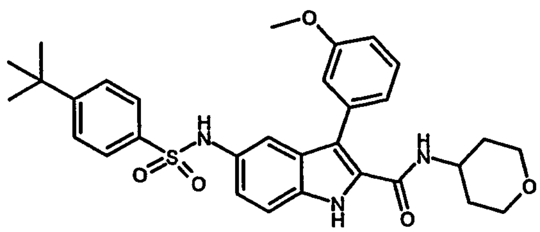

Gemäß Vorschrift 1 werden nach Umsetzung von 5-(4-tert-Butylbenzolsulfonylamino)-3-(3-methoxyphenyl)-1*H*-indol-2-carbonsäure (125 mg, 0.26 mmol) mit 4-Aminotetrahydropyran (29 mg, 0.29 mmol) und chromatographischer Reinigung (Kieselgel, Dichlormethan/Methanol (0-20% Methanol)) 110 mg (75%) des Produkts erhalten.
NMR (300 MHz, DMSO-d6): δ 1.24-1.36 (m, 11H), 1.67-1.70 (m, 2H), 3.31-3.38 (m, 2H+$H_2O$), 3.69-3.79 (m, 2H), 3.79 (s, 3H), 3.86-3.98 (m, 1H), 6.86-6.96 (m, 3H), 7.04 (dd, 1H), 7.16 (d, 1H), 7.31-7.38 (m, 3H), 7.51 (d, 2H), 7.57 (d, 2H), 9.83 (s, 1H), 11.73 (s, 1H).

**Beispiel 62:** 5-(4-tert-Butylbenzolsulfonylamino)-3-(3-methoxyphenyl)-1*H*-indol-2-carbonsäure-(2-morpholin-4-ylethyl) amid

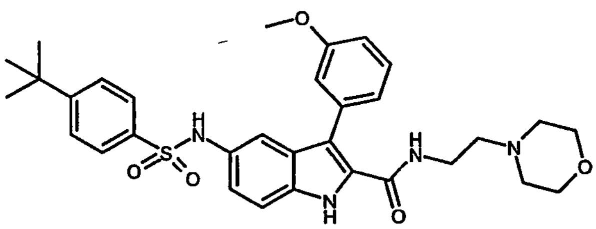

Gemäß Vorschrift 1 werden nach Umsetzung von *5-(4*-tert-Butylbenzolsulfonylamino)-3-(3-methoxyphenyl)-1*H*-indol-2-carbonsäure (125 mg, 0.26 mmol) mit 4-(2-Aminoethyl)morpholin (0.034 mL, 0.26 mmol) und chromatographischer Reinigung (Kieselgel, Dichlormethan/Methanol (0-20% Methanol)) 60 mg (39%) des Produkts erhalten. NMR (300 MHz, DMSO-d6): δ 1.24 (s, 9H), 2.21 (br, 4H), 2.28 (t, 2H), 3.26-3.30 (m, 2H+$H_2O$), 3.40 (br, 4H), 3.79 (s, 3H), 6.86-6.90 (m, 2H), 6.97-7.08 (m, 4H), 7.31-7.42 (m, 2H), 7.51 (d, 2H), 7.56 (d, 2H), 9.81 (d, 1H), 11.72 (s, 1H).

Folgende Verbindungen wurden in analoger Verfahrensweise zu den beschriebenen Beispielen hergestellt:

| Beispiel | Struktur | Name |
|---|---|---|
| 63 | 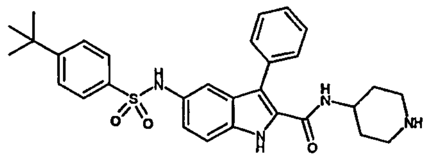 | 5-(4-tert-Butylbenzolsulfonylamino)-3-phenyl-1H-indol-2-carbonsäurepiperidin-4-ylamid |
| 64 | 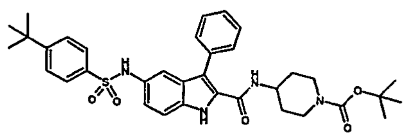 | 4-{[5-(4-tert-Butylbenzolsulfonylamino)-3-phenyl-1H-indol-2carbonyl]-amino}piperidin-1-carbonsäure-tert-butylester |
| 65 | 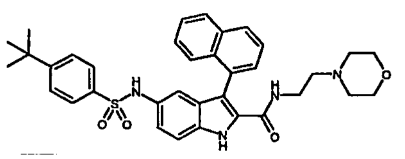 | 5-(4-tert-Butyl-phenylsulfonyl-amino)-3-naphthalin-1-yl-1H-indol-2-carbonsäure-(2-morpholin-4-yl-ethyl)-amid |
| 66 | 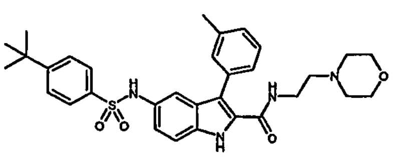 | 5-(4-tert-Butyl-phenylsulfonyl-amino)-3-m-tolyl-1H-indol-2-carbonsäure-(2-morpholin-4-yl-ethyl)-amid |
| 67 | 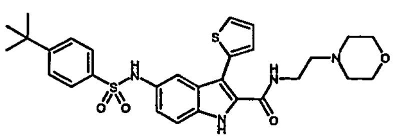 | 5-(4-tert-Butyl-phenylsulfonyl-amino)-3-thiophen-2-yl-1H-indol-2-carbonsäure-(2-morpholin-4-yl-ethyl)-amid |
| 68 | 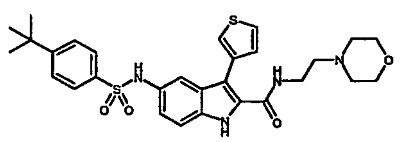 | 5-(4-tert-Butyl-phenylsulfonyl-amino)-3-thiophen-3-yl-1H-indol-2-carbonsäure-(2-morpholin-4-yl-ethyl)-amid |

(fortgesetzt)

| Beispiel | Struktur | Name |
|---|---|---|
| 69 | 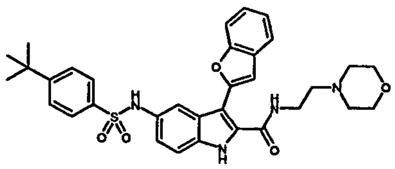 | 3-Benzofuran-2-yl-5-(4-tert-butyl-phenylsulfonylamino)-1H-indol-2-carbonsäure-(2-morpholin-4-yl-ethyl)-amid |
| 70 | 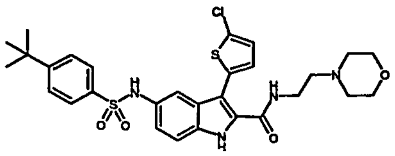 | 5-(4-tert-Butyl-phenylsulfonyl-amino)-3-(5-chlorthiophen-2-yl)-1H-indol-2-carbonsäure-(2-morpholin-4-yl-ethyl)-amid |
| 71 | 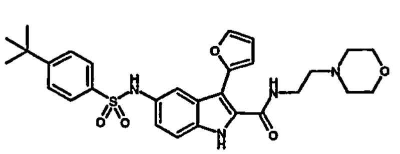 | 5-(4-tert-Butyl-phenylsulfonyl-amino)-3-furan-2-yl-1H-indol-2-carbonsäure-(2-morpholin-4-yl-ethyl)-amid |
| 72 | 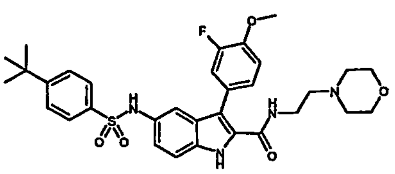 | 5-(4-tert-Butyl-phenylsulfonyl-amino)-3-(3-fluor-4-methoxy-phenyl)-1H-indol-2-carbonsäure-(2-morpholin-4-yl-ethyl)-amid |
| 73 | 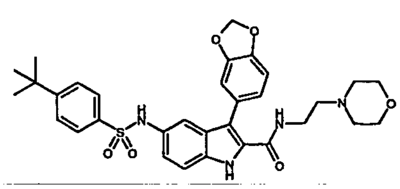 | 3-Benzo[1,3]dioxol-5-yl-5-(4-tert-butylphenylsulfonylamino)-1H-indol-2-carbonsäure-(2-morpholin-4-yl-ethyl)-amid |
| 74 | 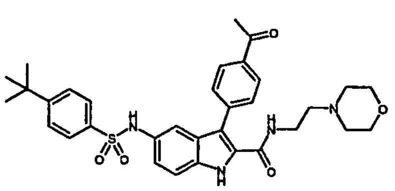 | 3-(4-Acetyl-phenyl)-5-(4-tert-butylphenylsulfonylamino)-1H-indol-2-carbonsäure-(2-morpholin-4-yl-ethyl)-amid |
| 75 | 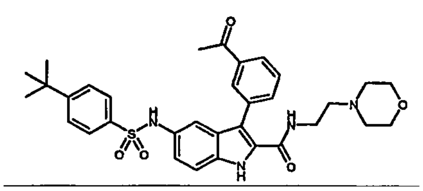 | 3-(3-Acetyl-phenyl)-5-(4-tert-butylphenylsulfonylamino)-1H-indol-2-carbonsäure-(2-morpholin-4-yl-ethyl)-amid |
| 76 | 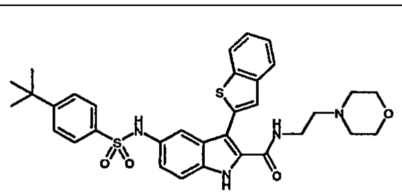 | 3-Benzo[b]thiophen-2-yl-5-(4-tert-butyl-phenylsulfonylamino)-1H-indol-2-carbonsäure-(2-morpholin-4-yl-ethyl)-amid |
| 77 | 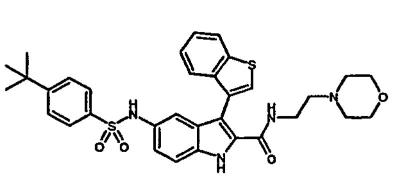 | 3-Benzo[b]thiophen-3-yl-5-(4-tert-butyl-phenylsulfonylamino)-1H-indol-2-carbonsäure-(2-morpholin-4-yl-ethyl)-amid |

(fortgesetzt)

| Beispiel | Struktur | Name |
|---|---|---|
| 78 | 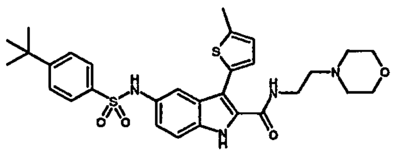 | 5-(4-tert-Butyl-phenylsulfonyl-amino)-3-(5-methyl-thiophen-2-yl)-1H-indol-2-carbonsäure-(2-morpholin-4-yl-ethyl)-amid |
| 79 | 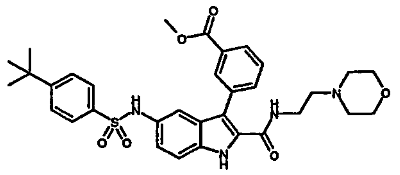 | 3-[5-(4-tert-Butyl-phenylsulfonyl-amino)-2-(2-morpholin-4-yl-ethylcarbamoyl)-1H-indol-3-yl]-benzoesäure methylester |
| 80 | 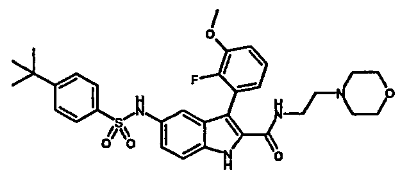 | 5-(4-tert-Butyl-phenylsulfonyl-amino)-3-(2-fluor-3-methoxy-phenyl)-1H-indol-2-carbonsäure-(2-morpholin-4-yl-ethyl)-amid |
| 81 | 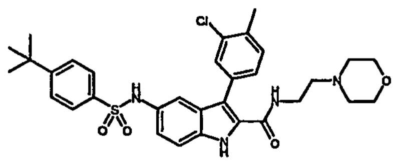 | 5-(4-tert-Butyl-phenylsulfonyl-amino)-3-(3-chlor-4-methyl-phenyl)-1H-indol-2-carbonsäure-(2-morpholin-4-yl-ethyl)-amid |
| 82 | 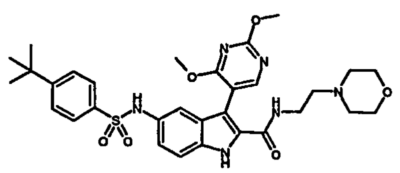 | 5-(4-tert-Butyl-phenylsulfonyl-amino)-3-(2,4-dimethoxy-pyrimidin-5-yl)-1H-indol-2-carbonsäure-(2-morpholin-4-yl-ethyl)-amid |
| 83 | 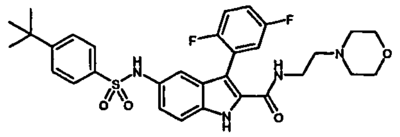 | 5-(4-tert-Butyl-phenylsulfonyl-amino)-3-(2,5-difluor-phenyl)-1H-indol-2-carbonsäure-(2-morpholin-4-yl-ethyl)-amid |
| 84 | 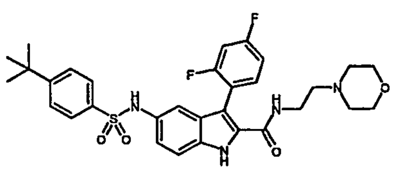 | 5-(4-tert-Butyl-phenylsulfonyl-amino)-3-(2,4-difluor-phenyl)-1H-indol-2-carbonsäure-(2-morpholin-4-yl-ethyl)-amid |
| 85 | 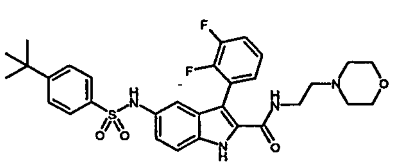 | 5-(4-tert-Butyl-phenylsulfonyl-amino)-3-(2,3-difluor-phenyl)-1H-indol-2-carbonsäure-(2-morpholin-4-yl-ethyl)-amid |
| 86 | 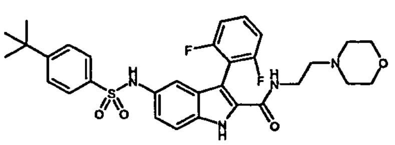 | 5-(4-tert-Butyl-phenylsulfonyl-amino)-3-(2,6-difluor-phenyl)-1H-indol-2-carbonsäure-(2-morpholin-4-yl-ethyl)-amid |

(fortgesetzt)

| Beispiel | Struktur | Name |
|---|---|---|
| 87 | 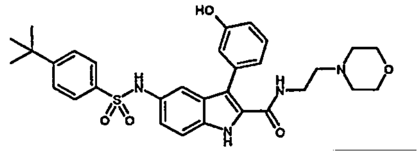 | 5-(4-tert-Butyl-phenylsulfonyl-amino)-3-(3-hydroxy-phenyl)-1H-indol-2-carbonsäure-(2-morpholin-4-yl-ethyl)-amid |
| 88 | 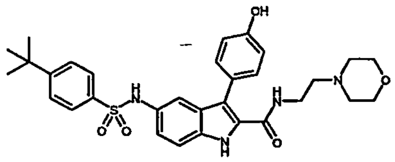 | 5-(4-tert-Butyl-phenylsulfonyl-amino)-3-(4-hydroxy-phenyl)-1H-indol-2-carbonsäure-(2-morpholin-4-yl-ethyl)-amid |
| 89 | 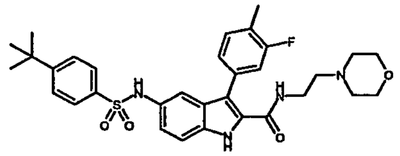 | 5-(4-tert-Butyl-phenylsulfonyl-amino)-3-(3-fluor-4-methyl-phenyl)-1H-indol-2-carbonsäure-(2-morpholin-4-yl-ethyl)-amid |
| 90 | 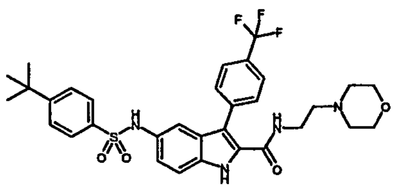 | 5-(4-tert-Butyl-phenylsulfonyl-amino)-3-(4-trifluormethyl-phenyl)-1H-indol-2-carbonsäure-(2-morpholin-4-yl-ethyl)-amid |
| 91 | 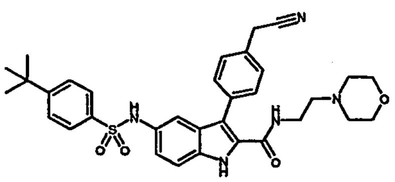 | 5-(4-tert-Butyl-phenylsulfonyl-amino)3-(4-cyanomethyl-phenyl)-1H-indol-2-carbonsäure-(2-morpholin-4-yl-ethyl)-amid |
| 92 | 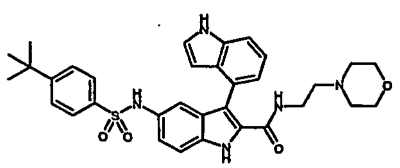 | 5-(4-tert-Butyl-phenylsulfonyl-amino)-1H,1'H-[3,4'] biindolyl-2-carbonsäure-(2-morpholin-4-yl-ethyl)-amid |
| 93 | 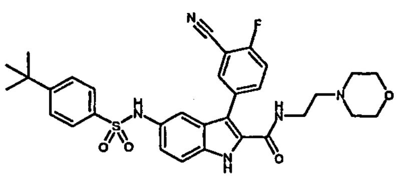 | 5-(4-tert-Butyl-phenylsulfonyl-amino)-3-(3-cyano-4-fluor-phenyl)-1H-indol-2-carbonsäure-(2-morpholin-4-yl-ethyl)-amid |
| 94 | 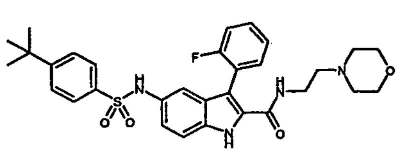 | 5-(4-tert-Butyl-phenylsulfonyl-amino)-3-(2-fluor-phenyl)-1H-indol-2-carbonsäure-(2-morpholin-4-yl-ethyl)-amid |
| 95 | 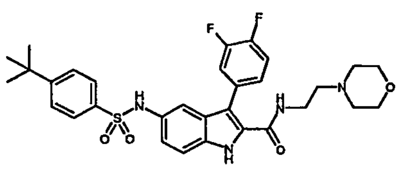 | 5-(4-tert-Butyl-phenylsulfonyl-amino)-3-(3,4-difluor-phenyl)-1H-indol-2-carbonsäure-(2-morpholin-4-yl-ethyl)-amid |

(fortgesetzt)

| Beispiel | Struktur | Name |
|---|---|---|
| 96 | 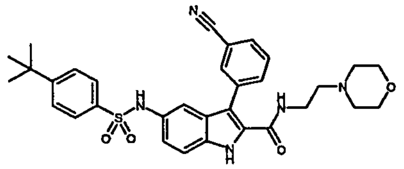 | 5-(4-tert-Butyl-phenylsulfonyl-amino)-3-(3-cyano-phenyl)-1H-indol-2-carbonsäure-(2-morpholin-4-yl-ethyl)-amid |
| 97 | 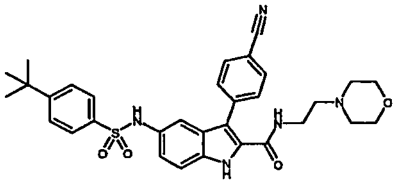 | 5-(4-tert-Butyl-phenylsulfonyl-amino)-3-(4-cyano-phenyl)-1H-indol-2-carbonsäure-(2-morpholin-4-yl-ethyl)-amid |
| 98 | 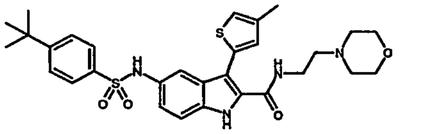 | 5-(4-tert-Butyl-phenylsulfonyl-amino)-3-(4-methyl-thiophen-2-yl)-1H-indol-2-carbonsäure-(2-morpholin-4-yl-ethyl)-amid |
| 99 | 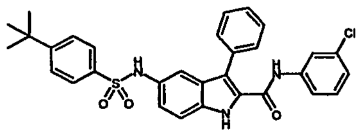 | 5-(4-tert-Butyl-phenylsulfonyl-amino)-3-phenyl-1H-indol-2-carbonsäure-(3-chloro-phenyl)-amid |
| 100 | 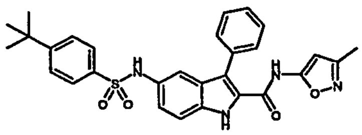 | 5-(4-tert-Butyl-phenylsulfonyl-amino)-3-phenyl-1H-indol-2-carbonsäure-(3-methyl-isoxazol-5-yl)-amid |
| 101 | 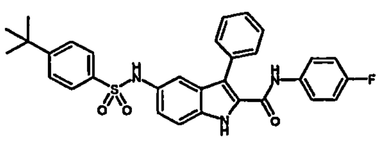 | 5-(4-tert-Butyl-phenylsulfonyl-amino)-3-phenyl-1H-indol-2-carbonsäure-(4-fluoro-phenyl)-amid |
| 102 | 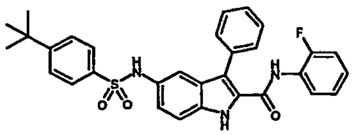 | 5-(4-tert-Butyl-phenylsulfonyl-amino)-3-phenyl-1H-indol-2-carbonsäure-(2-fluoro-phenyl)-amid |
| 103 | 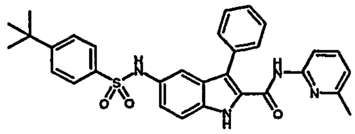 | 5-(4-tert-Butyl-phenylsulfonyl-amino)-3-phenyl-1H-indol-2-carbonsäure-(6-methyl-pyridin-2-yl)-amid |
| 104 | 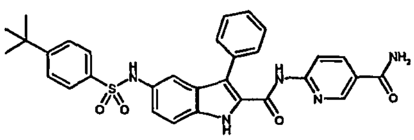 | 5-(4-tert-Butyl-phenylsulfonyl-amino)-3-phenyl-1H-indol-2-carbonsäure-(5-carbamoyl-pyridin-2-yl)-amid |
| 105 | 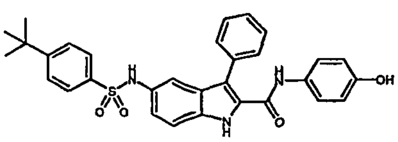 | 5-(4-tert-Butyl-phenylsulfonyl-amino)-3-phenyl-1H-indol-2-carbonsäure-(4-hydroxy-phenyl)-amid |

(fortgesetzt)

| Beispiel | Struktur | Name |
|---|---|---|
| 106 | 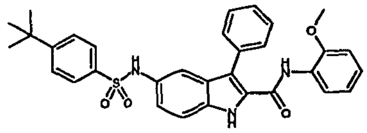 | 5-(4-tert-Butyl-phenylsulfonyl-amino)-3-phenyl-1H-indol-2-carbonsäure-(2-methoxy-phenyl)-amid |
| 107 | 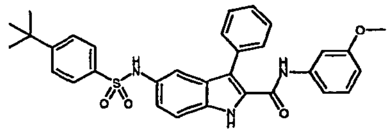 | 5-(4-tert-Butyl-phenylsulfonyl-amino)-3-phenyl-1H-indol-2-carbonsäure-(3-methoxy-phenyl)-amid |
| 108 | 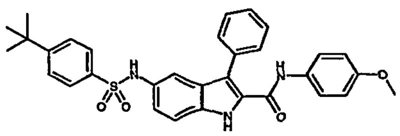 | 5-(4-tert-Butyl-phenylsulfonyl-amino)-3-phenyl-1H-indol-2-carbonsäure-(4-methoxy-phenyl)-amid |
| 109 | 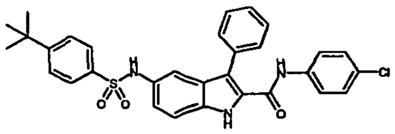 | 5-(4-tert-Butyl-phenylsulfonyl-amino)-3-phenyl-1H-indol-2-carbonsäure-(4-chloro-phenyl)-amid |
| 110 | 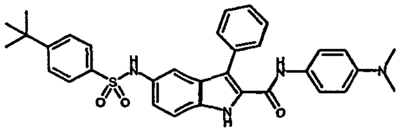 | 5-(4-tert-Butyl-phenylsulfonyl-amino)-3-phenyl-1H-indol-2-carbonsäure-(4-dimethylamino-phenyl)-amid |
| 111 | 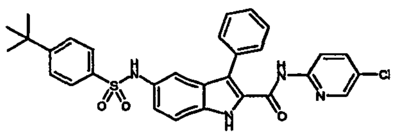 | 5-(4-tert-Butyl-phenylsulfonyl-amino)-3-phenyl-1H-indol-2-carbonsäure-(5-chlor-pyridin-2-yl)-amid |
| 112 | 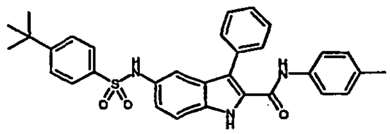 | 5-(4-tert-Butyl-phenylsulfonyl-amino)-3-phenyl-1H-indol-2-carbonsäure-p-tolylamid |
| 113 | 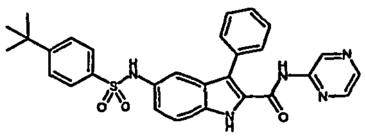 | 5-(4-tert-Butyl-phenylsulfonyl-amino)-3-phenyl-1H-indol-2-carbonsäure-pyrazin-2-ylamid |
| 114 | 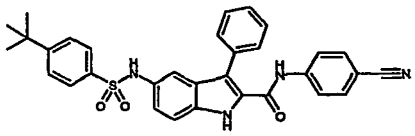 | 5-(4-tert-Butyl-phenylsulfonyl-amino)-3-phenyl-1H-indol-2-carbonsäure-(4-cyano-phenyl)-amid |
| 115 | 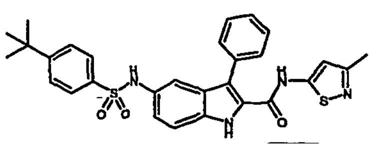 | 5-(4-tert-Butyl-phenylsulfonyl-amino)-3-phenyl-1H-indol-2-carbonsäure-(3-methyl-isothiazol-5-yl)-amid |

(fortgesetzt)

| Beispiel | Struktur | Name |
|---|---|---|
| 116 | 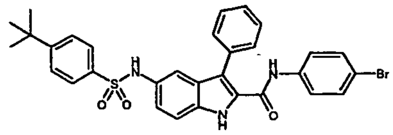 | 5-(4-tert-Butyl-phenylsulfonyl-amino)-3-phenyl-1H-indol-2-carbonsäure-(4-brom-phenyl)-amid |
| 117 | 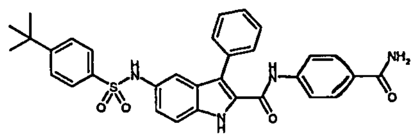 | 5-(4-tert-Butyl-phenylsulfonyl-amino)-3-phenyl-1H-indol-2-carbonsäure-(4-carbamoyl-phenyl)-amid |
| 118 | 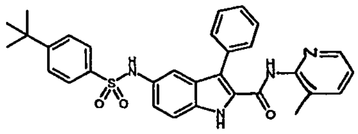 | 5-(4-tert-Butyl-phenylsulfonyl-amino)-3-phenyl-1H-indol-2-carbonsäure-(3-methyl-pyridin-2-yl)-amid |
| 119 | 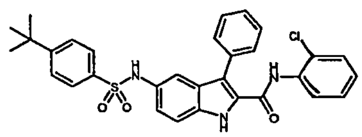 | 5-(4-tert-Butyl-phenylsulfonyl-amino)-3-phenyl-1H-indol-2-carbonsäure-(2-chlor-phenyl)-amid |
| 120 | 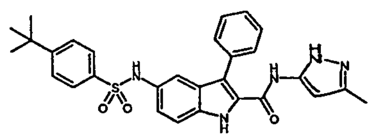 | 5-(4-tert-Butyl-phenylsulfonyl-amino)-3-phenyl-1H-indol-2-carbonsäure-(5-methyl-2H-pyrazol-3-yl)-amid |
| 121 | 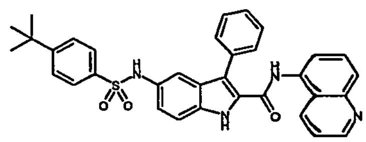 | 5-(4-tert-Butyl-phenylsulfonyl-amino)-3-phenyl-1H-indol-2-carbonsäure-quinolin-5-ylamid |
| 122 | 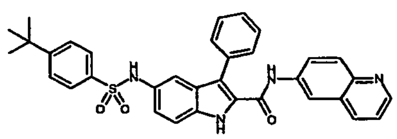 | 5-(4-tert-Butyl-phenylsulfonyl-amino)-3-phenyl-1H-indol-2-carbonsäure-quinolin-6-ylamid |
| 123 | 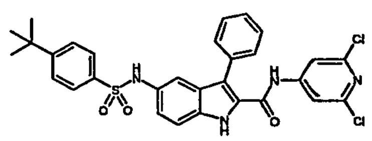 | 5-(4-tert-Butyl-phenylsulfonyl-amino)-3-phenyl-1H-indol-2-carbonsäure-(2,6-dichlor-pyridin-4-yl)-amid |
| 124 | 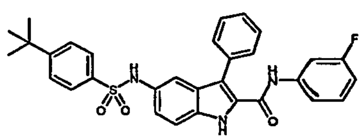 | 5-(4-tert-Butyl-phenylsulfonyl-amino)-3-phenyl-1H-indol-2-carbonsäure-(3-fluor-phenyl)-amid |
| 125 | 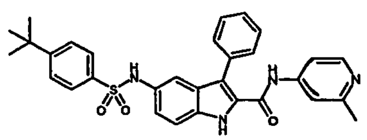 | 5-(4-tert-Butyl-phenylsulfonyl-amino)-3-phenyl-1H-indol-2-carbonsäure-(2-methyl-pyridin-4-yl)-amid |

(fortgesetzt)

| Beispiel | Struktur | Name |
|---|---|---|
| 126 | 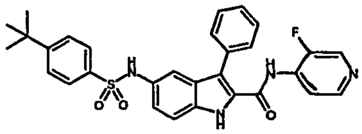 | 5-(4-tert-Butyl-phenylsulfonyl-amino)-3-phenyl-1H-indol-2-carbonsäure-(3-fluor-pyridin-4-yl)-amid |
| 127 | 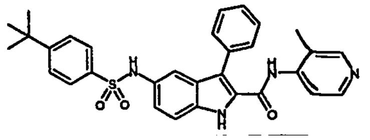 | 5-(4-tert-Butyl-phenylsulfonyl-amino)-3-phenyl-1H-indol-2-carbonsäure-(3-methyl-pyridin-4-yl)-amid |
| 128 | 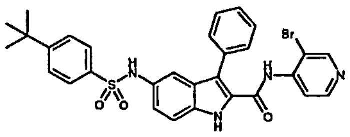 | 5-(4-tert-Butyl-phenylsulfonyl-amino)-3-phenyl-1H-indol-2-carbonsäure-(3-brom-pyridin-4-yl)-amid |
| 129 | 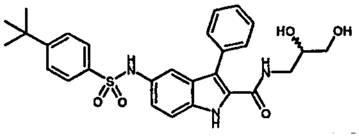 | 5-(4-tert-Butyl-phenylsulfonyl-amino)-3-phenyl-1H-indol-2-carbonsäure-(2,3-dihydroxy-propyl)-amid |
| 130 | 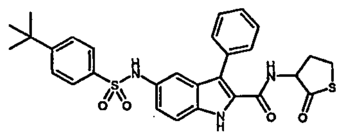 | 5-(4-tert-Butyl-phenylsulfonyl-amino)-3-phenyl-1H-indol-2-carbonsäure-(2-oxo-tetrahydro-thiophen-3-yl)-amid |
| 131 | 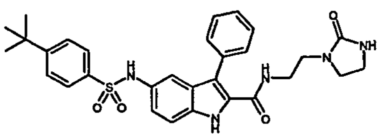 | 5-(4-tert-Butyl-phenylsulfonyl-amino)-3-phenyl-1H-indol-2-carbonsäure-[2-(2-oxo-imidazolidin-1-yl)-ethyl]-amid |
| 132 | 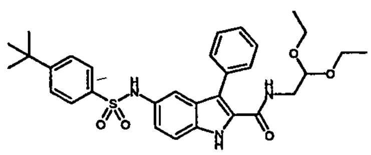 | 5-(4-tert-Butyl-phenylsulfonyl-amino)-3-phenyl-1H-indol-2-carbonsäure-(2,2-diethoxy-ethyl)-amid |
| 133 | 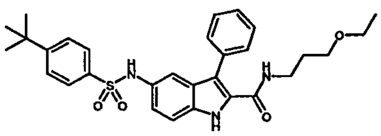 | 5-(4-tert-Butyl-phenylsulfonyl-amino)-3-phenyl-1H-indol-2-carbonsäure-(3-ethoxy-propyl)-amid |
| 134 | 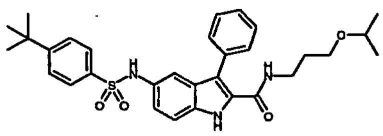 | 5-(4-tert-Butyl-phenylsulfonyl-amino)-3-phenyl-1H-indol-2-carbonsäure-(3-isopropoxy-propyl)-amid |
| 135 | 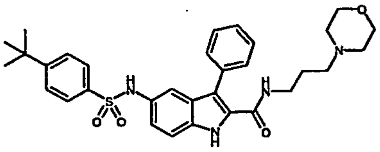 | 5-(4-tert-Butyl-phenylsulfonyl-amino)-3-phenyl-1H-indol-2-carbonsäure-(3-morpholin-4-yl-propyl)-amid |

(fortgesetzt)

| Beispiel | Struktur | Name |
|---|---|---|
| 136 | 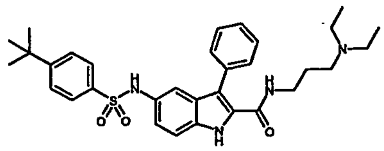 | 5-(4-tert-Butyl-phenylsulfonyl-amino)-3-phenyl-1H-indol-2-carbonsäure-(3-diethylamino-propyl)-amid |
| 137 | 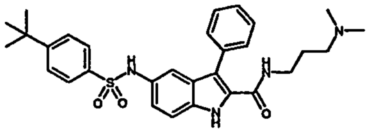 | 5-(4-tert-Butyl-phenylsulfonyl-amino)-3-phenyl-1H-indol-2-carbonsäure-(3-dimethylamino-propyl)-amid |
| 138 | 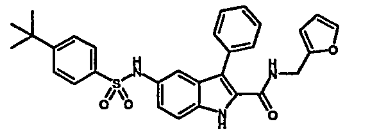 | 5-(4-tert-Butyl-phenylsulfonyl-amino)-3-phenyl-1H-indol-2-carbonsäure-(furan-2-ylmethyl)-amid |
| 139 | 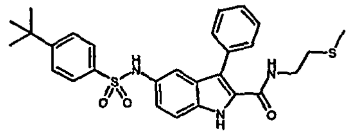 | 5-(4-tert-Butyl-phenylsulfonyl-amino)-3-phenyl-1H-indol-2-carbonsäure-(2-methyls ulfanyl-ethyl)-amid |
| 140 | 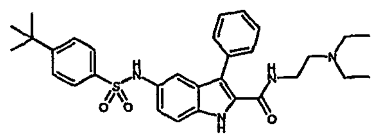 | 5-(4-tert-Butyl-phenylsulfonyl-amino)-3-phenyl-1H-indol-2-carbonsäure-(2-diethylamino-ethyl)-amid |
| 141 | 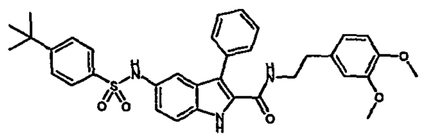 | 5-(4-tert-Butyl-phenylsulfonyl-amino)-3-phenyl-1H-indol-2-carbonsäure-[2-(3,4-dim ethoxy-phenyl)-ethyl]-amid |
| 142 | 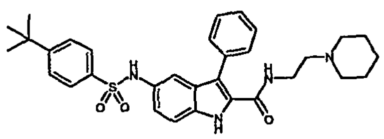 | 5-(4-tert-Butyl-phenylsulfonyl-amino)-3-phenyl-1H-indol-2-carbonsäure-(2-piperidin-1-yl-ethyl)-amid |
| 143 | 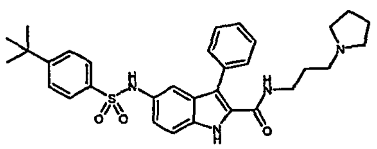 | 5-(4-tert-Butyl-phenylsulfonyl-amino)-3-phenyl-1H-indol-2-carbonsäure-(3-pyrrolidin-1-yl-propyl)-amid |
| 144 | 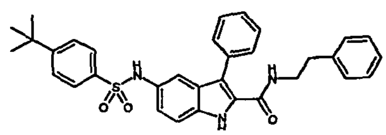 | 5-(4-tert-Butyl-phenylsulfonyl-amino)-3-phenyl-1H-indol-2-carbonsäure-phenethyl-amid |
| 145 | 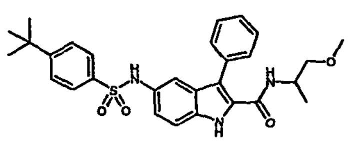 | 5-(4-tert-Butyl-phenylsulfonyl-amino)-3-phenyl-1H-indol-2-carbonsäure-(2-methoxy-1-methyl-ethyl)-amid |

(fortgesetzt)

| Beispiel | Struktur | Name |
|---|---|---|
| 146 | 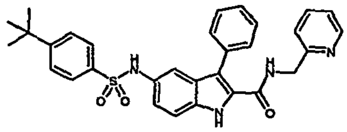 | 5-(4-tert-Butyl-phenylsulfonyl-amino)-3-phenyl-1H-indol-2-carbonsäure-(pyridin-2-ylmethyl)-amid |
| 147 | 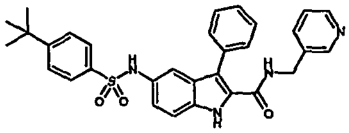 | 5-(4-tert-Butyl-phenylsulfonyl-amino)-3-phenyl-1H-indol-2-carbonsäure-(pyridin-3-ylmethyl)-amid |
| 148 | 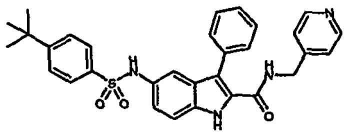 | 5-(4-tert-Butyl-phenylsulfonyl-amino)-3-phenyl-1H-indol-2-carbonsäure-(pyridin-4-ylmethyl)-amid |
| 149 | 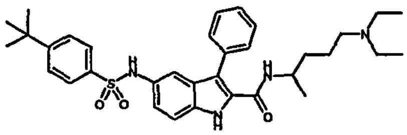 | 5-(4-tert-Butyl-phenylsulfonyl-amino)-3-phenyl-1H-indol-2-carbonsäure-(4-diethyla mino-1-methyl-butyl)-amid |
| 150 | 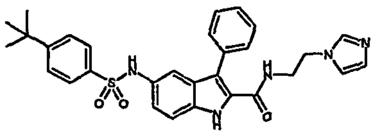 | 5-(4-tert-Butyl-phenylsulfonyl-amino)-3-phenyl-1H-indol-2-carbonsäure-(2-imidazol-1-yl-ethyl)-amid |
| 151 | 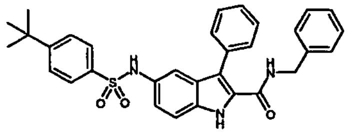 | 5-(4-tert-Butyl-phenylsulfonyl-amino)-3-phenyl-1H-indol-2-carbonsäure-benzylamid |
| 152 | 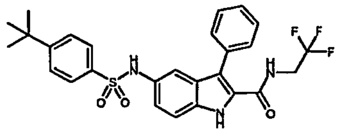 | 5-(4-tert-Butyl-phenylsulfonyl-amino)-3-phenyl-1H-indol-2-carbonsäure-(2,2,2-trifluor-ethyl)-amid |
| 153 | 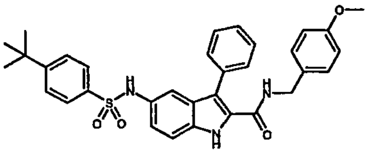 | 5-(4-tert-Butyl-phenylsulfonyl-amino)-3-phenyl-1H-indol-2-carbonsäure-4-methoxy-benzylamid |
| 154 | 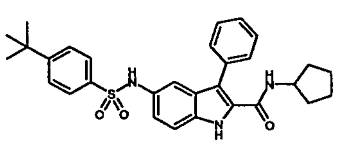 | 5-(4-tert-Butyl-phenylsulfonyl-amino)-3-phenyl-1H-indol-2-carbonsäure-cyclopentylamid |
| 155 | 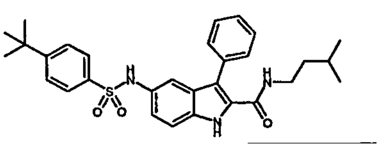 | 5-(4-tert-Butyl-phenylsulfonyl-amino)-3-phenyl-1H-indol-2-carbonsäure-(3-methyl-butyl)-amid |

(fortgesetzt)

| Beispiel | Struktur | Name |
|---|---|---|
| 156 | 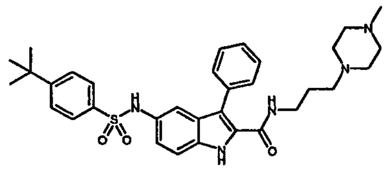 | 5-(4-tert-Butyl-phenylsulfonyl-amino)-3-phenyl-1H-indol-2-carbonsäure-[3-(4-methyl-piperazin-1-yl)-propyl]-amid |
| 157 | 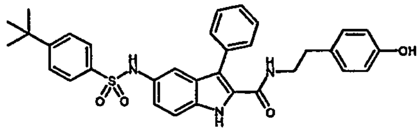 | 5-(4-tert-Butyl-phenylsulfonyl-amino)-3-phenyl-1H-indol-2-carbonsäure-[2-(4-hydroxy-phenyl)-ethyl]-amid |
| 158 | 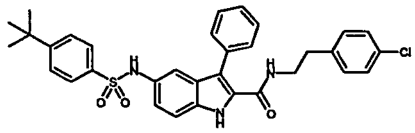 | 5-(4-tert-Butyl-phenylsulfonyl-amino)-3-phenyl-1H-indol-2-carbonsäure-[2-(4-chlor-phenyl)-ethyl]-amid |
| 159 | 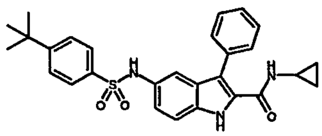 | 5-(4-tert-Butyl-phenylsulfonyl-amino)-3-phenyl-1H-indol-2-carbonsäure-cyclopropylamid |
| 160 | 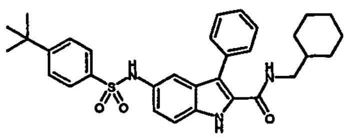 | 5-(4-tert-Butyl-phenylsulfonyl-amino)-3-phenyl-1H-indol-2-carbonsäure-cyclohexylmethyl-amid |
| 161 | 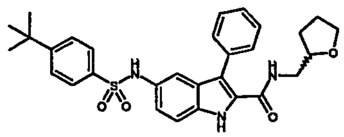 | 5-(4-tert-Butyl-phenylsulfonyl-amino)-3-phenyl-1H-indol-2-carbonsäure-(tetrahydro-furan-2-ylmethyl)-amid |
| 162 | 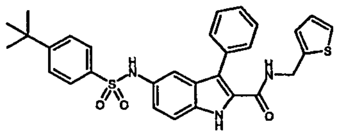 | 5-(4-tert-Butyl-phenylsulfonyl-amino)-3-phenyl-1H-indol-2-carbonsäure-(thiophen-2-ylmethyl)-amid |
| 163 | 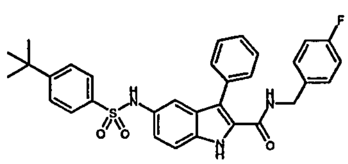 | 5-(4-tert-Butyl-phenylsulfonyl-amino)-3-phenyl-1H-indol-2-carbonsäure-4-fluoro-benzylamid |
| 164 | 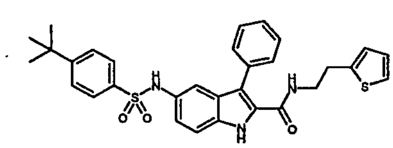 | 5-(4-tert-Butyl-phenylsulfonyl-amino)-3-phenyl-1H-indol-2-carbonsäure-(2-thiophen-2-yl-ethyl)-amid |
| 165 | 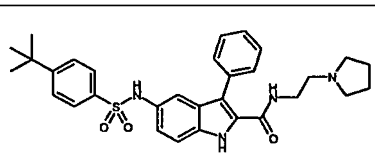 | 5-(4-tert-Butyl-phenylsulfonyl-amino)-3-phenyl-1H-indol-2-carbonsäure-(2-pyrrolidin-1-yl-ethyl)-amid |

(fortgesetzt)

| Beispiel | Struktur | Name |
|---|---|---|
| 166 | 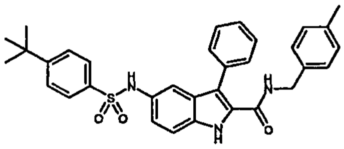 | 5-(4-tert-Butyl-phenylsulfonyl-amino)-3-phenyl-1H-indol-2-carbonsäure-4-methyl-benzylamid |
| 167 | 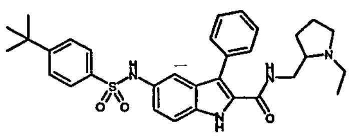 | 5-(4-tert-Butyl-phenylsulfonyl-amino)-3-phenyl-1H-indol-2-carbonsäure-(1-ethyl-pyrrolidin-2-ylmethyl) amid |
| 168 | 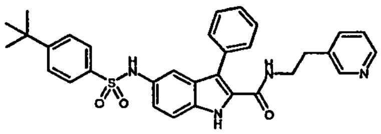 | 5-(4-tert-Butyl-phenylsulfonyl-amino)-3-phenyl-1H-indol-2-carbonsäure-(2-pyridin-3-yl-ethyl)-amid |
| 169 | 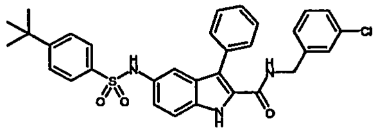 | 5-(4-tert-Butyl-phenylsulfonyl-amino)-3-phenyl-1H-indol-2-carbonsäure-3-chlor-benzylamid |
| 170 | 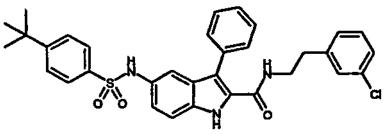 | 5-(4-tert-Butyl-phenylsulfonyl-amino)-3-phenyl-1H-indol-2-carbonsäure-[2-(3-chlor-phenyl)-ethyl]-amid |
| 171 | 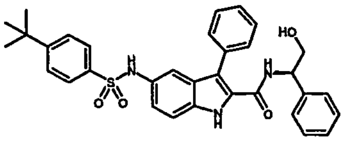 | 5-(4-tert-Butyl-phenylsulfonyl-amino)-3-phenyl-1H-indol-2-carbonsäure-((R)-2-hydroxy-1-phenyl-ethyl)-amid |
| 172 | 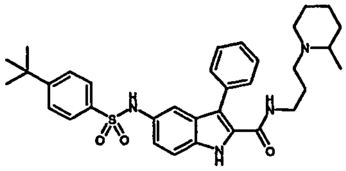 | 5-(4-tert-Butyl-phenylsulfonyl-amino)-3-phenyl-1H-indol-2-carbonsäure-[3-(2-methyl-piperidin-1-yl)-propyl]-amid |
| 173 | 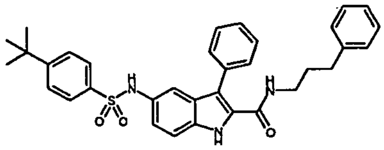 | 5-(4-tert-Butyl-phenylsulfonyl-amino)-3-phenyl-1H-indol-2-carbonsäure-(3-phenyl-propyl)-amid |
| 174 | 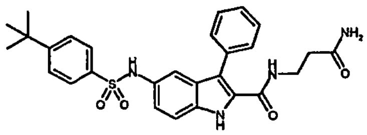 | 5-(4-tert-Butyl-phenylsulfonyl-amino)-3-phenyl-1H-indol-2-carbonsäure-(2-carbamoyl-ethyl)-amid |
| 175 | 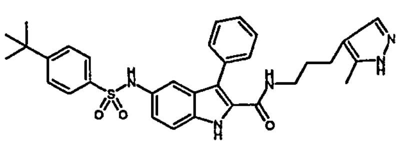 | 5-(4-tert-Butyl-phenylsulfonyl-amino)-3-phenyl-1H-indol-2-carbonsäure-[3-(5-methyl-1H-pyrazol-4-yl)-propyl]-amid |

(fortgesetzt)

| Beispiel | Struktur | Name |
|---|---|---|
| 176 | 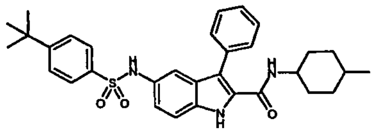 | 5-(4-tert-Butyl-phenylsulfonyl.-amino)-3-phenyl-1H-indol-2-carbonsäure-(4-methyl-cyclohexyl)-amid |
| 177 | 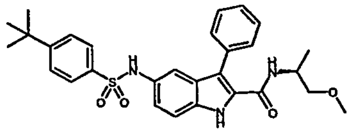 | 5-(4-tert-Butyl-phenylsulfonyl-amino)-3-phenyl-1H-indol-2-carbonsäure-((S)-2-methoxy-1-methyl-ethyl)-amid |
| 178 | 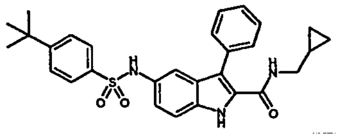 | 5-(4-tert-Butyl-phenylsulfonyl-amino)-3-phenyl-1H-indol-2-carbonsäure-cyclopropylmethyl-amid |
| 179 | 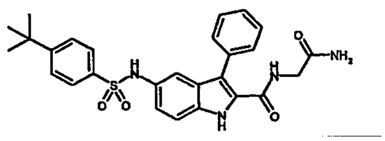 | 5-(4-tert-Butyl-phenylsulfonyl.-amino)-3-phenyl-1H-indol-2-carbonsäure-carbamoylmethyl-amid |
| 180 | 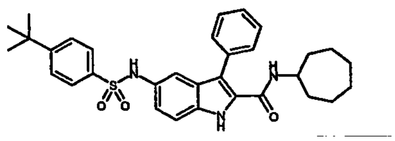 | 5-(4-tert-Butyl-phenylsulfonyl-amino)-3-phenyl-1H-indol-2-carbonsäure-cycloheptylamid |
| 181 | 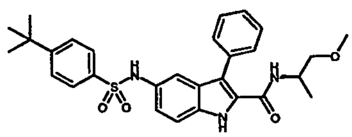 | 5-(4-tert-Butyl-phenylsulfonyl-amino)-3-phenyl-1H-indol-2-carbonsäure-((R)-2-methoxy-1-methyl-ethyl)-amid |
| 182 | 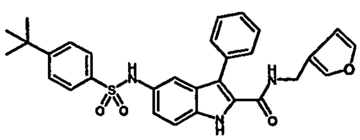 | 5-(4-tert-Butyl-phenylsulfonyl-amino)-3-phenyl-1H-indol-2-carbonsäure-(furan-3-ylmethyl)-amid |
| 183 | 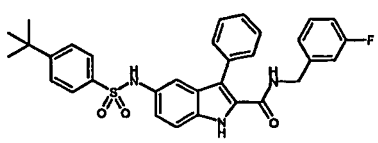 | 5-(4-tert-Butyl-phenylsulfonyl-amino)-3-phenyl-1H-indol-2-carbonsäure-3-fluor-benzylamid |
| 184 | 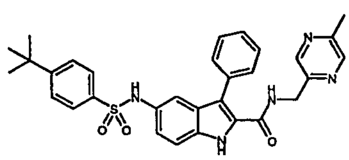 | 5-(4-tert-Butyl-phenylsulfonyl-amino)-3-phenyl-1H-indol-2-carbonsäure-(5-methyl-pyrazin-2-ylmethyl)-amid |
| 185 | 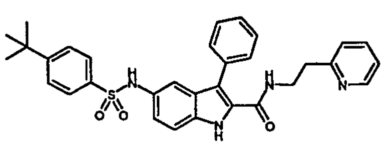 | 5-(4-tert-Butyl-phenylsulfonyl-amino)-3-phenyl-1H-indol-2-carbonsäure-(2-pyridin-2-yl-ethyl)-amid |

(fortgesetzt)

| Beispiel | Struktur | Name |
|---|---|---|
| 186 | 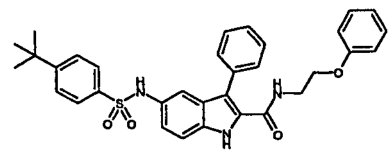 | 5-(4-tert-Butyl-phenylsulfonyl-amino)-3-phenyl-1H-indol-2-carbonsäure-(2-phenoxy-ethyl)-amid |
| 187 | 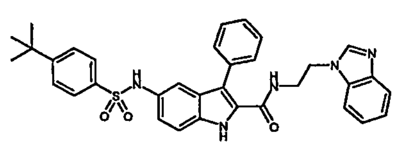 | 5-(4-tert-Butyl-phenylsulfonyl-amino)-3-phenyl-1H-indol-2-carbonsäure-(2-benzoimidazol-1-yl-ethyl)-amid |
| 188 | 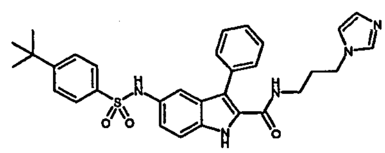 | 5-(4-tert-Butyl-phenylsulfonyl-amino)-3-phenyl-1H-indol-2-carbonsäure-(3-imidazol-1-yl-propyl)-amid |
| 189 | 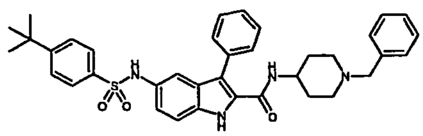 | 5-(4-tert-Butyl-phenylsulfonyl-amino)-3-phenyl-1H-indol-2-carbonsäure-(1-benzyl-piperidin-4-yl)-amid |
| 190 | 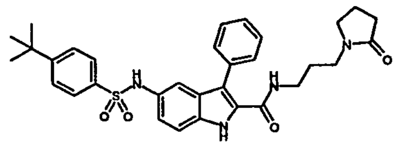 | 5-(4-tert-Butyl-phenylsulfonyl-amino)-3-phenyl-1H-indol-2-carbonsäure-[3-(2-oxo-pyrrolidin-1-yl)-propyl]-amid |
| 191 | 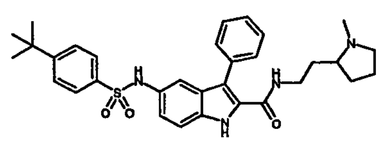 | 5-(4-tert-Butyl-phenylsulfonyl-amino)-3-phenyl-1H-indol-2-carbonsäure-[2-(1-methyl-pyrrolidin-2-yl)-ethyl]-amid |
| 192 | 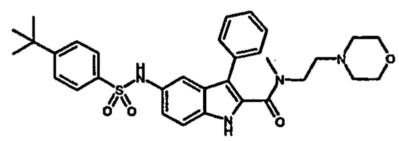 | 5-(4-tert-Butyl-phenylsulfonyl-amino)-3-phenyl-1H-indol-2-carbonsäure methyl-(2-morpholin-4-yl-ethyl)-amid |

**Biologische Beispiele:**

**Beispiel 1: sAC-Assay**

In einem geeigneten Puffersystem katalysiert die lösliche, spermienspezifische Adenylatzyklase die Umsetzung von Adenosintriphosphat (ATP) zu zyklischem Adenosinmonophosphat (cAMP) und Pyrophosphat. Auf diese Weise generiertes, freies cAMP wird anschließend in einem kompetitiven Nachweisverfahren eingesetzt, bei dem die Bindung eines mit Europiumkryptate (Eu[K]) markierten anti-cAMP Antikörpers (anti cAMP-Eu[K]-AK) an ein mit cAMP-Molekülen markiertes, modifiziertes Allophycocyanin-1 Molekül (cAMP-XL665) verhindert wird. In Abwesenheit von exogenem cAMP kommt es nach Anregung bei 335 nm zu einem Fluoreszenz Resonanz Energie Transfer (FRET) zwischen dem anti cAMP-Eu[K]-AK (FRET-Donor) und dem cAMP-XL665 Molekül (FRET-Akzeptor). Dieser Prozess wird zeitlich versetzt (timeresolved) anhand der Emission des FRET-Akzeptors XL665 (665nm und 620nm) quantifiziert. Ein Signal-Abfall (gemessen als Well-Ratio; Berechnungsformel: [(E665nm/E620nm) X 10000] ) lässt sich auf das Vorhandensein von cAMP und somit auf die Aktivität der sAC zurückführen. Pro Vertiefung einer 384-Loch Testplatte (Polystyrol; 384, NV) werden zunächst 1,5 µl der Testsubstanz (in 30% DMSO) vorgelegt, bei den Lösemittelkontrollen lediglich 30% DMSO. Anschließend werden 10 µl einer verdünnten sAC Enzymlösung ausgebracht (Enzym-Stocklösung in 300 mM NaCl, 10 % Glycerin; pH 7,6; Enzym-Zwischen- und Endverdünnung a) 1:10 und b) 1:2000 jeweils in: 1.0 mM $MnCl_2$;

0.2 % BSA; 50 mM Tris pH 7,5 in $H_2O$). Die Enzymreaktion wird durch Zugabe von 5 µl der ATP-Substrat-Lösung (200 µM ATP in $H_2O$) gestartet und nach einer Inkubation (25 Min. bei Raumtemperatur) durch die Zugabe von 5 µl der Stop-Lösung (200 µM EDTA in PBS) beendet. Zum Schluss wird die ganze Reaktion durch die Zugabe von 70 µl PBS auf ein Gesamtvolumen von 91,5 µl eingestellt.
Anschließend werden 8 µl der Detektionslösung 1 in eine Vertiefung der 384-Loch Mess-Platte vorgelegt (Messplatte: Polystyrol; 384, SV - black; Detektionslösung 1: 50 µl cAMP-XL665; 950 µl Rekonsititutionspuffer; 2200 µl PBS; cAMP-XL665: Herstellung durch Zugabe von 5 ml $H_2O$ zum lyophyliserten Produkt gemäß Vorschrift Cis bio Kit: #62AMPPEC; Lagerung: aliquotiert bei - 80˚C). Anschließend werden 3µl aus den 91,5 µl der entsprechenden Vertiefung der Testplatte zugegeben. Zum Schluss erfolgt die Zugabe von 8 µl der Detektionslösung2 (Detektionslösung 2: 50 µl anti cAMP-Eu [K]-AK; 950 µl Rekonsititutionspuffer; 2200 µl PBS; anti cAMP-Eu[K]-AK: Herstellung gemäß Vorschrift Cis bio Kit: #62AMPPEC; Lagerung: aliquotiert bei -80˚C).
Nach einer weiteren Inkubation von 90 Min. bei Raumtemperatur wird das HTRF-Ergebnis entweder am Packard Discovery oder mit dem RubiStar HTRF-Messgerät gemessen (Delay: 50 µs; Integration time: 400 µs).

**Beispiel 2. Isolierung von humanen Spermien aus Ejakulaten und Kapazitation**

2.1. Isolierung der Spermien:

Humane Spermien werden aus dem Ejakulat durch ein zweischichtiges Gradientensystem auf Basis von colloidalen Silica-Partikeln gereinigt (Handelsname: Percoll oder ISolate).
Pro Ejakulat werden in einem15 ml Zentrifugenröhrchen ( konisch, Kunststoff) je 2,5 ml vorgewärmte untere Schicht ("90% ISolate lower layer", Fa. Irvine) vorgelegt und vorsichtig mit 2,5 ml vorgewärmter oberer Schicht ("50% ISolate upper layer", Fa. Irvine) überschichtet und im Wasserbad bei 37˚C für < 1h vorgehalten. Der Gradient wird vorsichtig mit maximal 3 ml normalen (in Bezug auf Spermienanzahl, Motilität und Verflüssigung) Ejakulates überschichtet. Die Sedimentation der Spermien erfolgt bei 1000 x *g* für 25 Min bei Raumtemperatur. Mittels einer Glaskapillare werden beide Schichten bis kurz oberhalb der Spermienpellets abgesaugt. Zum Auswaschen des ISolate-Gradienten werden die in je ca. 200 µl resuspendierten Spermienpellets in ein 15 ml Kunststoffröhrchen mit 12ml mHTF Medium (4mM $NaHCO_3$; 0,01% BSA; 37˚C) überführt und die Spermien werden bei 1000 x *g* für 20 Min sedimentiert. Das Medium wird bis kurz über dem Pellet abgesaugt und mit mHTF Medium Medium (4mM $NaHCO_3$; 0,01% BSA; 37˚C) auf 1000 µl eingestellt. Die Anzahl der Spermien wird in einer Neubauer-Zählkammer bestimmt und für die folgende Kapazitation gegebenenfalls mit mHTF-Medium (4mM $NaHCO_3$; 0,01% BSA; 37˚C) auf 4x106 Spermien/150 µl eingestellt.

2.2. Kapazitation

Falls der Einfluss von Testsubstanzen auf die akrosomale Reaktion getestet werden soll, müssen die Spermien mit den Testsubstanzen vorinkubiert werden. Diese Vorinkubation (15 min im Wärmeschrank bei 37˚C) ist notwendig, um das Eindringen der Testsubstanzen in die Spermien vor Beginn der Kapazitation zu ermöglichen, d.h. eine Präsättigung der Bindungsstellen im Spermium zu erreichen, insbesondere bei Substanzen, die schlecht durch die Membran gehen. Sie ist außerdem notwendig, da die Erhöhung der BSA-Konzentration bei der Kapazitation durch die hohe Lipidbindung des BSA zur Abnahme der effektiven Testsubstanzkonzentration im Ansatz führen könnte.
Die Testsubstanzen werden in DMSO gelöst und mit mHTF-Medium (4mM $NaHCO_3$; 0,01 % BSA; 37˚C) verdünnt, so dass im finalen Kapazitationsansatz von 400 µl die DMSO-Konzentration 0.5% beträgt. Je 150 µl der temperierten obigen Testsubstanzlösung werden zu jeweils 150 µl Spermiensuspension pipettiert und für 15 min bei 37˚C vorinkubiert. Die Kapazitation der Spermien wird gestartet durch Zugabe von 100 µl mHTF-Medium (88mM $NaHCO_3$; 4% BSA; 37˚C). In dem finalen 400 µl Kapazitationsansatz beträgt die Spermienkonzentration 10x106/ml, die Bicarbonatkonzentration 4 mM und die BSA-Konzentration 1%. Die Kapazitation erfolgt bei 37˚C für 3 Stunden im Wärmeschrank.
Zum Beenden der Kapazitation werden die Ansätze (je 400 µl) komplett in jeweils ein 15 ml Probenröhren mit 1,5 ml mHTF (4mM $NaHCO_3$; 37˚C) überführt, 5 min bei 1000 x g zentrifugiert und der Überstand abgenommen. Mit diesem Schritt werden sowohl die hohe Proteinmenge als auch die TestSubstanzen entfernt.

**Beispiel 3. Flow cytometrische Bestimmung der akrosomalen Reaktion**

3.1. Einleitung der akrosomalen Reaktion durch Ionophorbehandlung und gleichzeitige CD46-FITC-Färbung

Die akrosomale Reaktion (AR) des Spermiums wird durch die Bindung des Spermiums an die Zona pellucida (ZP) ausgelöst. Dabei werden aus dem Akrosom Enzyme freigesetzt, die es dem Spermium ermöglichen, durch die ZP bis zur Eizelle vorzudringen. Bei der AR kommt es beim Spermium zu einer teilweisen Verschmelzung der Plasmamembran mit der äußeren akrosomalen Membran (OAM). Der Spermienkopf wird am Ende nur noch durch die innere akrosomale Membran (IAM) begrenzt. Nur an der IAM ist das CD46-Antigen nachweisbar.

*In vitro* lässt sich mit einer geeigneten Konzentration des Calcium-Ionophors A23187 an kapazitierten, aber nicht an unkapazitierten bzw. an durch Testsubstanzen an der Kapazitation gehemmten Spermien die akrosomale Reaktion induzieren. Mit Hilfe des FITC markierten Anti-CD46 Antikörpers (Fa. Pharmingen) gegen die IAM können die Akrosom -reagierten Spermien von den Akrosom -intakten Spermien, bei denen die IAM nicht exponiert ist, im Flow Cytometer unterschieden werden. Durch die gleichzeitige Färbung der Spermien mit dem DNA-Farbstoff Ethidium Homodimer (EhD), der nur die DNA membran-defekter, also toter Zellen färbt, können die toten von den lebenden Spermien unterschieden werden.

Weil die Ionophorverdünnungen zum Auslösen der AR sehr instabil zu sein scheinen und für die gleichzeitige Färbung mit der CD46-FITC Lösung gemischt werden müssen, können die Lösungen nicht vor Versuchsbeginn angesetzt werden, sondern müssen während der Aufarbeitung der Kapazitationsansätze hergestellt werden.

Die Spermienpellets werden im Restüberstand resuspendiert und im Wasserbad (37˚C) mit 450 µl mHTF (4mM $NaHCO_3$; 0,01% BSA; 37˚C) verdünnt. 100 µl Aliquots der Spermiensuspensionen werden in vorbereitete Proben-FACS-Flow-Röhrchen pipettiert (im Wasserbad). Zu den Spermien werden 150 µl einer Lösung mit Ionophor und FITC-markiertem Anti-CD46 Antikörper pipettiert. Die Endkonzentration beträgt 800nM Ionophor und eine 1:125 Verdünnung des Anti-CD46-Antikörpers in mHTF (4mM $NaHCO_3$; 0,01% BSA; 37˚C). Die Spermien werden darin für 30 min lichtgeschützt im Wasserbad bei 37˚C inkubiert.

Die Inkubation wird durch Zugabe von 3,5 ml PBS [0,1% BSA] / Ansatz gestoppt, gefolgt von einer Zentrifugation für 5 min bei 700 *x* g (Raumtemperatur) und anschließendem Absaugen der Überstände. Nach der Zentrifugation werden die Proben bis zur Messung auf der Wärmeplatte warmgehalten.

3.2. EhD-Färbung (zur Differenzierung der toten/lebenden akrosomal reagierten Spermien).

Die Spermienpellets werden nach dem Absaugen mit je 500 µl frisch angesetzter EhD-Lösung (1 50 nM EhD in PBS [w/o BSA]; 37˚C) versetzt. Die Proben können anschließend am Flow Cytometer (BD Facs Calibur) vermessen werden. Die Messung erfolgt bei einer Anregungswellenlänge des Lasers von 488nm, es werden 10000 Spermien pro Messung erfasst. Akrosomreagierte Spermien werden über CD46-FITC im Filter FL-1 bei 530nm gemessen. Tote Spermien werden mittels der EhD - DNA-Färbung im Filter FL-2 bei 634nm gemessen. Die Messkanäle werden zuvor entsprechend gegeneinander kompensiert.

3.3 Auswertung:

Die Spermien werden als sehr einheitliche Zellpopulation in einem FSC-H (forward scatter) gegen SSC-H (sideward scatter) Dotblot ausgewählt. Da eine Zweifarbenfluoreszenzfärbung genutzt wird, erfolgt die Auswertung mit Hilfe der Quadrantenanalyse in einem FL-1 (EhD, X-Achse) vs. FL-2 (FITC-CD46, Y-Achse) Dotblot mit der ausgewählten Spermienpopulation aus dem FSC vs SSC Dotblot:

| | Quadrant im FL-1 vs. FL-2 Dotblot | Färbung | Analyse |
|---|---|---|---|
| Q1 = UL | upper left | nur EhD | tote, nicht akrosom reagierte Spermien |
| Q2 = UR | upper right | EhD und FITC-CD46 | tote, akrosom reagierte Spermien |
| Q3 = LL | lower left | ungefärbt | lebende, nicht akrosom reagierte Spermien |
| Q4 = LR | lower right | nur FITC-CD46 | lebende, akrosom reagierte Spermien |

Zur Berechnung der % induziert akrosomal reagierter Spermien (= "IAR[%]") werden nur die lebenden Spermien aus Q3 und Q4 herangezogen und ihre Gesamtzahl gleich 100% gesetzt. IAR berechnet sich dann wie folgt:

$$IAR[\%] = \frac{LR \times 100}{LL + LR}$$

Ein Teil der Spermien reagiert bereits ohne Ionophorzugabe spontan akrosomal (= "SAR[%]"). Daher erfolgt immer auch eine Kontrollmessung gleichbehandelter Spermien ohne Ionophorzugabe. Die SAR berechnet analog zur IAR. Die wirklich durch das Ionophor ausgelöste akrosomale Reaktion ( = "ARIC[%]") berechnet sich als Differenz: ARIC

= IAR - SAR

Für die folgende Analyse des Einflusses unserer Inhibitoren auf die sAC vermittelte Kapazitation (gemessen als Fähigkeit des Spermiums zur Ionophorinduzierten akrosomalen Reaktion) wird der Prozentsatz akrosomal reagierter Spermien in der positiven Kapazitationskontrolle (= Inkubation mit mHTF-Medium mit 25mM NaHCO3; 1 % BSA ohne Prüfsubstanzen) = 100% gesetzt. Die Fähigkeit der mit den Prüfsubstanzen versetzten Spermien zur akrosomalen Reaktion wird relativ zu dieser maximalen akrosomalen Reaktion angegeben.

Verwendete Materialien:

mHTF = modif. Human tubular fluid (Fa. Irvine Scientific), Dulbeccos's Phosphate-Buffered-Saline (Fa. Gibco) (mit $Ca^{2}$+, $Mg^{2}$+, 1 g/L D-Glucose, 36 mg/L Na-Pyruvate, w/o Phenolrot, w/o $NaHCO_3$); Rinderserumalbumin, Fraktion V (Fa. Fluka); Dimethylsulfoxid (DMSO), wasserfrei (Fa. Merck);
Sodium Bicarbonate 7,5%ige Lsg.(893mM) (Fa. Irvine Scientific); Isolate-Gradient (Fa. Irvine Scientific); Ionophor-A23187 free acid, (Fa. Calbiochem); Ethidium Homodimer (EhD) (Fa. Molecular Probe), Mouse Anti Human CD46:FITC (Fa. Pharmingen).

Literaturzitat:

J. W. Carver-Ward, Human Reproduction Vol. 11, No. 9, pp:1923 ff, 1996 High fertilization prediction by flow cytometric analysis of the CD46 antigen on the inner acrosomal membrane of spermatozoa

O. J. D'Cruz, G. G. Haas, Fertility and Sterility Vol. 65, No. 4, pp: 843 ff, 1996 Fluorescence-labeled fucolectins are superior markers for flow cytometric quantitation of the sperm acrosome reaction

E. Nieschlag, H.M. Behre, Andrologie, Springer Verlag 1996

**Biologische Daten**

| # | $IC_{50}$ [M] | Löslichkeit (g/l) |
|---|---|---|
| 1 | 3,4E-6 | 0,001 |
| 2 | 4,9E-6 | 0,001 |
| 3 | 2,0E-6 | 0,001 |
| 6 | 3,7E-7 | 0,0001 |
| 9 | 5,3E-6 | 0,0015 |
| 10 | 2,2E-6 | 0,0021 |
| 19 | 1.5E-7 | 0.004 |
| 20 | 2,6E-6 | |
| 21 | 3,4E-6 | 0.0001 |
| 24 | 1,6E-6 | 0,008 |
| 25 | 6,3E-7 | 0,005 |
| 26 | 9,9E-6 | 0,003 |
| 28 | 2,7E-6 | 0,005 |
| 29 | 2E-6 | 0,007 |
| 31 | 1,2E-6 | 0,007 |
| 32 | 1,3E-6 | 0,055 |
| 37 | 7E-8 | 0,013 |
| 40 | 8,6E-8 | 0,005 |

**Vergleich mit bekannten Verbindungen**

Die erfindungsgemäßen Verbindungen wurden mit bekannten Verbindungen im Enzymtest verglichen. Das Ergebnis ist in der folgenden Tabelle aufgeführt.

| Beispiel | R4 | R3 | IC50 [M] | Löslichkeit (g/l) |
|---|---|---|---|---|
| 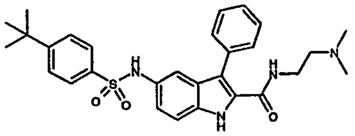 #6 | $(CH_2)_2N(CH_3)_2$ | Phenyl | 3,7E-7 | 0,0001 |
| 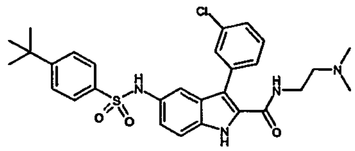 #25 | $CH_2)_2N(CH_3)_2$ | Cl-Phenyl | 6,3E-7 | 0,005 |
| 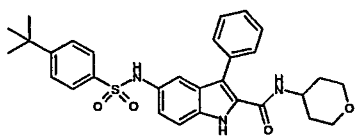 #40 | 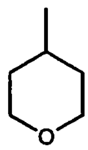 | Phenyl | 8,6E-8 | 0.005 |
| 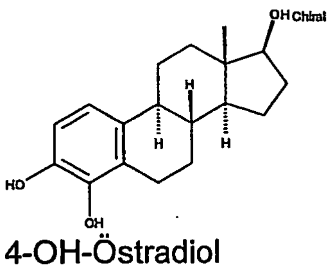 4-OH-Östradiol | | | 1,3E-5 | |
| 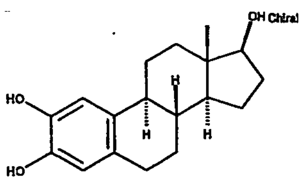 2-OH-Östradiol | | | 1.1 E-5 | |

Aus der Tabelle ist zu erkennen, dass die erfindungsgemäßen Verbindungen in bezug auf die Inhibition der löslichen Adenylatzyklase, ausgedrückt durch den $IC_{50}$-Wert, eine zum Teil 150 fach höhere Aktivität aufweisen als die bereits bekannten Catecholöstrogene (OH-Östradiole). Die Catacholöstrogene sind toxisch, daher sind die erfindungsgemäßen Verbindungen den bekannten Verbindungen weit überlegen. Die erfindungsgemäßen Verbindungen sind auch etwa 100 fach potenter als die von Zippin vorgestellten Verbindungen.

## Patentansprüche

1. Verbindungen der allgemeinen Formel 1

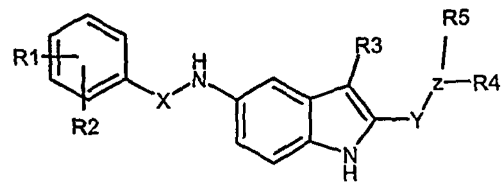

in der

$R^1$ für Wasserstoff, Halogen, $CF_3$, $C_3$-$C_6$-Cycloalkyl, welches gegebenenfalls mehrfach gesättigt und gegebenenfalls mehrfach substituiert ist, oder für die Gruppe $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl, $C_1$-$C_6$-Aryl-$C_1$-$C_6$-Alkyl oder $CF_3$, in der $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl oder $C_1$-$C_6$-Aryl-$C_1$-$C_6$-Alkyl gegebenenfalls ein- oder mehrfach, gleich oder verschieden durch Sauerstoff, Schwefel oder Stickstoff unterbrochen sein können, oder für die Gruppe Sulfonyl-$C_1$-$C_6$-Alkyl, Sulfonamid, oder Cyano steht,
$R^2$ für Halogen, $CF_3$, $C_3$-$C_6$-Cycloalkyl, welches gegebenenfalls mehrfach gesättigt und gegebenenfalls mehrfach substituiert ist, oder für die Gruppe $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl, $C_1$-$C_6$-Aryl-$C_1$-$C_6$-Alkyl oder $CF_3$, in der $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl oder $C_1$-$C_6$-Aryl-$C_1$-$C_6$-Alkyl gegebenenfalls ein- oder mehrfach, gleich oder verschieden durch Sauerstoff, Schwefel oder Stickstoff unterbrochen sein können, oder für die Gruppe Sulfonyl-$C_1$-$C_6$-Alkyl, Sulfonamid, oder Cyano steht,
$R^3$ für $C_6$-$C_{12}$-Aryl, welches gegebenenfalls ein- oder mehrfach, gleich oder verschieden mit Halogen, mit $C_1$-$C_6$-Alkyl oder $C_1$-$C_6$-Acyl, welches gegebenenfalls ein- oder mehrfach substituiert sein kann, oder mit $C_1$-$C_6$-Alkoxy, Hydroxy, Cyano, $CO_2$-($C_1$-$C_6$-Alkyl), N-($C_1$-$C_6$-Alkyl)$_2$, CO-$NR^4R^5$ oder mit $CF_3$ substituiert sein kann, für $C_5$-$C_{12}$-Heteroaryl, welches gegebenenfalls ein- oder mehrfach, gleich oder verschieden mit Halogen, mit $C_1$-$C_6$-Alkyl, $C_1$-$C_6$-Acyl, $C_1$-$C_6$-Alkoxy, Hydroxy, Cyano, $CO_2$-($C_1$-$C_6$-Alkyl), N-($C_1$-$C_6$-Alkyl)$_2$, CO-$NR^4R^5$ oder mit $CF_3$ substituiert sein kann oder für $C_3$-$C_6$-Cycloalkyl, welches gegebenenfalls ein- oder mehrfach, gleich oder verschieden mit Halogen, $CF_3$, Hydroxy, Cyano, $CO_2$-($C_1$-$C_6$-Alkyl), $C_1$-$C_6$-Alkyl, $C_1$-$C_6$-Acyl, N-($C_1$-$C_6$-Alkyl)$_2$, CO-$NR^4R^5$ oder $C_1$-$C_6$-Alkoxy substituiert sein kann, steht
$R^4$ für Wasserstoff, $C_3$-$C_6$-Cycloalkyl, welches gegebenenfalls ein- oder mehrfach, gleich oder verschieden mit $C_1$-$C_6$-Alkyl, $C_1$-$C_6$-Acyl, $C_1$-$C_6$-Alkoxy oder $CF_3$ substituiert ist, für $C_6$-$C_{12}$-Aryl, welches gegebenenfalls ein- oder mehrfach, gleich oder verschieden mit Halogen, mit $C_1$-$C_6$-Alkyl, $C_1$-$C_6$-Acyl, $C_1$-$C_6$-Alkoxy, N-$C_1$-$C_6$-Alkyl-$C_1$-$C_6$-Alkyl, $CF_3$ oder Cyano substituiert ist, oder für $C_5$-$C_{12}$-Heteroaryl, welches gegebenenfalls ein- oder mehrfach, gleich oder verschieden mit Halogen, mit $C_1$-$C_6$-Alkyl, $C_1$-$C_6$-Acyl, $C_1$-$C_6$-Alkoxy, N-$C_1$-$C_6$-Alkyl-$C_1$-$C_6$-Alkyl, $CF_3$ oder Cyano, substituiert ist, oder für $C_1$-$C_6$-Alkyl, welches beliebig substituiert sein kann, steht,
$R^5$ für Wasserstoff, $C_3$-$C_6$-Cycloalkyl, welches gegebenenfalls ein- oder mehrfach, gleich oder verschieden mit $C_1$-$C_6$-Alkyl, $C_1$-$C_6$-Acyl, $C_1$-$C_6$-Alkoxy oder $CF_3$ substituiert ist, für $C_6$-$C_{12}$-Aryl, welches gegebenenfalls ein- oder mehrfach, gleich oder verschieden mit Halogen, mit $C_1$-$C_6$-Alkyl, $C_1$-$C_6$-Acyl, $C_1$-$C_6$-Alkoxy, N-$C_1$-$C_6$-Alkyl-$C_1$-$C_6$-Alkyl, $CF_3$ oder Cyano substituiert ist, oder für $C_5$-$C_{12}$-Heteroaryl, welches gegebenenfalls ein- oder mehrfach, gleich oder verschieden mit Halogen, mit $C_1$-$C_6$-Alkyl, $C_1$-$C_6$-Acyl, $C_1$-$C_6$-Alkoxy, N-$C_1$-$C_6$-Alkyl-$C_1$-$C_6$-Alkyl, $CF_3$ oder Cyano, substituiert ist, oder für $C_1$-$C_6$-Alkyl, welches beliebig substituiert sein kann, steht,
$R^4$ und $R^5$ gemeinsam einen 5-8 gliedrigen Ring bilden, der weitere Heteroatome enthalten kann, stehen und
X für die Gruppen Sulfonyl, $(CH_2)_n$ oder Carbonyl steht,
Y für Carbonyl oder $(CH_2)_n$ steht,
Z für Stickstoff steht, und
n für 0 - 4 steht.

**2.** Verbindungen gemäß Anspruch 1, wobei

$R^1$ für Wasserstoff, Halogen, $CF_3$, $C_3$-$C_6$-Cycloalkyl, welches gegebenenfalls mehrfach gesättigt und gegebenenfalls mehrfach substituiert ist, oder für die Gruppe $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl, $C_1$-$C_6$-Aryl-$C_1$-$C_6$-Alkyl oder $CF_3$, in der $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl oder $C_1$-$C_6$-Aryl-$C_1$-$C_6$-Alkyl gegebe-

nenfalls ein- oder mehrfach, gleich oder verschieden durch Sauerstoff, Schwefel oder Stickstoff unterbrochen sein können, oder für die Gruppe Sulfonyl-$C_1$-$C_6$-Alkyl, Sulfonamid, oder Cyano steht,

$R^2$ Halogen, $CF_3$, $C_3$-$C_6$-Cycloalkyl, welches gegebenenfalls mehrfach gesättigt und gegebenenfalls mehrfach substituiert ist, oder für die Gruppe $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl, $C_1$-$C_6$-Aryl-$C_1$-$C_6$-Alkyl oder $CF_3$, in der $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl oder $C_1$-$C_6$-Aryl-$C_1$-$C_6$-Alkyl gegebenenfalls ein- oder mehrfach, gleich oder verschieden durch Sauerstoff, Schwefel oder Stickstoff unterbrochen sein können, oder für die Gruppe Sulfonyl-$C_1$-$C_6$-Alkyl, Sulfonamid, oder Cyano steht,

$R^3$ für $C_6$-$C_{12}$-Aryl, welches gegebenenfalls ein- oder mehrfach, gleich oder verschieden mit Halogen, mit $C_1$-$C_6$-Alkyl, $C_1$-$C_3$-Acyl, $C_1$-$C_3$-Alkoxy, Cyano, Hydroxy, N-$(CH_3)_2$, $CO_2$-($C_1$-$C_3$-Alkyl), CO-$NR^4R^5$ oder mit $CF_3$ substituiert sein kann, $C_5$-$C_{12}$-Heteroaryl, welches gegebenenfalls ein- oder mehrfach, gleich oder verschieden mit Chlor und / oder Fluor, mit $C_1$-$C_6$-Alkyl, $C_1$-$C_3$-Acyl, $C_1$-$C_3$-Alkoxy, Cyano, Hydroxy, N-$(CH_3)_2$, $CO_2$-($C_1$-$C_3$-Alkyl), CO-$NR^4R^5$ oder mit $CF_3$ substituiert sein kann, $C_3$-$C_6$-Cycloalkyl, welches gegebenenfalls ein- oder mehrfach, gleich oder verschieden mit Chlor und / oder Fluor, $CF_3$. Cyano, $C_1$-$C_3$-Alkyl, $C_1$-$C_3$-Acyl, Hydroxy, N-$(CH_3)_2$, $CO_2$-($C_1$-$C_3$-Alkyl), CO-$NR^4R^5$ oder $C_1$-$C_3$-Alkoxy substituiert sein kann, steht,

$R^4$ für Wasserstoff, $C_3$-$C_6$-Cycloalkyl, welches gegebenenfalls ein- oder mehrfach, gleich oder verschieden mit $C_1$-$C_3$-Alkyl, $C_1$-$C_3$-Acyl, $C_1$-$C_3$-Alkoxy oder $CF_3$ substituiert ist, für $C_6$-$C_{12}$-Aryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano substituiert ist, oder für $C_5$-$C_{12}$-Heteroaryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano, substituiert ist, oder für $C_1$-$C_6$-Alkyl, welches beliebig substituiert sein kann, steht,

$R^5$ für Wasserstoff, $C_3$-$C_6$-Cycloalkyl, welches gegebenenfalls ein- oder mehrfach, gleich oder verschieden mit $C_1$-$C_3$-Alkyl, $C_1$-$C_3$-Acyl, $C_1$-$C_3$-Alkoxy oder $CF_3$ substituiert ist, für $C_6$-$C_{12}$-Aryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano substituiert ist, oder für $C_5$-$C_{12}$-Heteroaryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano, substituiert ist, oder für $C_1$-$C_6$-Alkyl, welches beliebig substituiert sein kann, steht,

$R^4$ und $R^5$ gemeinsam einen 5-8 gliedrigen Ring bilden, der weitere Heteroatome enthalten kann, steht,

X für die Gruppen Sulfonyl, $(CH_2)_n$ oder Carbonyl steht,

Y für Carbonyl oder $(CH_2)_n$ steht,

Z für Stickstoff steht, und

n für 0 - 2 steht.

**3.** Verbindungen gemäß Anspruch 1 oder 2 wobei

$R^1$ für Wasserstoff steht,

$R^2$ für tertiär Butyl, Cyano, Brom, oder für die Gruppe -O-$CF_3$, -$SO_2$-$CH_3$ steht und in para-Stellung steht,

$R^3$ für die Gruppe

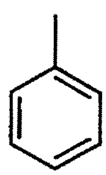
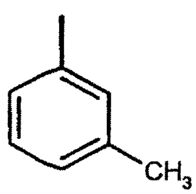
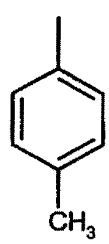
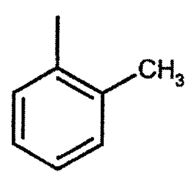
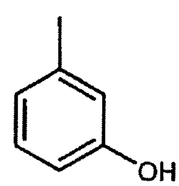
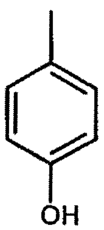
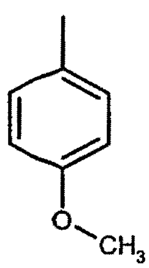
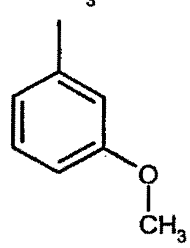
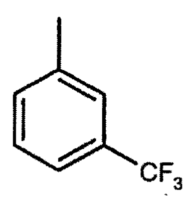
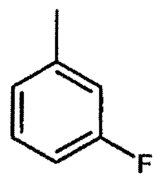

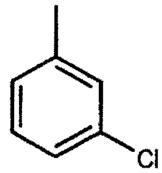 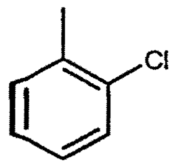 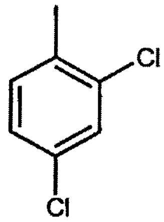 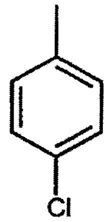

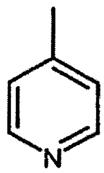 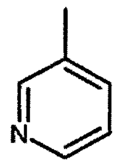 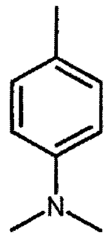

steht,
$R^4$ für Wasserstoff oder für die Gruppe $-(CH_2)_n-N-(CH_3)_2$, $-(CH_2)_2-CH_3$, $-(CH_2)_2-NH-COCH_3$, $-(CH_2)-CHCH_3-OH$, $-(CH_2)_2-O-CH_3$, $-(CH_2)_2-OH$, $--CHCH_3-CH_2-OH$,

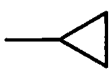 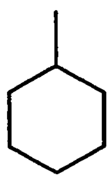 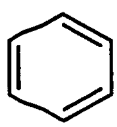 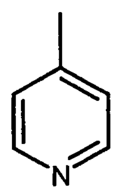

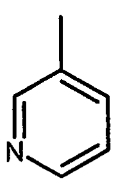 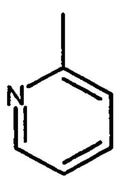 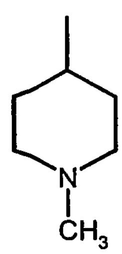 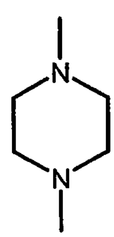 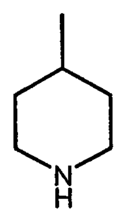

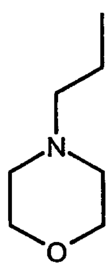 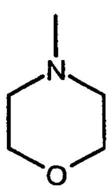 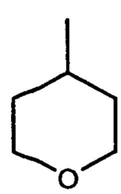 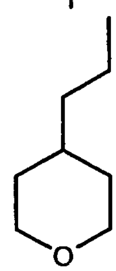 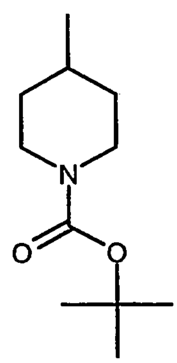

steht,
$R^5$ für Wasserstoff steht,
X für Sulfonyl, Carbonyl oder für die Gruppe $CH_2$ steht,

Y für Carbonyl oder für die Gruppe $(CH_2)$n steht,
Z für Stickstoff bzw. für

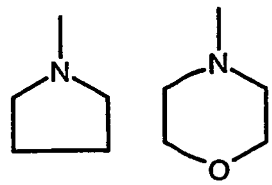

steht, und
n 1-2 steht.

4. Verbindungen gemäß Anspruch 1, in denen

$R^1$ für Wasserstoff, tertiär Butyl, Cyano, Brom, oder für die Gruppe $-O-CF_3$, $-SO_2-CH_3$ steht,
$R^2$ für tertiär Butyl, Cyano, Brom, oder für die Gruppe $-O-CF_3$, $-SO_2-CH_3$ steht, und
$R^3$ für $C_6$-$C_{12}$-Aryl, welches gegebenenfalls ein- oder mehrfach, gleich oder verschieden mit Halogen, mit $C_1$-$C_6$-Alkyl, $C_1$-$C_3$-Acyl, $C_1$-$C_3$-Alkoxy, Cyano, Hydroxy, $N(CH_3)_2$, $CO_2$-($C_1$-$C_3$-Alkyl), CO-$NR^4R^5$ oder mit $CF_3$ substituiert sein kann, $C_5$-$C_{12}$-Heteroaryl, welches gegebenenfalls ein- oder mehrfach, gleich oder verschieden mit Chlor und / oder Fluor, mit $C_1$-$C_6$-Alkyl, $C_1$-$C_3$-Acyl, $C_1$-$C_3$-Alkoxy, Cyano, Hydroxy, $N(CH_3)_2$, $CO_2$-($C_1$-$C_3$-Alkyl), CO-$NR^4R^5$ oder mit $CF_3$ substituiert sein kann, $C_3$-$C_6$-Cycloalkyl, welches gegebenenfalls ein- oder mehrfach, gleich oder verschieden mit Chlor und / oder Fluor, $CF_3$, Cyano, $C_1$-$C_3$-Alkyl, $C_1$-$C_3$-Acyl, Hydroxy, $N(CH_3)_2$, $CO_2$-($C_1$-$C_3$-Alkyl), CO-$NR^4R^5$ oder $C_1$-$C_3$-Alkoxy substituiert sein kann, steht,
$R^4$ für Wasserstoff, $C_3$-$C_6$-Cycloalkyl, welches gegebenenfalls ein- oder mehrfach, gleich oder verschieden mit $C_1$-$C_3$-Alkyl, $C_1$-$C_3$-Acyl, $C_1$-$C_3$-Alkoxy oder $CF_3$ substituiert ist, für $C_6$-$C_{12}$-Aryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano substituiert ist, oder für $C_5$-$C_{12}$-Heteroaryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano, substituiert ist, oder für $C_1$-$C_6$-Alkyl, welches beliebig substituiert sein kann, steht,
$R^5$ für Wasserstoff, $C_3$-$C_6$-Cycloalkyl, welches gegebenenfalls ein- oder mehrfach, gleich oder verschieden mit $C_1$-$C_3$-Alkyl, $C_1$-$C_3$-Acyl, $C_1$-$C_3$-Alkoxy oder $CF_3$ substituiert ist, für $C_6$-$C_{12}$-Aryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano substituiert ist, oder für $C_5$-$C_{12}$-Heteroaryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano, substituiert ist, oder für $C_1$-$C_6$-Alkyl, welches beliebig substituiert sein kann, steht,
$R^4$ und $R^5$ gemeinsam einen 5-8 gliedrigen Ring bilden, der weitere Heteroatome enthalten kann, steht,
X für die Gruppen Sulfonyl, $(CH_2)_n$ oder Carbonyl steht,
Y für Carbonyl oder $(CH_2)_n$ steht,
Z für Stickstoff steht, und
n für 0 - 2 steht.

5. Verbindungen gemäß Anspruch 1, in denen

$R^1$ für Wasserstoff steht,
$R^2$ für tertiär Butyl, Cyano, Brom, oder für die Gruppe $-O-CF_3$, $-SO_2-CH_3$ steht, und in para steht, und
$R^3$ für $C_6$-$C_{12}$-Aryl, welches gegebenenfalls ein- oder mehrfach, gleich oder verschieden mit Halogen, mit $C_1$-$C_6$-Alkyl, $C_1$-$C_3$-Acyl, $C_1$-$C_3$-Alkoxy, Cyano, Hydroxy, $N(CH_3)_2$, $CO_2$-($C_1$-$C_3$-Alkyl), CO-$NR^4R^5$ oder mit $CF_3$ substituiert sein kann, $C_5$-$C_{12}$-Heteroaryl, welches gegebenenfalls ein- oder mehrfach, gleich oder verschieden mit Chlor und / oder Fluor, mit $C_1$-$C_6$-Alkyl, $C_1$-$C_3$-Acyl, $C_1$-$C_3$-Alkony, Cyano, Hydroxy, $N(CH_3)_2$, $CO_2$-($C_1$-$C_3$-Alkyl), CO-$NR^4R^5$ oder mit $CF_3$ substituiert sein kann, $C_3$-$C_6$-Cycloalkyl, welches gegebenenfalls ein- oder mehrfach, gleich oder verschieden mit Chlor und / oder Fluor, $CF_3$, Cyano, $C_1$-$C_3$-Alkyl, $C_1$-$C_3$-Acyl, Hydroxy, $N(CH_3)_2$, $CO_2$-($C_1$-$C_3$-Alkyl), CO-$NR^4R^5$ oder $C_1$-$C_3$-Alkoxy substituiert sein kann, steht,
$R^4$ für Wasserstoff, $C_3$-$C_6$-Cycloalkyl, welches gegebenenfalls ein- oder mehrfach, gleich oder verschieden mit $C_1$-$C_3$-Alkyl, $C_1$-$C_3$-Acyl, $C_1$-$C_3$-Alkoxy oder $CF_3$ substituiert ist, für $C_6$-$C_{12}$-Aryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano substituiert ist, oder für $C_5$-$C_{12}$-Heteroaryl, welches gegebenenfalls ein- oder

mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano, substituiert ist, oder für $C_1$-$C_6$-Alkyl, welches beliebig substituiert sein kann, steht,
$R^5$ für Wasserstoff, $C_3$-$C_6$-Cycloalkyl, welches gegebenenfalls ein- oder mehrfach, gleich oder verschieden mit $C_1$-$C_3$-Alkyl, $C_1$-$C_3$-Acyl, $C_1$-$C_3$-Alkoxy oder $CF_3$ substituiert ist, für $C_6$-$C_{12}$-Aryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano substituiert ist, oder für $C_5$-$C_{12}$-Heteroaryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano, substituiert ist, oder für $C_1$-$C_6$-Alkyl, welches beliebig substituiert sein kann, steht,
$R^4$ und $R^5$ gemeinsam einen 5-8 gliedrigen Ring bilden, der weitere Heteroatome enthalten kann, steht,
X für die Gruppen Sulfonyl, $(CH_2)_n$ oder Carbonyl steht,
Y für Carbonyl oder $(CH_2)_n$ steht,
Z für Stickstoff steht, und
n für 0 - 2 steht.

**6.** Verbindungen gemäß Anspruch 1, wobei

$R^1$ für Wasserstoff, Halogen, $CF_3$, $C_3$-$C_6$-Cycloalkyl, welches gegebenenfalls mehrfach gesättigt und gegebenenfalls mehrfach substituiert ist, oder für die Gruppe $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl, $C_1$-$C_6$-Aryl-$C_1$-$C_6$-Alkyl oder $CF_3$, in der $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl oder $C_1$-$C_6$-Aryl-$C_1$-$C_6$-Alkyl gegebenenfalls ein- oder mehrfach, gleich oder verschieden durch Sauerstoff, Schwefel oder Stickstoff unterbrochen sein können, oder für die Gruppe Sulfonyl-$C_1$-$C_6$-Alkyl, Sulfonamid, oder Cyano steht,
$R^2$ für Halogen, $CF_3$, $C_3$-$C_6$-Cycloalkyl, welches gegebenenfalls mehrfach gesättigt und gegebenenfalls mehrfach substituiert ist, oder für die Gruppe $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl, $C_1$-$C_6$-Aryl-$C_1$-$C_6$-Alkyl oder $CF_3$, in der $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl oder $C_1$-$C_6$-Aryl-$C_1$-$C_6$-Alkyl gegebenenfalls ein- oder mehrfach, gleich oder verschieden durch Sauerstoff, Schwefel oder Stickstoff unterbrochen sein können, oder für die Gruppe Sulfonyl-$C_1$-$C_6$-Alkyl, Sulfonamid, oder Cyano steht,
$R^3$ für $C_6$-$C_{12}$-Aryl, welches gegebenenfalls ein- oder mehrfach, gleich oder verschieden mit Halogen, mit $C_1$-$C_6$-Alkyl, $C_1$-$C_3$-Acyl, $C_1$-$C_3$-Alkoxy, Cyano, Hydroxy, N-$(CH_3)_2$, $CO_2$-($C_1$-$C_3$-Alkyl), CO-$NR^4R^5$ oder mit $CF_3$ substituiert sein kann, $C_5$-$C_{12}$-Heteroaryl, welches gegebenenfalls ein- oder mehrfach, gleich oder verschieden mit Chlor und / oder Fluor, mit $C_1$-$C_6$-Alkyl, $C_1$-$C_3$-Acyl, $C_1$-$C_3$-Alkoxy, Cyano, Hydroxy, N-$(CH_3)_2$, $CO_2$-($C_1$-$C_3$-Alkyl), CO-$NR^4R^5$ oder mit $CF_3$ substituiert sein kann, $C_3$-$C_6$-Cycloalkyl, welches gegebenenfalls ein- oder mehrfach, gleich oder verschieden mit Chlor und / oder Fluor, $CF_3$, Cyano, $C_1$-$C_3$-Alkyl, $C_1$-$C_3$-Acyl, Hydroxy, N-$(CH_3)_2$, $CO_2$-($C_1$-$C_3$-Alkyl), CO-$NR^4R^5$ oder $C_1$-$C_3$-Alkoxy substituiert sein kann, steht,
$R^4$ für Wasserstoff, $C_3$-$C_6$-Cycloalkyl, welches gegebenenfalls ein- oder mehrfach, gleich oder verschieden mit $C_1$-$C_6$-Alkyl, $C_1$-$C_6$-Acyl, $C_1$-$C_6$-Alkoxy oder $CF_3$ substituiert ist, für $C_6$-$C_{12}$-Aryl, welches gegebenenfalls ein- oder mehrfach, gleich oder verschieden mit Halogen, mit $C_1$-$C_6$-Alkyl, $C_1$-$C_6$-Acyl, $C_1$-$C_6$-Alkoxy, N-$C_1$-$C_6$-Alkyl-$C_1$-$C_6$-Alkyl, $CF_3$ oder Cyano substituiert ist, oder für $C_5$-$C_{12}$-Heteroaryl, welches gegebenenfalls ein- oder mehrfach, gleich oder verschieden mit Halogen, mit $C_1$-$C_6$-Alkyl, $C_1$-$C_6$-Acyl, $C_1$-$C_6$-Alkoxy, N-$C_1$-$C_6$-Alkyl-$C_1$-$C_6$-Alkyl, $CF_3$ oder Cyano, substituiert ist, oder für $C_1$-$C_6$-Alkyl, welches beliebig substituiert sein kann, steht,
$R^5$ für Wasserstoff, $C_3$-$C_6$-Cycloalkyl, welches gegebenenfalls ein- oder mehrfach, gleich oder verschieden mit $C_1$-$C_6$-Alkyl, $C_1$-$C_6$-Acyl, $C_1$-$C_6$-Alkoxy oder $CF_3$ substituiert ist, für $C_6$-$C_{12}$-Aryl, welches gegebenenfalls ein- oder mehrfach, gleich oder verschieden mit Halogen, mit $C_1$-$C_6$-Alkyl, $C_1$-$C_6$-Acyl, $C_1$-$C_6$-Alkoxy, N-$C_1$-$C_6$-Alkyl-$C_1$-$C_6$-Alkyl, $CF_3$ oder Cyano substituiert ist, oder für $C_5$-$C_{12}$-Heteroaryl, welches gegebenenfalls ein- oder mehrfach, gleich oder verschieden mit Halogen, mit $C_1$-$C_6$-Alkyl, $C_1$-$C_6$-Acyl, $C_1$-$C_6$-Alkoxy, N-$C_1$-$C_6$-Alkyl-$C_1$-$C_6$-Alkyl, $CF_3$ oder Cyano, substituiert ist, oder für $C_1$-$C_6$-Alkyl, welches beliebig substituiert sein kann, steht,
$R^4$ und $R^5$ gemeinsam einen 5-8 gliedrigen Ring bilden, der weitere Heteroatome enthalten kann, stehen und
X für die Gruppen Sulfonyl, $(CH_2)_n$ oder Carbonyl steht,
Y für Carbonyl oder $(CH_2)_n$ steht,
Z für Stickstoff steht, und
n für 0 - 4 steht.

**7.** Verbindungen gemäß Anspruch 1, wobei

$R^1$ für Wasserstoff steht,

$R^2$ für tertiär Butyl, Cyano, Brom, oder für die Gruppe $-O-CF_3$, $-SO_2-CH_3$ steht und in para-Stellung steht, und
$R^3$ für $C_6$-$C_{12}$-Aryl, welches gegebenenfalls ein- oder mehrfach, gleich oder verschieden mit Halogen, mit $C_1$-$C_6$-Alkyl, $C_1$-$C_3$-Acyl, $C_1$-$C_3$-Alkoxy, Cyano, Hydroxy, $N-(CH_3)_2$, $CO_2$-($C_1$-$C_3$-Alkyl), $CO-NR^4R^5$ oder mit $CF_3$ substituiert sein kann, $C_5$-$C_{12}$-Heteroaryl, welches gegebenenfalls ein- oder mehrfach, gleich oder verschieden mit Chlor und / oder Fluor, mit $C_1$-$C_6$-Alkyl, $C_1$-$C_3$-Acyl, $C_1$-$C_3$-Alkoxy, Cyano, Hydroxy, $N-(CH_3)_2$, $CO_2$-($C_1$-$C_3$-Alkyl), $CO-NR^4R^5$ oder mit $CF_3$ substituiert sein kann, $C_3$-$C_6$-Cycloalkyl, welches gegebenenfalls ein- oder mehrfach, gleich oder verschieden mit Chlor und / oder Fluor, $CF_3$, Cyano, $C_1$-$C_3$-Alkyl, $C_1$-$C_3$-Acyl, Hydroxy, $N-(CH_3)_2$, $CO_2$-($C_1$-$C_3$-Alkyl), $CO-NR^4R^5$ oder $C_1$-$C_3$-Alkoxy substituiert sein kann, steht,
$R^4$ für Wasserstoff oder für die Gruppe $-(CH_2)_n-N-(CH_3)_2$, $-(CH_2)_2-CH_3$, $-(CH_2)_2-NH-COCH_3$, $-(CH_2)-CHCH_3-OH$, $-(CH_2)_2-O-CH_3$,$-(CH_2)_2-OH$, $-CHCH_3-CH_2-OH$,

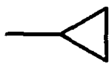
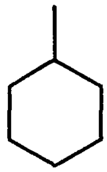
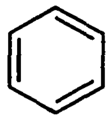
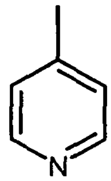

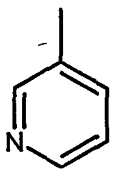
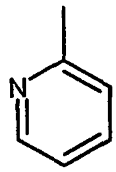
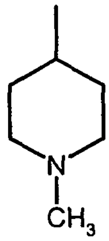
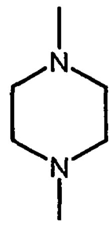
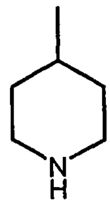

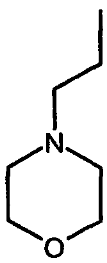
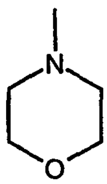
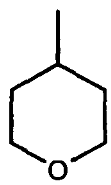
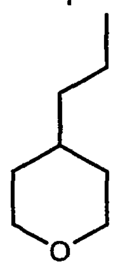
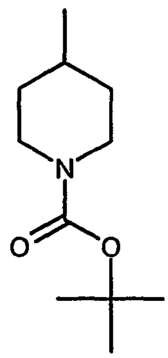

steht,
$R^5$ für Wasserstoff steht,
X für Sulfonyl, Carbonyl oder für die Gruppe $CH_2$ steht,
Y für Carbonyl oder für die Gruppe $(CH_2)_n$ steht,
Z für Stickstoff bzw. für

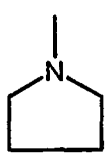
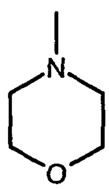

steht, und
n 1-2 steht.

**8.** Verbindungen gemäß Anspruch 1, wobei

$R^1$ für Wasserstoff, Halogen, $CF_3$, $C_3$-$C_6$-Cycloalkyl, welches gegebenenfalls mehrfach gesättigt und gegebenenfalls mehrfach substituiert ist, oder für die Gruppe $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl, $C_1$-$C_6$-Aryl-$C_1$-$C_6$-Alkyl oder $CF_3$, in der $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl oder $C_1$-$C_6$-Aryl-$C_1$-$C_6$-Alkyl gegebenenfalls ein- oder mehrfach, gleich oder verschieden durch Sauerstoff, Schwefel oder Stickstoff unterbrochen sein können, oder für die Gruppe Sulfonyl-$C_1$-$C_6$-Alkyl, Sulfonamid, oder Cyano steht,
$R^2$ Halogen, $CF_3$, $C_3$-$C_6$-Cycloalkyl, welches gegebenenfalls mehrfach gesättigt und gegebenenfalls mehrfach substituiert ist, oder für die Gruppe $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl, $C_1$-$C_6$-Aryl-$C_1$-$C_6$-Alkyl oder $CF_3$, in der $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl oder $C_1$-$C_6$-Aryl-$C_1$-$C_6$-Alkyl gegebenenfalls ein- oder mehrfach, gleich oder verschieden durch Sauerstoff, Schwefel oder Stickstoff unterbrochen sein können, oder für die Gruppe Sulfonyl-$C_1$-$C_6$-Alkyl, Sulfonamid, oder Cyano steht,
$R^3$ für die Gruppe

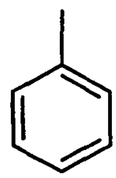
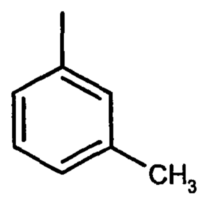
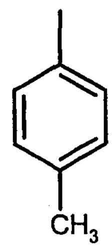
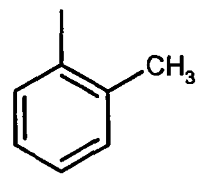
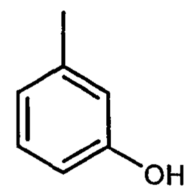
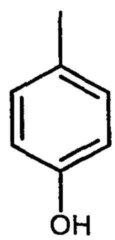
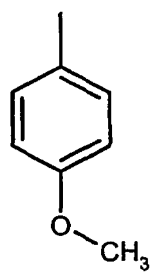
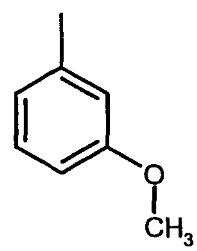
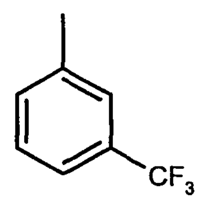
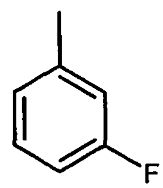
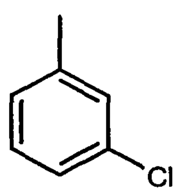
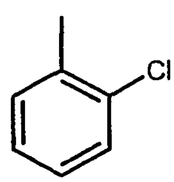
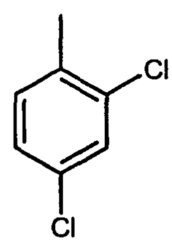
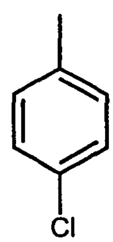

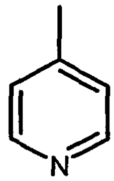 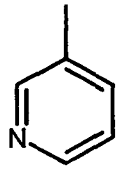 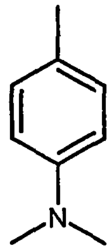

steht,

$R^4$ für Wasserstoff, $C_3$-$C_6$-Cycloalkyl, welches gegebenenfalls ein- oder mehrfach, gleich oder verschieden mit $C_1$-$C_3$-Alkyl, $C_1$-$C_3$-Acyl, $C_1$-$C_3$-Alkoxy oder $CF_3$ substituiert ist, für $C_6$-$C_{12}$-Aryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano substituiert ist, oder für $C_5$-$C_{12}$-Heteroaryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano, substituiert ist, oder für $C_1$-$C_6$-Alkyl, welches beliebig substituiert sein kann, steht,

$R^5$ für Wasserstoff, $C_3$-$C_6$-Cycloalkyl, welches gegebenenfalls ein- oder mehrfach, gleich oder verschieden mit $C_1$-$C_3$-Alkyl, $C_1$-$C_3$-Acyl, $C_1$-$C_3$-Alkoxy oder $CF_3$ substituiert ist, für $C_6$-$C_{12}$-Aryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano substituiert ist, oder für $C_5$-$C_{12}$-Heteroaryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano, substituiert ist, oder für $C_1$-$C_6$-Alkyl, welches beliebig substituiert sein kann, steht,

$R^4$ und $R^5$ gemeinsam einen 5-8 gliedrigen Ring bilden, der weitere Heteroatome enthalten kann, steht,

X für die Gruppen Sulfonyl, $(CH_2)_n$ oder Carbonyl steht,

Y für Carbonyl oder $(CH_2)_n$ steht,

Z für Stickstoff steht, und

n für 0 - 2 steht.

**9.** Verbindungen gemäß Anspruch 1, wobei

$R^1$ für Wasserstoff steht,

$R^2$ für tertiär Butyl, Cyano, Brom, oder für die Gruppe $-O-CF_3$, $-SO_2-CH_3$ steht und in para-Stellung steht, und

$R^3$ für die Gruppe

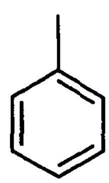 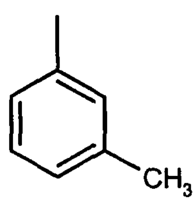 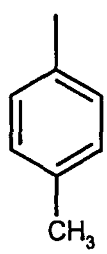 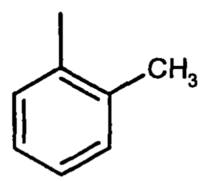 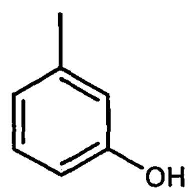

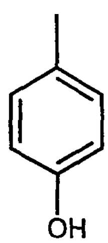 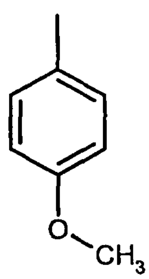 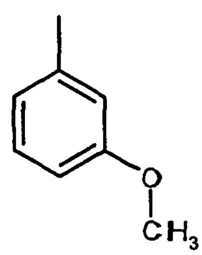 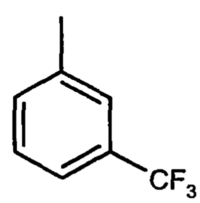 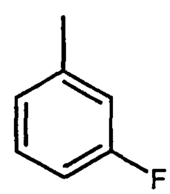

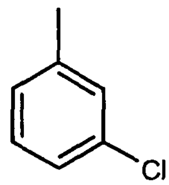 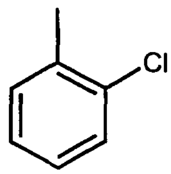 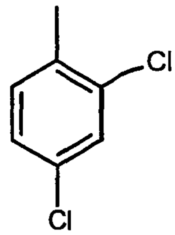 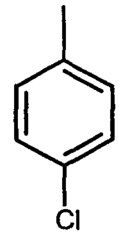

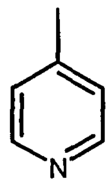 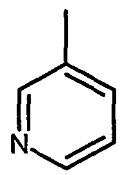 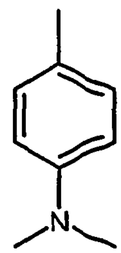

steht,

$R^4$ für Wasserstoff, $C_3$-$C_6$-Cycloalkyl, welches gegebenenfalls ein- oder mehrfach, gleich oder verschieden mit $C_1$-$C_3$-Alkyl, $C_1$-$C_3$-Acyl, $C_1$-$C_3$-Alkoxy oder $CF_3$ substituiert ist, für $C_6$-$C_{12}$-Aryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano substituiert ist, oder für $C_5$-$C_{12}$-Heteroaryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano, substituiert ist, oder für $C_1$-$C_6$-Alkyl, welches beliebig substituiert sein kann, steht,

$R^5$ für Wasserstoff, $C_3$-$C_6$-Cycloalkyl, welches gegebenenfalls ein- oder mehrfach, gleich oder verschieden mit $C_1$-$C_3$-Alkyl, $C_1$-$C_3$-Acyl, $C_1$-$C_3$-Alkoxy oder $CF_3$ substituiert ist, für $C_6$-$C_{12}$-Aryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano substituiert ist, oder für $C_5$-$C_{12}$-Heteroaryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano, substituiert ist, oder für $C_1$-$C_6$-Alkyl, welches beliebig substituiert sein kann, steht,

X für Sulfonyl, Carbonyl oder für die Gruppe $CH_2$ steht,

Y für Carbonyl oder für die Gruppe $(CH_2)n$ steht,

Z für Stickstoff bzw. für

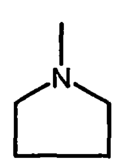 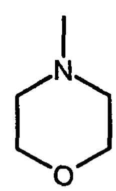

steht, und

n 1-2 steht.

**10.** Verbindungen gemäß Anspruch 1, wobei

$R^1$ für Wasserstoff, Halogen, $CF_3$, $C_3$-$C_6$-Cycloalkyl, welches gegebenenfalls mehrfach gesättigt und gegebenenfalls mehrfach substituiert ist, oder für die Gruppe $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl, $C_1$-$C_6$-Aryl-$C_1$-$C_6$-Alkyl oder $CF_3$, in der $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl oder $C_1$-$C_6$-Aryl-$C_1$-$C_6$-Alkyl gegebenenfalls ein- oder mehrfach, gleich oder verschieden durch Sauerstoff, Schwefel oder Stickstoff unterbrochen sein können, oder für die Gruppe Sulfonyl-$C_1$-$C_6$-Alkyl, Sulfonamid, oder Cyano steht,

$R^2$ Halogen, $CF_3$, $C_3$-$C_6$-Cycloalkyl, welches gegebenenfalls mehrfach gesättigt und gegebenenfalls mehrfach substituiert ist, oder für die Gruppe $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl, $C_1$-$C_6$-Aryl-$C_1$-$C_6$-Alkyl oder $CF_3$, in der $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl oder $C_1$-$C_6$-Aryl-$C_1$-$C_6$-Alkyl gegebenenfalls ein- oder mehrfach, gleich oder verschieden durch Sauerstoff, Schwefel oder Stickstoff unterbrochen sein können, oder für die Gruppe Sulfonyl-$C_1$-$C_6$-Alkyl, Sulfonamid, oder Cyano steht,

$R^3$ für $C_6$-$C_{12}$-Aryl, welches gegebenenfalls ein- oder mehrfach, gleich oder verschieden mit Halogen, mit $C_1$-$C_6$-Alkyl, $C_1$-$C_3$-Acyl, $C_1$-$C_3$-Alkoxy, Cyano, Hydroxy, N-$(CH_3)_2$, $CO_2$-($C_1$-$C_3$-Alkyl), CO-$NR^4R^5$ oder mit $CF_3$ substituiert sein kann, $C_5$-$C_{12}$-Heteroaryl, welches gegebenenfalls ein- oder mehrfach, gleich oder verschieden mit Chlor und / oder Fluor, mit $C_1$-$C_6$-Alkyl, $C_1$-$C_3$-Acyl, $C_1$-$C_3$-Alkoxy, Cyano, Hydroxy, N-$(CH_3)_2$, $CO_2$-($C_1$-$C_3$-Alkyl), CO-$NR^4R^5$ oder mit $CF_3$ substituiert sein kann, $C_3$-$C_6$-Cycloalkyl, welches gegebenenfalls ein- oder mehrfach, gleich oder verschieden mit Chlor und / oder Fluor, $CF_3$, Cyano, $C_1$-$C_3$-Alkyl, $C_1$-$C_3$-Acyl, Hydroxy, N-$(CH_3)_2$, $CO_2$-($C_1$-$C_3$-Alkyl), CO-$NR^4R^5$ oder $C_1$-$C_3$-Alkoxy substituiert sein kann, steht,

$R^4$ für Wasserstoff oder für die Gruppe $-(CH_2)_n-N-(CH_3)_2$, $-(CH_2)_2-CH_3$, $-(CH_2)_2-NH-COCH_3$, $-(CH_2)-CHCH_3-OH$, $-(CH_2)_2-O-CH_3$, $-(Ch_2)_2-OH$, $- CHCH_3-CH_2-OH$,

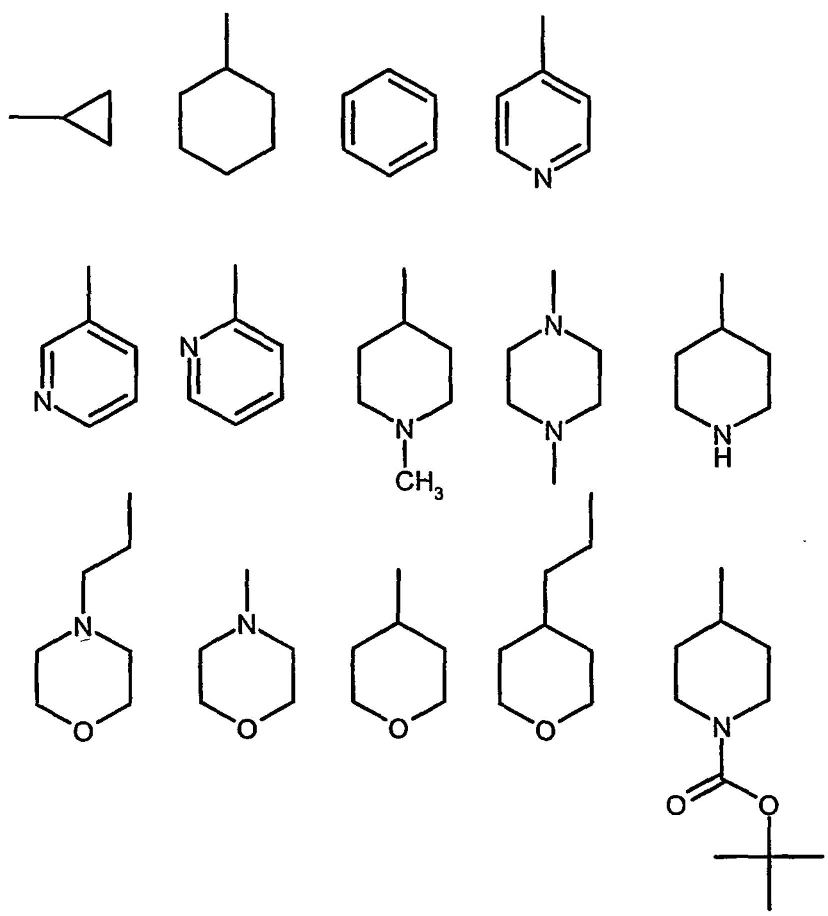

steht,

$R^5$ für Wasserstoff steht

X für die Gruppen Sulfonyl, $(CH_2)_n$ oder Carbonyl steht,

Y für Carbonyl oder $(CH_2)_n$ steht,

Z für Stickstoff steht, und

n für 0 - 2 steht.

**11.** Verbindungen gemäß Anspruch 1, wobei

$R^1$ für Wasserstoff, Halogen, $CF_3$, $C_3$-$C_6$-Cycloalkyl, welches gegebenenfalls mehrfach gesättigt und gegebenenfalls mehrfach substituiert ist, oder für die Gruppe $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl, $C_1$-$C_6$-Aryl-$C_1$-$C_6$-Alkyl oder $CF_3$, in der $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl oder $C_1$-$C_6$-Aryl-$C_1$-$C_6$-Alkyl gegebenenfalls ein- oder mehrfach, gleich oder verschieden durch Sauerstoff, Schwefel oder Stickstoff unterbrochen sein können, oder für die Gruppe Sulfonyl-$C_1$-$C_6$-Alkyl, Sulfonamid, oder Cyano steht,

$R^2$ Halogen, $CF_3$, $C_3$-$C_6$-Cycloalkyl, welches gegebenenfalls mehrfach gesättigt und gegebenenfalls mehrfach substituiert ist, oder für die Gruppe $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl, $C_1$-$C_6$-Aryl-$C_1$-$C_6$-Alkyl oder $CF_3$, in der $C_1$-$C_6$-Alkyl, $C_1$-$C_6$-Aryl, $C_1$-$C_6$-Acyl, Halo-$C_1$-$C_6$-Alkyl, $C_1$-$C_6$-Alkyl-$C_1$-$C_6$-Alkyl, $C_1$-$C_6$-Alkyl-$C_1$-$C_6$-Acyl, $C_1$-$C_6$-Acyl-$C_1$-$C_6$-Acyl, $C_1$-$C_6$-Alkyl-$C_1$-$C_6$-Aryl oder $C_1$-$C_6$-Aryl-$C_1$-$C_6$-Alkyl gegebenenfalls ein- oder mehrfach, gleich oder verschieden durch Sauerstoff, Schwefel oder Stickstoff unterbrochen sein können, oder für die Gruppe Sulfonyl-$C_1$-$C_6$-Alkyl, Sulfonamid, oder Cyano steht,

$R^3$ für $C_6$-$C_{12}$-Aryl, welches gegebenenfalls ein- oder mehrfach, gleich oder verschieden mit Halogen, mit $C_1$-$C_6$-Alkyl, $C_1$-$C_3$-Acyl, $C_1$-$C_3$-Alkoxy, Cyano, Hydroxy, N-$(CH_3)_2$, $CO_2$-($C_1$-$C_3$-Alkyl), CO-$NR^4R^5$ oder mit $CF_3$ substituiert sein kann, $C_5$-$C_{12}$-Heteroaryl, welches gegebenenfalls ein- oder mehrfach, gleich oder verschieden mit Chlor und / oder Fluor, mit $C_1$-$C_6$-Alkyl, $C_1$-$C_3$-Acyl, $C_1$-$C_3$-Alkoxy, Cyano, Hydroxy, N-$(CH_3)_2$, $CO_2$-($C_1$-$C_3$-Alkyl), CO=$NR^4R^5$ oder mit $CF_3$ substituiert sein kann, $C_3$-$C_6$-Cycloalkyl, welches gegebenenfalls ein- oder mehrfach, gleich oder verschieden mit Chlor und / oder Fluor, $CF_3$, Cyano, $C_1$-$C_3$-Alkyl, $C_1$-$C_3$-Acyl, Hydroxy, N-$(CH_3)_2$, $CO_2$-($C_1$-$C_3$-Alkyl), CO-$NR^4R^5$ oder $C_1$-$C_3$-Alkoxy substituiert sein kann, steht,

$R^4$ für Wasserstoff, $C_3$-$C_6$-Cycloalkyl, welches gegebenenfalls ein- oder mehrfach, gleich oder verschieden mit $C_1$-$C_3$-Alkyl, $C_1$-$C_3$-Acyl, $C_1$-$C_3$-Alkoxy oder $CF_3$ substituiert ist, für $C_6$-$C_{12}$-Aryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano substituiert ist, oder für $C_5$-$C_{12}$-Heteroaryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano, substituiert ist, oder für $C_1$-$C_6$-Alkyl, welches beliebig substituiert sein kann, steht,

$R^5$ für Wasserstoff, $C_3$-$C_6$-Cycloalkyl, welches gegebenenfalls ein- oder mehrfach, gleich oder verschieden mit $C_1$-$C_3$-Alkyl, $C_1$-$C_3$-Acyl, $C_1$-$C_3$-Alkoxy oder $CF_3$ substituiert ist, für $C_6$-$C_{12}$-Aryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano substituiert ist, oder für $C_5$-$C_{12}$-Heteroaryl, welches gegebenenfalls ein- oder mehrfach, gleich oder verschieden mit Halogen, mit $C_1$-$C_3$-Alkyl, $C_1$-$C_3$-Acyl, $C_1$-$C_3$-Alkoxy, N-$C_1$-$C_3$-Alkyl-$C_1$-$C_3$-Alkyl, $CF_3$ oder Cyano, substituiert ist, oder für $C_1$-$C_6$-Alkyl, welches beliebig substituiert sein kann, steht,

$R^4$ und $R^5$ gemeinsam einen 5-8 gliedrigen Ring bilden, der weitere Heteroatome enthalten kann, steht,

X für die Gruppen Sulfonyl, $(CH_2)_n$ oder Carbonyl steht,

Y für Carbonyl oder $(CH_2)_n$ steht,

Z für Stickstoff bzw. für

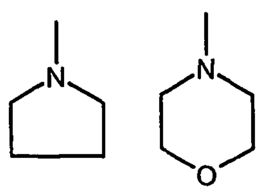

steht und

n für 0 - 2 steht.

**12.** Verbindungen gemäß den Ansprüchen 1- 11 ausgewählt aus der Gruppe, die folgende Verbindungen enthalten:

1. 5-(4-tert-Butylbenzolsulfonylamino)-3-phenyl-1*H*-indol-2-carbonsäurecyclopropylamid
2. 5-(4-tert-Butylbenzolsulfonylamino)-3-phenyl-1*H*-indol-2-carbonsäure-pyridin-3-ylamid
3. 5-(4-tert-Butylbenzolsulfonylamino)-3-phenyl-1*H*-indol-2-carbonsäurecyclohexylamid
4. 4-tert-Butyl-N-[3-phenyl-2-(pyrrolidin-1-carbonyl)-1*H*-indol-5-yl]-benzolsulfonamid
5. 4-tert-Butyl-N-[2-(morpholin-4-carbonyl)-3-phenyl-1*H*-indol-5-yl]-benzolsulfonamid
6. 5-(4-tert-Butylbenzolsulfonylamino)-3-phenyl-1*H*-indol-2-carbonsäure-(2-dimethylaminoethyl)amid
7. 5-(4-Cyanobenzolsulfonylamino)-3-phenyl-1*H*-indol-2-carbonsäurepropylamid

8. 5-(4-Brombenzolsulfonylamino)-3-phenyl-1*H*-indol-2-carbonsäurepropylamid
9. 5-(4-tert-Butylbenzolsulfonylamino)-3-phenyl-1*H*-indol-2-carbonsäurepropylamid
10. 5-(4-(Trifluormethoxy)benzolsulfonylamino)-3-phenyl-1*H*-indol-2-carbonsäurepropylamid
11. 5-(4-(Methylsulfonyl)benzolsulfonylamino)-3-phenyl-1*H*-indol-2-carbonsäurepropylamid
12.5-(4-tert-Butylbenzolsulfonylamino)-3-phenyl-1*H*-indol-2-carbonsäurephenylamid
13.5-(4-Cyanobenzolsulfonylamino)-3-phenyl-1*H*-indol-2-carbonsäurephenylamid
14.5-(4-Brombenzolsulfonylamino)-3-phenyl-1*H*-indol-2-carbonsäurephenylamid
15. 5-(4-(Trifluormethoxy)benzolsulfonylamino)-3-phenyl-1*H*-indol-2-carbonsäurephenylamid
16.5-(4-(Methylsulfonyl)benzolsulfonylamino)-3-phenyl-1*H*-indol-2-carbonsäurephenylamid
17.5-(4-tert-Butylbenzolsulfonylamino-3-phenyl-1*H*-indol-2-carbonsäure-pyridin-2-ylamid
18.5-(4-Cyanobenzolsulfonylamino)-3-phenyl-1*H*-indol-2-carbonsäure-pyridin-2-ylamid
19.5-(4-tert-Butylbenzolsulfonylamino)-3-phenyl-1*H*-indol-2-carbonsäure-(2-morpholin-4-ylethyl)amid
20.5-(4-tert-Butylbenzolsulfonylamino-3-phenyl-1H-indol-2-carbonsäure-(4-methylpiperazin-1-yl)amid
21.5-(4-tert-Butylbenzolsulfonylamino)-3-phenyl-1*H*-indol-2-carbonsäurepyrindin-4-ylamid
22.5-(4-tert-Butylbenzylamino)-3-phenyl-1*H*-indol-2carbonsäurepyridin-4-ylamid
23.5-(4-tert-Butylbenzoylamino-3-phenyl-1*H*-indol-2-carbonsäure-(2-dimethylaminoethyl)amid
24.5-(4- tert- Butylbenzolsulfonylamino)- 3-(2- chlorophenyl)- 1*H*-indol- 2- carbonsäure-(2- dimethylaminoethyl) amid
25.5-(4- tert- Butylbenzolsulfonylamino)- 3-(3- chlorphenyl)- 1*H*-indol- 2- carbonsäure-(2- dimethylaminoethyl) amid
26.5-(4-tert-Butylbenzolsulfonylamino-3-(4-chlorphenyl)-1*H*-indol-2-carbonsäure-(2-dimethylaminoethyl)amid
27.5-(4-tert-Butylbenzolsulfonylamino-3-(2,4-dichlorphenyl)-1H-indol-2-carbonsäure-(2-dimethylaminoethyl) amid
28.5-(4-tert- Butylbenzolsulfonylamino)- 3-(2- methylphenyl)- 1H- indol- 2- carbonsäure-(2- dimethylaminoethyl) amid
29.5-(4- tert- Butylbenzolsulfonylamino)- 3-(4- methylphenyl)- 1*H*-indol- 2- carbonsäure-(2- dimethylaminoethyl) amid
30.5-(4-tert-Butylbenzolsulfonylamino)-3-(4-methylphenyl)-1*H*-indol-2-carbonsäurepyridin-4ylamid
31.5-(4-tert-Butylbenzolsulfonylamino)-3-(4-methoxyphenyl)-1*H*-indol-2-carbonsäure-(2-dimethylaminoethyl) amid
32.5-(4-tert-Butylbenzolsulfonylamino)-3-pyridin-3-yl-1*H*-indol-2-carbon-säure-(2-dimethylaminoethyl)amid
33.5-(4-tert-Butylbenzolsulfonylamino)-3-(4-hydroxyphenyl)-1*H*-indol-2-carbonsäure-(2-dimethylaminoethyl) amid
34.5-(4-tert-Butylbenzolsulfonylamino)-3-(3-fluorphenyl)-1*H*-indol-2-carbonsäure-(2-dimethylaminoethyl)amid
35.5-(4-tert-Butylbenzolsulfonylamino)-3-phenyl-1*H*-indol-2-carbonsäure-(2-hydroxypropyl)amid
36.5-(4-tert-Butylbenzolsulfonylamino)-3-phenyl-1*H*-indol-2-carbonsäure-(2-methoxyethyl)amid
37.5-(4-tert-Butylbenzolsulfonylamino)-3-phenyl-1*H*-indol-2-carbonsäure-(2-hydroxyethyl)amid
38.5-(4-tert-Butylbenzolsulfonylamino)-3-phenyl-1*H*-indol-2-carbonsäure-(2-hydroxy-1-methylethyl)amid
39.5-(4-tert-Butylbenzolsulfonylamino)-3-phenyl-1*H*-indol-2-carbonsäure-(2-acetylaminoethyl)amid
40.5-(4-tert-Butylbenzolsulfonylamino)-3-phenyl-1*H*-indol-2-carbonsäure-(tetrahydropyran-4-yl)amid
41.5-(4-tert-Butylbenzolsulfonylamino)-3-phenyl-1*H*-indol-2-carbonsäure-(1-methylpiperidin-4-yl)amid
42.5-(4-tert-Butylbenzolsulfonylamino)-3-(4-N,N-dimethylaminophenyl)-1*H*-indol-2-carbonsäure-(2-dimethylaminoethyl)amid
43.5-(4-tert-Butylbenzolsulfonylamino)-3-(3-methoxyphenyl)-1*H*-indol-2-carbonsäure-(2-dimethylaminoethyl) amid
44.5-(4-tert-Butylbenzolsulfonylamino)-3-(3-trifluormethylphenyl)-1*H*-indol-2-carbonsäure-(2-dimethylaminoethyl)amid
45.5-(4-tert-Butylbenzolsulfonylamino)-3-(3-fluorphenyl)-1*H*-indol-2-carbonsäure-(2-hydroxyethyl)amid
46.5-(4-tert-Butylbenzolsulfonylamino)-3-(3-fluorphenyl)-1*H*-indol-2-carbonsäure-(tetrahydropyran-4-yl)amid
47.5-(4-tert-Butylbenzolsulfonylamino)-3-(3-fluorphenyl)-1*H*-indol-2-carbonsäure-(2-acetylaminoethyl)amid
48. 5-(4-tert-Butylbenzolsulfonylamino)-3-(3-fluorphenyl)-1*H*-indol-2-carbonsäure-(2-morpholin-4-ylethyl)amid
49. 5-(4-tert-Butylbenzolsulfonylamino)-3-(3-methoxyphenyl)-1*H*-indol-2-carbonsäure-(2-hydroxyethyl)amid
50. 5-(4-tert-Butylbenzolsulfonylamino)-3-(3-methoxyphenyl)-1*H*-indol-2-carbonsäure-(2-acetylaminoethyl) amid
51. 5-(4-tert-Butylbenzolsulfonylamino)-3-pyridin-3-yl-1*H*-indol-2-carbon-säure-(2-acetylaminoethyl)amid
52. 5-(4-tert-Butylbenzolsulfonylamino)-3-pyridin-3-yl-1*H*-indol-2-carbon-säure-(tetrahydropyran-4yl)amid
53. 5-(4-tert-Butylbenzolsulfonylamino)-3-pyridin-4-yl-1*H*-indol-2-carbon-säure-(2-acetylaminoethyl)amid
54. 5-(4-tert-Butylbenzolsulfonylamino)-3-pyridin-4-yl-1*H*-indol-2-carbon-säure-(2-morpholin-4-ylethyl)amid

55. 5-(4-tert-Butylbenzolsulfonylamino)-3-pyridin-4-yl-1*H*-indol-2-carbon-säure-(tetrahydropyran-4-yl)amid
56. 5-(4-tert-Butylbenzolsulfonylamino)-3-(3-methylphenyl)-1*H*-indol-2-carbonsäure-(2-acetylaminoethyl)amid
57. 5-(4-tert-Butylbenzolsulfonylamino)-3-(3-methylphenyl)-1*H*-indol-2-carbonsäure-(2-hydroxyethyl)amid
58. 5-(4-tert-Butylbenzolsulfonylamino)-3-(3-methylphenyl)-1*H*-indol-2-carbonsäure-(2-morpholin-4-ylethyl) amid
59. 5-(4-tert-Butylbenzolsulfonylamino)-3-(3-methylphenyl)-1*H*-indol-2-carbonsäure-(tetrahydropyran-4-yl) amid
60. 5-(4-tert-Butylbenzolsulfonylamino)-3-pyridin-3-yl-1*H*-indol-2-carbon-säure-(2-morpholin-4-ylethyl)amid
61.5-(4-tert-Butylbenzolsulfonylamino)-3-(3-methoxyphenyl)-1*H*-indol-2-carbonsäure-(tetrahydropyran-4-yl) amid
62.5-(4-tert-Butylbenzolsulfonylamino)-3-(3-methoxyphenyl)-1*H*-indol-2-carbonsäure-(2-morpholin-4-ylethyl) amid
63. 5-(4-tert-Butylbenzolsulfonylamino)-3-phenyl-1H-indol-2-carbonsäurepiperidin-4-ylamid
64.4-{[5-(4-tert-Butylbenzolsulfonylamino)-3-phenyl-1H-indol-2carbonyl]-amino}piperidin-1-carbonsäure-tert-butylester
65. 5-(4-tert-Butyl-phenylsulfonyl-amino)-3-naphthalin-1-yl-1H-indol-2-carbonsäure-(2-morpholin-4-yl-ethyl)-amid
66.5-(4-tert-Butyl-phenylsulfonyl-amino)-3-m-tolyl-1H-indol-2-carbonsäure-(2-morpholin-4-yl-ethyl)-amid
67.5-(4-tert-Butyl-phenylsulfonyl-amino)-3-thiophen-2-yl-1H-indol-2-carbonsäure-(2-morpholin-4-yl-ethyl)-amid
68.5-(4-tert-Butyl-phenylsulfonyl-amino)-3-thiophen-3-yl-1H-indol-2-carbonsäure-(2-morpholin-4-yl-ethyl)-amid
69.3-Benzofuran-2-yl-5-(4-tert-butyl-phenylsulfonylamino)-1 H-indol-2-carbonsäure-(2-morpholin-4-yl-ethyl)-amid
70.5-(4-tert-Butyl-phenylsulfonyl-amino)-3-(5-chlor-thiophen-2-yl)-1H-indol-2-carbonsäure-(2-morpholin-4-yl-ethyl)-amid
71.5-(4-tert-Butyl-phenylsulfonyl-amino)-3-furan-2-yl-1H-indol-2-carbonsäure-(2-morpholin-4-yl-ethyl)-amid
72.5-(4-tert-Butyl-phenylsulfonyl-amino)-3-(3-fluor-4-methoxy-phenyl)-1H-indol-2-carbonsäure-(2-morpholin-4-yl-ethyl)-amid
73.3-Benzo[1,3]dioxol-5-yl-5-(4-tert-butyl-phenylsulfonylamino)-1H-indol-2-carbonsäure-(2-morpholin-4-yl-ethyl)-amid
74.3-(4-Acetyl-phenyl)-5-(4-tert-butyl-phenylsulfonylamino)-1H-indol-2-carbonsäure-(2-morpholin-4-yl-ethyl)-amid
75.3-(3-Acetyl-phenyl)-5-(4-tert-butyl-phenylsulfonylamino)-1H-indol-2-carbonsäure-(2-morpholin-4-yl-ethyl)-amid
76. 3-Benzo[b]thiophen-2-yl-5-(4-tert-butyl-phenylsulfonylamino)-1 H-indol-2-carbonsäure-(2-morpholin-4-yl-ethyl)-amid
77.3-Benzo[b]thiophen-3-yl-5-(4-tert-butyl-phenylsulfonylamino)-1H-indol-2-carbonsäure-(2-morpholin-4-yl-ethyl)-amid
78.5-(4-tert-Butyl-phenylsulfonyl-amino)-3-(5-methyl-thiophen-2-yl)-1H-indol-2-carbonsäure-(2-morpholin-4-yl-ethyl)-amid
79.3-[5-(4-tert-Butyl-phenylsulfonyl-amino)-2-(2-morpholin-4-yl-ethylcarbamoyl)-1 H-indol-3-yl]-benzoesäure methylester
80.5-(4-tert-Butyl-phenylsulfonyl-amino)-3-(2-fluor-3-methoxy-phenyl)-1 H-indol-2-carbonsäure-(2-morpholin-4-yl-ethyl)-amid
81.5-(4-tert-Butyl-phenylsulfonyl-amino)-3-(3-chlor-4-methyl-phenyl)-1H-indol-2-carbonsäure-(2-morpholin-4-yl-ethyl)-amid
82.5-(4-tert-Butyl-phenylsulfonyl-amino)-3-(2,4-dimethoxy-pyrimidin-5-yl)-1 H-indol-2-carbonsäure-(2-morpholin-4-yl-ethyl)-amid
83.5-(4-tert-Butyl-phenylsulfonyl-amino)-3-(2,5-difluor-phenyl)-1H-indol-2-carbonsäure-(2-morpholin-4-yl-ethyl)-amid
84.5-(4-tert-Butyl-phenylsulfonyl-amino)-3-(2,4-difluor-phenyl)-1H-indol-2-carbonsäure-(2-morpholin-4-yl-ethyl)-amid
85.5-(4-tert-Butyl-phenylsulfonyl-amino)-3-(2,3-difluor-phenyl)-1H-indol-2-carbonsäure-(2-morpholin-4-yl-ethyl)-amid
86.5-(4-tert-Butyl-phenylsulfonyl-amino)-3-(2,6-difluor-phenyl)-1H-indol-2-carbonsäure-(2-morpholin-4-yl-ethyl)-amid
87.5-(4-tert-Butyl-phenylsulfonyl-amino)-3-(3-hydroxy-phenyl)-1H-indol-2-carbonsäure-(2-morpholin-4-yl-ethyl)-amid

88.5-(4-tert-Butyl-phenylsulfonyl-amino)-3-(4-hydroxy-phenyl)-1H-indol-2-carbonsäure-(2-morpholin-4-yl-ethyl)-amid

89.5-(4-tert-Butyl-phenylsulfonyl-amino)-3-(3-fluor-4-methyl-phenyl)-1H-indol-2-carbonsäure-(2-morpholin-4-yl-ethyl)-amid

90.5-(4-tert-Butyl-phenylsulfonyl-amino)-3-(4-trifluormethyl-phenyl)-1H-indol-2-carbonsäure-(2-morpholin-4-yl-ethyl)-amid

91.5-(4-tert-Butyl-phenylsulfonyl-amino)-3-(4-cyanomethyl-phenyl)-1H-indol-2-carbonsäure-(2-morpholin-4-yl-ethyl)-amid

92.5-(4-tert-Butyl-phenylsulfonyl-amino)-1H,1'H-[3,4']biindolyl-2-carbonsäure-(2-morpholin-4-yl-ethyl)-amid

93.5-(4-tert-Butyl-phenylsulfonyl-amino)-3-(3-cyano-4-fluor-phenyl)-1H-indol-2-carbonsäure-(2-morpholin-4-yl-ethyl)-amid

94.5-(4-tert-Butyl-phenylsulfonyl-amino)-3-(2-fluor-phenyl)-1H-indol-2-carbonsäure-(2-morpholin-4-yl-ethyl)-amid

95.5-(4-tert-Butyl-phenylsulfonyl-amino)-3-(3,4-difluor-phenyl)-1H-indol-2-carbonsäure-(2-morpholin-4-yl-ethyl)-amid

96.5-(4-tert-Butyl-phenylsulfonyl-amino)-3-(3-cyano-phenyl)-1H-indol-2-carbonsäure-(2-morpholin-4-yl-ethyl)-amid

97.5-(4-tert-Butyl-phenylsulfonyl-amino)-3-(4-cyano-phenyl)-1H-indol-2-carbonsäure-(2-morpholin-4-yl-ethyl)-amid

98.5-(4-tert-Butyl-phenylsulfonyl-amino)-3-(4-methyl-thiophen-2-yl)-1 H-indol-2-carbonsäure-(2-morpholin-4-yl-ethyl)-amid

99.5-(4-tert-Butyl-phenylsulfonyl-amino)-3-phenyl-1H-indol-2-carbonsäure-(3-chloro-phenyl)-amid

100. 5-(4-tert-Butyl-phenylsulfonyl-amino)-3-phenyl-1H-indol-2-carbonsäure-(3-methyl-isoxazol-5-yl)-amid

101. 5-(4-tert-Butyl-phenylsulfonyl-amino)-3-phenyl-1H-indol-2-carbonsäure-(4-fluoro-phenyl)-amid

102. 5-(4-tert-Butyl-phenylsulfonyl-amino)-3-phenyl-1H-indol-2-carbonsäure-(2-fluoro-phenyl)-amid

103. 5-(4-tert-Butyl-phenylsulfonyl-amino)-3-phenyl-1H-indol-2-carbonsäure-(6-methyl-pyridin-2-yl)-amid

104. 5-(4-tert-Butyl-phenylsulfonyl-amino)-3-phenyl-1H-indol-2-carbonsäure-(5-carbamoyl-pyridin-2-yl)-amid

105. 5-(4-tert-Butyl-phenylsulfonyl-amino)-3-phenyl-1H-indol-2-carbonsäure-(4-hydroxy-phenyl)-amid

106. 5-(4-tert-Butyl-phenylsulfonyl-amino)-3-phenyl-1H-indol-2-carbonsäure-(2-methoxy-phenyl)-amid

107. 5-(4-tert-Butyl-phenylsulfonyl-amino)-3-phenyl-1H-indol-2-carbonsäure-(3-methoxy-phenyl)-amid

108. 5-(4-tert-Butyl-phenylsulfonyl-amino)-3-phenyl-1H-indol-2-carbonsäure-(4-methoxy-phenyl)-amid

109. 5-(4-tert-Butyl-phenylsulfonyl-amino)-3-phenyl-1H-indol-2-carbonsäure-(4-chloro-phenyl)-amid

110. 5-(4-tert-Butyl-phenylsulfonyl-amino)-3-phenyl-1H-indol-2-carbonsäure-(4-dimethylamino-phenyl)-amid

111. 5-(4-tert-Butyl-phenylsulfonyl-amino)-3-phenyl-1H-indol-2-carbonsäure-(5-chlor-pyridin-2-yl)-amid

112. 5-(4-tert-Butyl-phenylsulfonyl-amino)-3-phenyl-1H-indol-2-carbonsäure-p-tolylamid

113. 5-(4-tert-Butyl-phenylsulfonyl-amino)-3-phenyl-1 H-indol-2-carbonsäurepyrazin-2-ylamid

114. 5-(4-tert-Butyl-phenylsulfonyl-amino)-3-phenyl-1H-indol-2-carbonsäure-(4-cyano-phenyl)-amid

115. 5-(4-tert-Butyl-phenylsulfonyl-amino)-3-phenyl-1H-indol-2-carbonsäure-(3-methyl-isothiazol-5-yl)-amid

116. 5-(4-tert-Butyl-phenylsulfonyl-amino)-3-phenyl-1H-indol-2-carbonsäure-(4-brom-phenyl)-amid

117. 5-(4-tert-Butyl-phenylsulfonyl-amino)-3-phenyl-1H-indol-2-carbonsäure-(4-carbamoyl-phenyl)-amid

118. 5-(4-tert-Butyl-phenylsulfonyl-amino)-3-phenyl-1H-indol-2-carbonsäure-(3-methyl-pyridin-2-yl)-amid

119. 5-(4-tert-Butyl-phenylsulfonyl-amino)-3-phenyl-1H-indol-2-carbonsäure-(2-chlor-phenyl)-amid

120. 5-(4-tert-Butyl-phenylsulfonyl-amino)-3-phenyl-1H-indol-2-carbonsäure-(5-methyl-2H-pyrazol-3-yl)-amid

121. 5-(4-tert-Butyl-phenylsulfonyl-amino)-3-phenyl-1H-indol-2-carbonsäurequinolin-5-ylamid

122. 5-(4-tert-Butyl-phenylsulfonyl-amino)-3-phenyl-1H-indol-2-carbonsäurequinolin-6-ylamid

123. 5-(4-tert-Butyl-phenylsulfonyl-amino)-3-phenyl-1H-indol-2-carbonsäure-(2,6-dichlor-pyridin-4-yl)-amid

124. 5-(4-tert-Butyl-phenylsulfonyl-amino)-3-phenyl-1H-indol-2-carbonsäure-(3-fluor-phenyl)-amid

125. 5-(4-tert-Butyl-phenylsulfonyl-amino)-3-phenyl-1 H-indol-2-carbonsäure-(2-methyl-pyridin-4-yl)-amid

126. 5-(4-tert-Butyl-phenylsulfonyl-amino)-3-phenyl-1H-indol-2-carbonsäure-(3-fluor-pyridin-4-yl)-amid

127. 5-(4-tert-Butyl-phenylsulfonyl-amino)-3-phenyl-1H-indol-2-carbonsäure-(3-methyl-pyridin-4-yl)-amid

128. 5-(4-tert-Butyl-phenylsulfonyl-amino)-3-phenyl-1H-indol-2-carbonsäure-(3-brom-pyridin-4-yl)-amid

129. 5-(4-tert-Butyl-phenylsulfonyl-amino)-3-phenyl-1H-indol-2-carbonsäure-(2,3-dihydroxy-propyl)-amid

130. 5-(4-tert-Butyl-phenylsulfonyl-amino)-3-phenyl-1H-indol-2-carbonsäure-(2-oxo-tetrahydro-thiophen-3-yl)-amid

131. 5-(4-tert-Butyl-phenylsulfonyl-amino)-3-phenyl-1H-indol-2-carbonsäure-[2-(2-oxo-imidazolidin-1-yl)-ethyl]-amid

132. 5-(4-tert-Butyl-phenylsulfonyl-amino)-3-phenyl-1H-indol-2-carbonsäure-(2,2-diethoxy-ethyl)-amid

133. 5-(4-tert-Butyl-phenylsulfonyl-amino)-3-phenyl-1H-indol-2-carbonsäure-(3-ethoxy-propyl)-amid
134. 5-(4-tert-Butyl-phenylsulfonyl-amino)-3-phenyl-1 H-indol-2-carbonsäure-(3-isopropoxy-propyl)-amid
135. 5-(4-tert-Butyl-phenylsulfonyl-amino)-3-phenyl-1H-indol-2-carbonsäure-(3-morpholin-4-yl-propyl)-amid
136. 5-(4-tert-Butyl-phenylsulfonyl-amino)-3-phenyl-1H-indol-2-carbonsäure-(3-diethylamino-propyl)-amid
137. 5-(4-tert-Butyl-phenylsulfonyl-amino)-3-phenyl-1 H-indol-2-carbonsäure-(3-dimethylamino-propyl)-amid
138. 5-(4-tert-Butyl-phenylsulfonyl-amino)-3-phenyl-1H-indol-2-carbonsäure-(furan-2-ylmethyl)-amid
139. 5-(4-tert-Butyl-phenylsulfonyl-amino)-3-phenyl-1H-indol-2-carbonsäure-(2-methylsulfanyl-ethyl)-amid
140. 5-(4-tert-Butyl-phenylsulfonyl-amino)-3-phenyl-1H-indol-2-carbonsäure-(2-diethylamino-ethyl)-amid
141. 5-(4-tert-Butyl-phenylsulfonyl-amino)-3-phenyl-1H-indol-2-carbonsäure-[2-(3,4-dimethoxy-phenyl)-ethyl]-amid
142. 5-(4-tert-Butyl-phenylsulfonyl-amino)-3-phenyl-1 H-indol-2-carbonsäure-(2-piperidin-1-yl-ethyl)-amid
143. 5-(4-tert-Butyl-phenylsulfonyl-amino)-3-phenyl-1H-indol-2-carbonsäure-(3-pyrrolidin-1-yl-propyl)-amid
144. 5-(4-tert-Butyl-phenylsulfonyl-amino)-3-phenyl-1H-indol-2-carbonsäurephenethyl-amid
145. 5-(4-tert-Butyl-phenylsulfonyl-amino)-3-phenyl-1H-indol-2-carbonsäure-(2-methoxy-1-methyl-ethyl)-amid
146. 5-(4-tert-Butyl-phenylsulfonyl-amino)-3-phenyl-1H-indol-2-carbonsäure-(pyridin-2-ylmethyl)-amid
147. 5-(4-tert-Butyl-phenylsulfonyl-amino)-3-phenyl-1H-indol-2-carbonsäure-(pyridin-3-ylmethyl)-amid
148. 5-(4-tert-Butyl-phenylsulfonyl-amino)-3-phenyl-1H-indol-2-carbonsäure-(pyridin-4-ylmethyl)-amid
149. 5-(4-tert-Butyl-phenylsulfonyl-amino)-3-phenyl-1H-indol-2-carbonsäure-(4-diethylamino-1-methyl-butyl)-amid
150. 5-(4-tert-Butyl-phenylsulfonyl-amino)-3-phenyl-1H-indol-2-carbonsäure-(2-imidazol-1-yl-ethyl)-amid
151. 5-(4-tert-Butyl-phenylsulfonyl-amino)-3-phenyl-1H-indol-2-carbonsäurebenzylamid
152. 5-(4-tert-Butyl-phenylsulfonyl-amino)-3-phenyl-1H-indol-2-carbonsäure-(2,2,2-trifluor-ethyl)-amid
153. 5-(4-tert-Butyl-phenylsulfonyl-amino)-3-phenyl-1H-indol-2-carbonsäure-4-methoxy-benzylamid
154. 5-(4-tert-Butyl-phenylsulfonyl-amino)-3-phenyl-1H-indol-2-carbonsäurecyclopentylamid
155. 5-(4-tert-Butyl-phenylsulfonyl-amino)-3-phenyl-1 H-indol-2-carbonsäure-(3-methyl-butyl)-amid
156. 5-(4-tert-Butyl-phenylsulfonyl-amino)-3-phenyl-1 H-indol-2-carbonsäure-[3-(4-methyl-piperazin-1-yl)-propyl]-amid
157. 5-(4-tert-Butyl-phenylsulfonyl-amino)-3-phenyl-1H-indol-2-carbonsäure-[2-(4-hydroxy-phenyl)-ethyl]-amid
158. 5-(4-tert-Butyl-phenylsulfonyl-amino)-3-phenyl-1H-indol-2-carbonsäure-[2-(4-chlor-phenyl)-ethyl]-amid
159. 5-(4-tert-Butyl-phenylsulfonyl-amino)-3-phenyl-1H-indol-2-carbonsäurecyclopropylamid
160. 5-(4-tert-Butyl-phenylsulfonyl-amino)-3-phenyl-1H-indol-2-carbonsäurecyclohexylmethyl-amid
161. 5-(4-tert-Butyl-phenylsulfonyl-amino)-3-phenyl-1H-indol-2-carbonsäure-(tetrahydro-furan-2-ylmethyl)-amid
162. 5-(4-tert-Butyl-phenylsulfonyl-amino)-3-phenyl-1H-indol-2-carbonsäure-(thiophen-2-ylmethyl)-amid
163. 5-(4-tert-Butyl-phenylsulfonyl-amino)-3-phenyl-1H-indol-2-carbonsäure-4-fluoro-benzylamid
164. 5-(4-tert-Butyl-phenylsulfonyl-amino)-3-phenyl-1H-indol-2-carbonsäure-(2-thiophen-2-yl-ethyl)-amid
165. 5-(4-tert-Butyl-phenylsulfonyl-amino)-3-phenyl-1H-indol-2-carbonsäure-(2-pyrrolidin-1-yl-ethyl)-amid
166. 5-(4-tert-Butyl-phenylsulfonyl-amino)-3-phenyl-1H-indol-2-carbonsäure-4-methyl-benzylamid
167. 5-(4-tert-Butyl-phenylsulfonyl-amino)-3-phenyl-1H-indol-2-carbonsäure-(1-ethyl-pyrrolidin-2-ylmethyl)-amid
168. 5-(4-tert-Butyl-phenylsulfonyl-amino)-3-phenyl-1H-indol-2-carbonsäure-(2-pyridin-3-yl-ethyl)-amid
169. 5-(4-tert-Butyl-phenylsulfonyl-amino)-3-phenyl-1H-indol-2-carbonsäure-3-chlor-benzylamid
170. 5-(4-tert-Butyl-phenylsulfonyl-amino)-3-phenyl-1H-indol-2-carbonsäure-[2-(3-chlor-phenyl)-ethyl]-amid
171. 5-(4-tert-Butyl-phenylsulfonyl-amino)-3-phenyl-1H-indol-2-carbonsäure-((R)-2-hydroxy-1-phenyl-ethyl)-amid
172. 5-(4-tert-Butyl-phenylsulfonyl-amino)-3-phenyl-1H-indol-2-carbonsäure-[3-(2-methyl-piperidin-1-yl)-propyl]-amid
173. 5-(4-tert-Butyl-phenylsulfonyl-amino)-3-phenyl-1H-indol-2-carbonsäure-(3-phenyl-propyl)-amid
174. 5-(4-tert-Butyl-phenylsulfonyl-amino)-3-phenyl-1H-indol-2-carbonsäure-(2-carbamoyl-ethyl)-amid
175. 5-(4-tert-Butyl-phenylsulfonyl-amino)-3-phenyl-1H-indol-2-carbonsäure-[3-(5-methyl-1 H-pyrazol-4-yl)-propyl]-amid
176. 5-(4-tert-Butyl-phenylsulfonyl.-amino)-3-phenyl-1H-indol-2-carbonsäure-(4-methyl-cyclohexyl)-amid
177. 5-(4-tert-Butyl-phenylsulfonyl-amino)-3-phenyl-1 H-indol-2-carbonsäure-((S)-2-methoxy-1-methyl-ethyl)-amid
178. 5-(4-tert-Butyl-phenylsulfonyl-amino)-3-phenyl-1H-indol-2-carbonsäurecyclopropylmethyl-amid
179. 5-(4-tert-Butyl-phenylsulfonyl.-amino)-3-phenyl-1H-indol-2-carbonsäurecarbamoylmethyl-amid
180. 5-(4-tert-Butyl-phenylsulfonyl-amino)-3-phenyl-1 H-indol-2-carbonsäurecycloheptylamid

181. 5-(4-tert-Butyl-phenylsulfonyl-amino)-3-phenyl-1 H-indol-2-carbonsäure-((R)-2-methoxy-1-methyl-ethyl)-amid
182. 5-(4-tert-Butyl-phenylsulfonyl-amino)-3-phenyl-1H-indol-2-carbonsäure-(furan-3-ylmethyl)-amid
183. 5-(4-tert-Butyl-phenylsulfonyl-amino)-3-phenyl-1H-indol-2-carbonsäure-3-fluor-benzylamid
184. 5-(4-tert-Butyl-phenylsulfonyl-amino-3-phenyl-1H-indol-2-carbonsäure-(5-methyl-pyrazin-2-ylmethyl)-amid
185. 5-(4-tert-Butyl-phenylsulfonyl-amino)-3-phenyl-1H-indol-2-carbonsäure-(2-pyridin-2-yl-ethyl)-amid
186. 5-(4-tert-Butyl-phenylsulfonyl-amino)-3-phenyl-1H-indol-2-carbonsäure-(2-phenoxy-ethyl)-amid
187. 5-(4-tert-Butyl-phenylsulfonyl-amino)-3-phenyl-1H-indol-2-carbonsäure-(2-benzoimidazol-1-yl-ethyl)-amid
188. 5-(4-tert-Butyl-phenylsulfonyl-amino)-3-phenyl-1H-indol-2-carbonsäure-(3-imidazol-1-yl-propyl)-amid
189. 5-(4-tert-Butyl-phenylsulfonyl-amino)-3-phenyl-1H-indol-2-carbonsäure-(1-benzyl-piperidin-4-yl)-amid
190. 5-(4-tert-Butyl-phenylsulfonyl-amino)-3-phenyl-1H-indol-2-carbonsäure-[3-(2-oxo-pyrrolidin-1-yl)-propyl]-amid
191. 5-(4-tert-Butyl-phenylsulfonyl-amino)-3-phenyl-1H-indol-2-carbonsäure-[2-(1-methyl-pyrrolidin-2-yl)-ethyl]-amid
192. 5-(4-tert-Butyl-phenylsulfonyl-amino)-3-phenyl-1H-indol-2-carbonsäure methyl-(2-morpholin-4-yl-ethyl)-amid

**13.** Arzneimittel, die mindestens eine der Verbindungen gemäß den Ansprüchen 1-12 enthalten.

**14.** Arzneimittel gemäß Anspruch 13 in der die Verbindung der allgemeinen Formel 1 in einer wirksamen Dosis enthalten ist.

**15.** Verwendung der Verbindungen der allgemeinen Formel 1 gemäß den Ansprüchen 1-12 für die Herstellung von Arzneimittels zur Behandlung von Erkrankungen, die verursacht werden durch Störungen im cAMP Stoffwechsel.

**16.** Verwendung der Verbindungen der allgemeinen Formel 1 gemäß den Ansprüchen 1-12 für die Herstellung von Arzneimittels für die nicht hormonelle Kontrazeption.

**17.** Verwendung der Verbindung der allgemeinen Formel 1 gemäß den Ansprüchen 1-12 für die Herstellung von Arzneimittels zur Inhibition der löslichen Adenylatzyklase.

**18.** Verbindungen gemäß Anspruch 1-12 als Arzneimittel gemäß Anspruch 13 und 14 mit geeigneten Formulierungs- und Trägerstoffen.

**19.** Verwendung der Verbindungen der allgemeinen Formel 1 gemäß Anspruch 1-12 zur Herstellung eines pharmazeutischen Präparates für die enterale, parenterale, vaginale und orale Applikation.

## Claims

**1.** Compounds of general formula 1

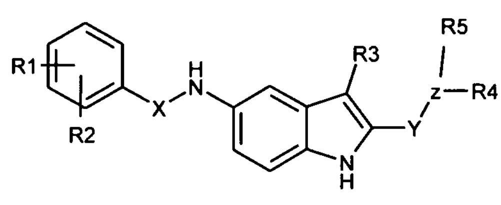

in which

$R^1$ stands for hydrogen, halogen, $CF_3$, $C_3$-$C_6$-cycloalkyl, which optionally is polysaturated and optionally is polysubstituted, or for the group $C_1$-$C_6$-alkyl, $C_1$-$C_6$-aryl, $C_1$-$C_6$-acyl, halo-$C_1$-$C_6$-alkyl, $C_1$-$C_6$-alkyl-$C_1$-$C_6$-alkyl, $C_1$-$C_6$-alkyl-$C_1$-$C_6$-acyl, $C_1$-$C_6$-acyl-$C_1$-$C_6$-acyl, $C_1$-$C_6$-alkyl-$C_1$-$C_6$-aryl, $C_1$-$C_6$-aryl-$C_1$-$C_6$-alkyl or $CF_3$, in which $C_1$-$C_6$-alkyl, $C_1$-$C_6$-aryl, $C_1$-$C_6$-acyl, halo-$C_1$-$C_6$-alkyl, $C_1$-$C_6$-alkyl-$C_1$-$C_6$-alkyl, $C_1$-$C_6$-alkyl-$C_1$-$C_6$-acyl,

$C_1$-$C_6$-acyl-$C_1$-$C_6$-acyl, $C_1$-$C_6$-alkyl-$C_1$-$C_6$-aryl or $C_1$-$C_6$-aryl-$C_1$-$C_6$-alkyl optionally can be interrupted in one or more places, in the same way or differently, by oxygen, sulfur or nitrogen, or for the group sulfonyl-$C_1$-$C_6$-alkyl, sulfonamide, or cyano,

$R^2$ stands for halogen, $CF_3$, $C_3$-$C_6$-cycloalkyl, which optionally is polysaturated and optionally is polysubstituted, or for the group $C_1$-$C_6$-alkyl, $C_1$-$C_6$-aryl, $C_1$-$C_6$-acyl, halo-$C_1$-$C_6$-alkyl, $C_1$-$C_6$-alkyl-$C_1$-$C_6$-alkyl, $C_1$-$C_6$-alkyl-$C_1$-$C_6$-acyl, $C_1$-$C_6$-acyl-$C_1$-$C_6$-acyl, $C_1$-$C_6$-alkyl-$C_1$-$C_6$-aryl, $C_1$-$C_6$-aryl-$C_1$-$C_6$-alkyl or $CF_3$, in which $C_1$-$C_6$-alkyl, $C_1$-$C_6$-aryl, $C_1$-$C_6$-acyl, halo-$C_1$-$C_6$-alkyl, $C_1$-$C_6$-alkyl-$C_1$-$C_6$-alkyl, $C_1$-$C_6$-alkyl-$C_1$-$C_6$-acyl, $C_1$-$C_6$-acyl-$C_1$-$C_6$-acyl, $C_1$-$C_6$-alkyl-$C_1$-$C_6$-aryl or $C_1$-$C_6$-aryl-$C_1$-$C_6$-alkyl optionally can be interrupted in one or more places, in the same way or differently, by oxygen, sulfur or nitrogen, or for the group sulfonyl-$C_1$-$C_6$-alkyl, sulfonamide, or cyano,

$R^3$ stands for $C_6$-$C_{12}$-aryl, which optionally can be substituted in one or more places, in the same way or differently, with halogen, with $C_1$-$C_6$-alkyl or $C_1$-$C_6$-acyl, which optionally can be substituted in one or more places, or can be substituted with $C_1$-$C_6$-alkoxy, hydroxy, cyano, $CO_2$-($C_1$-$C_6$-alkyl), N-($C_1$-$C_6$-alkyl$)_2$, CO-$NR^4R^5$ or with $CF_3$, for $C_5$-$C_{12}$-heteroaryl, which optionally can be substituted in one or more places, in the same way or differently, with halogen, with $C_1$-$C_6$-alkyl, $C_1$-$C_6$-acyl, $C_1$-$C_6$-alkoxy, hydroxy, cyano, $CO_2$-($C_1$-$C_6$-alkyl), N-($C_1$-$C_6$-alkyl$)_2$, CO-$NR^4R^5$ or with $CF_3$, or for $C_3$-$C_6$-cycloalkyl, which optionally can be substituted in one or more places, in the same way or differently, with halogen, $CF_3$, hydroxy, cyano, $CO_2$-($C_1$-$C_6$-alkyl), $C_1$-$C_6$-alkyl, $C_1$-$C_6$-acyl, N-($C_1$-$C_6$-alkyl$)_2$, CO-$NR^4R^5$ or $C_1$-$C_6$-alkoxy,

$R^4$ stands for hydrogen, $C_3$-$C_6$-cycloalkyl, which optionally is substituted in one or more places, in the same way or differently, with $C_1$-$C_6$-alkyl, $C_1$-$C_6$-acyl, $C_1$-$C_6$-alkoxy or $CF_3$, for $C_6$-$C_{12}$-aryl, which optionally is substituted in one or more places, in the same way or differently, with halogen, with $C_1$-$C_6$-alkyl, $C_1$-$C_6$-acyl, $C_1$-$C_6$-alkoxy, N-$C_1$-$C_6$-alkyl-$C_1$-$C_6$-alkyl, $CF_3$ or cyano, or for $C_5$-$C_{12}$-heteroaryl, which optionally is substituted in one or more places, in the same way or differently, with halogen, with $C_1$-$C_6$-alkyl, $C_1$-$C_6$-acyl, $C_1$-$C_6$-alkoxy, N-$C_1$-$C_6$-alkyl-$C_1$-$C_6$-alkyl, $CF_3$ or cyano, or for $C_1$-$C_6$-alkyl, which can be substituted in any way desired,

$R^5$ stands for hydrogen, $C_3$-$C_6$-cycloalkyl, which optionally is substituted in one or more places, in the same way or differently, with $C_1$-$C_6$-alkyl, $C_1$-$C_6$-acyl, $C_1$-$C_6$-alkoxy or $CF_3$, for $C_6$-$C_{12}$-aryl, which optionally is substituted in one or more places, in the same way or differently, with halogen, with $C_1$-$C_6$-alkyl, $C_1$-$C_6$-acyl, $C_1$-$C_6$-alkoxy, N-$C_1$-$C_6$-alkyl-$C_1$-$C_6$-alkyl, $CF_3$ or cyano, or for $C_5$-$C_{12}$-heteroaryl, which optionally is substituted in one or more places, in the same way or differently, with halogen, with $C_1$-$C_6$-alkyl, $C_1$-$C_6$-acyl, $C_1$-$C_6$-alkoxy, N-$C_1$-$C_6$-alkyl-$C_1$-$C_6$-alkyl, $CF_3$ or cyano, or for $C_1$-$C_6$-alkyl, which can be substituted in any way desired,

$R^4$ and $R^5$ together form a 5- to 8-membered ring, which can contain additional heteroatoms, and

X stands for the groups sulfonyl, $(CH_2)_n$ or carbonyl,

Y stands for carbonyl or $(CH_2)_n$,

Z stands for nitrogen, and

n stands for 0 - 4.

**2.** Compounds according to claim 1, whereby

$R^1$ stands for hydrogen, halogen, $CF_3$, $C_3$-$C_6$-cycloalkyl, which optionally is polysaturated and optionally is polysubstituted, or for the group $C_1$-$C_6$-alkyl, $C_1$-$C_6$-aryl, $C_1$-$C_6$-acyl, halo-$C_1$-$C_6$-alkyl, $C_1$-$C_6$-alkyl-$C_1$-$C_6$-alkyl, $C_1$-$C_6$-alkyl-$C_1$-$C_6$-acyl, $C_1$-$C_6$-acyl-$C_1$-$C_6$-acyl, $C_1$-$C_6$-alkyl-$C_1$-$C_6$-aryl, $C_1$-$C_6$-aryl-$C_1$-$C_6$-alkyl or $CF_3$, in which $C_1$-$C_6$-alkyl, $C_1$-$C_6$-aryl, $C_1$-$C_6$-acyl, halo-$C_1$-$C_6$-alkyl, $C_1$-$C_6$-alkyl-$C_1$-$C_6$-alkyl, $C_1$-$C_6$-alkyl-$C_1$-$C_6$-acyl, $C_1$-$C_6$-acyl-$C_1$-$C_6$-acyl, $C_1$-$C_6$-alkyl-$C_1$-$C_6$-aryl or $C_1$-$C_6$-aryl-$C_1$-$C_6$-alkyl optionally can be interrupted in one or more places, in the same way or differently, by oxygen, sulfur or nitrogen, or for the group sulfonyl-$C_1$-$C_6$-alkyl, sulfonamide, or cyano,

$R^2$ stands for halogen, $CF_3$, $C_3$-$C_6$-cycloalkyl, which optionally is polysaturated and optionally is polysubstituted, or for the group $C_1$-$C_6$-alkyl, $C_1$-$C_6$-aryl, $C_1$-$C_6$-acyl, halo-$C_1$-$C_6$-alkyl, $C_1$-$C_6$-alkyl-$C_1$-$C_6$-alkyl, $C_1$-$C_6$-alkyl-$C_1$-$C_6$-acyl, $C_1$-$C_6$-acyl-$C_1$-$C_6$-acyl, $C_1$-$C_6$-alkyl-$C_1$-$C_6$-aryl, $C_1$-$C_6$-aryl-$C_1$-$C_6$-alkyl or $CF_3$, in which $C_1$-$C_6$-alkyl, $C_1$-$C_6$-aryl, $C_1$-$C_6$-acyl, halo-$C_1$-$C_6$-alkyl, $C_1$-$C_6$-alkyl-$C_1$-$C_6$-alkyl, $C_1$-$C_6$-alkyl-$C_1$-$C_6$-acyl, $C_1$-$C_6$-acyl-$C_1$-$C_6$-acyl, $C_1$-$C_6$-alkyl-$C_1$-$C_6$-aryl or $C_1$-$C_6$-aryl-$C_1$-$C_6$-alkyl optionally can be interrupted in one or more places, in the same way or differently, by oxygen, sulfur or nitrogen, or for the group sulfonyl-$C_1$-$C_6$-alkyl, sulfonamide, or cyano,

$R^3$ stands for $C_6$-$C_{12}$-aryl, which optionally can be substituted in one or more places, in the same way or differently, with halogen, with $C_1$-$C_6$-alkyl, $C_1$-$C_3$-acyl, $C_1$-$C_3$-alkoxy, cyano, hydroxy, N- $(CH_3)_2$, $CO_2$-($C_1$-$C_3$-alkyl), CO-$NR^4R^5$ or with $CF_3$, $C_5$-$C_{12}$-heteroaryl, which optionally can be substituted in one or more places, in the same way or differently, with chlorine and/or fluorine, with $C_1$-$C_6$-alkyl, $C_1$-$C_3$-acyl, $C_1$-$C_3$-alkoxy, cyano, hydroxy, N- $(CH_3)_2$, $CO_2$-($C_1$-$C_3$-alkyl), CO-$NR^4R^5$ or with $CF_3$, $C_3$-$C_6$-cycloalkyl, which optionally can be substituted in one or more places, in the same way or differently, with chlorine and/or fluorine,

$CF_3$, cyano, $C_1$-$C_3$-alkyl, $C_1$-$C_3$-acyl, hydroxy, N- $(CH_3)_2$, $CO_2$-($C_1$-$C_3$-alkyl), CO-$NR^4R^5$ or $C_1$-$C_3$-alkoxy,
$R^4$ stands for hydrogen, $C_3$-$C_6$-cycloalkyl, which optionally is substituted in one or more places, in the same way or differently, with $C_1$-$C_3$-alkyl, $C_1$-$C_3$-acyl, $C_1$-$C_3$-alkoxy or $CF_3$, for $C_6$-$C_{12}$-aryl, which optionally is substituted in one or more places, in the same way or differently, with halogen, with $C_1$-$C_3$-alkyl, $C_1$-$C_3$-acyl, $C_1$-$C_3$-alkoxy, N-$C_1$-$C_3$-alkyl-$C_1$-$C_3$-alkyl, $CF_3$ or cyano, or for $C_5$-$C_{12}$-heteroaryl, which optionally is substituted in one or more places, in the same way or differently, with halogen, with $C_1$-$C_3$-alkyl, $C_1$-$C_3$-acyl, $C_1$-$C_3$-alkoxy, N-$C_1$-$C_3$-alkyl-$C_1$-$C_3$-alkyl, $CF_3$ or cyano, or for $C_1$-$C_6$-alkyl, which can be substituted in any way desired,
$R^5$ stands for hydrogen, $C_3$-$C_6$-cycloalkyl, which optionally is substituted in one or more places, in the same way or differently, with $C_1$-$C_3$-alkyl, $C_1$-$C_3$-acyl, $C_1$-$C_3$-alkoxy or $CF_3$, for $C_6$-$C_{12}$-aryl, which optionally is substituted in one or more places, in the same way or differently, with halogen, with $C_1$-$C_3$-alkyl, $C_1$-$C_3$-acyl, $C_1$-$C_3$-alkoxy, N-$C_1$-$C_3$-alkyl-$C_1$-$C_3$-alkyl, $CF_3$ or cyano, or for $C_5$-$C_{12}$-heteroaryl, which optionally is substituted in one or more places, in the same way or differently, with halogen, with $C_1$-$C_3$-alkyl, $C_1$-$C_3$-acyl, $C_1$-$C_3$-alkoxy, N-$C_1$-$C_3$-alkyl-$C_1$-$C_3$-alkyl, $CF_3$ or cyano, or for $C_1$-$C_6$-alkyl, which can be substituted in any way desired,
$R^4$ and $R^5$ together form a 5- to 8-membered ring, which can contain additional heteroatoms,
X stands for the groups sulfonyl, $(CH_2)_n$ or carbonyl,
Y stands for carbonyl or $(CH_2)_n$,
Z stands for nitrogen, and
n stands for 0 - 2.

**3.** Compounds according to claim 1 or 2, whereby

$R^1$ stands for hydrogen,
$R^2$ stands for tert-butyl, cyano, bromine, or for the group -O-$CF_3$, -$SO_2$-$CH_3$ and is in para-position,
$R^3$ stands for the group

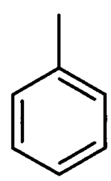
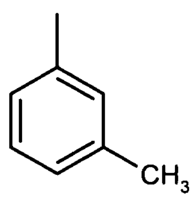
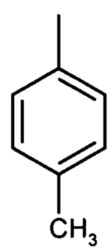
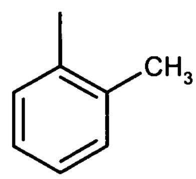
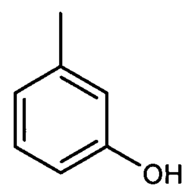

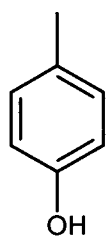
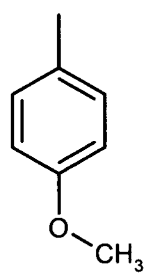
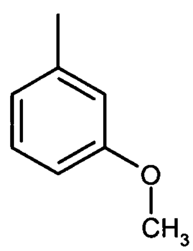
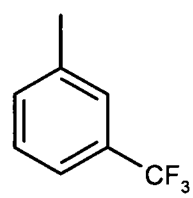
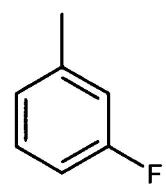

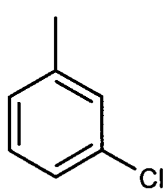
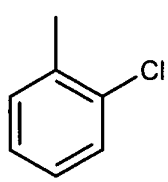
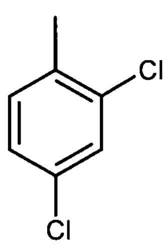
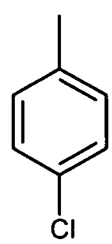

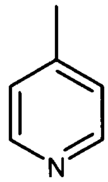 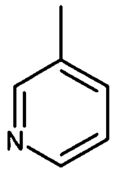 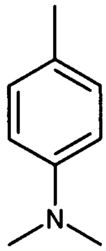

$R^4$ stands for hydrogen or for the group - $(CH_2)_n$-N-$(CH_3)_2$, -$(CH_2)_2$-$CH_3$, -$(CH_2)_2$-NH-$COCH_3$, -$(CH_2)$-$CHCH_3$-OH, - $(CH_2)_2$-O-$CH_3$, -$(CH_2)_2$-OH, -$CHCH_3$-$CH_2$-OH,

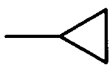 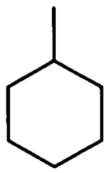 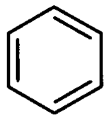 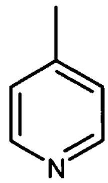

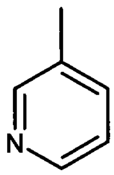 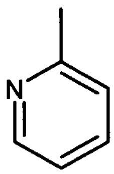 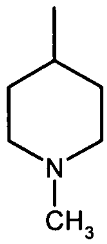 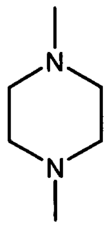 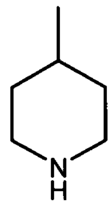

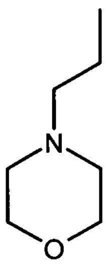 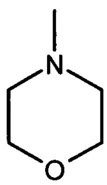 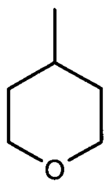 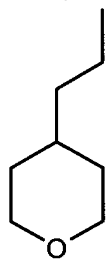 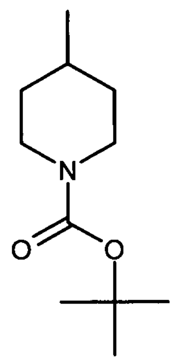

$R^5$ stands for hydrogen,
X stands for sulfonyl, carbonyl or for the group $CH_2$,
Y stands for carbonyl or for the group $(CH_2)_n$,
Z stands for nitrogen or for

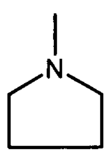 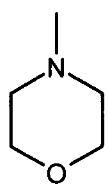 ,

and

n is 1-2.

**4.** Compounds according to claim 1, in which

$R^1$ stands for hydrogen, tert-butyl, cyano, bromine, or for the group $-O-CF_3$ or $-SO_2-CH_3$,
$R^2$ stands for tert-butyl, cyano, bromine, or for the group $-O-CF_3$ or $-SO_2-CH_3$, and
$R^3$ stands for $C_6$-$C_{12}$-aryl, which optionally can be substituted in one or more places, in the same way or differently, with halogen, with $C_1$-$C_6$-alkyl, $C_1$-$C_3$-acyl, $C_1$-$C_3$-alkoxy, cyano, hydroxy, $N-(CH_3)_2$, $CO_2$-($C_1$-$C_3$-alkyl), CO-$NR^4R^5$ or with $CF_3$, $C_5$-$C_{12}$-heteroaryl, which optionally can be substituted in one or more places, in the same way or differently, with chlorine and/or fluorine, with $C_1$-$C_6$-alkyl, $C_1$-$C_3$-acyl, $C_1$-$C_3$-alkoxy, cyano, hydroxy, $N-(CH_3)_2$, $CO_2$-($C_1$-$C_3$-alkyl), CO-$NR^4R^5$ or with $CF_3$, $C_3$-$C_6$-cycloalkyl, which optionally can be substituted in one or more places, in the same way or differently, with chlorine and/or fluorine, $CF_3$, cyano, $C_1$-$C_3$-alkyl, $C_1$-$C_3$-acyl, hydroxy, $N-(CH_3)_2$, $CO_2$-($C_1$-$C_3$-alkyl), CO-$NR^4R^5$ or $C_1$-$C_3$-alkoxy,
$R^4$ stands for hydrogen, $C_3$-$C_6$-cycloalkyl, which optionally is substituted in one or more places, in the same way or differently, with $C_1$-$C_3$-alkyl, $C_1$-$C_3$-acyl, $C_1$-$C_3$-alkoxy or $CF_3$, for $C_6$-$C_{12}$-aryl, which optionally is substituted in one or more places, in the same way or differently, with halogen, with $C_1$-$C_3$-alkyl, $C_1$-$C_3$-acyl, $C_1$-$C_3$-alkoxy, N-$C_1$-$C_3$-alkyl-$C_1$-$C_3$-alkyl, $CF_3$ or cyano, or for $C_5$-$C_{12}$-heteroaryl, which optionally is substituted in one or more places, in the same way or differently, with halogen, with $C_1$-$C_3$-alkyl, $C_1$-$C_3$-acyl, $C_1$-$C_3$-alkoxy, N-$C_1$-$C_3$-alkyl-$C_1$-$C_3$-alkyl, $CF_3$ or cyano, or for $C_1$-$C_6$-alkyl, which can be substituted in any way desired,
$R^5$ stands for hydrogen, $C_3$-$C_6$-cycloalkyl, which optionally is substituted in one or more places, in the same way or differently, with $C_1$-$C_3$-alkyl, $C_1$-$C_3$-acyl, $C_1$-$C_3$-alkoxy or $CF_3$, for $C_6$-$C_{12}$-aryl, which optionally is substituted in one or more places, in the same way or differently, with halogen, with $C_1$-$C_3$-alkyl, $C_1$-$C_3$-acyl, $C_1$-$C_3$-alkoxy, N-$C_1$-$C_3$-alkyl-$C_1$-$C_3$-alkyl, $CF_3$ or cyano, or for $C_5$-$C_{12}$-heteroaryl, which optionally is substituted in one or more places, in the same way or differently, with halogen, with $C_1$-$C_3$-alkyl, $C_1$-$C_3$-acyl, $C_1$-$C_3$-alkoxy, N-$C_1$-$C_3$-alkyl-$C_1$-$C_3$-alkyl, $CF_3$ or cyano, or for $C_1$-$C_6$-alkyl, which can be substituted in any way desired,
$R^4$ and $R^5$ together form a 5- to 8-membered ring, which can contain additional heteroatoms,
X stands for the groups sulfonyl, $(CH_2)_n$ or carbonyl,
Y stands for carbonyl or $(CH_2)_n$,
Z stands for nitrogen, and
n stands for 0 - 2.

**5.** Compounds according to claim 1, in which

$R^1$ stands for hydrogen,
$R^2$ stands for tert-butyl, cyano, bromine, or for the group $-O-CF_3$ or $-SO_2-CH_3$ and is in para-position, and
$R^3$ stands for $C_6$-$C_{12}$-aryl, which optionally can be substituted in one or more places, in the same way or differently, with halogen, with $C_1$-$C_6$-alkyl, $C_1$-$C_3$-acyl, $C_1$-$C_3$-alkoxy, cyano, hydroxy, $N-(CH_3)_2$, $CO_2$-($C_1$-$C_3$-alkyl), CO-$NR^4R^5$ or with $CF_3$, $C_5$-$C_{12}$-heteroaryl, which optionally can be substituted in one or more places, in the same way or differently, with chlorine and/or fluorine, with $C_1$-$C_6$-alkyl, $C_1$-$C_3$-acyl, $C_1$-$C_3$-alkoxy, cyano, hydroxy, $N-(CH_3)_2$, $CO_2$-($C_1$-$C_3$-alkyl), CO-$NR^4R^5$ or with $CF_3$, $C_3$-$C_6$-cycloalkyl, which optionally can be substituted in one or more places, in the same way or differently, with chlorine and/or fluorine, $CF_3$, cyano, $C_1$-$C_3$-alkyl, $C_1$-$C_3$-acyl, hydroxy, $N-(CH_3)_2$, $CO_2$-($C_1$-$C_3$-alkyl), CO-$NR^4R^5$ or $C_1$-$C_3$-alkoxy,
$R^4$ stands for hydrogen, $C_3$-$C_6$-cycloalkyl, which optionally is substituted in one or more places, in the same way or differently, with $C_1$-$C_3$-alkyl, $C_1$-$C_3$-acyl, $C_1$-$C_3$-alkoxy or $CF_3$, for $C_6$-$C_{12}$-aryl, which optionally is substituted in one or more places, in the same way or differently, with halogen, with $C_1$-$C_3$-alkyl, $C_1$-$C_3$-acyl, $C_1$-$C_3$-alkoxy, N-$C_l$-$C_3$-alkyl-$C_1$-$C_3$-alkyl, $CF_3$ or cyano, or for $C_5$-$C_{12}$-heteroaryl, which optionally is substituted in one or more places, in the same way or differently, with halogen, with $C_1$-$C_3$-alkyl, $C_1$-$C_3$-acyl, $C_1$-$C_3$-alkoxy, N-$C_1$-$C_3$-alkyl-$C_1$-$C_3$-alkyl, $CF_3$ or cyano, or for $C_1$-$C_6$-alkyl, which can be substituted in any way desired,
$R^5$ stands for hydrogen, $C_3$-$C_6$-cycloalkyl, which optionally is substituted in one or more places, in the same way or differently, with $C_1$-$C_3$-alkyl, $C_1$-$C_3$-acyl, $C_1$-$C_3$-alkoxy or $CF_3$, for $C_6$-$C_{12}$-aryl, which optionally is substituted in one or more places, in the same way or differently, with halogen, with $C_1$-$C_3$-alkyl, $C_1$-$C_3$-acyl, $C_1$-$C_3$-alkoxy, N-$C_1$-$C_3$-alkyl-$C_1$-$C_3$-alkyl, $CF_3$ or cyano, or for $C_5$-$C_{12}$-heteroaryl, which optionally is substituted in one or more places, in the same way or differently, with halogen, with $C_1$-$C_3$-alkyl, $C_1$-$C_3$-acyl, $C_1$-$C_3$-alkoxy, N-$C_1$-$C_3$-alkyl-$C_1$-$C_3$-alkyl, $CF_3$ or cyano, or for $C_1$-$C_6$-alkyl, which can be substituted in any way desired,
$R^4$ and $R^5$ together form a 5- to 8-membered ring, which can contain additional heteroatoms,
X stands for the groups sulfonyl, $(CH_2)_n$ or carbonyl,
Y stands for carbonyl or $(CH_2)_n$,
Z stands for nitrogen, and

n stands for 0 - 2.

**6.** Compounds according to claim 1, whereby

$R^1$ stands for hydrogen, halogen, $CF_3$, $C_3$-$C_6$-cycloalkyl, which optionally is polysaturated and optionally is polysubstituted, or for the group $C_1$-$C_6$-alkyl, $C_1$-$C_6$-aryl, $C_1$-$C_6$-acyl, halo-$C_1$-$C_6$-alkyl, $C_1$-$C_6$-alkyl-$C_1$-$C_6$-alkyl, $C_1$-$C_6$-alkyl-$C_1$-$C_6$-acyl, $C_1$-$C_6$-acyl-$C_1$-$C_6$-acyl, $C_1$-$C_6$-alkyl-$C_1$-$C_6$-aryl, $C_1$-$C_6$-aryl-$C_1$-$C_6$-alkyl or $CF_3$, in which $C_1$-$C_6$-alkyl, $C_1$-$C_6$-aryl, $C_1$-$C_6$-acyl, halo-$C_1$-$C_6$-alkyl, $C_1$-$C_6$-alkyl-$C_1$-$C_6$-alkyl, $C_1$-$C_6$-alkyl-$C_1$-$C_6$-acyl, $C_1$-$C_6$-acyl-$C_1$-$C_6$-acyl, $C_1$-$C_6$-alkyl-$C_1$-$C_6$-aryl or $C_1$-$C_6$-aryl-$C_1$-$C_6$-alkyl optionally can be interrupted in one or more places, in the same way or differently, by oxygen, sulfur or nitrogen, or for the group sulfonyl-$C_1$-$C_6$-alkyl, sulfonamide, or cyano,
$R^2$ stands for halogen, $CF_3$, $C_3$-$C_6$-cycloalkyl, which optionally is polysaturated and optionally is polysubstituted, or for the group $C_1$-$C_6$-alkyl, $C_1$-$C_6$-aryl, $C_1$-$C_6$-acyl, halo-$C_1$-$C_6$-alkyl, $C_1$-$C_6$-alkyl-$C_1$-$C_6$-alkyl, $C_1$-$C_6$-alkyl-$C_1$-$C_6$-acyl, $C_1$-$C_6$-acyl-$C_1$-$C_6$-acyl, $C_1$-$C_6$-alkyl-$C_1$-$C_6$-aryl, $C_1$-$C_6$-aryl-$C_1$-$C_6$-alkyl or $CF_3$, in which $C_1$-$C_6$-alkyl, $C_1$-$C_6$-aryl, $C_1$-$C_6$-acyl, halo-$C_1$-$C_6$-alkyl, $C_1$-$C_6$-alkyl-$C_1$-$C_6$-alkyl, $C_1$-$C_6$-alkyl-$C_1$-$C_6$-acyl, $C_1$-$C_6$-acyl-$C_1$-$C_6$-acyl, $C_1$-$C_6$-alkyl-$C_1$-$C_6$-aryl or $C_1$-$C_6$-aryl-$C_1$-$C_6$-alkyl optionally can be interrupted in one or more places, in the same way or differently, by oxygen, sulfur or nitrogen, or for the group sulfonyl-$C_1$-$C_6$-alkyl, sulfonamide, or cyano,
$R^3$ stands for $C_6$-$C_{12}$-aryl, which optionally can be substituted in one or more places, in the same way or differently, with halogen, with $C_1$-$C_6$-alkyl, $C_1$-$C_3$-acyl, $C_1$-$C_3$-alkoxy, cyano, hydroxy, $N(CH_3)_2$, $CO_2$-($C_1$-$C_3$-alkyl), CO-$NR^4R^5$ or with $CF_3$, $C_5$-$C_{12}$-heteroaryl, which optionally can be substituted in one or more places, in the same way or differently, with chlorine and/or fluorine, with $C_1$-$C_6$-alkyl, $C_1$-$C_3$-acyl, $C_1$-$C_3$-alkoxy, cyano, hydroxy, $N(CH_3)_2$, $CO_2$-($C_1$-$C_3$-alkyl), CO-$NR^4R^5$ or with $CF_3$, $C_3$-$C_6$-cycloalkyl, which optionally can be substituted in one or more places, in the same way or differently, with chlorine and/or fluorine, $CF_3$, cyano, $C_1$-$C_3$-alkyl, $C_1$-$C_3$-acyl, hydroxy, $N(CH_3)_2$, $CO_2$-($C_1$-$C_3$-alkyl), CO-$NR^4R^5$ or $C_1$-$C_3$-alkoxy,
$R^4$ stands for hydrogen, $C_3$-$C_6$-cycloalkyl, which optionally is substituted in one or more places, in the same way or differently, with $C_1$-$C_6$-alkyl, $C_1$-$C_6$-acyl, $C_1$-$C_6$-alkoxy or $CF_3$, for $C_6$-$C_{12}$-aryl, which optionally is substituted in one or more places, in the same way or differently, with halogen, with $C_1$-$C_6$-alkyl, $C_1$-$C_6$-acyl, $C_1$-$C_6$-alkoxy, N-$C_1$-$C_6$-alkyl-$C_1$-$C_6$-alkyl, $CF_3$ or cyano, or for $C_5$-$C_{12}$-heteroaryl, which optionally is substituted in one or more places, in the same way or differently, with halogen, with $C_1$-$C_6$-alkyl, $C_1$-$C_6$-acyl, $C_1$-$C_6$-alkoxy, N-$C_1$-$C_6$-alkyl-$C_1$-$C_6$-alkyl, $CF_3$ or cyano, or for $C_1$-$C_6$-alkyl, which can be substituted in any way desired,
$R^5$ stands for hydrogen, $C_3$-$C_6$-cycloalkyl, which optionally is substituted in one or more places, in the same way or differently, with $C_1$-$C_6$-alkyl, $C_1$-$C_6$-acyl, $C_1$-$C_6$-alkoxy or $CF_3$, for $C_6$-$C_{12}$-aryl, which optionally is substituted in one or more places, in the same way or differently, with halogen, with $C_1$-$C_6$-alkyl, $C_1$-$C_6$-acyl, $C_1$-$C_6$-alkoxy, N-$C_1$-$C_6$-alkyl-$C_1$-$C_6$-alkyl, $CF_3$ or cyano, or for $C_5$-$C_{12}$-heteroaryl, which optionally is substituted in one or more places, in the same way or differently, with halogen, with $C_1$-$C_6$-alkyl, $C_1$-$C_6$-acyl, $C_1$-$C_6$-alkoxy, N-$C_1$-$C_6$-alkyl-$C_1$-$C_6$-alkyl, $CF_3$ or cyano, or for $C_1$-$C_6$-alkyl, which can be substituted in any way desired,
$R^4$ and $R^5$ together form a 5- to 8-membered ring, which can contain additional heteroatoms, and
X stands for the groups sulfonyl, $(CH_2)_n$ or carbonyl,
Y stands for carbonyl or $(CH_2)_n$,
Z stands for nitrogen, and
n stands for 0 - 4.

**7.** Compounds according to claim 1, whereby

$R^1$ stands for hydrogen,
$R^2$ stands for tert-butyl, cyano, bromine, or for the group $-O-CF_3$ or $-SO_2-CH_3$ and is in para-position, and
$R^3$ stands for $C_6$-$C_{12}$-aryl, which optionally can be substituted in one or more places, in the same way or differently, with halogen, with $C_1$-$C_6$-alkyl, $C_1$-$C_3$-acyl, $C_1$-$C_3$-alkoxy, cyano, hydroxy, $N(CH_3)_2$, $CO_2$-($C_1$-$C_3$-alkyl), CO-$NR^4R^5$ or with $CF_3$, $C_5$-$C_{12}$-heteroaryl, which optionally can be substituted in one or more places, in the same way or differently, with chlorine and/or fluorine, with $C_1$-$C_6$-alkyl, $C_1$-$C_3$-acyl, $C_1$-$C_3$-alkoxy, cyano, hydroxy, $N(CH_3)_2$, $CO_2$-($C_1$-$C_3$-alkyl), CO-$NR^4R^5$ or with $CF_3$, $C_3$-$C_6$-cycloalkyl, which optionally can be substituted in one or more places, in the same way or differently, with chlorine and/or fluorine, $CF_3$, cyano, $C_1$-$C_3$-alkyl, $C_1$-$C_3$-acyl, hydroxy, $N(CH_3)_2$, $CO_2$-($C_1$-$C_3$-alkyl), CO-$NR^4R^5$ or $C_1$-$C_3$-alkoxy,
$R^4$ stands for hydrogen or for the group $-(CH_2)_n-N(CH_3)_2$, $-(CH_2)_2-CH_3$, $-(CH_2)_2-NH-COCH_3$, $-(CH_2)-CHCH_3-OH$, $-(CH_2)_2-O-CH_3$, $-(CH_2)_2-OH$, $-CHCH_3-CH_2-OH$,

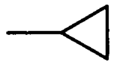 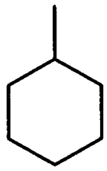 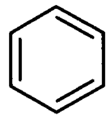 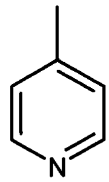

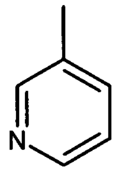 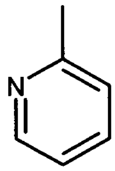 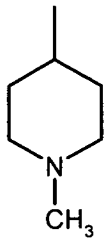 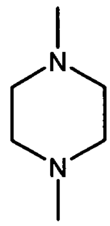 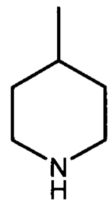

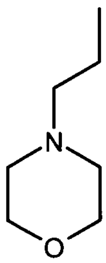 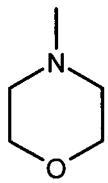 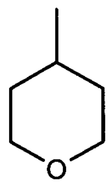 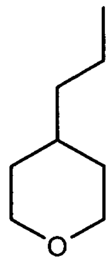 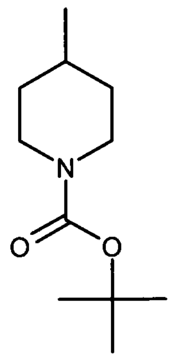

$R^5$ stands for hydrogen,
X stands for sulfonyl, carbonyl or for the group $CH_2$,
Y stands for carbonyl or for the group $(CH_2)_n$,
Z stands for nitrogen or for

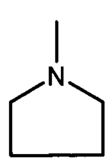 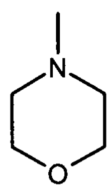 ,

and
n stands for 1-2.

**8.** Compounds according to claim 1, whereby

$R^1$ stands for hydrogen, halogen, $CF_3$, $C_3$-$C_6$-cycloalkyl, which optionally is polysaturated and optionally is polysubstituted, or for the group $C_1$-$C_6$-alkyl, $C_1$-$C_6$-aryl, $C_1$-$C_6$-acyl, halo-$C_1$-$C_6$-alkyl, $C_1$-$C_6$-alkyl-$C_1$-$C_6$alkyl, $C_1$-$C_6$-alkyl-$C_1$-$C_6$-acyl, $C_1$-$C_6$-acyl-$C_1$-$C_6$-acyl, $C_1$-$C_6$-alkyl-$C_1$-$C_6$-aryl, $C_1$-$C_6$-aryl-$C_1$-$C_6$-alkyl or $CF_3$, in which $C_1$-$C_6$-alkyl, $C_1$-$C_6$-aryl, $C_1$-$C_6$-acyl, halo-$C_1$-$C_6$-alkyl, $C_1$-$C_6$-alkyl-$C_1$-$C_6$-alkyl, $C_1$-$C_6$-alkyl-$C_1$-$C_6$-acyl, $C_1$-$C_6$-acyl-$C_1$-$C_6$-acyl, $C_1$-$C_6$-alkyl-$C_1$-$C_6$-aryl or $C_1$-$C_6$-aryl-$C_1$-$C_6$-alkyl optionally can be interrupted in one or more places, in the same way or differently, by oxygen, sulfur or nitrogen, or for the group sulfonyl-$C_1$-$C_6$-alkyl, sulfonamide, or cyano,
$R^2$ stands for halogen, $CF_3$, $C_3$-$C_6$-cycloalkyl, which optionally is polysaturated and optionally is polysubstituted,

or for the group $C_1$-$C_6$-alkyl, $C_1$-$C_6$-aryl, $C_1$-$C_6$-acyl, halo-$C_1$-$C_6$-alkyl, $C_1$-$C_6$-alkyl-$C_1$-$C_6$-alkyl, $C_1$-$C_6$-alkyl-$C_1$-$C_6$-acyl, $C_1$-$C_6$-acyl-$C_1$-$C_6$-acyl, $C_1$-$C_6$-alkyl-$C_1$-$C_6$-aryl, $C_1$-$C_6$-aryl-$C_1$-$C_6$-alkyl or $CF_3$, in which $C_1$-$C_6$-alkyl, $C_1$-$C_6$-aryl, $C_1$-$C_6$-acyl, halo-$C_1$-$C_6$-alkyl, $C_1$-$C_6$-alkyl-$C_1$-$C_6$-alkyl, $C_1$-$C_6$-alkyl-$C_1$-$C_6$-acyl, $C_1$-$C_6$-acyl-$C_1$-$C_6$-acyl, $C_1$-$C_6$-alkyl-$C_1$-$C_6$-aryl or $C_1$-$C_6$-aryl-$C_1$-$C_6$-alkyl optionally can be interrupted in one or more places, in the same way or differently, by oxygen, sulfur or nitrogen, or for the group sulfonyl-$C_1$-$C_6$-alkyl, sulfonamide, or cyano,
$R^3$ stands for the group

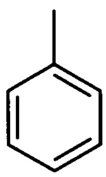
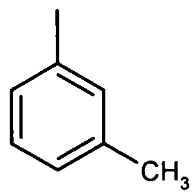
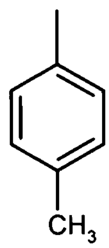
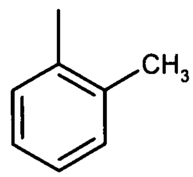
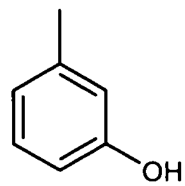

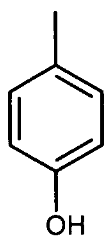
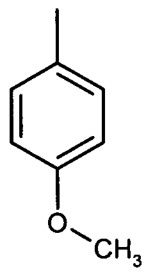
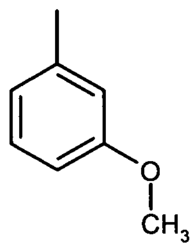
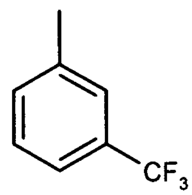
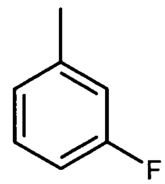

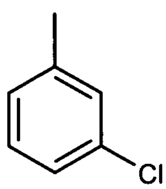
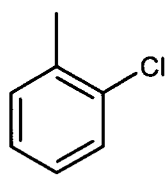
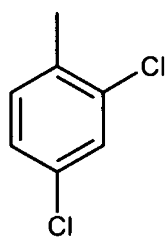
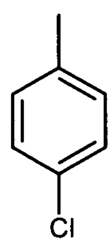

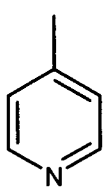
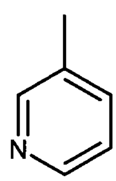
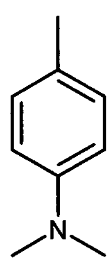

$R^4$ stands for hydrogen, $C_3$-$C_6$-cycloalkyl, which optionally is substituted in one or more places, in the same way or differently, with $C_1$-$C_3$-alkyl, $C_1$-$C_3$-acyl, $C_1$-$C_3$-alkoxy or $CF_3$, for $C_6$-$C_{12}$-aryl, which optionally is substituted in one or more places, in the same way or differently, with halogen, with $C_1$-$C_3$-alkyl, $C_1$-$C_3$-acyl, $C_1$-$C_3$-alkoxy, N-$C_1$-$C_3$-alkyl-$C_1$-$C_3$-alkyl, $CF_3$ or cyano, or for $C_5$-$C_{12}$-heteroaryl, which optionally is substituted in one or more places, in the same way or differently, with halogen, with $C_1$-$C_3$-alkyl, $C_1$-$C_3$-acyl, $C_1$-$C_3$-alkoxy, N-$C_1$-$C_3$-alkyl-$C_1$-$C_3$-alkyl, $CF_3$ or cyano, or for $C_1$-$C_6$-alkyl, which can be substituted in any way desired,
$R^5$ stands for hydrogen, $C_3$-$C_6$-cycloalkyl, which optionally is substituted in one or more places, in the same way or differently, with $C_1$-$C_3$-alkyl, $C_1$-$C_3$-acyl, $C_1$-$C_3$-alkoxy or $CF_3$, for $C_6$-$C_{12}$-aryl, which optionally is substituted in one or more places, in the same way or differently, with halogen, with $C_1$-$C_3$-alkyl, $C_1$-$C_3$-acyl,

$C_1$-$C_3$-alkoxy, N-$C_1$-$C_3$-alkyl-$C_1$-$C_3$-alkyl, $CF_3$ or cyano, or for $C_5$-$C_{12}$-heteroaryl, which optionally is substituted in one or more places, in the same way or differently, with halogen, with $C_1$-$C_3$-alkyl, $C_1$-$C_3$-acyl, $C_1$-$C_3$-alkoxy, N-$C_1$-$C_3$-alkyl-$C_1$-$C_3$-alkyl, $CF_3$ or cyano, or for $C_1$-$C_6$-alkyl, which can be substituted in any way desired,
$R^4$ and $R^5$ together form a 5- to 8-membered ring, which can contain additional heteroatoms,
X stands for the groups sulfonyl, $(CH_2)_n$ or carbonyl,
Y stands for carbonyl or $(CH_2)_n$,
Z stands for nitrogen, and
n stands for 0 - 2.

**9.** Compounds according to claim 1, whereby

$R^1$ stands for hydrogen,
$R^2$ stands for tert-butyl, cyano, bromine or for the group -O-$CF_3$ or -$SO_2$-$CH_3$ and is in para-position, and
$R^3$ stands for the group

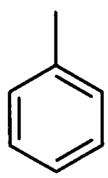
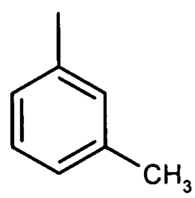
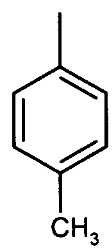
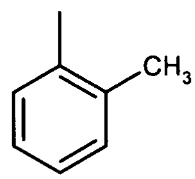
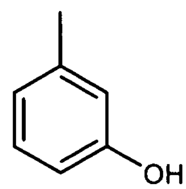

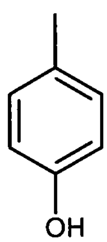
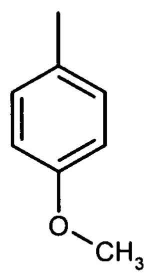
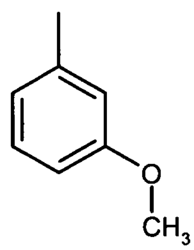
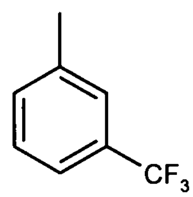
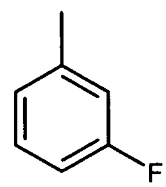

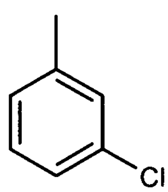
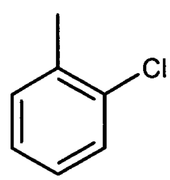
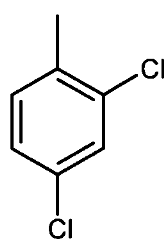
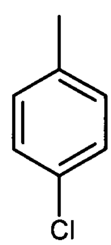

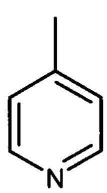
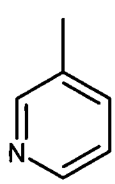
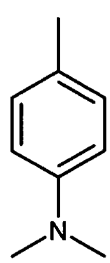

$R^4$ stands for hydrogen, $C_3$-$C_6$-cycloalkyl, which optionally is substituted in one or more places, in the same way or differently, with $C_1$-$C_3$-alkyl, $C_1$-$C_3$-acyl, $C_1$-$C_3$-alkoxy or $CF_3$, for $C_6$-$C_{12}$-aryl, which optionally is substituted in one or more places, in the same way or differently, with halogen, with $C_1$-$C_3$-alkyl, $C_1$-$C_3$-acyl, $C_1$-$C_3$-alkoxy, N-$C_1$-$C_3$-alkyl-$C_1$-$C_3$-alkyl, $CF_3$ or cyano, or for $C_5$-$C_{12}$-heteroaryl, which optionally is substituted in one or more places, in the same way or differently, with halogen, with $C_1$-$C_3$-alkyl, $C_1$-$C_3$-acyl, $C_1$-$C_3$-alkoxy, N-$C_1$-$C_3$-alkyl-$C_1$-$C_3$-alkyl, $CF_3$ or cyano, or for $C_1$-$C_6$-alkyl, which can be substituted in any way desired,
$R^5$ stands for hydrogen, $C_3$-$C_6$-cycloalkyl, which optionally is substituted in one or more places, in the same way or differently, with $C_1$-$C_3$-alkyl, $C_1$-$C_3$-acyl, $C_1$-$C_3$-alkoxy or $CF_3$, for $C_6$-$C_{12}$-aryl, which optionally is substituted in one or more places, in the same way or differently, with halogen, with $C_1$-$C_3$-alkyl, $C_1$-$C_3$-acyl, $C_1$-$C_3$-alkoxy, N-$C_1$-$C_3$-alkyl-$C_1$-$C_3$-alkyl, $CF_3$ or cyano, or for $C_5$-$C_{12}$-heteroaryl, which optionally is substituted in one or more places, in the same way or differently, with halogen, with $C_1$-$C_3$-alkyl, $C_1$-$C_3$-acyl, $C_1$-$C_3$-alkoxy, N-$C_1$-$C_3$-alkyl-$C_1$-$C_3$-alkyl, $CF_3$ or cyano, or for $C_1$-$C_6$-alkyl, which can be substituted in any way desired,
X stands for sulfonyl, carbonyl or for the group $CH_2$,
Y stands for carbonyl or for the group $(CH_2)n$,
Z stands for nitrogen or for

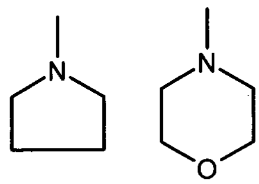,

and
n stands for 1-2.

**10.** Compounds according to claim 1, whereby

$R^1$ stands for hydrogen, halogen, $CF_3$, $C_3$-$C_6$-cycloalkyl, which optionally is polysaturated and optionally is polysubstituted, or for the group $C_1$-$C_6$-alkyl, $C_1$-$C_6$-aryl, $C_1$-$C_6$-acyl, halo-$C_1$-$C_6$-alkyl, $C_1$-$C_6$-alkyl-$C_1$-$C_6$-alkyl, $C_1$-$C_6$-alkyl-$C_1$-$C_6$-acyl, $C_1$-$C_6$-acyl-$C_1$-$C_6$-acyl, $C_1$-$C_6$-alkyl-$C_1$-$C_6$-aryl, $C_1$-$C_6$-aryl-$C_1$-$C_6$-alkyl or $CF_3$, in which $C_1$-$C_6$-alkyl, $C_1$-$C_6$-aryl, $C_1$-$C_6$-acyl, halo-$C_1$-$C_6$-alkyl, $C_1$-$C_6$-alkyl-$C_1$-$C_6$-alkyl, $C_1$-$C_6$-alkyl-$C_1$-$C_6$-acyl, $C_1$-$C_6$-acyl-$C_1$-$C_6$-acyl, $C_1$-$C_6$-alkyl-$C_1$-$C_6$-aryl or $C_1$-$C_6$-aryl-$C_1$-$C_6$-alkyl optionally can be interrupted in one or more places, in the same way or differently, by oxygen, sulfur or nitrogen, or for the group sulfonyl-$C_1$-$C_6$-alkyl, sulfonamide, or cyano,
$R^2$ stands for halogen, $CF_3$, $C_3$-$C_6$-cycloalkyl, which optionally is polysaturated and optionally is polysubstituted, or for the group $C_1$-$C_6$-alkyl, $C_1$-$C_6$-aryl, $C_1$-$C_6$-acyl, halo-$C_1$-$C_6$-alkyl, $C_1$-$C_6$-alkyl-$C_1$-$C_6$-alkyl, $C_1$-$C_6$-alkyl-$C_1$-$C_6$-acyl, $C_1$-$C_6$-acyl-$C_1$-$C_6$-acyl, $C_1$-$C_6$-alkyl-$C_1$-$C_6$-aryl, $C_1$-$C_6$-aryl-$C_1$-$C_6$-alkyl or $CF_3$, in which $C_1$-$C_6$-alkyl, $C_1$-$C_6$-aryl, $C_1$-$C_6$-acyl, halo-$C_1$-$C_6$-alkyl, $C_1$-$C_6$-alkyl-$C_1$-$C_6$-alkyl, $C_1$-$C_6$-alkyl-$C_1$-$C_6$-acyl, $C_1$-$C_6$-acyl-$C_1$-$C_6$-acyl, $C_1$-$C_6$-alkyl-$C_1$-$C_6$-aryl or $C_1$-$C_6$-aryl-$C_1$-$C_6$-alkyl optionally can be interrupted in one or more places, in the same way or differently, by oxygen, sulfur or nitrogen, or for the group sulfonyl-$C_1$-$C_6$-alkyl, sulfonamide, or cyano,
$R^3$ stands for $C_6$-$C_{12}$-aryl, which optionally can be substituted in one or more places, in the same way or differently, with halogen, with $C_1$-$C_6$-alkyl, $C_1$-$C_3$-acyl, $C_1$-$C_3$-alkoxy, cyano, hydroxy, N-$(CH_3)_2$, $CO_2$-($C_1$-$C_3$-alkyl), CO-$NR^4R^5$ or with $CF_3$, $C_5$-$C_{12}$-heteroaryl, which optionally can be substituted in one or more places, in the same way or differently, with chlorine and/or fluorine, with $C_1$-$C_6$-alkyl, $C_1$-$C_3$-acyl, $C_1$-$C_3$-alkoxy, cyano, hydroxy, N-$(CH_3)_2$, $CO_2$-($C_1$-$C_3$-alkyl), CO-$NR^4R^5$ or with $CF_3$, $C_3$-$C_6$-cycloalkyl, which optionally can be substituted in one or more places, in the same way or differently, with chlorine and/or fluorine, $CF_3$, cyano, $C_1$-$C_3$-alkyl, $C_1$-$C_3$-acyl, hydroxy, N-$(CH_3)_2$, $CO_2$-($C_1$-$C_3$-alkyl), CO-$NR^4R^5$ or $C_1$-$C_3$-alkoxy,
$R^4$ stands for hydrogen or for the group $-(CH_2)_n$-N-$(CH_3)_2$, $-(CH_2)_2$-$CH_3$, $-(CH_2)_2$-NH-$COCH_3$, $-(CH_2)$-$CHCH_3$-OH, $-(CH_2)_2$-O-$CH_3$, $-(CH_2)_2$-OH, -$CHCH_3$-$CH_2$-OH,

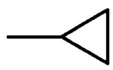 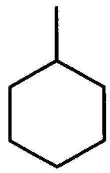 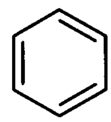 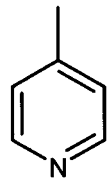

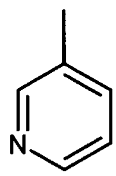 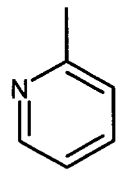 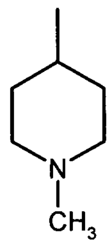 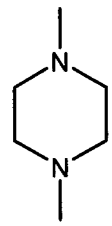 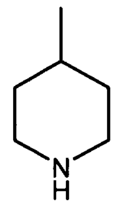

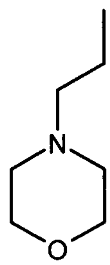 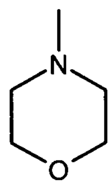 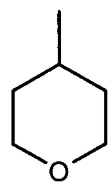 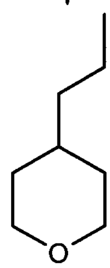 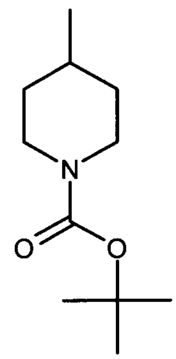

$R^5$ stands for hydrogen,
X stands for the groups sulfonyl, $(CH_2)_n$ or carbonyl,
Y stands for carbonyl or $(CH_2)_n$,
Z stands for nitrogen, and
n stands for 0 - 2.

**11.** Compounds according to claim 1, whereby

$R^1$ stands for hydrogen, halogen, $CF_3$, $C_3$-$C_6$-cycloalkyl, which optionally is polysaturated and optionally is polysubstituted, or for the group $C_1$-$C_6$-alkyl, $C_1$-$C_6$-aryl, $C_1$-$C_6$-acyl, halo-$C_1$-$C_6$-alkyl, $C_1$-$C_6$-alkyl-$C_1$-$C_6$-alkyl, $C_1$-$C_6$-alkyl-$C_1$-$C_6$-acyl, $C_1$-$C_6$-acyl-$C_1$-$C_6$-acyl, $C_1$-$C_6$-alkyl-$C_1$-$C_6$-aryl, $C_1$-$C_6$-aryl-$C_1$-$C_6$-alkyl or $CF_3$, in which $C_1$-$C_6$-alkyl, $C_1$-$C_6$-aryl, $C_1$-$C_6$-acyl, halo-$C_1$-$C_6$-alkyl, $C_1$-$C_6$-alkyl-$C_1$-$C_6$-alkyl, $C_1$-$C_6$-alkyl-$C_1$-$C_6$-acyl, $C_1$-$C_6$-acyl-$C_1$-$C_6$-acyl, $C_1$-$C_6$-alkyl-$C_1$-$C_6$-aryl or $C_1$-$C_6$-aryl-$C_1$-$C_6$-alkyl optionally can be interrupted in one or more places, in the same way or differently, by oxygen, sulfur or nitrogen, or for the group sulfonyl-$C_1$-$C_6$-alkyl, sulfonamide, or cyano,
$R^2$ stands for halogen, $CF_3$, $C_3$-$C_6$-cycloalkyl, which optionally is polysaturated and optionally is polysubstituted, or for the group $C_1$-$C_6$-alkyl, $C_1$-$C_6$-aryl, $C_1$-$C_6$-acyl, halo-$C_1$-$C_6$-alkyl, $C_1$-$C_6$-alkyl-$C_1$-$C_6$-alkyl, $C_1$-$C_6$-alkyl-$C_1$-$C_6$-acyl, $C_1$-$C_6$-acyl-$C_1$-$C_6$-acyl, $C_1$-$C_6$-alkyl-$C_1$-$C_6$-aryl, $C_1$-$C_6$-aryl-$C_1$-$C_6$-alkyl or $CF_3$, in which $C_1$-$C_6$-alkyl, $C_1$-$C_6$-aryl, $C_1$-$C_6$-acyl, halo-$C_1$-$C_6$-alkyl, $C_1$-$C_6$-alkyl-$C_1$-$C_6$-alkyl, $C_1$-$C_6$-alkyl-$C_1$-$C_6$-acyl, $C_1$-$C_6$-acyl-$C_1$-$C_6$-acyl, $C_1$-$C_6$-alkyl-$C_1$-$C_6$-aryl or $C_1$-$C_6$-aryl-$C_1$-$C_6$-alkyl optionally can be interrupted in one or more places, in the same way or differently, by oxygen, sulfur or nitrogen, or for the group sulfonyl-$C_1$-$C_6$-alkyl, sulfonamide, or cyano,
$R^3$ stands for $C_6$-$C_{12}$-aryl, which optionally can be substituted in one or more places, in the same way or differently, with halogen, with $C_1$-$C_6$-alkyl, $C_1$-$C_3$-acyl, $C_1$-$C_3$-alkoxy, cyano, hydroxy, N- $(CH_3)_2$, $CO_2$-($C_1$-$C_3$-alkyl), CO-$NR^4R^5$ or with $CF_3$, $C_5$-$C_{12}$-heteroaryl, which optionally can be substituted in one or more places, in the same way or differently, with chlorine and/or fluorine, with $C_1$-$C_6$-alkyl, $C_1$-$C_3$-acyl, $C_1$-$C_3$-alkoxy, cyano, hydroxy, N-$(CH_3)_2$, $CO_2$-($C_1$-$C_3$-alkyl), CO-$NR^4R^5$ or with $CF_3$, $C_3$-$C_6$-cycloalkyl, which

optionally can be substituted in one or more places, in the same way or differently, with chlorine and/or fluorine, $CF_3$, cyano, $C_1$-$C_3$-alkyl, $C_1$-$C_3$-acyl, hydroxy, N-$(CH_3)_2$, $CO_2$-($C_1$-$C_3$-alkyl), CO-$NR^4R^5$ or $C_1$-$C_3$-alkoxy,
$R^4$ stands for hydrogen, $C_3$-$C_6$-cycloalkyl, which optionally is substituted in one or more places, in the same way or differently, with $C_1$-$C_3$-alkyl, $C_1$-$C_3$-acyl, $C_1$-$C_3$-alkoxy or $CF_3$, for $C_6$-$C_{12}$-aryl, which optionally is substituted in one or more places, in the same way or differently, with halogen, with $C_1$-$C_3$-alkyl, $C_1$-$C_3$-acyl, $C_1$-$C_3$-alkoxy, N-$C_1$-$C_3$-alkyl-$C_1$-$C_3$-alkyl, $CF_3$ or cyano, or for $C_5$-$C_{12}$-heteroaryl, which optionally is substituted in one or more places, in the same way or differently, with halogen, with $C_1$-$C_3$-alkyl, $C_1$-$C_3$-acyl, $C_1$-$C_3$-alkoxy, N-$C_1$-$C_3$-alkyl-$C_1$-$C_3$-alkyl, $CF_3$ or cyano, or for $C_1$-$C_6$-alkyl, which can be substituted in any way desired,
$R^5$ stands for hydrogen, $C_3$-$C_6$-cycloalkyl, which optionally is substituted in one or more places, in the same way or differently, with $C_1$-$C_3$-alkyl, $C_1$-$C_3$-acyl, $C_1$-$C_3$-alkoxy or $CF_3$, for $C_6$-$C_{12}$-aryl, which optionally is substituted in one or more places, in the same way or differently, with halogen, with $C_1$-$C_3$-alkyl, $C_1$-$C_3$-acyl, $C_1$-$C_3$-alkoxy, N-$C_1$-$C_3$-alkyl-$C_1$-$C_3$-alkyl, $CF_3$ or cyano, or for $C_5$-$C_{12}$-heteroaryl, which optionally is substituted in one or more places, in the same way or differently, with halogen, with $C_1$-$C_3$-alkyl, $C_1$-$C_3$-acyl, $C_1$-$C_3$-alkoxy, N-$C_1$-$C_3$-alkyl-$C_1$-$C_3$-alkyl, $CF_3$ or cyano, or for $C_1$-$C_6$-alkyl, which can be substituted in any way desired,
$R^4$ and $R^5$ together form a 5- to 8-membered ring that can contain additional heteroatoms,
X stands for the groups sulfonyl, $(CH_2)_n$ or carbonyl,
Y stands for carbonyl or $(CH_2)_n$,
Z stands for nitrogen or for

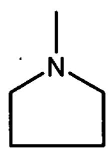 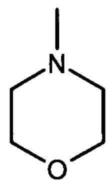 ,

and
n stands for 0 - 2.

**12.** Compounds according to claims 1-11 that are selected from the group that contains the following compounds:

1. 5-(4-tert-Butylbenzenesulfonylamino)-3-phenyl-1*H*-indole-2-carboxylic acid-cyclopropylamide
2. 5-(4-tert-Butylbenzenesulfonylamino)-3-phenyl-1*H*-indole-2-carboxylic acid-pyridin-3-yl amide
3. 5-(4-tert-Butylbenzenesulfonylamino)-3-phenyl-1*H*-indole-2-carboxylic acid-cyclohexylamide
4. 4-tert-Butyl-N-[3-phenyl-2-(pyrrolidine-1-carbonyl)-1*H*-indol-5-yl]-benzenesulfonamide
5. 4-tert-Butyl-N-[2-(morpholine-4-carbonyl)-3-phenyl-1*H*-indol-5-yl]-benzene-sulfonamide
6. 5-(4-tert-Butylbenzenesulfonylamino)-3-phenyl-1*H*-indole-2-carboxylic acid-(2-dimethylaminoethyl)amide
7. 5-(4-Cyanobenzenesulfonylamino)-3-phenyl-1*H*-indole-2-carboxylic acid-propylamide
8. 5-(4-Bromobenzenesulfonylamino)-3-phenyl-1*H*-indole-2-carboxylic acid-propylamide
9. 5-(4-tert-Butylbenzenesulfonylamino)-3-phenyl-1*H*-indole-2-carboxylic acid-propylamide
10. 5-(4-(Trifluoromethoxy)benzenesulfonylamino)-3-phenyl-1*H*-indole-2-carboxylic acid-propylamide
11. 5-(4-(Methylsulfonyl)benzenesulfonylamino)-3-phenyl-1*H*-indole-2-carboxylic acid-propylamide
12. 5-(4-tert-Butylbenzenesulfonylamino)-3-phenyl-1*H*-indole-2-carboxylic acid-phenylamide
13. 5-(4-Cyanobenzenesulfonylamino)-3-phenyl-1*H*-indole-2-carboxylic acid-phenylamide
14. 5-(4-Bromobenzenesulfonylamino)-3-phenyl-1*H*-indole-2-carboxylic acid-phenylamide
15. 5-(4-(Trifluoromethoxy)benzenesulfonylamino)-3-phenyl-1*H*-indole-2-carboxylic acid-phenylamide
16. 5-(4-(Methylsulfonyl)benzenesulfonylamino)-3-phenyl-1*H*-indole-2-carboxylic acid-phenylamide
17. 5-(4-tert-Butylbenzenesulfonylamino)-3-phenyl-1*H*-indole-2-carboxylic acid-pyridin-2-ylamide
18. 5-(4-Cyanobenzenesulfonylamino)-3-phenyl-1*H*-indole-2-carboxylic acid-pyridin-2-ylamide
19. 5-(4-tert-Butylbenzenesulfonylamino)-3-phenyl-1*H*-indole-2-carboxylic acid-(2-morpholin-4-ylethyl)-amide
20. 5-(4-tert-Butylbenzenesulfonylamino)-3-phenyl-1H-indole-2-carboxylic acid-(4-methylpiperazin-1-yl)amide
21. 5-(4-tert-Butylbenzenesulfonylamino)-3-phenyl-1*H*-indole-2-carboxylic acid pyridin-4-ylamide
22. 5-(4-tert-Butylbenzylamino)-3-phenyl-1*H*-indole-2-carboxylic acid-pyridin-4-ylamide
23. 5-(4-tert-Butylbenzoylamino)-3-phenyl-1*H*-indole-2-carboxylic acid-(2-dimethylaminoethyl)amide
24. 5-(4-tert-Butylbenzenesulfonylamino)-3-(2-chlorophenyl)-1*H*-indole-2-carboxylic acid-(2-dimethylaminoethyl)amide
25. 5-(4-tert-Butylbenzenesulfonylamino)-3-(3-chlorophenyl)-1*H*-indole-2-carboxylic acid-(2-dimethylaminoethyl)amide

26. 5-(4-tert-Butylbenzenesulfonylamino)-3-(4-chlorophenyl)-1*H*-indole-2-carboxylic acid-(2-dimethylaminoethyl)amide

27. 5-(4-tert-Butylbenzenesulfonylamino)-3-(2,4-dichlorophenyl)-1H-indole-2-carboxylic acid-(2-dimethylaminoethyl)amide

28. 5-(4-tert-Butylbenzenesulfonylamino)-3-(2-methylphenyl)-1H-indole-2-carboxylic acid-(2-dimethylaminoethyl)amide

29. 5-(4-tert-Butylbenzenesulfonylamino)-3-(4-methylphenyl)-1*H*-indole-2-carboxylic acid-(2-dimethylaminoethyl)amide

30. 5-(4-tert-Butylbenzenesulfonylamino)-3-(4-methylphenyl)-1*H*-indole-2-carboxylic acid pyridin-4-ylamide

31. 5-(4-tert-Butylbenzenesulfonylamino)-3-(4-methoxyphenyl)-1*H*-indole-2-carboxylic acid-(2-dimethylaminoethyl)amide

32. 5-(4-tert-Butylbenzenesulfonylamino)-3-pyridin-3-yl-1*H*-indole-2-carboxylic acid-(2-dimethylaminoethyl) amide

33. 5-(4-tert-Butylbenzenesulfonylamino)-3-(4-hydroxyphenyl)-1*H*-indole-2-carboxylic acid-(2-dimethylaminoethyl)amide

34. 5-(4-tert-Butylbenzenesulfonylamino)-3-(3-fluorophenyl)-1*H*-indole-2-carboxylic acid-(2-dimethylaminoethyl)amide

35. 5-(4-tert-Butylbenzenesulfonylamino)-3-phenyl-1*H*-indole-2-carboxylic acid-(2-hydroxypropyl)amide

36. 5-(4-tert-Butylbenzenesulfonylamino)-3-phenyl-1*H*-indole-2-carboxylic acid-(2-methoxyethyl)amide

37. 5-(4-tert-Butylbenzenesulfonylamino)-3-phenyl-1*H*-indole-2-carboxylic acid-(2-hydroxyethyl)amide

38. 5-(4-tert-Butylbenzenesulfonylamino)-3-phenyl-1*H*-indole-2-carboxylic acid-(2-hydroxy-1-methylethyl) amide

39. 5-(4-tert-Butylbenzenesulfonylamino)-3-phenyl-1*H*-indole-2-carboxylic acid-(2-acetylaminoethyl)amide

40. 5-(4-tert-Butylbenzenesulfonylamino)-3-phenyl-1*H*-indole-2-carboxylic acid-(tetrahydropyran-4-yl)-amide

41. 5-(4-tert-Butylbenzenesulfonylamino)-3-phenyl-1*H*-indole-2-carboxylic acid-(1-methylpiperidin-4-yl)amide

42. 5-(4-tert-Butylbenzenesulfonylamino)-3-(4-N,N-dimethylamino-phenyl)-1*H*-indole-2-carboxylic acid-(2-dimethylaminoethyl)amide

43. 5-(4-tert-Butylbenzenesulfonylamino)-3-(3-methoxyphenyl)-1*H*-indole-2-carboxylic acid-(2-dimethylaminoethyl)amide

44. 5-(4-tert-Butylbenzenesulfonylamino)-3-(3-trifluoromethylphenyl)-1*H*-indole-2-carboxylic acid-(2-dimethylaminoethyl)amide

45. 5-(4-tert-Butylbenzenesulfonylamino)-3-(3-fluorophenyl)-1*H*-indole-2-carboxylic acid-(2-hydroxyethyl) amide

46. 5-(4-tert-Butylbenzenesulfonylamino)-3-(3-fluorophenyl)-1*H*-indole-2-carboxylic acid-(tetrahydropyran-4-yl)amide

47. 5-(4-tert-Butylbenzenesulfonylamino)-3-(3-fluorophenyl)-1*H*-indole-2-carboxylic acid-(2-acetylaminoethyl) amide

48. 5-(4-tert-Butylbenzenesulfonylamino)-3-(3-fluorophenyl)-1*H*-indole-2-carboxylic acid-(2-morpholin-4-ylethyl)amide

49. 5-(4-tert-Butylbenzenesulfonylamino)-3-(3-methoxyphenyl)-1*H*-indole-2-carboxylic acid-(2-hydroxyethyl) amide

50. 5-(4-tert-Butylbenzenesulfonylamino)-3-(3-methoxyphenyl)-1*H*-indole-2-carboxylic acid-(2-acetylaminoethyl)amide

51. 5-(4-tert-Butylbenzenesulfonylamino)-3-pyridin-3-yl-1*H*-indole-2-carboxylic acid-(2-acetylaminoethyl) amide

52. 5-(4-tert-Butylbenzenesulfonylamino)-3-pyridin-3-yl-1*H*-indole-2-carboxylic acid-(tetrahydropyran-4-yl) amide

53. 5-(4-tert-Butylbenzenesulfonylamino)-3-pyridin-4-yl-1*H*-indole-2-carboxylic acid-(2-acetylaminoethyl) amide

54. 5-(4-tert-Butylbenzenesulfonylamino)-3-pyridin-4-yl-1*H*-indole-2-carboxylic acid-(2-morpholin-4-yl-ethyl) amide

55. 5-(4-tert-Butylbenzenesulfonylamino)-3-pyridin-4-yl-1*H*-indole-2-carboxylic acid-(tetrahydropyran-4-yl) amide

56. 5-(4-tert-Butylbenzenesulfonylamino)-3-(3-methylphenyl)-1*H*-indole-2-carboxylic acid-(2-acetylaminoethyl)amide

57. 5-(4-tert-Butylbenzenesulfonylamino)-3-(3-methylphenyl)-1*H*-indole-2-carboxylic acid-(2-hydroxyethyl) amide

58. 5-(4-tert-Butylbenzenesulfonylamino)-3-(3-methylphenyl)-1*H*-indole-2-carboxylic acid-(2-morpholin-4-ylethyl)amide
59. 5-(4-tert-Butylbenzenesulfonylamino)-3-(3-methylphenyl)-1*H*-indole-2-carboxylic acid-(tetrahydropyran-4-yl)amide
60. 5-(4-tert-Butylbenzenesulfonylamino)-3-pyridin-3-yl-1*H*-indole-2-carboxylic acid-(2-morpholin-4-ylethyl) amide
61. 5-(4-tert-Butylbenzenesulfonylamino)-3-(3-methoxyphenyl)-1*H*-indole-2-carboxylic acid-(tetrahydropyran-4-yl)amide
62. 5-(4-tert-Butylbenzenesulfonylamino)-3-(3-methoxyphenyl)-1*H*-indole-2-carboxylic acid-(2-morpholin-4-ylethyl)amide
63. 5-(4-tert-Butylbenzenesulfonylamino)-3-phenyl-1H-indole-2-carboxylic acid piperidin-4-ylamide
64. 4-{[5-(4-tert-Butylbenzenesulfonylamino)-3-phenyl-1H-indole-2-carbonyl]amino}piperidine-1-carboxylic acid-tert-butyl ester
65. 5-(4-tert-Butyl-phenylsulfonyl-amino)-3-naphthalin-1-yl-1H-indole-2-carboxylic acid-(2-morpholin-4-yl-ethyl)-amide
66. 5-(4-tert-Butyl-phenylsulfonyl-amino)-3-m-tolyl-1H-indole-2-carboxylic acid-(2-morpholin-4-yl-ethyl)-amide
67. 5-(4-tert-Butyl-phenylsulfonyl-amino)-3-thiophen-2-yl-1H-indole-2-carboxylic acid-(2-morpholin-4-yl-ethyl)-amide
68. 5-(4-tert-Butyl-phenylsulfonyl-amino)-3-thiophen-3-yl-1H-indole-2-carboxylic acid-(2-morpholin-4-yl-ethyl)-amide
69. 3-Benzofuran-2-yl-5-(4-tert-butyl-phenylsulfonyl-amino)-1H-indole-2-carboxylic acid-(2-morpholin-4-yl-ethyl)-amide
70. 5-(4-tert-Butyl-phenylsulfonyl-amino)-3-(5-chloro-thiophen-2-yl)-1H-indole-2-carboxylic acid-(2-morpholin-4-yl-ethyl)-amide
71. 5-(4-tert-Butyl-phenylsulfonyl-amino)-3-furan-2-yl-1H-indole-2-carboxylic acid-(2-morpholin-4-yl-ethyl)-amide
72. 5-(4-tert-Butyl-phenylsulfonyl-amino)-3-(3-fluoro-4-methoxy-phenyl)-1H-indole-2-carboxylic acid-(2-morpholin-4-yl-ethyl)-amide
73. 3-Benzo[1,3]dioxol-5-yl-5-(4-tert-butyl-phenylsulfonylamino)-1H-indole-2-carboxylic acid-(2-morpholin-4-yl-ethyl)-amide
74. 3-(4-Acetyl-phenyl)-5-(4-tert-butyl-phenylsulfonylamino)-1H-indole-2-carboxylic acid-(2-morpholin-4-yl-ethyl)-amide
75. 3-(3-Acetyl-phenyl)-5-(4-tert-butyl-phenylsulfonylamino)-1H-indole-2-carboxylic acid-(2-morpholin-4-yl-ethyl)-amide
76. 3-Benzo[b]thiophen-2-yl-5-(4-tert-butyl-phenylsulfonylamino)-1H-indole-2-carboxylic acid-(2-morpholin-4-yl-ethyl)-amide
77. 3-Benzo[b]thiophen-3-yl-5-(4-tert-butyl-phenylsulfonylamino)-1H-indole-2-carboxylic acid-(2-morpholin-4-yl-ethyl)-amide
78. 5-(4-tert-Butyl-phenylsulfonylamino)-3-(5-methylthiophen-2-yl)-1H-indole-2-carboxylic acid-(2-morpholin-4-yl-ethyl)-amide
79. 3-[5-(4-tert-Butyl-phenylsulfonyl-amino)-2-(2-morpholin-4-yl-ethylcarbamoyl)-1H-indol-3-yl]-benzoic acid methyl ester
80. 5-(4-tert-Butyl-phenylsulfonyl-amino)-3-(2-fluoro-3-methoxyphenyl)-1H-indole-2-carboxylic acid-(2-morpholin-4-yl-ethyl)-amide
81. 5-(4-tert-Butyl-phenylsulfonyl-amino)-3-(3-chloro-4-methyl-phenyl)-1H-indole-2-carboxylic acid-(2-morpholin-4-yl-ethyl)-amide
82. 5-(4-tert-Butyl-phenylsulfonyl-amino)-3-(2,4-dimethoxy-pyrimidin-5-yl)-1H-indole-2-carboxylic acid-(2-morpholin-4-yl-ethyl)-amide
83. 5-(4-tert-Butyl-phenylsulfonyl-amino)-3-(2,5-difluoro-phenyl)-1H-indole-2-carboxylic acid-(2-morpholin-4-yl-ethyl)-amide
84. 5-(4-tert-Butyl-phenylsulfonyl-amino)-3-(2,4-difluoro-phenyl)-1H-indole-2-carboxylic acid-(2-morpholin-4-yl-ethyl)-amide
85. 5-(4-tert-Butyl-phenylsulfonyl-amino)-3-(2,3-difluoro-phenyl)-1H-indole-2-carboxylic acid-(2-morpholin-4-yl-ethyl)-amide
86. 5-(4-tert-Butyl-phenylsulfonyl-amino)-3-(2,6-difluoro-phenyl)-1H-indole-2-carboxylic acid-(2-morpholin-4-yl-ethyl)-amide
87. 5-(4-tert-Butyl-phenylsulfonyl-amino)-3-(3-hydroxy-phenyl)-1H-indole-2-carboxylic acid-(2-morpholin-4-yl-ethyl)-amide

88. 5-(4-tert-Butyl-phenylsulfonyl-amino)-3-(4-hydroxyphenyl)-1H-indole-2-carboxylic acid-(2-morpholin-4-yl-ethyl)-amide
89. 5-(4-tert-Butyl-phenylsulfonyl-amino)-3-(3-fluoro-4-methylphenyl)-1H-indole-2-carboxylic acid-(2-morpholin-4-yl-ethyl)-amide
90. 5-(4-tert-Butyl-phenylsulfonyl-amino)-3-(4-trifluoromethyl-phenyl)-1H-indole-2-carboxylic acid-(2-morpholin-4-yl-ethyl)-amide
91. 5-(4-tert-Butyl-phenylsulfonyl-amino)-3-(4-cyanomethyl-phenyl)-1H-indole-2-carboxylic acid-(2-morpholin-4-yl-ethyl)-amide
92. 5-(4-tert-Butyl-phenylsulfonyl-amino)-1H,1'H-[3,4']biindolyl-2-carboxylic acid-(2-morpholin-4-yl-ethyl)-amide
93. 5-(4-tert-Butyl-phenylsulfonyl-amino)-3-(3-cyano-4-fluoro-phenyl)-1H-indole-2-carboxylic acid-(2-morpholin-4-yl-ethyl)-amide
94. 5-(4-tert-Butyl-phenylsulfonyl-amino)-3-(2-fluorophenyl)-1H-indole-2-carboxylic acid-(2-morpholin-4-yl-ethyl)-amide
95. 5-(4-tert-Butyl-phenylsulfonylamino)-3-(3,4-difluoro-phenyl)-1H-indole-2-carboxylic acid-(2-morpholin-4-yl-ethyl)-amide
96. 5-(4-tert-Butyl-phenylsulfonyl-amino)-3-(3-cyanophenyl)-1H-indole-2-carboxylic acid-(2-morpholin-4-yl-ethyl)-amide
97. 5-(4-tert-Butyl-phenylsulfonyl-amino)-3-(4-cyanophenyl)-1H-indole-2-carboxylic acid-(2-morpholin-4-yl-ethyl)-amide
98. 5-(4-tert-Butyl-phenylsulfonyl-amino)-3-(4-methylthiophen-2-yl)-1H-indole-2-carboxylic acid-(2-morpholin-4-yl-ethyl)-amide
99. 5-(4-tert-Butyl-phenylsulfonyl-amino)-3-phenyl-1H-indole-2-carboxylic acid-(3-chloro-phenyl)-amide
100. 5-(4-tert-Butyl-phenylsulfonyl-amino)-3-phenyl-1H-indole-2-carboxylic acid-(3-methyl-isoxazol-5-yl)-amide
101. 5-(4-tert-Butyl-phenylsulfonyl-amino)-3-phenyl-1H-indole-2-carboxylic acid-(4-fluoro-phenyl)-amide
102. 5-(4-tert-Butyl-phenylsulfonyl-amino)-3-phenyl-1H-indole-2-carboxylic acid-(2-fluoro-phenyl)-amide
103. 5-(4-tert-Butyl-phenylsulfonyl-amino)-3-phenyl-1H-indole-2-carboxylic acid-(6-methyl-pyridin-2-yl)-amide
104. 5-(4-tert-Butyl-phenylsulfonyl-amino)-3-phenyl-1H-indole-2-carboxylic acid-(5-carbamoyl-pyridin-2-yl)-amide
105. 5-(4-tert-Butyl-phenylsulfonyl-amino)-3-phenyl-1H-indole-2-carboxylic acid-(4-hydroxy-phenyl)-amide
106. 5-(4-tert-Butyl-phenylsulfonyl-amino)-3-phenyl-1H-indole-2-carboxylic acid-(2-methoxy-phenyl)-amide
107. 5-(4-tert-Butyl-phenylsulfonyl-amino)-3-phenyl-1H-indole-2-carboxylic acid-(3-methoxy-phenyl)-amide
108. 5-(4-tert-Butyl-phenylsulfonyl-amino)-3-phenyl-1H-indole-2-carboxylic acid-(4-methoxy-phenyl)-amide
109. 5-(4-tert-Butyl-phenylsulfonyl-amino)-3-phenyl-1H-indole-2-carboxylic acid-(4-chloro-phenyl)-amide
110. 5-(4-tert-Butyl-phenylsulfonyl-amino)-3-phenyl-1H-indole-2-carboxylic acid-(4-dimethylamino-phenyl)-amide
111. 5-(4-tert-Butyl-phenylsulfonyl-amino)-3-phenyl-1H-indole-2-carboxylic acid-(5-chloro-pyridin-2-yl)-amide
112. 5-(4-tert-Butyl-phenylsulfonyl-amino)-3-phenyl-1H-indole-2-carboxylic acid-p-tolylamide
113. 5-(4-tert-Butyl-phenylsulfonyl-amino)-3-phenyl-1H-indole-2-carboxylic acid-pyrazin-2-ylamide
114. 5-(4-tert-Butyl-phenylsulfonyl-amino)-3-phenyl-1H-indole-2-carboxylic acid-(4-cyano-phenyl)-amide
115. 5-(4-tert-Butyl-phenylsulfonyl-amino)-3-phenyl-1H-indole-2-carboxylic acid-(3-methyl-isothiazol-5-yl)-amide
116. 5-(4-tert-Butyl-phenylsulfonyl-amino)-3-phenyl-1H-indole-2-carboxylic acid-(4-bromo-phenyl)-amide
117. 5-(4-tert-Butyl-phenylsulfonyl-amino)-3-phenyl-1H-indole-2-carboxylic acid-(4-carbamoyl-phenyl)-amide
118. 5-(4-tert-Butyl-phenylsulfonyl-amino)-3-phenyl-1H-indole-2-carboxylic acid-(3-methyl-pyridin-2-yl)-amide
119. 5-(4-tert-Butyl-phenylsulfonyl-amino)-3-phenyl-1H-indole-2-carboxylic acid-(2-chlorophenyl)-amide
120. 5-(4-tert-Butyl-phenylsulfonyl-amino)-3-phenyl-1H-indole-2-carboxylic acid-(5-methyl-2H-pyrazol-3-yl)-amide
121. 5-(4-tert-Butyl-phenylsulfonyl-amino)-3-phenyl-1H-indole-2-carboxylic acid-quinolin-5-ylamide
122. 5-(4-tert-Butyl-phenylsulfonyl-amino)-3-phenyl-1H-indole-2-carboxylic acid-quinolin-6-ylamide
123. 5-(4-tert-Butyl-phenylsulfonyl-amino)-3-phenyl-1H-indole-2-carboxylic acid-(2,6-dichloro-pyridin-4-yl)-amide
124. 5-(4-tert-Butyl-phenylsulfonyl-amino)-3-phenyl-1H-indole-2-carboxylic acid-(3-fluoro-phenyl)-amide
125. 5-(4-tert-Butyl-phenylsulfonyl-amino)-3-phenyl-1H-indole-2-carboxylic acid-(2-methyl-pyridin-4-yl)-amide
126. 5-(4-tert-Butyl-phenylsulfonyl-amino)-3-phenyl-1H-indole-2-carboxylic acid-(3-fluoro-pyridin-4-yl)-amide
127. 5-(4-tert-Butyl-phenylsulfonyl-amino)-3-phenyl-1H-indole-2-carboxylic acid-(3-methyl-pyridin-4-yl)-amide
128. 5-(4-tert-Butyl-phenylsulfonyl-amino)-3-phenyl-1H-indole-2-carboxylic acid-(3-bromo-pyridin-4-yl)-amide

129. 5-(4-tert-Butyl-phenylsulfonyl-amino)-3-phenyl-1H-indole-2-carboxylic acid-(2,3-dihydroxy-propyl)-amide
130. 5-(4-tert-Butyl-phenylsulfonyl-amino)-3-phenyl-1H-indole-2-carboxylic acid-(2-oxo-tetrahydrothiophen-3-yl)-amide
131. 5-(4-tert-Butyl-phenylsulfonyl-amino)-3-phenyl-1H-indole-2-carboxylic acid-[2-(2-oxo-imidazolidin-1-yl)-ethyl]-amide
132. 5-(4-tert-Butyl-phenylsulfonyl-amino)-3-phenyl-1H-indole-2-carboxylic acid-(2,2-diethoxy-ethyl)-amide
133. 5-(4-tert-Butyl-phenylsulfonyl-amino)-3-phenyl-1H-indole-2-carboxylic acid-(3-ethoxy-propyl)-amide
134. 5-(4-tert-Butyl-phenylsulfonyl-amino)-3-phenyl-1H-indole-2-carboxylic acid-(3-isopropoxy-propyl)-amide
135. 5-(4-tert-Butyl-phenylsulfonyl-amino)-3-phenyl-1H-indole-2-carboxylic acid-(3-morpholin-4-yl-propyl)-amide
136. 5-(4-tert-Butyl-phenylsulfonyl-amino)-3-phenyl-1H-indole-2-carboxylic acid-(3-diethylamino-propyl)-amide
137. 5-(4-tert-Butyl-phenylsulfonyl-amino)-3-phenyl-1H-indole-2-carboxylic acid-(3-dimethylamino-propyl)-amide
138. 5-(4-tert-Butyl-phenylsulfonyl-amino)-3-phenyl-1H-indole-2-carboxylic acid-(furan-2-ylmethyl)-amide
139. 5-(4-tert-Butyl-phenylsulfonyl-amino)-3-phenyl-1H-indole-2-carboxylic acid-(2-methylsulfanyl-ethyl)-amide
140. 5-(4-tert-Butyl-phenylsulfonyl-amino)-3-phenyl-1H-indole-2-carboxylic acid-(2-diethylamino-ethyl)-amide
141. 5-(4-tert-Butyl-phenylsulfonyl-amino)-3-phenyl-1H-indole-2-carboxylic acid-[2-(3,4-dimethoxyphenyl)-ethyl]-amide
142. 5-(4-tert-Butyl-phenylsulfonyl-amino)-3-phenyl-1H-indole-2-carboxylic acid-(2-piperidin-1-yl-ethyl)-amide
143. 5-(4-tert-Butyl-phenylsulfonyl-amino)-3-phenyl-1H-indole-2-carboxylic acid-(3-pyrrolidin-1-yl-propyl)-amide
144. 5-(4-tert-Butyl-phenylsulfonyl-amino)-3-phenyl-1H-indole-2-carboxylic acid-phenethyl-amide
145. 5-(4-tert-Butyl-phenylsulfonyl-amino)-3-phenyl-1H-indole-2-carboxylic acid-(2-methoxy-1-methylethyl)-amide
146. 5-(4-tert-Butyl-phenylsulfonyl-amino)-3-phenyl-1H-indole-2-carboxylic acid-(pyridin-2-ylmethyl)-amide
147. 5-(4-tert-Butyl-phenylsulfonyl-amino)-3-phenyl-1H-indole-2-carboxylic acid-(pyridin-3-ylmethyl)-amide
148. 5-(4-tert-Butyl-phenylsulfonyl-amino)-3-phenyl-1H-indole-2-carboxylic acid-(pyridin-4-ylmethyl)-amide
149. 5-(4-tert-Butyl-phenylsulfonyl-amino)-3-phenyl-1H-indole-2-carboxylic acid-(4-diethylamino-1-methyl-butyl)-amide
150. 5-(4-tert-Butyl-phenylsulfonyl-amino)-3-phenyl-1H-indole-2-carboxylic acid-(2-imidazol-1-yl-ethyl)-amide
151. 5-(4-tert-Butyl-phenylsulfonyl-amino)-3-phenyl-1H-indole-2-carboxylic acid-benzylamide
152. 5-(4-tert-Butyl-phenylsulfonyl-amino)-3-phenyl-1H-indole-2-carboxylic acid-(2,2,2-trifluoro-ethyl)-amide
153. 5-(4-tert-Butyl-phenylsulfonyl-amino)-3-phenyl-1H-indole-2-carboxylic acid-4-methoxy-benzylamide
154. 5-(4-tert-Butyl-phenylsulfonyl-amino)-3-phenyl-1H-indole-2-carboxylic acid-cyclopentylamide
155. 5-(4-tert-Butyl-phenylsulfonyl-amino)-3-phenyl-1H-indole-2-carboxylic acid-(3-methyl-butyl)-amide
156. 5-(4-tert-Butyl-phenylsulfonyl-amino)-3-phenyl-1H-indole-2-carboxylic acid-[3-(4-methyl-piperazin-1-yl)-propyl]-amide
157. 5-(4-tert-Butyl-phenylsulfonyl-amino)-3-phenyl-1H-indole-2-carboxylic acid-[2-(4-hydroxy-phenyl)-ethyl]-amide
158. 5-(4-tert-Butyl-phenylsulfonyl-amino)-3-phenyl-1H-indole-2-carboxylic acid-[2-(4-chloro-phenyl)-ethyl]-amide
159. 5-(4-tert-Butyl-phenylsulfonyl-amino)-3-phenyl-1H-indole-2-carboxylic acid-cyclopropylamide
160. 5-(4-tert-Butyl-phenylsulfonyl-amino)-3-phenyl-1H-indole-2-carboxylic acid-cyclohexylmethyl-amide
161. 5-(4-tert-Butyl-phenylsulfonyl-amino)-3-phenyl-1H-indole-2-carboxylic acid-(tetrahydro-furan-2-ylmethyl)-amide
162. 5-(4-tert-Butyl-phenylsulfonyl-amino)-3-phenyl-1H-indole-2-carboxylic acid-(thiophen-2-ylmethyl)-amide
163. 5-(4-tert-Butyl-phenylsulfonyl-amino)-3-phenyl-1H-indole-2-carboxylic acid-4-fluoro-benzylamide
164. 5-(4-tert-Butyl-phenylsulfonyl-amino)-3-phenyl-1H-indole-2-carboxylic acid-(2-thiophen-2-yl-ethyl)-amide
165. 5-(4-tert-Butyl-phenylsulfonyl-amino)-3-phenyl-1H-indole-2-carboxylic acid-(2-pyrrolidin-1-yl-ethyl)-amide
166. 5-(4-tert-Butyl-phenylsulfonyl-amino)-3-phenyl-1H-indole-2-carboxylic acid-4-methyl-benzylamide
167. 5-(4-tert-Butyl-phenylsulfonyl-amino)-3-phenyl-1H-indole-2-carboxylic acid-(1-ethyl-pyrrolidin-2-ylmethyl)-amide
168. 5-(4-tert-Butyl-phenylsulfonyl-amino)-3-phenyl-1H-indole-2-carboxylic acid-(2-pyridin-3-yl-ethyl)-amide
169. 5-(4-tert-Butyl-phenylsulfonyl-amino)-3-phenyl-1H-indole-2-carboxylic acid-3-chloro-benzylamide
170. 5-(4-tert-Butyl-phenylsulfonyl-amino)-3-phenyl-1H-indole-2-carboxylic acid-[2-(3-chlorophenyl)-ethyl]-amide

171. 5-(4-tert-Butyl-phenylsulfonyl-amino)-3-phenyl-1H-indole-2-carboxylic acid-((R)-2-hydroxy-1-phenylethyl)-amide
172. 5-(4-tert-Butyl-phenylsulfonyl-amino)-3-phenyl-1H-indole-2-carboxylic acid-[3-(2-methyl-piperidin-1-yl)-propyl]-amide
173. 5-(4-tert-Butyl-phenylsulfonyl-amino)-3-phenyl-1H-indole-2-carboxylic acid-(3-phenyl-propyl)-amide
174. 5-(4-tert-Butyl-phenylsulfonyl-amino)-3-phenyl-1H-indole-2-carboxylic acid-(2-carbamoyl-ethyl)-amide
175. 5-(4-tert-Butyl-phenylsulfonyl-amino)-3-phenyl-1H-indole-2-carboxylic acid-[3-(5-methyl-1H-pyrazol-4-yl)-propyl]-amide
176. 5-(4-tert-Butyl-phenylsulfonyl-amino)-3-phenyl-1H-indole-2-carboxylic acid-(4-methyl-cyclohexyl)-amide
177. 5-(4-tert-Butyl-phenylsulfonyl-amino)-3-phenyl-1H-indole-2-carboxylic acid-((S)-2-methoxy-1-methylethyl)-amide
178. 5-(4-tert-Butyl-phenylsulfonyl-amino)-3-phenyl-1H-indole-2-carboxylic acid-cyclopropylmethyl-amide
179. 5-(4-tert-Butyl-phenylsulfonyl-amino)-3-phenyl-1H-indole-2-carboxylic acid-carbamoylmethyl-amide
180. 5-(4-tert-Butyl-phenylsulfonyl-amino)-3-phenyl-1H-indole-2-carboxylic acid-cycloheptylamide
181. 5-(4-tert-Butyl-phenylsulfonyl-amino)-3-phenyl-1H-indole-2-carboxylic acid-((R)-2-methoxy-1-methylethyl)-amide
182. 5-(4-tert-Butyl-phenylsulfonyl-amino)-3-phenyl-1H-indole-2-carboxylic acid-(furan-3-ylmethyl)-amide
183. 5-(4-tert-Butyl-phenylsulfonyl-amino)-3-phenyl-1H-indole-2-carboxylic acid-3-fluoro-benzylamide
184. 5-(4-tert-Butyl-phenylsulfonyl-amino)-3-phenyl-1H-indole-2-carboxylic acid-(5-methyl-pyrazin-2-ylmethyl)-amide
185. 5-(4-tert-Butyl-phenylsulfonyl-amino)-3-phenyl-1H-indole-2-carboxylic acid-(2-pyridin-2-yl-ethyl)-amide
186. 5-(4-tert-Butyl-phenylsulfonyl-amino)-3-phenyl-1H-indole-2-carboxylic acid-(2-phenoxy-ethyl)-amide
187. 5-(4-tert-Butyl-phenylsulfonyl-amino)-3-phenyl-1H-indole-2-carboxylic acid-(2-benzoimidazol-1-yl-ethyl)-amide
188. 5-(4-tert-Butyl-phenylsulfonyl-amino)-3-phenyl-1H-indole-2-carboxylic acid-(3-imidazol-1-yl-propyl)-amide
189. 5-(4-tert-Butyl-phenylsulfonyl-amino)-3-phenyl-1H-indole-2-carboxylic acid-(1-benzyl-piperidin-4-yl)-amide
190. 5-(4-tert-Butyl-phenylsulfonyl-amino)-3-phenyl-1H-indole-2-carboxylic acid-[3-(2-oxo-pyrrolidin-1-yl)-propyl]-amide
191. 5-(4-tert-Butyl-phenylsulfonyl-amino)-3-phenyl-1H-indole-2-carboxylic acid-[2-(1-methyl-pyrrolidin-2-yl)-ethyl]-amide
192. 5-(4-tert-Butyl-phenylsulfonyl-amino)-3-phenyl-1H-indole-2-carboxylic acid methyl-(2-morpholin-4-yl-ethyl)-amide

**13.** Pharmaceutical agents that contain at least one of the compounds according to claims 1-12.

**14.** Pharmaceutical agents according to claim 13 in which the compound of general formula 1 is contained in an effective dose.

**15.** Use of the compounds of general formula 1 according to claims 1-12 for the production of a pharmaceutical agent for treating diseases that are caused by disorders in cAMP metabolism.

**16.** Use of the compounds of general formula 1 according to claims 1-12 for the production of a pharmaceutical agent for non-hormonal contraception.

**17.** Use of the compound of general formula 1 according to claims 1-12 for the production of a pharmaceutical agent for inhibition of soluble adenylate cyclase.

**18.** Compounds according to claims 1-12 as pharmaceutical agents according to claims 13 and 14 with suitable formulation substances and vehicles.

**19.** Use of the compounds of general formula 1 according to claims 1-12 for the production of a pharmaceutical preparation for enteral, parenteral, vaginal and oral administration.

## Revendications

**1.** Composés de formule générale 1

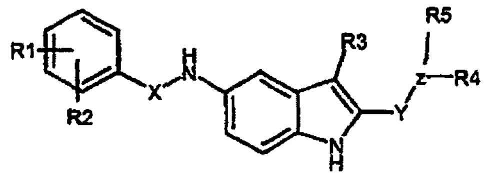

où

$R^1$ représente hydrogène, halogène, $CF_3$, $C_3$-$C_6$-cycloalkyle, qui est le cas échéant polyinsaturé et le cas échéant polysubstitué, ou représente le groupe $C_1$-$C_6$-alkyle, $C_1$-$C_6$-aryle, $C_1$-$C_6$-acyle, halogéno-$C_1$-$C_6$-alkyle, $C_1$-$C_6$-alkyl-$C_1$-$C_6$-alkyle, $C_1$-$C_6$-alkyl-$C_1$-$C_6$-acyle, $C_1$-$C_6$-acyl-$C_1$-$C_6$-acyle, $C_1$-$C_6$-alkyl-$C_1$-$C_6$-aryle, $C_1$-$C_6$-aryl-$C_1$-$C_6$-alkyle ou $CF_3$, où les groupes $C_1$-$C_6$-alkyle, $C_1$-$C_6$-aryle, $C_1$-$C_6$-acyle, halogéno-$C_1$-$C_6$-alkyle, $C_1$-$C_6$-alkyl-$C_1$-$C_6$-alkyle, $C_1$-$C_6$-alkyl-$C_1$-$C_6$-acyle, $C_1$-$C_6$-acyl-$C_1$-$C_6$-acyle, $C_1$-$C_6$-alkyl-$C_1$-$C_6$-aryle ou $C_1$-$C_6$-aryl-$C_1$-$C_6$-alkyle peuvent le cas échéant être interrompus une ou plusieurs fois, de manière identique ou différente, par oxygène, soufre ou azote, ou représente le groupe sulfonyl-$C_1$-$C_6$-alkyle, sulfonamide, ou cyano,

$R^2$ représente halogène, $CF_3$, $C_3$-$C_6$-cycloalkyle, qui est le cas échéant polyinsaturé et le cas échéant polysubstitué, ou représente le groupe $C_1$-$C_6$-alkyle, $C_1$-$C_6$-aryle, $C_1$-$C_6$-acyle, halogéno-$C_1$-$C_6$-alkyle, $C_1$-$C_6$-alkyl-$C_1$-$C_6$-alkyle, $C_1$-$C_6$-alkyl-$C_1$-$C_6$-acyle, $C_1$-$C_6$-acyl-$C_1$-$C_6$-acyle, $C_1$-$C_6$-alkyl-$C_1$-$C_6$-aryle, $C_1$-$C_6$-aryl-$C_1$-$C_6$-alkyle ou $CF_3$, où les groupes $C_1$-$C_6$-alkyle, $C_1$-$C_6$-aryle, $C_1$-$C_6$-acyle, halogéno-$C_1$-$C_6$-alkyle, $C_1$-$C_6$-alkyl-$C_1$-$C_6$-alkyle, $C_1$-$C_6$-alkyl-$C_1$-$C_6$-acyle, $C_1$-$C_6$-acyl-$C_1$-$C_6$-acyle, $C_1$-$C_6$-alkyl-$C_1$-$C_6$-aryle ou $C_1$-$C_6$-aryl-$C_1$-$C_6$-alkyle peuvent le cas échéant être interrompus une ou plusieurs fois, de manière identique ou différente, par oxygène, soufre ou azote, ou représente le groupe sulfonyl-$C_1$-$C_6$-alkyle, sulfonamide, ou cyano,

$R^3$ représente $C_6$-$C_{12}$-aryle, qui peut le cas échéant être monosubstitué ou polysubstitué, de manière identique ou différente, par halogène, par $C_1$-$C_6$-alkyle ou $C_1$-$C_6$-acyle, qui peut le cas échéant être monosubstitué ou polysubstitué, ou par $C_1$-$C_6$-alcoxy, hydroxy, cyano, $CO_2$-($C_1$-$C_6$-alkyle), N-($C_1$-$C_6$-alkyle)$_2$, CO-$NR^4R^6$ ou par $CF_3$, représente $C_5$-$C_{12}$-hétéroaryle, qui peut le cas échéant être monosubstitué ou polysubstitué, de manière identique ou différente, par halogène, par $C_1$-$C_6$-alkyle, $C_1$-$C_6$-acyle, $C_1$-$C_6$-alcoxy, hydroxy, cyano, $CO_2$-($C_1$-$C_6$-alkyle), N- ($C_1$-$C_6$-alkyle)$_2$, CO-$NR^4R^5$ ou par $CF_3$ ou représente $C_3$-$C_6$-cycloalkyle, qui peut le cas échéant être monosubstitué ou polysubstitué, de manière identique ou différente, par halogène, $CF_3$, hydroxy, cyano, $CO_2$-($C_1$-$C_6$-alkyle), $C_1$-$C_6$-alkyle, $C_1$-$C_6$-acyle, N-($C_1$-$C_6$-alkyle)$_2$, CO-$NR^4R^5$ ou $C_1$-$C_6$-alcoxy

$R^4$ représente hydrogène, $C_3$-$C_6$-cycloalkyle, qui est le cas échéant monosubstitué ou polysubstitué, de manière identique ou différente, par $C_1$-$C_6$-alkyle, $C_1$-$C_6$-acyle, $C_1$-$C_6$-alcoxy ou $CF_3$, représente $C_6$-$C_{12}$-aryle, qui est le cas échéant monosubstitué ou polysubstitué, de manière identique ou différente, par halogène, par $C_1$-$C_6$-alkyle, $C_1$-$C_6$-acyle, $C_1$-$C_6$-alcoxy, N-$C_1$-$C_6$-alkyl-$C_1$-$C_6$-alkyle, $CF_3$ ou cyano, ou représente $C_5$-$C_{12}$-hétéroaryle, qui est le cas échéant monosubstitué ou polysubstitué, de manière identique ou différente, par halogène, par $C_1$-$C_6$-alkyle, $C_1$-$C_6$-acyle, $C_1$-$C_6$-alcoxy, N-$C_1$-$C_6$-alkyl-$C_1$-$C_6$-alkyle, $CF_3$ ou cyano, ou représente $C_1$-$C_6$-alkyle, qui peut être substitué de manière quelconque,

$R^5$ représente hydrogène, $C_3$-$C_6$-cycloalkyle, qui est le cas échéant monosubstitué ou polysubstitué, de manière identique ou différente, par $C_1$-$C_6$-alkyle, $C_1$-$C_6$-acyle, $C_1$-$C_6$-alcoxy ou $CF_3$, représente $C_6$-$C_{12}$-aryle, qui est le cas échéant monosubstitué ou polysubstitué, de manière identique ou différente, par halogène, par $C_1$-$C_6$-alkyle, $C_1$-$C_6$-acyle, $C_1$-$C_6$-alcoxy, N-$C_1$-$C_6$-alkyl-$C_1$-$C_6$-alkyle, $CF_3$ ou cyano, ou représente $C_5$-$C_{12}$-hétéroaryle, qui est le cas échéant monosubstitué ou polysubstitué, de manière identique ou différente, par halogène, par $C_1$-$C_6$-alkyle, $C_1$-$C_6$-acyle, $C_1$-$C_6$-alcoxy, N-$C_1$-$C_6$-alkyl-$C_1$-$C_6$-alkyle, $CF_3$ ou cyano, ou représente $C_1$-$C_6$-alkyle, qui peut être substitué de manière quelconque,

$R^4$ et $R^5$ forment ensemble un cycle de 5-8 chaînons, qui peut contenir d'autres hétéroatomes et

X représente les groupes sulfonyle, $(CH_2)_n$ ou carbonyle,

Y représente carbonyle ou $(CH_2)$,

Z représente azote, et

n vaut 0-4.

**2.** Composés selon la revendication 1, où

$R^1$ représente hydrogène, halogène, $CF_3$, $C_3$-$C_6$-cycloalkyle, qui est le cas échéant polyinsaturé et le cas échéant polysubstitué, ou représente le groupe $C_1$-$C_6$-alkyle, $C_1$-$C_6$-aryle, $C_1$-$C_6$-acyle, halogéno-$C_1$-$C_6$-alkyle, $C_1$-$C_6$-alkyl-$C_1$-$C_6$-alkyle, $C_1$-$C_6$-alkyl-$C_1$-$C_6$-acyle, $C_1$-$C_6$-acyl-$C_1$-$C_6$-acyle, $C_1$-$C_6$-alkyl-$C_1$-$C_6$-aryle, $C_1$-$C_6$-aryl-$C_1$-$C_6$-alkyle ou $CF_3$, où les groupes $C_1$-$C_6$-alkyle, $C_1$-$C_6$-aryle, $C_1$-$C_6$-acyle, halogéno-$C_1$-$C_6$-alkyle, $C_1$-$C_6$-alkyl-$C_1$-$C_6$-alkyle, $C_1$-$C_6$-alkyl-$C_1$-$C_6$-acyle, $C_1$-$C_6$-acyl-$C_1$-$C_6$-acyle, $C_1$-$C_6$-alkyl-$C_1$-$C_6$-aryle ou $C_1$-$C_6$-aryl-$C_1$-$C_6$-alkyle peuvent le cas échéant être interrompus une ou plusieurs fois, de manière identique ou différente, par oxygène, soufre ou azote, ou représente le groupe sulfonyl-$C_1$-$C_6$-alkyle, sulfonamide, ou cyano,

$R^2$ représente halogène, $CF_3$, $C_3$-$C_6$-cycloalkyle, qui est le cas échéant polyinsaturé et le cas échéant polysubstitué, ou représente le groupe $C_1$-$C_6$-alkyle, $C_1$-$C_6$-aryle, $C_1$-$C_6$-acyle, halogéno-$C_1$-$C_6$-alkyle, $C_1$-$C_6$-alkyl-$C_1$-$C_6$-alkyle, $C_1$-$C_6$-alkyl-$C_1$-$C_6$-acyle, $C_1$-$C_6$-acyl-$C_1$-$C_6$-acyle, $C_1$-$C_6$-alkyl-$C_1$-$C_6$-aryle, $C_1$-$C_6$-aryl-$C_1$-$C_6$-alkyle ou $CF_3$, où les groupes $C_1$-$C_6$-alkyle, $C_1$-$C_6$-aryle, $C_1$-$C_6$-acyle, halogéno-$C_1$-$C_6$-alkyle, $C_1$-$C_6$-alkyl-$C_1$-$C_6$-alkyle, $C_1$-$C_6$-alkyl-$C_1$-$C_6$-acyle, $C_1$-$C_6$-acyl-$C_1$-$C_6$-acyle, $C_1$-$C_6$-alkyl-$C_1$-$C_6$-aryle ou $C_1$-$C_6$-aryl-$C_1$-$C_6$-alkyle peuvent le cas échéant être interrompus une ou plusieurs fois, de manière identique ou différente, par oxygène, soufre ou azote, ou représente le groupe sulfonyl-$C_1$-$C_6$-alkyle, sulfonamide, ou cyano,

$R^3$ représente $C_6$-$C_{12}$-aryle, qui peut le cas échéant être monosubstitué ou polysubstitué, de manière identique ou différente, par halogène, par $C_1$-$C_6$-alkyle, $C_1$-$C_3$-acyle, $C_1$-$C_3$-alcoxy, cyano, hydroxy, $N(CH_3)_2$, $CO_2$-($C_1$-$C_3$-alkyle), CO-$NR^4R^5$ ou par $CF_3$, représente $C_5$-$C_{12}$-hétéroaryle, qui peut le cas échéant être monosubstitué ou polysubstitué, de manière identique ou différente, par chlore et/ou fluor, par $C_1$-$C_6$-alkyle, $C_1$-$C_3$-acyle, $C_1$-$C_3$-alcoxy, cyano, hydroxy, $N(CH_3)_2$, $CO_2$-($C_1$-$C_3$-alkyle), CO-$NR^4R^5$ ou par $CF_3$, représente $C_3$-$C_6$-cycloalkyle, qui peut le cas échéant être monosubstitué ou polysubstitué, de manière identique ou différente, par chlore et/ou fluor, $CF_3$, cyano, $C_1$-$C_3$-alkyle, $C_1$-$C_3$-acyle, hydroxy, $N(CH_3)_2$, $CO_2$-($C_1$-$C_3$-alkyle), CO-$NR^4R^5$ ou $C_1$-$C_3$-alcoxy,

$R^4$ représente hydrogène, $C_3$-$C_6$-cycloalkyle, qui est le cas échéant monosubstitué ou polysubstitué, de manière identique ou différente, par $C_1$-$C_3$-alkyle, $C_1$-$C_3$-acyle, $C_1$-$C_3$-alcoxy ou $CF_3$, représente $C_6$-$C_{12}$-aryle, qui est le cas échéant monosubstitué ou polysubstitué, de manière identique ou différente, par halogène, par $C_1$-$C_3$-alkyle, $C_1$-$C_3$-acyle, $C_1$-$C_3$-alcoxy, N-$C_1$-$C_3$-alkyl-$C_1$-$C_3$-alkyle, $CF_3$ ou cyano, ou représente $C_5$-$C_{12}$-hétéroaryle, qui est le cas échéant monosubstitué ou polysubstitué, de manière identique ou différente, par halogène, par $C_1$-$C_3$-alkyle, $C_1$-$C_3$-acyle, $C_1$-$C_3$-alcoxy, N-$C_1$-$C_3$-alkyl-$C_1$-$C_3$-alkyle, $CF_3$ ou cyano, ou représente $C_1$-$C_6$-alkyle, qui peut être substitué de manière quelconque,

$R^5$ représente hydrogène, $C_3$-$C_6$-cycloalkyle, qui est le cas échéant monosubstitué ou polysubstitué, de manière identique ou différente, par $C_1$-$C_3$-alkyle, $C_1$-$C_3$-acyle, $C_1$-$C_3$-alcoxy ou $CF_3$, représente $C_6$-$C_{12}$-aryle, qui est le cas échéant monosubstitué ou polysubstitué, de manière identique ou différente, par halogène, par $C_1$-$C_3$-alkyle, $C_1$-$C_3$-acyle, $C_1$-$C_3$-alcoxy, N-$C_1$-$C_3$-alkyl-$C_1$-$C_3$-alkyle, $CF_3$ ou cyano, ou représente $C_5$-$C_{12}$-hétéroaryle, qui est le cas échéant monosubstitué ou polysubstitué, de manière identique ou différente, par halogène, par $C_1$-$C_3$-alkyle, $C_1$-$C_3$-acyle, $C_1$-$C_3$-alcoxy, N-$C_1$-$C_3$-alkyl-$C_1$-$C_3$-alkyle, $CF_3$ ou cyano, ou représente $C_1$-$C_6$-alkyle, qui peut être substitué de manière quelconque,

$R^4$ et $R^5$ forment ensemble un cycle de 5-8 chaînons, qui peut contenir d'autres hétéroatomes et

X représente les groupes sulfonyle, $(CH_2)_n$ ou carbonyle,

Y représente carbonyle ou $(CH_2)_n$

Z représente azote, et

n vaut 0-2.

**3.** Composés selon la revendication 1 ou 2, où

$R^1$ représente hydrogène,

$R^2$ représente tert-butyle, cyano, brome, ou représente le groupe $-O-CF_3$, $-SO_2-CH_3$ et se trouve en position para,

$R^3$ représente le groupe

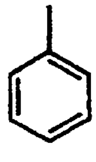
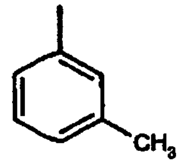
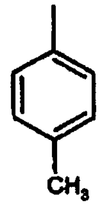
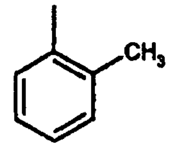
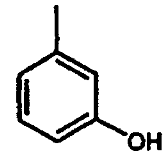

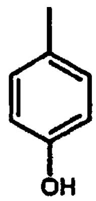 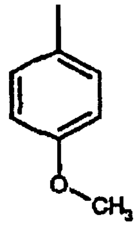 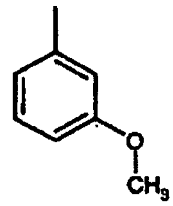 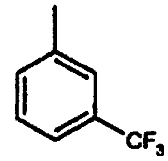 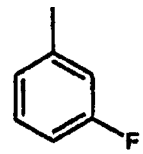

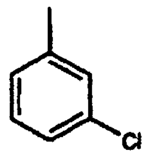 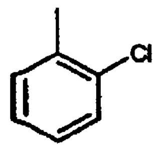 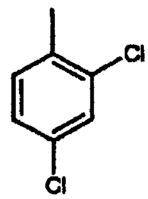 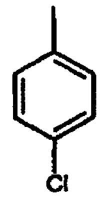

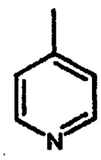 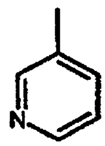 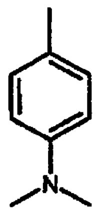

$R^4$ représente hydrogène ou le groupe $-(CH_2)_n-N-(CH_3)_2$, $-(CH_2)_2-CH_3$, $-(CH_2)_2-NH-COCH_3$, $-(CH_2)-CHCH_3-OH$, $-(CH_2)_2-O-CH_3$, $-(CH_2)_2-OH$, $-CHCH_3-CH_2-OH$,

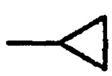 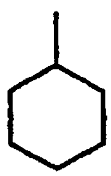 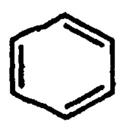 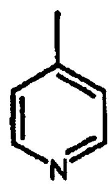

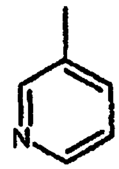 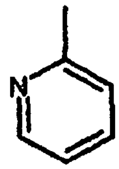 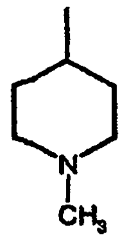 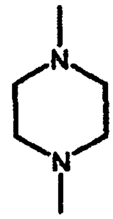 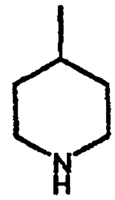

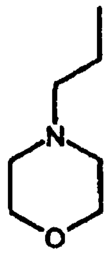 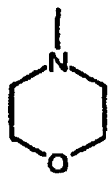 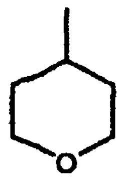 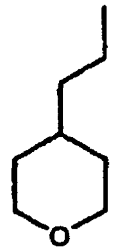 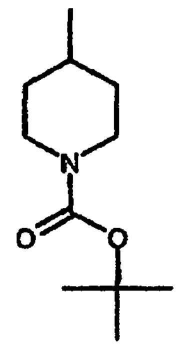

$R^5$ représente hydrogène,
X représente sulfonyle, carbonyle ou le groupe $CH_2$,
Y représente carbonyle ou le groupe $(CH_2)_n$,
Z représente azote ou

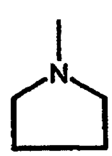 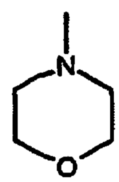

et
n vaut 1-2.

**4.** Composés selon la revendication 1, dans lesquels

$R^1$ représente hydrogène, tert-butyle, cyano, brome, ou représente le groupe $-O-CF_3$, $-SO_2-CH_3$,
$R^2$ représente tert-butyle, cyano, brome, ou représente le groupe $-O-CF_3$, $-SO_2-CH_3$ et
$R^3$ représente $C_6$-$C_{12}$-aryle, qui peut le cas échéant être monosubstitué ou polysubstitué, de manière identique ou différente, par halogène, par $C_1$-$C_6$-alkyle, $C_1$-$C_3$-acyle, $C_1$-$C_3$-alcoxy, cyano, hydroxy, $N-(CH_3)_2$, $CO_2$-($C_1$-$C_3$-alkyle), CO-$NR^4R^5$ ou par $CF_3$, représente $C_5$-$C_{12}$-hétéroaryle, qui peut le cas échéant être monosubstitué ou polysubstitué, de manière identique ou différente, par chlore et/ou fluor, par $C_1$-$C_6$-alkyle, $C_1$-$C_3$-acyle, $C_1$-$C_3$-alcoxy, cyano, hydroxy, $N-(CH_3)_2$, $CO_2$-($C_1$-$C_3$-alkyle), CO-$NR^4R^5$ ou par $CF_3$, représente $C_3$-$C_6$-cycloalkyle, qui peut le cas échéant être monosubstitué ou polysubstitué, de manière identique ou différente, par chlore et/ou fluor, $CF_3$, cyano, $C_1$-$C_3$-alkyle, $C_1$-$C_3$-acyle, hydroxy, $N-(CH_3)_2$, $CO_2$-($C_1$-$C_3$-alkyle), CO-$NR^4R^5$ ou $C_1$-$C_3$-alcoxy,
$R^4$ représente hydrogène, $C_3$-$C_6$-cycloalkyle, qui est le cas échéant monosubstitué ou polysubstitué, de manière identique ou différente, par $C_1$-$C_3$-alkyle, $C_1$-$C_3$-acyle, $C_1$-$C_3$-alcoxy ou $CF_3$, représente $C_6$-$C_{12}$-aryle, qui est le cas échéant monosubstitué ou polysubstitué, de manière identique ou différente, par halogène, par $C_1$-$C_3$-alkyle, $C_1$-$C_3$-acyle, $C_1$-$C_3$-alcoxy, N-$C_1$-$C_3$-alkyl-$C_1$-$C_3$-alkyle, $CF_3$ ou cyano, ou représente $C_5$-$C_{12}$-hétéroaryle, qui est le cas échéant monosubstitué ou polysubstitué, de manière identique ou différente, par halogène, par $C_1$-$C_3$-alkyle, $C_1$-$C_3$-acyle, $C_1$-$C_3$-alcoxy, N-$C_1$-$C_3$-alkyl-$C_1$-$C_3$-alkyle, $CF_3$ ou cyano, ou représente $C_1$-$C_6$-alkyle, qui peut être substitué de manière quelconque,
$R^5$ représente hydrogène, $C_3$-$C_6$-cycloalkyle, qui est le cas échéant monosubstitué ou polysubstitué, de manière

identique ou différente, par $C_1$-$C_3$-alkyle, $C_1$-$C_3$-acyle, $C_1$-$C_3$-alcoxy ou $CF_3$, représente $C_6$-$C_{12}$-aryle, qui est le cas échéant monosubstitué ou polysubstitué, de manière identique ou différente, par halogène, par $C_1$-$C_3$-alkyle, $C_1$-$C_3$-acyle, $C_1$-$C_3$-alcoxy, N-$C_1$-$C_3$-alkyl-$C_1$-$C_3$-alkyle, $CF_3$ ou cyano, ou représente $C_5$-$C_{12}$-hétéroaryle, qui est le cas échéant monosubstitué ou polysubstitué, de manière identique ou différente, par halogène, par $C_1$-$C_3$-alkyle, $C_1$-$C_3$-acyle, $C_1$-$C_3$-alcoxy, N-$C_1$-$C_3$-alkyl-$C_1$-$C_3$-alkyle, $CF_3$ ou cyano, ou représente $C_1$-$C_6$-alkyle, qui peut être substitué de manière quelconque,
$R^4$ et $R^5$ forment ensemble un cycle de 5-8 chaînons, qui peut contenir d'autres hétéroatomes et
X représente les groupes sulfonyle, $(CH_2)_n$ ou carbonyle,
Y représente carbonyle ou $(CH_2)_n$
Z représente azote, et
n vaut 0-2.

**5.** Composés selon la revendication 1, dans lesquels

$R^1$ représente hydrogène,
$R^2$ représente tert-butyle, cyano, brome, ou représente le groupe -O-$CF_3$, -$SO_2$-$CH_3$ et se trouve en position para, et
$R^3$ représente $C_6$-$C_{12}$-aryle, qui peut le cas échéant être monosubstitué ou polysubstitué, de manière identique ou différente, par halogène, par $C_1$-$C_6$-alkyle, $C_1$-$C_3$-acyle, $C_1$-$C_3$-alcoxy, cyano, hydroxy, N-$(CH_3)_2$, $CO_2$-($C_1$-$C_3$-alkyle), CO-$NR^4R^5$ ou par $CF_3$, représente $C_5$-$C_{12}$-hétéroaryle, qui peut le cas échéant être monosubstitué ou polysubstitué, de manière identique ou différente, par chlore et/ou fluor, par $C_1$-$C_6$-alkyle, $C_1$-$C_3$-acyle, $C_1$-$C_3$-alcoxy, cyano, hydroxy, N-$(CH_3)_2$, $CO_2$-($C_1$-$C_3$-alkyle), CO-$NR^4R^5$ ou par $CF_3$, représente $C_3$-$C_6$-cycloalkyle, qui peut le cas échéant être monosubstitué ou polysubstitué, de manière identique ou différente, par chlore et/ou fluor, $CF_3$, cyano, $C_1$-$C_3$-alkyle, $C_1$-$C_3$-acyle, hydroxy, N-$(CH_3)_2$, $CO_2$-($C_1$-$C_3$-alkyle), CO-$NR^4R^5$ ou $C_1$-$C_3$-alcoxy,
$R^4$ représente hydrogène, $C_3$-$C_6$-cycloalkyle, qui est le cas échéant monosubstitué ou polysubstitué, de manière identique ou différente, par $C_1$-$C_3$-alkyle, $C_1$-$C_3$-acyle, $C_1$-$C_3$-alcoxy ou $CF_3$, représente $C_6$-$C_{12}$-aryle, qui est le cas échéant monosubstitué ou polysubstitué, de manière identique ou différente, par halogène, par $C_1$-$C_3$-alkyle, $C_1$-$C_3$-acyle, $C_1$-$C_3$-alcoxy, N-$C_1$-$C_3$-alkyl-$C_1$-$C_3$-alkyle, $CF_3$ ou cyano, ou représente $C_5$-$C_{12}$-hétéroaryle, qui est le cas échéant monosubstitué ou polysubstitué, de manière identique ou différente, par halogène, par $C_1$-$C_3$-alkyle, $C_1$-$C_3$-acyle, $C_1$-$C_3$-alcoxy, N-$C_1$-$C_3$-alkyl-$C_1$-$C_3$-alkyle, $CF_3$ ou cyano, ou représente $C_1$-$C_6$-alkyle, qui peut être substitué de manière quelconque,
$R^5$ représente hydrogène, $C_3$-$C_6$-cycloalkyle, qui est le cas échéant monosubstitué ou polysubstitué, de manière identique ou différente, par $C_1$-$C_3$-alkyle, $C_1$-$C_3$-acyle, $C_1$-$C_3$-alcoxy ou $CF_3$, représente $C_6$-$C_{12}$-aryle, qui est le cas échéant monosubstitué ou polysubstitué, de manière identique ou différente, par halogène, par $C_1$-$C_3$-alkyle, $C_1$-$C_3$-acyle, $C_1$-$C_3$-alcoxy, N-$C_1$-$C_3$-alkyl-$C_1$-$C_3$-alkyle, $CF_3$ ou cyano, ou représente $C_5$-$C_{12}$-hétéroaryle, qui est le cas échéant monosubstitué ou polysubstitué, de manière identique ou différente, par halogène, par $C_1$-$C_3$-alkyle, $C_1$-$C_3$-acyle, $C_1$-$C_3$-alcoxy, N-$C_1$-$C_3$-alkyl-$C_1$-$C_3$-alkyle, $CF_3$ ou cyano, ou représente $C_1$-$C_6$-alkyle, qui peut être substitué de manière quelconque,
$R^4$ et $R^5$ forment ensemble un cycle de 5-8 chaînons, qui peut contenir d'autres hétéroatomes et
X représente les groupes sulfonyle, $(CH_2)_n$ ou carbonyle,
Y représente carbonyle ou $(CH_2)_n$
Z représente azote, et
n vaut 0-2.

**6.** Composés selon la revendication 1, où

$R^1$ représente hydrogène, halogène, $CF_3$, $C_3$-$C_6$-cycloalkyle, qui est le cas échéant polyinsaturé et le cas échéant polysubstitué, ou représente le groupe $C_1$-$C_6$-alkyle, $C_1$-$C_6$-aryle, $C_1$-$C_6$-acyle, halogéno-$C_1$-$C_6$-alkyle, $C_1$-$C_6$-alkyl-$C_1$-$C_6$-alkyle, $C_1$-$C_6$-alkyl-$C_1$-$C_6$-acyle, $C_1$-$C_6$-acyl-$C_1$-$C_6$-acyle, $C_1$-$C_6$-alkyl-$C_1$-$C_6$-aryle, $C_1$-$C_6$-aryl-$C_1$-$C_6$-alkyle ou $CF_3$, où les groupes $C_1$-$C_6$-alkyle, $C_1$-$C_6$-aryle, $C_1$-$C_6$-acyle, halogéno-$C_1$-$C_6$-alkyle, $C_1$-$C_6$-alkyl-$C_1$-$C_6$-alkyle, $C_1$-$C_6$-alkyl-$C_1$-$C_6$-acyle, $C_1$-$C_6$-acyl-$C_1$-$C_6$-acyle, $C_1$-$C_6$-alkyl-$C_1$-$C_6$-aryle ou $C_1$-$C_6$-aryl-$C_1$-$C_6$-alkyle peuvent le cas échéant être interrompus une ou plusieurs fois, de manière identique ou différente, par oxygène, soufre ou azote, ou représente le groupe sulfonyl-$C_1$-$C_6$-alkyle, sulfonamide, ou cyano,
$R^2$ représente halogène, $CF_3$, $C_3$-$C_6$-cycloalkyle, qui est le cas échéant polyinsaturé et le cas échéant polysubstitué, ou représente le groupe $C_1$-$C_6$-alkyle, $C_1$-$C_6$-aryle, $C_1$-$C_6$-acyle, halogéno-$C_1$-$C_6$-alkyle, $C_1$-$C_6$-alkyl-$C_1$-$C_6$-alkyle, $C_1$-$C_6$-alkyl-$C_1$-$C_6$-acyle, $C_1$-$C_6$-acyl-$C_1$-$C_6$-acyle, $C_1$-$C_6$-alkyl-$C_1$-$C_6$-aryle, $C_1$-$C_6$-aryl-$C_1$-$C_6$-alk-

yle ou $CF_3$, où les groupes $C_1$-$C_6$-alkyle, $C_1$-$C_6$-aryle, $C_1$-$C_6$-acyle, halogéno-$C_1$-$C_6$-alkyle, $C_1$-$C_6$-alkyl-$C_1$-$C_6$-alkyle, $C_1$-$C_6$-alkyl-$C_1$-$C_6$-acyle, $C_1$-$C_6$-acyl-$C_1$-$C_6$-acyle, $C_1$-$C_6$-alkyl-$C_1$-$C_6$-aryle ou $C_1$-$C_6$-aryl-$C_1$-$C_6$-alkyle peuvent le cas échéant être interrompus une ou plusieurs fois, de manière identique ou différente, par oxygène, soufre ou azote, ou représente le groupe sulfonyl-$C_1$-$C_6$-alkyle, sulfonamide, ou cyano,

$R^3$ représente $C_6$-$C_{12}$-aryle, qui peut le cas échéant être monosubstitué ou polysubstitué, de manière identique ou différente, par halogène, par $C_1$-$C_6$-alkyle, $C_1$-$C_3$-acyle, $C_1$-$C_3$-alcoxy, cyano, hydroxy, N-$(CH_3)_2$, $CO_2$-($C_1$-$C_3$-alkyle), CO-$NR^4R^5$ ou par $CF_3$, $C_5$-$C_{12}$-hétéroaryle, qui peut le cas échéant être monosubstitué ou polysubstitué, de manière identique ou différente, par chlore et/ou fluor, par $C_1$-$C_6$-alkyle, $C_1$-$C_3$-acyle, $C_1$-$C_3$-alcoxy, cyano, hydroxy, N-$(CH_3)_2$, $CO_2$-($C_1$-$C_3$-alkyle), CO-$NR^4R^5$ ou par $CF_3$, représente $C_3$-$C_6$-cycloalkyle, qui peut le cas échéant être monosubstitué ou polysubstitué, de manière identique ou différente, par chlore et/ou fluor, $CF_3$, cyano, $C_1$-$C_3$-alkyle, $C_1$-$C_3$-acyle, hydroxy, N-$(CH_3)_2$, $CO_2$-($C_1$-$C_3$-alkyle), CO-$NR^4R^5$ ou $C_1$-$C_3$-alcoxy,

$R^4$ représente hydrogène, $C_3$-$C_6$-cycloalkyle, qui est le cas échéant monosubstitué ou polysubstitué, de manière identique ou différente, par $C_1$-$C_6$-alkyle, $C_1$-$C_6$-acyle, $C_1$-$C_6$-alcoxy ou $CF_3$, représente $C_6$-$C_{12}$-aryle, qui est le cas échéant monosubstitué ou polysubstitué, de manière identique ou différente, par halogène, par $C_1$-$C_6$-alkyle, $C_1$-$C_6$-acyle, $C_1$-$C_6$-alcoxy, N-$C_1$-$C_6$-alkyl-$C_1$-$C_6$-alkyle, $CF_3$ ou cyano, ou représente $C_5$-$C_{12}$-hétéroaryle, qui est le cas échéant monosubstitué ou polysubstitué, de manière identique ou différente, par halogène, par $C_1$-$C_6$-alkyle, $C_1$-$C_6$-acyle, $C_1$-$C_6$-alcoxy, N-$C_1$-$C_6$-alkyl-$C_1$-$C_6$-alkyle, $CF_3$ ou cyano, ou représente $C_1$-$C_6$-alkyle, qui peut être substitué de manière quelconque,

$R^5$ représente hydrogène, $C_3$-$C_6$-cycloalkyle, qui est le cas échéant monosubstitué ou polysubstitué, de manière identique ou différente, par $C_1$-$C_6$-alkyle, $C_1$-$C_6$-acyle, $C_1$-$C_6$-alcoxy ou $CF_3$, représente $C_6$-$C_{12}$-aryle, qui est le cas échéant monosubstitué ou polysubstitué, de manière identique ou différente, par halogène, par $C_1$-$C_6$-alkyle, $C_1$-$C_6$-acyle, $C_1$-$C_6$-alcoxy, N-$C_1$-$C_6$-alkyl-$C_1$-$C_6$-alkyle, $CF_3$ ou cyano, ou représente $C_5$-$C_{12}$-hétéroaryle, qui est le cas échéant monosubstitué ou polysubstitué, de manière identique ou différente, par halogène, par $C_1$-$C_6$-alkyle, $C_1$-$C_6$-acyle, $C_1$-$C_6$-alcoxy, N-$C_1$-$C_6$-alkyl-$C_1$-$C_6$-alkyle, $CF_3$ ou cyano, ou représente $C_1$-$C_6$-alkyle, qui peut être substitué de manière quelconque,

$R^4$ et $R^5$ forment ensemble un cycle de 5-8 chaînons, qui peut contenir d'autres hétéroatomes et

X représente les groupes sulfonyle, $(CH_2)_n$ ou carbonyle,

Y représente carbonyle ou $(CH_2)_n$

Z représente azote, et

n vaut 0-4.

**7.** Composés selon la revendication 1, où

$R^1$ représente hydrogène,

$R^2$ représente tert-butyle, cyano, brome, ou représente le groupe -O-$CF_3$, -$SO_2$-$CH_3$ et se trouve en position para, et

$R^3$ représente $C_6$-$C_{12}$-aryle, qui peut le cas échéant être monosubstitué ou polysubstitué, de manière identique ou différente, par halogène, par $C_1$-$C_6$-alkyle, $C_1$-$C_3$-acyle, $C_1$-$C_3$-alcoxy, cyano, hydroxy, N-$(CH_3)_2$, $CO_2$-($C_1$-$C_3$-alkyle), CO-$NR^4R^5$ ou par $CF_3$, représente $C_5$-$C_{12}$-hétéroaryle, qui peut le cas échéant être monosubstitué ou polysubstitué, de manière identique ou différente, par chlore et/ou fluor, par $C_1$-$C_6$-alkyle, $C_1$-$C_3$-acyle, $C_1$-$C_3$-alcoxy, cyano, hydroxy, N-$(CH_3)_2$, $CO_2$-($C_1$-$C_3$-alkyle), CO-$NR^4R^5$ ou par $CF_3$, représente $C_3$-$C_6$-cycloalkyle, qui peut le cas échéant être monosubstitué ou polysubstitué, de manière identique ou différente, par chlore et/ou fluor, $CF_3$, cyano, $C_1$-$C_3$-alkyle, $C_1$-$C_3$-acyle, hydroxy, N-$(CH_3)_2$, $CO_2$-($C_1$-$C_3$-alkyle), CO-$NR^4R^5$ ou $C_1$-$C_3$-alcoxy,

$R^4$ représente hydrogène ou le groupe -$(CH_2)_n$-N-$(CH_3)_2$, - $(CH_2)_2$-$CH_3$, - $(CH_2)_2$-NH-$COCH_3$, -$(CH_2)$-$CHCH_3$-OH, -$(CH_2)_2$-O-$CH_3$, -$(CH_2)_2$-OH, -$CHCH_3$-$CH_2$-OH,

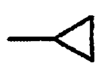 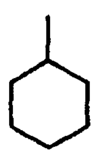 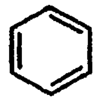 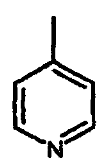

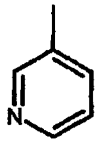 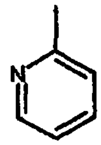 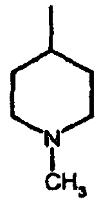 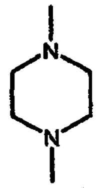 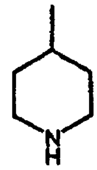

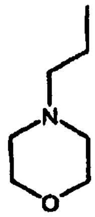 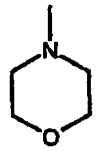 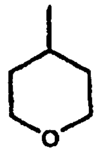 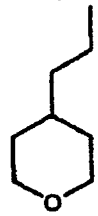 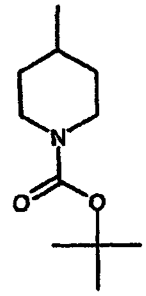

$R^5$ représente hydrogène,
X représente sulfonyle, carbonyle ou le groupe $CH_2$,
Y représente carbonyle ou le groupe $(CH_2)_n$,
Z représente azote ou

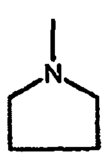 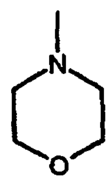

et
n vaut 1-2.

**8.** Composés selon la revendication 1, où

$R^1$ représente hydrogène, halogène, $CF_3$, $C_3$-$C_6$-cycloalkyle, qui est le cas échéant polyinsaturé et le cas échéant polysubstitué, ou représente le groupe $C_1$-$C_6$-alkyle, $C_1$-$C_6$-aryle, $C_1$-$C_6$-acyle, halogéno-$C_1$-$C_6$-alkyle, $C_1$-$C_6$-alkyl-$C_1$-$C_6$-alkyle, $C_1$-$C_6$-alkyl-$C_1$-$C_6$-acyle, $C_1$-$C_6$-acyl-$C_1$-$C_6$-acyle, $C_1$-$C_6$-alkyl-$C_1$-$C_6$-aryle, $C_1$-$C_6$-aryl-$C_1$-$C_6$-alkyle ou $CF_3$, où les groupes $C_1$-$C_6$-alkyle, $C_1$-$C_6$-aryle, $C_1$-$C_6$-acyle, halogéno-$C_1$-$C_6$-alkyle, $C_1$-$C_6$-alkyl-$C_1$-$C_6$-alkyle, $C_1$-$C_6$-alkyl-$C_1$-$C_6$-acyle, $C_1$-$C_6$-acyl-$C_1$-$C_6$-acyle, $C_1$-$C_6$-alkyl-$C_1$-$C_6$-aryle ou $C_1$-$C_6$-aryl-$C_1$-$C_6$-alkyle peuvent le cas échéant être interrompus une ou plusieurs fois, de manière identique ou différente, par oxygène, soufre ou azote, ou représente le groupe sulfonyl-$C_1$-$C_6$-alkyle, sulfonamide, ou cyano,
$R^2$ représente halogène, $CF_3$, $C_3$-$C_6$-cycloalkyle, qui est le cas échéant polyinsaturé et le cas échéant polysubstitué, ou représente le groupe $C_1$-$C_6$-alkyle, $C_1$-$C_6$-aryle, $C_1$-$C_6$-acyle, halogéno-$C_1$-$C_6$-alkyle, $C_1$-$C_6$-alkyl-$C_1$-$C_6$-alkyle, $C_1$-$C_6$-alkyl-$C_1$-$C_6$-acyle, $C_1$-$C_6$-acyl-$C_1$-$C_6$-acyle, $C_1$-$C_6$-alkyl-$C_1$-$C_6$-aryle, $C_1$-$C_6$-aryl-$C_1$-$C_6$-alkyle ou $CF_3$, où les groupes $C_1$-$C_6$-alkyle, $C_1$-$C_6$-aryle, $C_1$-$C_6$-acyle, halogéno-$C_1$-$C_6$-alkyle, $C_1$-$C_6$-alkyl-$C_1$-$C_6$-alkyle, $C_1$-$C_6$-alkyl-$C_1$-$C_6$-acyle, $C_1$-$C_6$-acyl-$C_1$-$C_6$-acyle, $C_1$-$C_6$-alkyl-$C_1$-$C_6$-aryle ou $C_1$-$C_6$-aryl-$C_1$-$C_6$-alkyle peuvent le cas échéant être interrompus une ou plusieurs fois, de manière identique ou différente, par oxygène, soufre ou azote, ou représente le groupe sulfonyl-$C_1$-$C_6$-alkyle, sulfonamide, ou cyano,
$R^3$ représente le groupe

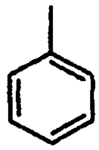 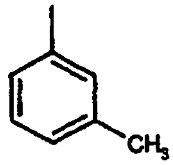 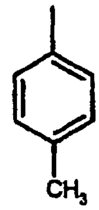 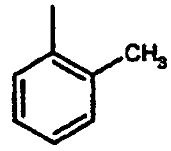 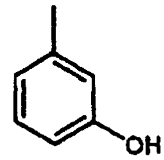

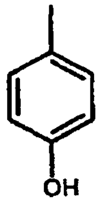 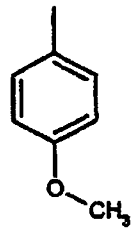 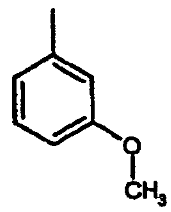 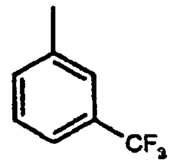 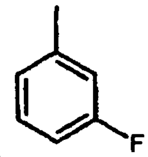

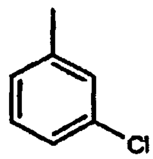 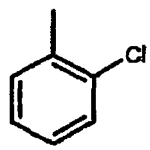 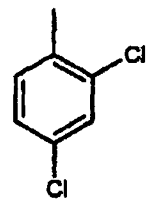 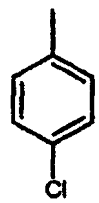

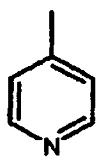 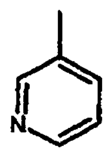 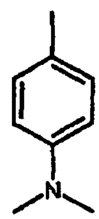

$R^4$ représente hydrogène, $C_3$-$C_6$-cycloalkyle, qui est le cas échéant monosubstitué ou polysubstitué, de manière identique ou différente, par $C_1$-$C_3$-alkyle, $C_1$-$C_3$-acyle, $C_1$-$C_3$-alcoxy ou $CF_3$, représente $C_6$-$C_{12}$-aryle, qui est le cas échéant monosubstitué ou polysubstitué, de manière identique ou différente, par halogène, par $C_1$-$C_3$-alkyle, $C_1$-$C_3$-acyle, $C_1$-$C_3$-alcoxy, N-$C_1$-$C_3$-alkyl-$C_1$-$C_3$-alkyle, $CF_3$ ou cyano, ou représente $C_5$-$C_{12}$-hétéroaryle, qui est le cas échéant monosubstitué ou polysubstitué, de manière identique ou différente, par halogène, par $C_1$-$C_3$-alkyle, $C_1$-$C_3$-acyle, $C_1$-$C_3$-alcoxy, N-$C_1$-$C_3$-alkyl-$C_1$-$C_3$-alkyle, $CF_3$ ou cyano, ou représente $C_1$-$C_6$-alkyle, qui peut être substitué de manière quelconque,
$R^5$ représente hydrogène, $C_3$-$C_6$-cycloalkyle, qui est le cas échéant monosubstitué ou polysubstitué, de manière identique ou différente, par $C_1$-$C_3$-alkyle, $C_1$-$C_3$-acyle, $C_1$-$C_3$-alcoxy ou $CF_3$, représente $C_6$-$C_{12}$-aryle, qui est le cas échéant monosubstitué ou polysubstitué, de manière identique ou différente, par halogène, par $C_1$-$C_3$-alkyle, $C_1$-$C_3$-acyle, $C_1$-$C_3$-alcoxy, N-$C_1$-$C_3$-alkyl-$C_1$-$C_3$-alkyle, $CF_3$ ou cyano, ou représente $C_5$-$C_{12}$-hétéroaryle, qui est le cas échéant monosubstitué ou polysubstitué, de manière identique ou différente, par halogène, par $C_1$-$C_3$-alkyle, $C_1$-$C_3$-acyle, $C_1$-$C_3$-alcoxy, N-$C_1$-$C_3$-alkyl-$C_1$-$C_3$-alkyle, $CF_3$ ou cyano, ou représente $C_1$-$C_6$-alkyle, qui peut être substitué de manière quelconque,
$R^4$ et $R^5$ forment ensemble un cycle de 5-8 chaînons, qui peut contenir d'autres hétéroatomes et
X représente les groupes sulfonyle, $(CH_2)_n$ ou carbonyle,
Y représente carbonyle ou $(CH_2)_n$
Z représente azote, et
n vaut 0-2.

**9.** Composés selon la revendication 1, où

$R^1$ représente hydrogène,

$R^2$ représente tert-butyle, cyano, brome, ou représente le groupe -O-$CF_3$, -$SO_2$-$CH_3$ et se trouve en position para, et

$R^3$ représente le groupe

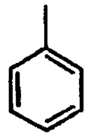 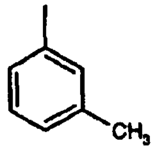 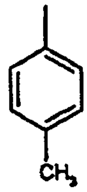 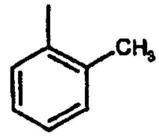 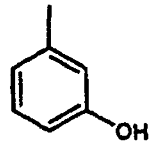

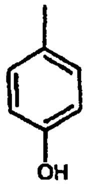 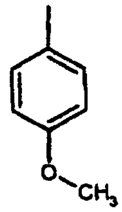 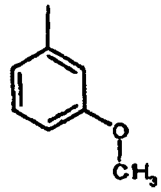 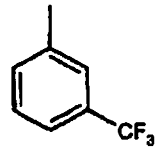 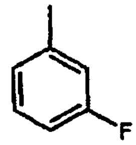

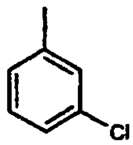 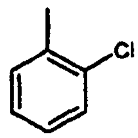 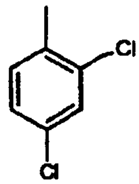 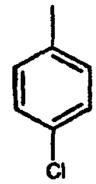

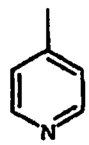 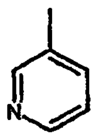 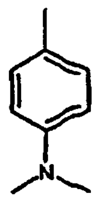

$R^4$ représente hydrogène, $C_3$-$C_6$-cycloalkyle, qui est le cas échéant monosubstitué ou polysubstitué, de manière identique ou différente, par $C_1$-$C_3$-alkyle, $C_1$-$C_3$-acyle, $C_1$-$C_3$-alcoxy ou $CF_3$, représente $C_6$-$C_{12}$-aryle, qui est le cas échéant monosubstitué ou polysubstitué, de manière identique ou différente, par halogène, par $C_1$-$C_3$-alkyle, $C_1$-$C_3$-acyle, $C_1$-$C_3$-alcoxy, N-$C_1$-$C_3$-alkyl-$C_1$-$C_3$-alkyle, $CF_3$ ou cyano, ou représente $C_5$-$C_{12}$-hétéroaryle, qui est le cas échéant monosubstitué ou polysubstitué, de manière identique ou différente, par halogène, par $C_1$-$C_3$-alkyle, $C_1$-$C_3$-acyle, $C_1$-$C_3$-alcoxy, N-$C_1$-$C_3$-alkyl-$C_1$-$C_3$-alkyle, $CF_3$ ou cyano, ou représente $C_1$-$C_6$-alkyle, qui peut être substitué de manière quelconque,

$R^5$ représente hydrogène, $C_3$-$C_6$-cycloalkyle, qui est le cas échéant monosubstitué ou polysubstitué, de manière identique ou différente, par $C_1$-$C_3$-alkyle, $C_1$-$C_3$-acyle, $C_1$-$C_3$-alcoxy ou $CF_3$, représente $C_6$-$C_{12}$-aryle, qui est le cas échéant monosubstitué ou polysubstitué, de manière identique ou différente, par halogène, par $C_1$-$C_3$-alkyle, $C_1$-$C_3$-acyle, $C_1$-$C_3$-alcoxy, N-$C_1$-$C_3$-alkyl-$C_1$-$C_3$-alkyle, $CF_3$ ou cyano, ou représente $C_5$-$C_{12}$-hétéroaryle, qui est le cas échéant monosubstitué ou polysubstitué, de manière identique ou différente, par halogène, par $C_1$-$C_3$-alkyle, $C_1$-$C_3$-acyle, $C_1$-$C_3$-alcoxy, N-$C_1$-$C_3$-alkyl-$C_1$-$C_3$-alkyle, $CF_3$ ou cyano, ou représente $C_1$-$C_6$-alkyle, qui peut être substitué de manière quelconque,

X représente sulfonyle, carbonyle ou le groupe $CH_2$,

Y représente carbonyle ou le groupe $(CH_2)_n$,

Z représente azote ou

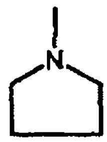 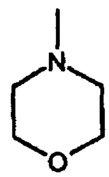

et
n vaut 1-2.

**10.** Composés selon la revendication 1, où

$R^1$ représente hydrogène, halogène, $CF_3$, $C_3$-$C_6$-cycloalkyle, qui est le cas échéant polyinsaturé et le cas échéant polysubstitué, ou représente le groupe $C_1$-$C_6$-alkyle, $C_1$-$C_6$-aryle, $C_1$-$C_6$-acyle, halogéno-$C_1$-$C_6$-alkyle, $C_1$-$C_6$-alkyl-$C_1$-$C_6$-alkyle, $C_1$-$C_6$-alkyl-$C_1$-$C_6$-acyle, $C_1$-$C_6$-acyl-$C_1$-$C_6$-acyle, $C_1$-$C_6$-alkyl-$C_1$-$C_6$-aryle, $C_1$-$C_6$-aryl-$C_1$-$C_6$-alkyle ou $CF_3$, où les groupes $C_1$-$C_6$-alkyle, $C_1$-$C_6$-aryle, $C_1$-$C_6$-acyle, halogéno-$C_1$-$C_6$-alkyle, $C_1$-$C_6$-alkyl-$C_1$-$C_6$-alkyle, $C_1$-$C_6$-alkyl-$C_1$-$C_6$-acyle, $C_1$-$C_6$-acyl-$C_1$-$C_6$-acyle, $C_1$-$C_6$-alkyl-$C_1$-$C_6$-aryle ou $C_1$-$C_6$-aryl-$C_1$-$C_6$-alkyle peuvent le cas échéant être interrompus une ou plusieurs fois, de manière identique ou différente, par oxygène, soufre ou azote, ou représente le groupe sulfonyl-$C_1$-$C_6$-alkyle, sulfonamide, ou cyano,
$R^2$ représente halogène, $CF_3$, $C_3$-$C_6$-cycloalkyle, qui est le cas échéant polyinsaturé et le cas échéant polysubstitué, ou représente le groupe $C_1$-$C_6$-alkyle, $C_1$-$C_6$-aryle, $C_1$-$C_6$-acyle, halogéno-$C_1$-$C_6$-alkyle, $C_1$-$C_6$-alkyl-$C_1$-$C_6$-alkyle, $C_1$-$C_6$-alkyl-$C_1$-$C_6$-acyle, $C_1$-$C_6$-acyl-$C_1$-$C_6$-acyle, $C_1$-$C_6$-alkyl-$C_1$-$C_6$-aryle, $C_1$-$C_6$-aryl-$C_1$-$C_6$-alkyle ou $CF_3$, où les groupes $C_1$-$C_6$-alkyle, $C_1$-$C_6$-aryle, $C_1$-$C_6$-acyle, halogéno-$C_1$-$C_6$-alkyle, $C_1$-$C_6$-alkyl-$C_1$-$C_6$-alkyle, $C_1$-$C_6$-alkyl-$C_1$-$C_6$-acyle, $C_1$-$C_6$-acyl-$C_1$-$C_6$-acyle, $C_1$-$C_6$-alkyl-$C_1$-$C_6$-aryle ou $C_1$-$C_6$-aryl-$C_1$-$C_6$-alkyle peuvent le cas échéant être interrompus une ou plusieurs fois, de manière identique ou différente, par oxygène, soufre ou azote, ou représente le groupe sulfonyl-$C_1$-$C_6$-alkyle, sulfonamide, ou cyano,
$R^3$ représente $C_6$-$C_{12}$-aryle, qui peut le cas échéant être monosubstitué ou polysubstitué, de manière identique ou différente, par halogène, par $C_1$-$C_6$-alkyle, $C_1$-$C_3$-acyle, $C_1$-$C_3$-alcoxy, cyano, hydroxy, $N(CH_3)_2$, $CO_2$-($C_1$-$C_3$-alkyle), CO-$NR^4R^5$ ou par $CF_3$, représente $C_5$-$C_{12}$-hétéroaryle, qui peut le cas échéant être monosubstitué ou polysubstitué, de manière identique ou différente, par chlore et/ou fluor, par $C_1$-$C_6$-alkyle, $C_1$-$C_3$-acyle, $C_1$-$C_3$-alcoxy, cyano, hydroxy, $N(CH_3)_2$, $CO_2$-($C_1$-$C_3$-alkyle), CO-$NR^4R^5$ ou par $CF_3$, représente $C_3$-$C_6$-cycloalkyle, qui peut le cas échéant être monosubstitué ou polysubstitué, de manière identique ou différente, par chlore et/ou fluor, $CF_3$, cyano, $C_1$-$C_3$-alkyle, $C_1$-$C_3$-acyle, hydroxy, $N(CH_3)_2$, $CO_2$-($C_1$-$C_3$-alkyle), CO-$NR^4R^5$ ou $C_1$-$C_3$-alcoxy,
$R^4$ représente hydrogène ou le groupe $-(CH_2)_n-N(CH_3)_2$, - $(CH_2)_2-CH_3$, $-(CH_2)_2-NH-COCH_3$, $-(CH_2)-CHCH_3-OH$, $-(CH_2)_2-O-CH_3$, $-(CH_2)_2-OH$, $-CHCH_3-CH_2-OH$,

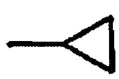 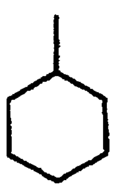 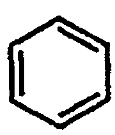 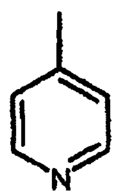

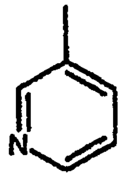
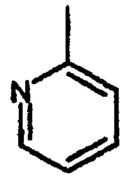
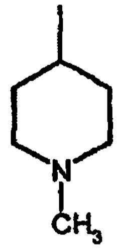
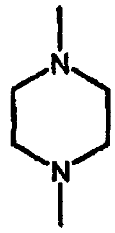
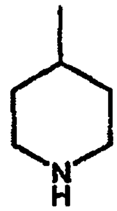
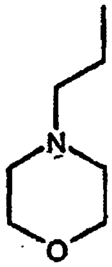
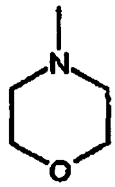
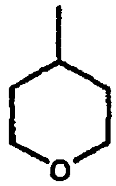
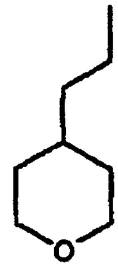
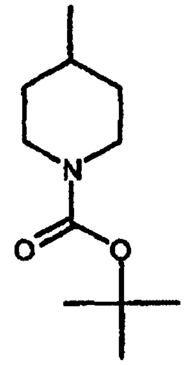

$R^5$ représente hydrogène,
X représente les groupes sulfonyle, $(CH_2)_n$ ou carbonyle,
Y représente carbonyle ou $(CH_2)_n$
Z représente azote, et
n vaut 0-2.

**11.** Composés selon la revendication 1, où

$R^1$ représente hydrogène, halogène, $CF_3$, $C_3$-$C_6$-cycloalkyle, qui est le cas échéant polyinsaturé et le cas échéant polysubstitué, ou représente le groupe $C_1$-$C_6$-alkyle, $C_1$-$C_6$-aryle, $C_1$-$C_6$-acyle, halogéno-$C_1$-$C_6$-alkyle, $C_1$-$C_6$-alkyl-$C_1$-$C_6$-alkyle, $C_1$-$C_6$-alkyl-$C_1$-$C_6$-acyle, $C_1$-$C_6$-acyl-$C_1$-$C_6$-acyle, $C_1$-$C_6$-alkyl-$C_1$-$C_6$-aryle, $C_1$-$C_6$-aryl-$C_1$-$C_6$-alkyle ou $CF_3$, où les groupes $C_1$-$C_6$-alkyle, $C_1$-$C_6$-aryle, $C_1$-$C_6$-acyle, halogéno-$C_1$-$C_6$-alkyle, $C_1$-$C_6$-alkyl-$C_1$-$C_6$-alkyle, $C_1$-$C_6$-alkyl-$C_1$-$C_6$-acyle, $C_1$-$C_6$-acyl-$C_1$-$C_6$-acyle, $C_1$-$C_6$-alkyl-$C_1$-$C_6$-aryle ou $C_1$-$C_6$-aryl-$C_1$-$C_6$-alkyle peuvent le cas échéant être interrompus une ou plusieurs fois, de manière identique ou différente, par oxygène, soufre ou azote, ou représente le groupe sulfonyl-$C_1$-$C_6$-alkyle, sulfonamide, ou cyano,
$R^2$ représente halogène, $CF_3$, $C_3$-$C_6$-cycloalkyle, qui est le cas échéant polyinsaturé et le cas échéant polysubstitué, ou représente le groupe $C_1$-$C_6$-alkyle, $C_1$-$C_6$-aryle, $C_1$-$C_6$-acyle, halogéno-$C_1$-$C_6$-alkyle, $C_1$-$C_6$-alkyl-$C_1$-$C_6$-alkyle, $C_1$-$C_6$-alkyl-$C_1$-$C_6$-acyle, $C_1$-$C_6$-acyl-$C_1$-$C_6$-acyle, $C_1$-$C_6$-alkyl-$C_1$-$C_6$-aryle, $C_1$-$C_6$-aryl-$C_1$-$C_6$-alkyle ou $CF_3$, où les groupes $C_1$-$C_6$-alkyle, $C_1$-$C_6$-aryle, $C_1$-$C_6$-acyle, halogéno-$C_1$-$C_6$-alkyle, $C_1$-$C_6$-alkyl-$C_1$-$C_6$-alkyle, $C_1$-$C_6$-alkyl-$C_1$-$C_6$-acyle, $C_1$-$C_6$-acyl-$C_1$-$C_6$-acyle, $C_1$-$C_6$-alkyl-$C_1$-$C_6$-aryle ou $C_1$-$C_6$-aryl-$C_1$-$C_6$-alkyle peuvent le cas échéant être interrompus une ou plusieurs fois, de manière identique ou différente, par oxygène, soufre ou azote, ou représente le groupe sulfonyl-$C_1$-$C_6$-alkyle, sulfonamide, ou cyano,
$R^3$ représente $C_6$-$C_{12}$-aryle, qui peut le cas échéant être monosubstitué ou polysubstitué, de manière identique ou différente, par halogène, par $C_1$-$C_6$-alkyle, $C_1$-$C_3$-acyle, $C_1$-$C_3$-alcoxy, cyano, hydroxy, $N$-$(CH_3)_2$, $CO_2$-($C_1$-$C_3$-alkyle), CO-$NR^4R^5$ ou par $CF_3$, représente $C_5$-$C_{12}$-hétéroaryle, qui peut le cas échéant être monosubstitué ou polysubstitué, de manière identique ou différente, par chlore et/ou fluor, par $C_1$-$C_6$-alkyle, $C_1$-$C_3$-acyle, $C_1$-$C_3$-alcoxy, cyano, hydroxy, $N$-$(CH_3)_2$, $CO_2$-($C_1$-$C_3$-alkyle), CO-$NR^4R^5$ ou par $CF_3$, représente $C_3$-$C_6$-cycloalkyle, qui peut le cas échéant être monosubstitué ou polysubstitué, de manière identique ou différente, par chlore et/ou fluor, $CF_3$, cyano, $C_1$-$C_3$-alkyle, $C_1$-$C_3$-acyle, hydroxy, N- $(CH_3)_2$, $CO_2$-($C_1$-$C_3$-alkyle), CO-$NR^4R^5$ ou $C_1$-$C_3$-alcoxy,
$R^4$ représente hydrogène, $C_3$-$C_6$-cycloalkyle, qui est le cas échéant monosubstitué ou polysubstitué, de manière identique ou différente, par $C_1$-$C_3$-alkyle, $C_1$-$C_3$-acyle, $C_1$-$C_3$-alcoxy ou $CF_3$, représente $C_6$-$C_{12}$-aryle, qui est le cas échéant monosubstitué ou polysubstitué, de manière identique ou différente, par halogène, par $C_1$-$C_3$-alk-

yle, $C_1$-$C_3$-acyle, $C_1$-$C_3$-alcoxy, N-$C_1$-$C_3$-alkyl-$C_1$-$C_3$-alkyle, $CF_3$ ou cyano, ou représente $C_5$-$C_{12}$-hétéroaryle, qui est le cas échéant monosubstitué ou polysubstitué, de manière identique ou différente, par halogène, par $C_1$-$C_3$-alkyle, $C_1$-$C_3$-acyle, $C_1$-$C_3$-alcoxy, N-$C_1$-$C_3$-alkyl-$C_1$-$C_3$-alkyle, $CF_3$ ou cyano, ou représente $C_1$-$C_6$-alkyle, qui peut être substitué de manière quelconque,

$R^5$ représente hydrogène, $C_3$-$C_6$-cycloalkyle, qui est le cas échéant monosubstitué ou polysubstitué, de manière identique ou différente, par $C_1$-$C_3$-alkyle, $C_1$-$C_3$-acyle, $C_1$-$C_3$-alcoxy ou $CF_3$, représente $C_6$-$C_{12}$-aryle, qui est le cas échéant monosubstitué ou polysubstitué, de manière identique ou différente, par halogène, par $C_1$-$C_3$-alkyle, $C_1$-$C_3$-acyle, $C_1$-$C_3$-alcoxy, N-$C_1$-$C_3$-alkyl-$C_1$-$C_3$-alkyle, $CF_3$ ou cyano, ou représente $C_5$-$C_{12}$-hétéroaryle, qui est le cas échéant monosubstitué ou polysubstitué, de manière identique ou différente, par $C_1$-$C_3$-alkyle, $C_1$-$C_3$-acyle, $C_1$-$C_3$-alcoxy, N-$C_1$-$C_3$-alkyl-$C_1$-$C_3$-alkyle, $CF_3$ ou cyano, ou représente $C_4$-$C_6$-alkyle, qui peut être substitué de manière quelconque,

$R^4$ et $R^5$ forment ensemble un cycle de 5-8 chaînons, qui peut contenir d'autres hétéroatomes et

X représente les groupes sulfonyle, $(CH_2)_n$ ou carbonyle,

Y représente carbonyle ou $(CH_2)_n$

Z représente azote ou

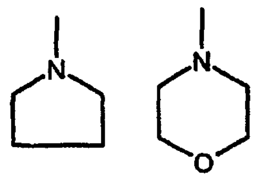

et

n vaut 0-2.

**12.** Composés selon les revendications 1-11 choisis dans le groupe qui contient les composés suivants :

1. cyclopropylamide de l'acide 5-(4-tert-butylbenzènesulfonylamino)-3-phényl-1H-indole-2-carboxylique
2. pyridin-3-ylamide de l'acide 5-(4-tert-butylbenzènesulfonylamino)-3-phényl-1H-indole-2-carboxylique
3. cyclohexylamide de l'acide 5-(4-tert-butylbenzènesulfonylamino)-3-phényl-1H-indole-2-carboxylique
4. 4-tert-butyl-N-[3-phényl-2-(pyrrolidine-1-carbonyl)-1H-indol-5-yl]-benzènesulfonamide
5. 4-tert-butyl-N-[2-(morpholine-4-carbonyl)-3-phényl-1H-indol-5-yl]-benzènesulfonamide
6. (2-diméthylaminoéthyl)amide de l'acide 5-(4-tert-butylbenzènesulfonylamino)-3-phényl-1H-indole-2-carboxylique
7. propylamide de l'acide 5-(4-cyanobenzènesulfonylamino)-3-phényl-1H-indole-2-carboxylique
8. propylamide de l'acide 5-(4-bromobenzènesulfonylamino)-3-phényl-1H-indole-2-carboxylique
9. propylamide de l'acide 5-(4-tert-butylbenzènesulfonylamino)-3-phényl-1H-indole-2-carboxylique
10. propylamide de l'acide 5-(4-(trifluorométhoxy)benzènesulfonylamino)-3-phényl-1H-indole-2-carboxylique
11. propylamide de l'acide 5-(4-(méthylsulfonyl)benzènesulfonylamino)-3-phényl-1H-indole-2-carboxylique
12. phénylamide de l'acide 5-(4-tert-butylbenzènesulfonylamino)-3-phényl-1H-indole-2-carboxylique
13. phénylamide de l'acide 5-(4-cyanobenzènesulfonylamino)-3-phényl-1H-indole-2-carboxylique
14. phénylamide de l'acide 5-(4-bromobenzènesulfonylamino)-3-phényl-1H-indole-2-carboxylique
15. phénylamide de l'acide 5-(4-(trifluorométhoxy)benzènesulfonylamino)-3-phényl-1H-indole-2-carboxylique
16. phénylamide de l'acide 5-(4-(méthylsulfonyl)benzènesulfonylamino)-3-phényl-1H-indole-2-carboxylique
17. pyridin-2-ylamide de l'acide 5-(4-tert-butylbenzènesulfonylamino)-3-phényl-1H-indole-2-carboxylique
18. pyridin-2-ylamide de l'acide 5-(4-cyanobenzènesulfonylamino)-3-phényl-1H-indole-2-carboxylique
19. (2-morpholin-4-yléthyl)amide de l'acide 5-(4-tert-butylbenzènesulfonylamino)-3-phényl-1H-indole-2-carboxylique
20. (4-méthylpipérazin-1-yl)amide de l'acide 5-(4-tert-butylbenzènesulfonylamino)-3-phényl-1H-indole-2-carboxylique
21. pyridin-4-ylamide de l'acide 5-(4-tert-butylbenzènesulfonylamino)-3-phényl-1H-indole-2-carboxylique
22. pyridin-4-ylamide de l'acide 5-(4-tert-butylbenzylamino)-3-phényl-1H-indole-2-carboxylique
23. (2-diméthylaminoéthyl)amide de l'acide 5-(4-tert-butylbenzoylamino)-3-phényl-1H-indole-2-carboxylique
24. (2-diméthylaminoéthyl)amide de l'acide 5-(4-tert-butylbenzènesulfonylamino)-3-(2-chlorophényl)-1H-indole-2-carboxylique
25. (2-diméthylaminoéthyl)amide de l'acide 5-(4-tert-butylbenzènesulfonylamino)-3-(3-chlorophényl)-1H-indole-2-carboxylique

26. (2-diméthylaminoéthyl)amide de l'acide 5-(4-tert-butylbenzènesulfonylamino)-3-(4-chlorophényl)-1H-indole-2-carboxylique

27. (2-diméthylaminoéthyl)amide de l'acide 5-(4-tert-butylbenzènesulfonylamino)-3-(2,4-dichlorophényl)-1H-indole-2-carboxylique

28. (2-diméthylaminoéthyl)amide de l'acide 5-(4-tert-butylbenzènesulfonylamino)-3-(2-méthylphényl)-1H-indole-2-carboxylique

29. (2-diméthylaminoéthyl)amide de l'acide 5-(4-tert-butylbenzènesulfonylamino)-3-(4-méthylphényl)-1H-indole-2-carboxylique

30. pyridin-4-ylamide de l'acide 5-(4-tert-butylbenzènesulfonylamino)-3-(4-méthylphényl)-1H-indole-2-carboxylique

31. (2-diméthylaminoéthyl)amide de l'acide 5-(4-tert-butylbenzènesulfonylamino)-3-(4-méthoxyphényl)-1H-indole-2-carboxylique

32. (2-diméthylaminoéthyl)amide de l'acide 5-(4-tert-butylbenzènesulfonylamino)-3-pyridin-3-yl-1H-indole-2-carboxylique

33. (2-diméthylaminoéthyl)amide de l'acide 5-(4-tert-butylbenzènesulfonylamino)-3-(4-hydroxyphényl)-1H-indole-2-carboxylique

34. (2-diméthylaminoéthyl)amide de l'acide 5-(4-tert-butylbenzènesulfonylamino)-3-(3-fluorophényl)-1H-indole-2-carboxylique

35. (2-hydroxypropyl)amide de l'acide 5-(4-tert-butylbenzènesulfonylamino)-3-phényl-1H-indole-2-carboxylique

36. (2-méthoxyéthyl)amide de l'acide 5-(4-tert-butylbenzènesulfonylamino)-3-phényl-1H-indole-2-carboxylique

37. (2-hydroxyéthyl)amide de l'acide 5-(4-tert-butylbenzènesulfonylamino)-3-phényl-1H-indole-2-carboxylique

38. (2-hydroxy-1-méthyléthyl)amide de l'acide 5-(4-tert-butylbenzènesulfonylamino)-3-phényl-1H-indole-2-carboxylique

39. (2-acétylaminoéthyl)amide de l'acide 5-(4-tert-butylbenzènesulfonylamino)-3-phényl-1H-indole-2-carboxylique

40. (tétrahydropyrann-4-yl)amide de l'acide 5-(4-tert-butylbenzènesulfonylamino)-3-phényl-1H-indole-2-carboxylique

41. (1-méthylpipéridin-4-yl)amide de l'acide 5-(4-tert-butylbenzènesulfonylamino)-3-phényl-1H-indole-2-carboxylique

42. (2-diméthylaminoéthyl)amide de l'acide 5-(4-tert-butylbenzènesulfonylamino)-3-(4-N,N-diméthylaminophényl)-1H-indole-2-carboxylique

43. (2-diméthylaminoéthyl)amide de l'acide 5-(4-tert-butylbenzènesulfonylamino)-3-(3-méthoxyphényl)-1H-indole-2-carboxylique

44. (2-diméthylaminoéthyl)amide de l'acide 5-(4-tert-butylbenzènesulfonylamino)-3-(3-trifluorométhylphényl)-1H-indole-2-carboxylique

45. (2-hydroxyéthyl)amide de l'acide 5-(4-tert-butylbenzènesulfonylamino)-3-(3-fluorophényl)-1H-indole-2-carboxylique

46. (tétrahydropyrann-4-yl)amide de l'acide 5-(4-tert-butylbenzènesulfonylamino)-3-(3-fluorophényl)-1H-indole-2-carboxylique

47. (2-acétylaminoéthyl)amide de l'acide 5-(4-tert-butylbenzènesulfonylamino)-3-(3-fluorophényl)-1H-indole-2-carboxylique

48. (2-morpholin-4-yléthyl)amide de l'acide 5-(4-tert-butylbenzènesulfonylamino)-3-(3-fluorophényl)-1H-indole-2-carboxylique

49. (2-hydroxyéthyl)amide de l'acide 5-(4-tert-butylbenzènesulfonylamino)-3-(3-méthoxyphényl)-1H-indole-2-carboxylique

50. (2-acétylaminoéthyl)amide de l'acide 5-(4-tert-butylbenzènesulfonylamino)-3-(3-méthoxyphényl)-1H-indole-2-carboxylique

51. (2-acétylaminoéthyl)amide de l'acide 5-(4-tert-butylbenzènesulfonylamino)3-pyridin-3-yl-1H-indole-2-carboxylique

52. (tétrahydropyrann-4-yl)amide de l'acide 5-(4-tert-butylbenzènesulfonylamino)-3-pyridin-3-yl-1H-indole-2-carboxylique

53. (2-acétylaminoéthyl)amide de l'acide 5-(4-tert-butylbenzènesulfonylamino)-3-pyridin-4-yl-1H-indole-2-carboxylique

54. (2-morpholin-4-yléthyl)amide de l'acide 5-(4-tert-butylbenzènesulfonylamino)-3-pyridin-4-yl-1H-indole-2-carboxylique

55. (tétrahydropyrann-4-yl)amide de l'acide 5-(4-tert-butylbenzènesulfonylamino)-3-pyridin-4-yl-1H-indole-2-carboxylique

56. (2-acétylaminoéthyl)amide de l'acide 5-(4-tert-butylbenzènesulfonylamino)-3-(3-méthylphényl)-1H-indole-2-carboxylique

57. (2-hydroxyéthyl)amide de l'acide 5-(4-tert-butylbenzènesulfonylamino)-3-(3-méthylphényl)-1H-indole-2-carboxylique

58. (2-morpholin-4-yléthyl)amide de l'acide 5-(4-tert-butylbenzènesulfonylamino)-3-(3-méthylphényl)-1H-indole-2-carboxylique

59. (tétrahydropyrann-4-yl)amide de l'acide 5-(4-tert-butylbenzènesulfonylamino)-3-(3-méthylphényl)-1H-indole-2-carboxylique

60. (2-morpholin-4-yléthyl)amide de l'acide 5-(4-tert-butylbenzènesulfonylamino)-3-pyridin-3-yl-1H-indole-2-carboxylique

61. (tétrahydropyrann-4-yl)amide de l'acide 5-(4-tert-butylbenzènesulfonylamino)-3-(3-méthoxyphényl)-1H-indole-2-carboxylique

62. (2-morpholin-4-yléthyl)amide de l'acide 5-(4-tert-butylbenzènesulfonylamino)-3-(3-méthoxyphényl)-1H-indole-2-carboxylique

63. pipéridin-4-ylamide de l'acide 5-(4-tert-butylbenzènesulfonylamino)-3-phényl-1H-indole-2-carboxylique

64. ester tert-butylique de l'acide 4-{[5-(4-tert-butylbenzènesulfonylamino)-3-phényl-1H-indole-2-carbonyl]-amino}pipéridine-1-carboxylique

65. (2-morpholin-4-yl-éthyl)-amide de l'acide 5-(4-tert-butylphénylsulfonylamino)-3-naphtalène-1-yl-1H-indole-2-carboxylique

66. (2-morpholin-4-yléthyl)-amide de l'acide 5-(4-tert-butylphénylsulfonylamino)-3-m-toluyl-1H-indole-2-carboxylique

67. (2-morpholin-4-yléthyl)-amide de l'acide 5-(4-tert-butylphénylsulfonylamino)-3-thiophén-2-yl-1H-indole-2-carboxylique

68. (2-morpholin-4-yléthyl)-amide de l'acide 5-(4-tert-butylphénylsulfonylamino)-3-thiophén-3-yl-1H-indole-2-carboxylique

69. (2-morpholin-4-yléthyl)-amide de l'acide 3-benzofurann-2-yl-5-(4-tert-butylphénylsulfonylamino)-1H-indole-2-carboxylique

70. (2-morpholin-4-yléthyl)-amide de l'acide 5-(4-tert-butylphénylsulfonylamino)-3-(5-chlorothiophén-2-yl)-1H-indole-2-carboxylique

71. (2-morpholin-4-yléthyl)-amide de l'acide 5-(4-tert-butylphénylsulfonylamino)-3-furann-2-yl-1H-indole-2-carboxylique

72. (2-morpholin-4-yléthyl)amide de l'acide 5-(4-tert-butylphénylsulfonylamino)-3-(3-fluoro-4-méthoxyphényl)-1H-indole-2-carboxylique

73. (2-morpholin-4-yléthyl)-amide de l'acide 3-benzo[1,3]dioxol-5-yl-5-(4-tert-butylphénylsulfonylamino)-1H-indole-2-carboxylique

74. (2-morpholin-4-yléthyl)-amide de l'acide 3-(4-acétylphényl)-5-(4-tert-butylphénylsulfonylamino)-1H-indole-2-carboxylique

75. (2-morpholin-4-yléthyl)-amide de l'acide 3-(3-acétylphényl)-5-(4-tert-butylphénylsulfonylamino)-1H-indole-2-carboxylique

76. (2-morpholin-4-yléthyl)-amide de l'acide 3-benzo[b]thiophén-2-yl-5-(4-tert-butylphénylsulfonylamino)-1H-indole-2-carboxylique

77. (2-morpholin-4-yléthyl)-amide de l'acide 3-benzo[b]thiophén-3-yl-5-(4-tert-butylphénylsulfonylamino)-1H-indole-2-carboxylique

78. (2-morpholin-4-yléthyl)-amide de l'acide 5-(4-tert-butylphénylsulfonylamino)-3-(5-méthylthiophén-2-yl)-1H-indole-2-carboxylique

79. ester méthylique de l'acide 3-[5-(4-tert-butylphénylsulfonylamino)-2-(2-morpholin-4-yléthylcarbamoyl)-1H-indol-3-yl]-benzoïque

80. (2-morpholin-4-yléthyl)-amide de l'acide 5-(4-tert-butylphénylsulfonylamino)-3-(2-fluoro-3-méthoxyphényl)-1H-indole-2-carboxylique

81. (2-morpholin-4-yléthyl)-amide de l'acide 5-(4-tert-butylphénylsulfonylamino)-3-(3-chloro-4-méthylphényl)-1H-indole-2-carboxylique

82. (2-morpholin-4-yléthyl)-amide de l'acide 5-(4-tert-butylphénylsulfonylamino)-3-(2,4-diméthoxypyrimidin-5-yl)-1H-indole-2-carboxylique

83. (2-morpholin-4-yléthyl)-amide de l'acide 5-(4-tert-butylphénylsulfonylamino)-3-(2,5-difluorophényl)-1H-indole-2-carboxylique

84. (2-morpholin-4-yléthyl)amide de l'acide 5-(4-tert-butylphénylsulfonylamino)-3-(2,4-difluorophényl)-1H-indole-2-carboxylique

85. (2-morpholin-4-yléthyl)-amide de l'acide 5-(4-tert-butylphénylsulfonylamino)-3-(2,3-difluorophényl)-1H-indole-2-carboxylique
86. (2-morpholin-4-yléthyl)-amide de l'acide 5-(4-tert-butylphénylsulfonylamino)-3-(2,6-difluorophényl)-1H-indole-2-carboxylique
87. (2-morpholin-4-yléthyl)-amide de l'acide 5-(4-tert-butylphénylsulfonylamino)-3-(3-hydroxyphényl)-1H-indole-2-carboxylique
88. (2-morpholin-4-yléthyl)-amide de l'acide 5-(4-tert-butylphénylsulfonylamino)-3-(4-hydroxyphényl)-1H-indole-2-carboxylique
89. (2-morpholin-4-yléthyl)-amide de l'acide 5-(4-tert-butylphénylsulfonylamino)-3-(3-fluoro-4-méthylphényl)-1H-indole-2-carboxylique
90. (2-morpholin-4-yléthyl)-amide de l'acide 5-(4-tert-butylphénylsulfonylamino)-3-(4-trifluorométhylphényl)-1H-indole-2-carboxylique
91. (2-morpholin-4-yléthyl)-amide de l'acide 5-(4-tert-butylphénylsulfonylamino)-3-(4-cyanométhylphényl)-1H-indole-2-carboxylique
92. (2-morpholin-4-yléthyl)-amide de l'acide 5-(4-tert-butylphénylsulfonylamino)-1H,1'H-[3,4']biindolyle-2-carboxylique
93. (2-morpholin-4-yléthyl)-amide de l'acide 5-(4-tert-butylphénylsulfonylamino)-3-(3-cyano-4-fluorophényl)-1H-indole-2-carboxylique
94. (2-morpholin-4-yléthyl)-amide de l'acide 5-(4-tert-butylphénylsulfonylamino)-3-(2-fluorophényl)-1H-indole-2-carboxylique
95. (2-morpholin-4-yléthyl)-amide de l'acide 5-(4-tert-butylphénylsulfonylamino)-3-(3,4-difluorophényl)-1H-indole-2-carboxylique
96. (2-morpholin-4-yléthyl)-amide de l'acide 5-(4-tert-butylphénylsulfonylamino)-3-(3-cyanophényl)-1H-indole-2-carboxylique
97. (2-morpholin-4-yléthyl)-amide de l'acide 5-(4-tert-butylphénylsulfonylamino)-3-(4-cyanophényl)-1H-indole-2-carboxylique
98. (2-morpholin-4-yléthyl)-amide de l'acide 5-(4-tert-butylphénylsulfonylamino)-3-(4-méthylthiophén-2-yl)-1H-indole-2-carboxylique
99. (3-chlorophényl)-amide de l'acide 5-(4-tert-butylphénylsulfonylamino)-3-phényl-1H-indole-2-carboxylique
100. (3-méthylisoxazol-5-yl)-amide de l'acide 5-(4-tert-butylphénylsulfonylamino)-3-phényl-1H-indole-2-carboxylique
101. (4-fluorophényl)-amide de l'acide 5-(4-tert-butylphénylsulfonylamino)-3-phényl-1H-indole-2-carboxylique
102. (2-fluorophényl)-amide de l'acide 5-(4-tert-butylphénylsulfonylamino)-3-phényl-1H-indole-2-carboxylique
103. (6-méthylpyridin-2-yl)-amide de l'acide 5-(4-tert-butylphénylsulfonylamino)-3-phényl-1H-indole-2-carboxylique
104. (5-carbamoylpyridin-2-yl)-amide de l'acide 5-(4-tert-butylphénylsulfonylamino)-3-phényl-1H-indole-2-carboxylique
105. (4-hydroxyphényl)-amide de l'acide 5-(4-tert-butylphénylsulfonylamino)-3-phényl-1H-indole-2-carboxylique
106. (2-méthoxyphényl)-amide de l'acide 5-(4-tert-butylphénylsulfonylamino)-3-phényl-1H-indole-2-carboxylique
107. (3-méthoxyphényl)-amide de l'acide 5-(4-tert-butylphénylsulfonylamino)-3-phényl-1H-indole-2-carboxylique
108. (4-méthoxyphényl)-amide de l'acide 5-(4-tert-butylphénylsulfonylamino)-3-phényl-1H-indole-2-carboxylique
109. (4-chlorophényl)-amide de l'acide 5-(4-tert-butylphénylsulfonylamino)-3-phényl-1H-indole-2-carboxylique
110. (4-diméthylaminophényl)-amide de l'acide 5-(4-tert-butylphénylsulfonylamino)-3-phényl-1H-indole-2-carboxylique
111. (5-chloropyridin-2-yl)-amide de l'acide 5-(4-tert-butylphénylsulfonylamino)-3-phényl-1H-indole-2-carboxylique
112. p-toluylamide de l'acide 5-(4-tert-butylphénylsulfonylamino)-3-phényl-1H-indole-2-carboxylique
113. pyrazin-2-ylamide de l'acide 5-(4-tert-butylphénylsulfonylamino)-3-phényl-1H-indole-2-carboxylique
114. (4-cyanophényl)-amide de l'acide 5-(4-tert-butylphénylsulfonylamino)-3-phényl-1H-indole-2-carboxylique
115. (3-méthylisothiazol-5-yl)-amide de l'acide 5-(4-tert-butylphénylsulfonylamino)-3-phényl-1H-indole-2-carboxylique
116. (4-bromophényl)-amide de l'acide 5-(4-tert-butylphénylsulfonylamino)-3-phényl-1H-indole-2-carboxylique
117. (4-carbamoylphényl)-amide de l'acide 5-(4-tert-butylphénylsulfonylamino)-3-phényl-1H-indole-2-carboxylique

118. (3-méthylpyridin-2-yl)-amide de l'acide 5-(4-tert-butylphénylsulfonylamino)-3-phényl-1H-indole-2-carboxylique
119. (2-chlorophényl)-amide de l'acide 5-(4-tert-butylphénylsulfonylamino)-3-phényl-1H-indole-2-carboxylique
120. (5-méthyl-2H-pyrazol-3-yl)-amide de l'acide 5-(4-tert-butylphénylsulfonylamino)-3-phényl-1H-indole-2-carboxylique
121. quinoléin-5-ylamide de l'acide 5-(4-tert-butylphénylsulfonylamino)-3-phényl-1H-indole-2-carboxylique
122. quinoléin-6-ylamide de l'acide 5-(4-tert-butylphénylsulfonylamino)-3-phényl-1H-indole-2-carboxylique
123. (2,6-dichloropyridin-4-yl)-amide de l'acide 5-(4-tert-butylphénylsulfonylamino)-3-phényl-1H-indole-2-carboxylique
124. (3-fluorophényl)-amide de l'acide 5-(4-tert-butylphénylsulfonylamino)-3-phényl-1H-indole-2-carboxylique
125. (2-méthylpyridin-4-yl)-amide de l'acide 5-(4-tert-butylphénylsulfonylamino)-3-phényl-1H-indole-2-carboxylique
126. (3-fluoropyridin-4-yl)-amide de l'acide 5-(4-tert-butylphénylsulfonylamino)-3-phényl-1H-indole-2-carboxylique
127. (3-méthylpyridin-4-yl)-amide de l'acide 5-(4-tert-butylphénylsulfonylamino)-3-phényl-1H-indole-2-carboxylique
128. (3-bromopyridin-4-yl)-amide de l'acide 5-(4-tert-butylphénylsulfonylamino)-3-phényl-1H-indole-2-carboxylique
129. (2,3-dihydroxypropyl)-amide de l'acide 5-(4-tert-butylphénylsulfonylamino)-3-phényl-1H-indole-2-carboxylique
130. (2-oxotétrahydrothiophén-3-yl)-amide de l'acide 5-(4-tert-butylphénylsulfonylamino)-3-phényl-1H-indole-2-carboxylique
131. [2-(2-oxoimidazolidin-1-yl)-éthyl]-amide de l'acide 5-(4-tert-butylphénylsulfonylamino)-3-phényl-1H-indole-2-carboxylique
132. (2,2-diéthoxyéthyl)-amide de l'acide 5-(4-tert-butylphénylsulfonylamino)-3-phényl-1H-indole-2-carboxylique
133. (3-éthoxypropyl)-amide de l'acide 5-(4-tert-butylphénylsulfonylamino)-3-phényl-1H-indole-2-carboxylique
134. (3-isopropoxypropyl)-amide de l'acide 5-(4-tert-butylphénylsulfonylamino)-3-phényl-1H-indole-2-carboxylique
135. (3-morpholin-4-yl-propyl)-amide de l'acide 5-(4-tert-butylphénylsulfonylamino)-3-phényl-1H-indole-2-carboxylique
136. (3-diéthylaminopropyl)-amide de l'acide 5-(4-tert-butylphénylsulfonylamino)-3-phényl-1H-indole-2-carboxylique
137. (3-diméthylaminopropyl)-amide de l'acide 5-(4-tert-butylphénylsulfonylamino)-3-phényl-1H-indole-2-carboxylique
138. (furann-2-ylméthyl)-amide de l'acide 5-(4-tert-butylphénylsulfonylamino)-3-phényl-1H-indole-2-carboxylique
139. (2-méthylsulfanyléthyl)-amide de l'acide 5-(4-tert-butylphénylsulfonylamino)-3-phényl-1H-indole-2-carboxylique
140. (2-diéthylaminoéthyl)-amide de l'acide 5-(4-tert-butylphénylsulfonylamino)-3-phényl-1H-indole-2-carboxylique
141. [2-(3,4-diméthoxyphényl)-éthyl]-amide de l'acide 5-(4-tert-butylphénylsulfonylamino)-3-phényl-1H-indole-2-carboxylique
142. (2-pipéridin-1-yléthyl)-amide de l'acide 5-(4-tert-butylphénylsulfonylamino)-3-phényl-1H-indole-2-carboxylique
143. (3-pyrrolidin-1-ylpropyl)-amide de l'acide 5-(4-tert-butylphénylsulfonylamino)-3-phényl-1H-indole-2-carboxylique
144. phénéthylamide de l'acide 5-(4-tert-butylphénylsulfonylamino)-3-phényl-1H-indole-2-carboxylique
145. (2-méthoxy-1-méthyléthyl)-amide de l'acide 5-(4-tert-butylphénylsulfonylamino)-3-phényl-1H-indole-2-carboxylique
146. (pyridin-2-ylméthyl)-amide de l'acide 5-(4-tert-butylphénylsulfonylamino)-3-phényl-1H-indole-2-carboxylique
147. (pyridin-3-ylméthyl)-amide de l'acide 5-(4-tert-butylphénylsulfonylamino)-3-phényl-1H-indole-2-carboxylique
148. (pyridin-4-ylméthyl)-amide de l'acide 5-(4-tert-butylphénylsulfonylamino)-3-phényl-1H-indole-2-carboxylique
149. (4-diéthylamino-1-méthylbutyl)-amide de l'acide 5-(4-tert-butylphénylsulfonylamino)-3-phényl-1H-indole-2-carboxylique

150. (2-imidazol-1-yléthyl)-amide de l'acide 5-(4-tert-butylphénylsulfonylamino)-3-phényl-1H-indole-2-carboxylique
151. benzylamide de l'acide 5-(4-tert-butylphénylsulfonylamino)-3-phényl-1H-indole-2-carboxylique
152. (2,2,2-trifluoroéthyl)-amide de l'acide 5-(4-tert-butylphénylsulfonylamino)-3-phényl-1H-indole-2-carboxylique
153. 4-méthoxybenzylamide de l'acide 5(4-tert-butylphénylsulfonylamino)-3-phényl-1H-indole-2-carboxylique
154. cyclopentylamide de l'acide 5-(4-tert-butylphénylsulfonylamino)-3-phényl-1H-indole-2-carboxylique
155. (3-méthylbutyl)-amide de l'acide 5-(4-tert-butylphénylsulfonylamino)-3-phényl-1H-indole-2-carboxylique
156. [3-(4-méthylpipérazin-1-yl)-propyl]-amide de l'acide 5-(4-tert-butylphénylsulfonylamino)-3-phényl-1H-indole-2-carboxylique
157. [2-(4-hydroxyphényl)-éthyl]-amide de l'acide 5-(4-tert-butylphénylsulfonylamino)-3-phényl-1H-indole-2-carboxylique
158. [2-(4-chlorophényl)-éthyl]-amide de l'acide 5-(4-tert-butylphénylsulfonylamino)-3-phényl-1H-indole-2-carboxylique
159. cyclopropylamide de l'acide 5-(4-tert-butylphénylsulfonylamino)-3-phényl-1H-indole-2-carboxylique
160. cyclohexylméthylamide de l'acide 5-(4-tert-butylphénylsulfonylamino)-3-phényl-1H-indole-2-carboxylique
161. (tétrahydrofurann-2-ylméthyl)-amide de l'acide 5-(4-tert-butylphénylsulfonylamino)-3-phényl-1H-indole-2-carboxylique
162. (thiophén-2-ylméthyl)-amide de l'acide 5-(4-tert-butylphénylsulfonylamino)-3-phényl-1H-indole-2-carboxylique
163. 4-fluorobenzylamide de l'acide 5-(4-tert-butylphénylsulfonylamino)-3-phényl-1H-indole-2-carboxylique
164. (2-thiophén-2-yléthyl)-amide de l'acide 5-(4-tert-butylphénylsulfonylamino)-3-phényl-1H-indole-2-carboxylique
165. (2-pyrrolidin-1-yléthyl)-amide de l'acide 5-(4-tert-butylphénylsulfonylamino)-3-phényl-1H-indole-2-carboxylique
166. 4-méthylbenzylamide de l'acide 5-(4-tert-butylphénylsulfonylamino)-3-phényl-1H-indole-2-carboxylique
167. (1-éthylpyrrolidin-2-ylméthyl)-amide de l'acide 5-(4-tert-butylphénylsulfonylamino)-3-phényl-1H-indole-2-carboxylique
168. (2-pyridin-3-yléthyl)-amide de l'acide 5-(4-tert-butylphénylsulfonylamino)-3-phényl-1H-indole-2-carboxylique
169. 3-chlorobenzylamide de l'acide 5-(4-tert-butylphénylsulfonylamino)-3-phényl-1H-indole-2-carboxylique
170. [2-(3-chlorophényl)-éthyl]-amide de l'acide 5-(4-tert-butylphénylsulfonylamino)-3-phényl-1H-indole-2-carboxylique
171. ((R)-2-hydroxy-1-phényléthyl)-amide de l'acide 5-(4-tert-butylphénylsulfonylamino)-3-phényl-1H-indole-2-carboxylique
172. [3-(2-méthylpipéridin-1-yl)-propyl]-amide de l'acide 5-(4-tert-butylphénylsulfonylamino)-3-phényl-1H-indole-2-carboxylique
173. (3-phénylpropyl)-amide de l'acide 5-(4-tert-butylphénylsulfonylamino)-3-phényl-1H-indole-2-carboxylique
174. (2-carbamoyléthyl)-amide de l'acide 5-(4-tert-butylphénylsulfonylamino)-3-phényl-1H-indole-2-carboxylique
175. [3-(5-méthyl-1H-pyrazol-4-yl)-propyl]-amide de l'acide 5-(4-tert-butylphénylsulfonylamino)-3-phényl-1H-indole-2-carboxylique
176. (4-méthylcyclohexyl)-amide de l'acide 5-(4-tert-butylphénylsulfonylamino)-3-phényl-1H-indole-2-carboxylique
177. ((S)-2-méthoxy-1-méthyléthyl)-amide de l'acide 5-(4-tert-butylphénylsulfonylamino)-3-phényl-1H-indole-2-carboxylique
178. cyclopropylméthylamide de l'acide 5-(4-tert-butylphénylsulfonylamino)-3-phényl-1H-indole-2-carboxylique
179. carbamoylméthylamide de l'acide 5-(4-tert-butylphénylsulfonylamino)-3-phényl-1H-indole-2-carboxylique
180. cycloheptylamide de l'acide 5-(4-tert-butylphénylsulfonylamino)-3-phényl-1H-indole-2-carboxylique
181. ((R)-2-méthoxy-1-méthyléthyl)-amide de l'acide 5-(4-tert-butylphénylsulfonylamino)-3-phényl-1H-indole-2-carboxylique
182. (furann-3-ylméthyl)-amide de l'acide 5-(4-tert-butylphénylsulfonylamino)-3-phényl-1H-indole-2-carboxylique
183. 3-fluorobenzylamide de l'acide 5-(4-tert-butylphénylsulfonylamino)-3-phényl-1H-indole-2-carboxylique
184. (5-méthylpyrazin-2-ylméthyl)-amide de l'acide 5-(4-tert-butylphénylsulfonylamino)-3-phényl-1H-indole-2-carboxylique
185. (2-pyridin-2-yléthyl)-amide de l'acide 5-(4-tert-butylphénylsulfonylamino)-3-phényl-1H-indole-2-carboxylique

186. (2-phénoxyéthyl)-amide de l'acide 5-(4-tert-butylphénylsulfonylamino)-3-phényl-1H-indole-2-carboxylique
187. (2-benzo-imidazol-1-yléthyl)-amide de l'acide 5-(4-tert-butylphénylsulfonylamino)-3-phényl-1H-indole-2-carboxylique
188. (3-imidazol-1-ylpropyl)-amide de l'acide 5-(4-tert-butylphénylsulfonylamino)-3-phényl-1H-indole-2-carboxylique
189. (1-benzylpipéridin-4-yl)-amide de l'acide 5-(4-tert-butylphénylsulfonylamino)-3-phényl-1H-indole-2-carboxylique
190. [3-(2-oxopyrrolidin-1-yl)-propyl]-amide de l'acide 5-(4-tert-butylphénylsulfonylamino)-3-phényl-1H-indole-2-carboxylique
191. [2-(1-méthylpyrrolidin-2-yl)-éthyl]-amide de l'acide 5-(4-tert-butylphénylsulfonylamino)-3-phényl-1H-indole-2-carboxylique
192. méthyl-(2-morpholin-4-yléthyl)-amide de l'acide 5-(4-tert-butylphénylsulfonylamino-3-phényl-1H-indole-2-carboxylique.

**13.** Médicaments qui contiennent au moins un des composés selon les revendications 1-12.

**14.** Médicaments selon la revendication 13 dans lesquels le composé de formule générale 1 est contenu en une dose active.

**15.** Utilisation des composés de formule générale 1 selon les revendications précédentes 1 à 12 pour la préparation de médicaments destinés au traitement de maladies qui sont provoquées par des troubles du métabolisme du cAMP.

**16.** Utilisation des composés de formule générale 1 selon les revendications précédentes 1 à 12 pour la préparation de médicaments destinés à la contraception non hormonale.

**17.** Utilisation du composé de formule générale 1 selon les revendications précédentes 1 à 12 pour la préparation de médicaments destinés à l'inhibition de l'adénylate-cyclase soluble.

**18.** Composés selon les revendications 1-12 comme médicament selon la revendication 13 et 14 avec des substances de formulation et support appropriées.

**19.** Utilisation des composés de formule générale 1 selon les revendications 1-12 pour la préparation d'une composition pharmaceutique destinée à l'administration entérale, parentérale, vaginale et orale.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 0185753 A
- US 6544768 B
- WO 0121829 A, Conti
- WO 0220745 A


### In der Beschreibung aufgeführte Nicht-Patentliteratur


- **LEVIN ; BUCK.** *Proc. Natl. Acad. Sci. USA,* vol. 96 (1), 79-84
- **T. BRAUN.** *Proc Soc Exp Biol Med,* 1990, vol. 194 (1), 58ff
- **KL OLGIATI.** *Arch Biochem Biophys,* 1984, vol. 231 (2), 411ff
- **MA BROWN ; ER CASILLAS.** *J Androl,* 1984, vol. 5, 361ff
- **JH ZIPPIN et al.** *J Cell Biol,* 2004, vol. 164 (4), 527ff
- **T. BRAUN et al.** *PNAS,* 1975, vol. 73, 1097ff
- **N. OKAMURA et al.** *J. Biol. Chem,* 1985, vol. 260 (17), 9699ff
- **J. BUCK et al.** *PNAS,* 1999, vol. 96, 79ff
- **Y. CHEN et al.** *Science,* 2000, vol. 289, 625ff
- **ML SINCLAIR et al.** *Mol Reprod Develop,* 2000, vol. 56, 6ff
- **N OKAMURA et al.** *J. Biol. Chem,* 1985, vol. 260 (17), 9699ff
- **PE VISCONTI ; GS KOPF.** *Biol Reprod,* 1998, vol. 59, 1ff
- **E DE LAMIRANDE et al.** *Mol Hum Reprod,* 1997, vol. 3 (3), 175ff
- **G ESPOSITO et al.** *PNAS,* 2004, vol. 101 (9), 2993ff
- **JH ZIPPIN et al.** *FASEB,* 2003, vol. 17, 82ff
- **J. W. CARVER-WARD.** *Human Reproduction,* 1996, vol. 11 (9), 1923 ff
- **O. J. D'CRUZ ; G. G. HAAS.** *Fertility and Sterility,* 1996, vol. 65 (4), 843 ff
- **E. NIESCHLAG ; H.M. BEHRE.** Andrologie. Springer Verlag, 1996